# EUROPÄISCHE PATENTSCHRIFT

(11) **EP 1 194 425 B1**
(45) Veröffentlichungstag und Bekanntmachung des Hinweises auf die Patenterteilung: **10.08.2005**
(21) Anmeldenummer: 00938780.4
(22) Anmeldetag: 09.06.2000
(51) Int. Cl.: C07D 401/04, A61K 31/415, A61P 29/00, C07D 401/14, C07D 413/14, C07D 471/04

(54) **SUBSTITUIERTE BENZIMIDAZOLE**
SUBSTITUTED BENZIMIDAZOLE
BENZIMIDAZOLES SUBSTITUES

(30) Priorität: 23.06.1999 DE 19928424; 12.02.2000 DE 10006297
(43) Veröffentlichungstag der Anmeldung: 10.04.2002
(73) Patentinhaber: Aventis Pharma Deutschland GmbH, 65929 Frankfurt am Main (DE)
(72) Erfinder: RITZELER, Olaf, D-65812 Bad Soden (DE); STILZ, Hans, Ulrich, D-65929 Frankfurt (DE); NEISES, Bernhard, D-77652 Offenburg (DE); BOCK, William, Jerome, Jr., Tucson, AZ 85737 (US); WALSER, Armin, Tucson, AZ 85718 (US); FLYNN, Gary, A., Tucson, AZ 85737 (US); HABERMANN, Jörg, 65812 Bad Soden (DE); JAHNE, Gerhard, 65929 Frankfurt am Main (DE)
(86) Internationale Anmeldenummer: PCT/EP2000/005340
(87) Internationale Veröffentlichungsnummer: WO 2001/000610

(56) Entgegenhaltungen:
- EP-A- 0 719 765
- WO-A-98/05335
- DE-A- 2 641 060
- GB-A- 2 164 648
- US-A- 5 852 011
- RAFALSKI, M. ET AL: "Synthesis and biological evaluation of substituted benzimidazoles - potential GPIIb/IIIa receptor antagonists" PEPT.: CHEM., STRUCT. BIOL., PROC. AM. PEPT. SYMP., 14TH (1996), MEETING DATE 1995, 707-708. EDITOR(S): KAUMAYA, PRAVIN T. P.;HODGES, ROBERT S. PUBLISHER: MAYFLOWER SCIENTIFIC, KINGSWINFORD, UK. , XP000942687
- DENNY W A ET AL: "POTENTIAL ANTITUMOR AGENTS 59. STRUCTURE-ACTIVITY RELATIONSHIPS FOR 2-PHENYLBENZIMIDAZOLE-4-CARB OXAMIDES, A NEW CLASS OF MINIMAL DNA-INTERCALATING AGENTS WHICH MAY NOT ACT VIA TOPOISOMERASE II" JOURNAL OF MEDICINAL CHEMISTRY,US,AMERICAN CHEMICAL SOCIETY. WASHINGTON, Bd. 33, Nr. 2, 1. Februar 1990 (1990-02-01), Seiten 814-819, XP002007402 ISSN: 0022-2623
- XUE C -B ET AL: "DESIGN, SYNTHESIS AND IN VITRO ACTIVITIES OF A SERIES OF BENZIMIDAZOLE/BENZOXAZOLE GLYCOPROTEIN IIB/IIA INHIBITORS" BIOORGANIC & MEDICINAL CHEMISTRY LETTERS,GB,OXFORD, Bd. 6, Nr. 3, 1996, Seiten 339-344, XP000576489 ISSN: 0960-894X
- GOEKER ET AL.: "Synthesis of 1,2-disubstituted benzimidazole..." IL FARMACO, Bd. 51, Nr. 1, 1996, Seiten 53-58, XP000942654
- GOEKER ET AL.: "Synthesis fo some new benzimidazole..." IL FARMACO, Bd. 53, Nr. 6, 1998, Seiten 415-420, XP000942653
- O'CONNOR ET AL.: "Damage of egg phosphatidylcholine..." BULL.CHEM.SOC.JPN, Bd. 64, Nr. 4, 1991, Seiten 1364-1369, XP000942666
- AKIMENKO ET AL.: "Interaction specificity of benzimidazole..." J. BIOMOL. STRUCT. DYN., Bd. 12, Nr. 5, 1995, Seiten 1121-1127, XP000942658
- VINOGRADOV A E ET AL: "SOME PROPERTIES OF NEW DNA-SPECIFIC BISBENZIMIDAZOLE FLUOROCHROMES WITHOUT A PIPERAZINE RING" BIOTECHNIC AND HISTOCHEMISTRY,US,WILLIAMS & WILKINS, BALTIMORE, MD, Bd. 68, Nr. 5, 1993, Seiten 265-270, XP000196599 ISSN: 1052-0295

## Beschreibung

Die Erfindung betrifft neue substituierte Benzimidazole, ein Verfahren zu ihrer Herstellung und Verwendung derselben als Arzneimittel.

In der Anmeldung WO 94/12478 werden unter anderem Benzimidazolderivate beschrieben, die die Blutplättchen-Aggregation inhibieren. NFκB ist ein heterodimerer Transkriptionsfaktor, der eine Vielzahl von Genen aktivieren kann, die unter anderen für proinflammatorische Cytokine wie IL-1, IL-2, TNFα oder IL-6 kodieren. NFκB liegt im Cytosol von Zellen komplexiert mit seinem natürlich vorkommenden Inhibitor IκB vor. Die Stimulation von Zellen, beispielsweise durch Cytokine, führt zur Phosphorylierung und anschließenden proteolytischen Abbau von IκB. Dieser proteolytische Abbau führt zur Aktivierung von NFκB, das anschließend in den Kern der Zelle wandert und dort eine Vielzahl von proinflammatorischen Genen aktiviert.

In Erkrankungen wie Rheumatoider Arthritis (bei der Entzündung), Osteoarthritis, Asthma, Herzinfarkt, Alzheimer Erkrankung oder Atherosklerose ist NFκB über das normale Maß hinaus aktiviert. Die Hemmung von NFκB ist auch in der Krebstherapie von Nutzen, da sie dort zur Verstärkung der Cytostatika Therapie eingesetzt wird. Es konnte gezeigt werden, daß Arzneimittel wie Glucocorticoide, Salicylate oder Goldsalze, die in der Rheumatherapie eingesetzt werden, an verschiedenen Stellen in die NFκB-aktivierende Signalkette inhibierend eingreifen oder direkt mit der Transkription der Gene interferieren.

Der erste Schritt in der genannten Signalkaskade ist der Abbau von IκB. Diese Phosphorylierung wird durch die spezifische IκB-Kinase reguliert. Bisher sind keine Inhibitoren bekannt, die spezifisch IκB-Kinase inhibieren.

In dem Bestreben wirksame Verbindungen zur Behandlung von Rheumatoider Arthritis (bei der Entzündung), Osteoarthritis, Asthma, Herzinfarkt, Alzheimer Erkrankung, Krebserkrankungen (Potenzierung von Cytotoxica-Therapien) oder Atherosklerose zu erhalten, wurde nun gefunden, daß die erfindungsgemäßen Benzimidazole starke und sehr spezifische Inhibitoren der IκB-Kinase sind.

Die Erfindung betrifft daher die Verbindung der Formel I 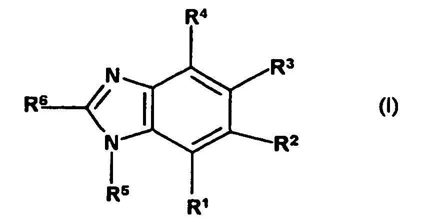 und/oder eine stereoisomere Form der Verbindung der Formel I und/oder ein physiologisch verträgliches Salz der Verbindung der Formel I, wobei
einer der Substituenten R¹, R², R³ und R⁴ für einen Rest der Formel II steht, 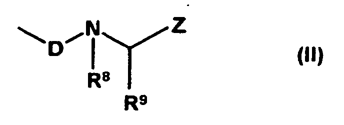 worin
- D: für -C(O)-, -S(O)- oder -S(O)₂- steht,
- R⁸: für Wasserstoffatom oder (C₁-C₄)-Alkyl steht,
- R⁹: für
1. charakteristischen Rest einer Aminosäure, der sich von einer natürlich vorkommenden α-Aminosäure aus der Gruppe Alanin, Valin, Leucin, Isoleucin, Phenylalanin, Tyrosin, Tryptophan, Serin, Threonin, Cystein, Methionin, Asparagin, Glutamin, Lysin, Histidin, Arginin und Glutaminsäure ableitet,
2. charakteristischen Rest einer Aminosäure, der sich von einer nicht natürlich vorkommenden Aminosäure aus der Gruppe 2-Aminoadipinsäure, 2-Aminobuttersäure, 2-Aminoisobuttersäure , 2,3-Diamino-propionsäure, 2,4-Diaminobuttersäure, 1,2,3,4-Tetra-hydroisochinolin-i-carbonsäure, 1,2,3,4-Tetrahydroisochinolin-3-carbonsäure, 2-Amino-pimelinsäure, Phenylglycin, 3-(2-Thienyl)-alanin, 3-(3-Thienyl)-alanin, Sarkosin, 2-(2-Thienyl)-glycin, 2-Aminoheptansäure, Pipecolinsäure, Hydroxylysin, N-Methylisoleucin, 6-N-Methyllysin, N-Methylvalin, Norvalin, Norleucin, Ornithin, allo-Isoleucin, 4-Hydroxy-prolin, allo-Hydroxylysin, allo-Threonin, 3-Hydroxyprolin, 3-(2-Naphtyl)-alanin, 3-(1-Naphtylalanin), Homophenylalanin, Homocystein, 2-Amino-3-phenylaminoethylpropion-säure, Homocysteinsäure, Homotryptophan, Cysteinsäure, 3-(2-Pyridyl)-alanin, 3-(3-Pyridyl)-alanin, 3-(4-Pyridyl)-alanin, Phosphinothricin, 4-Fluorphenylalanin, 3-Fluorphenyl-alanin, 2-Fluor-phenylalanin, 4-Chlorphenylalanin, 4-Nitrophenylalanin, 4-Aminophenylalanin, Cyclo-hexylalanin, Citrullin, 5-Fluortryptophan, 5-Methoxytryptophan, 2-Amino-3-phenylamino-propionsäure, Methionin-Sulfon, Methionin-Sulfoxid oder -NH-NR¹¹-CON(R¹¹)₂ ableitet,
3. -(C₁-C₆)-Alkyl, worin Alkyl gerade oder verzweigt ist und ein-, zwei- oder dreifach unabhängig voneinander substituiert ist durch
   3.1 Aryl, worin Aryl für einen aromatischen Kohlenstoffrest mit 6 bis 14 Kohlenstoffatomen im Ring steht aus der Gruppe Phenyl, 1-Naphthyl, 2-Naphthyl, 2-Biphenylyl, 3-Biphenylyl, 4-Biphenylyl, Anthryl oder Fluorenyl, worin Aryl unsubstituiert oder ein-, zwei- oder dreifach durch gleiche oder verschiedene Reste substituiert ist aus der Reihe (C₁-C₈)-Alkyl, (C₁-C₈)-Alkoxy, Halogen, Nitro, Amino, Trifluormethyl, Hydroxy, Hydroxy-methyl, 1-Hydroxyethyl, 2-Hydroxyethyl, Methylendioxy, Ethylendioxy, Formyl, Acetyl, Cyano, Hydroxycarbonyl, Aminocarbonyl, (C₁-C₄)-Alkoxy-carbonyl, Phenyl, Phenoxy, Benzyl, Benzyloxy oder Tetrazolyl,
   3.2 Heteroaryl mit 5 bis 14 Ringgliedern, worin Heteroaryl für einen Rest steht aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen,
      worin Heteroary unsubstituiert oder ein-, zwei- oder dreifach durch gleiche oder verschiedene Reste substituiert ist aus der Reihe (C₁-C₈)-Alkyl, (C₁-C₈)-Alkoxy, Halogen, Nitro, -N(R¹¹)₂, Trifluormethyl, Hydroxy, Hydroxymethyl, 1-Hydroxyethyl, 2-Hydroxyethyl, Methylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy oder Tetrazolyl,
   3.3 ein Rest aus der Gruppe Azetidin, Pyrrolin, Pyrrolidin, Piperidin, isothiazot, Diazepin, Thiomorpholin, Morpholin oder Azepin,
   3.4 -O-R¹¹,
   3.5 =0,
   3.6 Halogen,
   3.7 -CN,
   3.8 -CF₃,
   3.9 -S(O)ₓ-R¹¹, worin x die ganze Zahl Null, 1 oder 2 ist,
   3.10 -C(O)-N(R¹¹)₂,
   3.11 -N(R¹¹)₂,
   3.12 (C₃-C₆)-Cycloalkyl,
   3.13 Rest der Formel 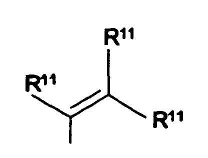 oder
   3.14 Rest der Formel 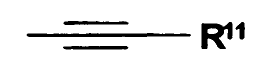 worin
- R¹¹: für
a) Wasserstoffatom,
b) (C₁-C₆)-Alkyl, worin Alkyl unsubstituiert oder ein-, zwei- oder dreifach substituiert ist durch
   1. Aryl, worin Aryl wie oben definiert ist und unsubstituiert oder wie oben substituiert ist,
   2. Heteroaryl mit 5 bis 14 Ringgliedern, worin Heteroaryl wie oben definiert ist,
   3. Halogen,
   4. -N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
   5. -O-(C₁-C₆)-Alkyl oder
   6. -COOH,
c) Aryl, worin Aryl wie oben definiert ist und unsubstituiert oder wie oben substituiert ist,
d) Heteroaryl mit 5 bis 14 Ringgliedern, worin Heteroaryl wie oben definiert ist,
e) Halogen oder
f) -N(R¹³)₂, worin
   R¹³ unabhängig voneinander für
   f) a) Wasserstoffatom,
   f) b) -(C₁-C₆)-Alkyl,
   f) c) -(C₁-C₄)-Alkyl-O-(C₁-C₄)-Alkyl,
   f) d) -(C₁-C₆)-Alkyl-N-(C₁-C₆).-Alkyl, worin n die ganze Zahl Null, oder 2 bedeutet und Alkyl unsubstituiert oder ein-, zwei- oder dreifach unabhängig voneinander durch Halogen oder durch -COOH substituiert ist,
   f) e) Halogen oder
   f) f) -(C₀-C₄)-Alkyl, ein- oder zweifach substituiert durch Phenyl, 1-Naphthyl, 2-Naphthyl, 2-Biphenylyl, 3-Biphenylyl, 4-Biphenylyl, Anthryl, Fluorenyl, Imidazolyl oder Morpholinyl, bedeutet, steht, und
für den Fall des (R¹¹)₂ hat R¹¹ unabhängig voneinander die Bedeutung von a) bis f)
- Z: für
1. Aryl, worin Aryl wie oben definiert ist und unsubstituiert oder wie oben substituiert ist,
2. Heteroaryl mit 5 bis 14 Ringgliedern, worin Heteroaryl wie oben definiert ist und unsubstituiert oder wie oben substituiert ist,
3. Heterocyclus mit 5 bis 12 Ringgliedern, worin Heterocyclus für einen monocyclischen oder bicyclischen 5-gliedrigen bis 12-gliedrigen heterocyclischen Ring steht, der teilweise gesättigt oder vollständig gesättigt ist aus der Gruppe Pyrrol, Furan, Thiophen, Imidazol, Pyrazol, Oxazol, Isoxazol, Thiazol, lsothiazol, Tetrazol, 1,3,4-Oxadiazol, 1,2,3,5-Oxathia-diazol-2-Oxide, Triazolone, oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen,
   worin Heterocyclus unsubstituiert oder ein-, zwei-, drei oder vierfach durch gleiche oder verschiedene Reste substituiert ist aus der Reihe (C₁-C₈)-Alkyl, (C₁-C₈)-Alkoxy, Phenyl-(C₁-C₄)-alkoxy, Hydroxy, Oxo, Halogen, Nitro, Amino oder Trifluormethyl, wobei die Stickstoffheterocyclen auch als N-Oxide vorliegen können oder als Quartärsalze,
4. 1,3,4-Oxadiazol, worin1,3,4- oxadiazol unsubstituiert oder einfach substituiert ist durch -NH-C(O)-(C₁-C₄)-Alkyl,- NH-C(O)-NH-(C₁-C₄)-Alkyl, -NH-C(O)-NH-(C₃-C₇)-Cyloalkyl, -NH-C(O)-NH-Aryl, worin Aryl wie oben definiert ist, -NH-C(O)-NH-Phenyl, -NH-SO₂-Aryl, worin Aryl wie oben definiert ist, oder -NH-SO₂-(C₁-C₄)-Alkyl,
5. -(C₁-C₆)-Alkyl, worin Alkyl gerade oder verzweigt ist und unsubstituiert oder ein- oder zweifach substituiert ist durch Phenyl oder -OH, oder
6. -C(O)-R¹⁰ steht, worin
   R¹⁰ für
   1. -O-R¹¹,
   2. -N(R¹¹)₂,
   3. Phenyl,
   4. Pyrimidinyl oder
   5. Morholinyl, steht oder
- R⁸ und R⁹: bilden zusammen mit dem Stickstoff- und Kohlenstoffatom an das sie jeweils gebunden sind einen heterocyclischen Ring der Formel IIa, 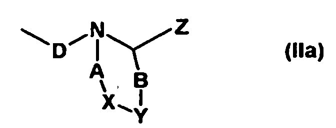 worin D und Z wie in Formel II definiert sind,
A für Stickstoffatom oder den Rest -CH₂- steht,
B für Sauerstoffatom, Schwefelatom, Stickstoffatom oder den Rest -CH₂- steht,
X für Sauerstoffatom, Schwefelatom, Stickstoffatom oder den Rest -CH₂- steht,
Y fehlt oder für Sauerstoffatom, Schwefelatom, Stickstoffatom oder den Rest -CH₂- steht, oder
X und Y zusammen einen Phenyl-, 1,2-Diazin-, 1,3-Diazin oder einen 1,4-Diazinrest bilden, wobei das durch N, A, X, Y, B und Kohlenstoffatom gebildete Ringsystem nicht mehr als ein Sauerstoffatom enthält, X nicht Sauerstoffatom, Schwefelatom oder Stickstoffatom darstellt, wenn A Stickstoffatom ist, nicht mehr als ein Schwefelatom enthält,1, 2, 3 oder 4 Stickstoffatome enthält und wobei nicht gleichzeitig ein Sauerstoff- und Schwefelatom vorkommen,
wobei das durch N, A, X, Y, B und Kohlenstoffatom gebildete Ringsystem unsubstituiert ist oder ein-, zwei oder dreifach unabhängig voneinander substituiert ist durch (C₁-C₈)-Alkyl, worin Alkyl unsubstituiert oder ein- oder zweifach substituiert ist durch
1.1. -OH,
1.2. (C₁-C₈)-Alkoxy,
1.3. Halogen,
1.4. -NO₂,
1.5. -NH₂,
1.6. -CF₃,
1.6. -OH,
1.7. Methylendioxy,
1.8. -C(O)-CH₃,
1.9. -CH(O),
1.10. -CN,
1.11. -COOH,
1.12. -C(O)-NH₂,
1.13. (C₁-C₄)-Alkoxycarbonyl.
1.14. Phenyl,
1.15. Phenoxy,
1.16. Benzyl,
1.17. Benzyloxy oder
1.18. Tetrazolyl, oder
- R⁹ und Z: bilden zusammen mit den Kohlenstoffatomen an das sie jeweils gebunden sind einen heterocyclischen Ring der Formel llc, 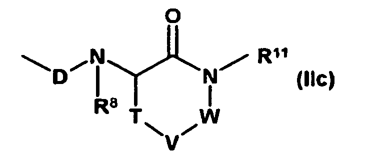 worin
D, R⁸ und R¹¹ wie in Formel II definiert sind,
T für Sauerstoffatom, Schwefelatom, Stickstoffatom oder den Rest -CH₂- steht,
W für Sauerstoffatom, Schwefelatom, Stickstoffatom oder den Rest -CH₂- steht,
V fehlt oder für Sauerstoffatom, Schwefelatom, Stickstoffatom oder den Rest -CH₂- steht, oder
T und V oder V und W zusammen einen Phenyl-, 1,2-Diazin-, 1,3-Diazin oder einen 1,4-Diazinrest bilden, wobei das durch N, T, V, W und zwei Kohlenstoffatome gebildete Ringsystem nicht mehr als ein Sauerstoffatom, nicht mehr als ein Schwefelatom und 1, 2, 3 oder 4 Stickstoffatome enthält, wobei nicht gleichzeitig ein Sauerstoff- und Schwefelatom vorkommen, und wobei das durch N, T, V, W und zwei Kohlenstoffatome gebildete Ringsystem unsubstituiert ist oder ein-, zwei oder dreifach unabhängig voneinander substituiert ist durch die oben unter 1.1. bis 1.18. definierten Substituenten, und
die jeweils anderen Substituenten R¹, R², R³ und R⁴ unabhängig voneinander für
1. Wasserstoffatom,
2. Halogen,
3. (C₁-C₄)-Alkyl,
4. Heteroaryl mit 5 bis 14 Ringgliedern, worin Heteroaryl wie oben definiert ist und unsubstituiert oder wie oben substituiert ist,
5. Heterocyclus mit 5 bis 12 Ringgliedern, worin Heterocyclus wie oben definiert ist und unsubstituiert oder wie oben substituiert ist,
6. (C₁-C₆)-Alkyl,
7. -CN,
8. -O-(C₀-C₄)-Alkyl-Aryl, worin Aryl wie oben definiert ist,
9. -O-(C₁-C₄)-Alkyl,
10. -O-R¹¹,
11. -N(R¹¹)₂,
12. -S(O)ₓ-R¹¹, worin x die ganze Zahl Null, 1 oder 2 ist,
13. -NO₂ oder
14. -CF₃ stehen,

- R⁵: für
1. Wasserstoffatom,
2. -OH oder
3. =O steht, und
- R⁶: für
1. Aryl, worin Aryl wie oben definiert ist und unsubstituiert oder wie oben substituiert ist,
2. Phenyl, ein- oder zweifach substituiert durch
   2.1 -N(R¹¹)₂ ,
   2.2 -NH-C(O)-R¹¹,
   2.3 -S(O)ₓ-R¹¹, worin x die ganze Zahl Null, 1 oder 2 ist,
   2.4 -C(O)-R¹¹oder
   2.5 -(C₁-C₄)-Alkyl-NH₂ ,
3. Heteroaryl mit 5 bis 14 Ringgliedern, worin Heteroaryl wie oben definiert ist und unsubstituiert oder durch -N-(C₃-C₆)-Cycloalkyl oder wie oben substituiert ist oder
4. Heterocyclus mit 5 bis 12 Ringgliedern, worin Heterocyclus wie oben
definiert ist und unsubstituiert oder durch -NH-(C₁-C₆)-Alkyl, -NH-Phenyl, -NH-(C₃-C₆)-Cycloalkyl oder ein-, zwei- oder dreifach wie oben substituiert ist, steht.

Bevorzugt ist eine Verbindung der Formel I, wobei
einer der Substituenten R¹, R², R³ und R⁴ für einen Rest der Formel II steht, worin
- R⁸: für Wasserstoffatom steht,
- R⁹: für
1. charakteristischen Rest einer Aminosäure, der sich von einer natürlich vorkommenden α-Aminosäure aus der Gruppe Histidin, Tryptophan, Serin,
Threonin, Cystein, Methionin, Asparagin, Glutamin, Lysin, Arginin und Glutaminsäure ableitet oder
2. (C₁-C₆)-Alkyl, worin Alkyl gerade oder verzweigt ist und unsubstituiert oder ein- oder zweifach substituiert ist durch einen Rest aus der Gruppe Pyrrol, Pyrrol ein- oder zweifach substituiert durch -(C₁-C₄)-Alkyl, Pyrazol, Phenyl, Imidazol, Triazol, Thiophen, Thiazol, Oxazol, Isoxazol, Pyridin, Pyrimidin, Indol, Benzothiophen, Benzimidazol, Benzoxazol, Benzothiazol, Azetidin, Pyrrolin, Pyrrolidin, Piperidin, Isothiazol, Diazepin, Thiomorpholin, -CN, Morpholin, Azepin, 1,3,4-Oxadiazol, Pyrazin, -N(R¹³)-Phenyl, (C₃-C₆)-Cycloalkyl, -OR¹¹, -NH(R¹¹), worin R¹¹ jeweils wie in Anspruch 1 definiert ist, -S(O)ₓ-R¹², worin x für Null, 1 oder 2 und R¹² Naphthyl, Pyrimidinyl, Morpholinyl oder Phenyl bedeutet, welche unsubstituiert oder ein- oder zweifach substituiert sind durch -OH, (C₁-C₄)-Alkyl, -CF₃, Halogen, -O-(C₁-C₄)-Alkyl, -COOH, -C(O)-O-(C₁-C₄)-Alkyl, -NH₂ oder -NH-C(O)-(C₁-C₄)-Alkyl,
- Z: für -C(O)-R¹⁰, Tetrazol, (C₁-C₆)-Alkyl, worin Alkyl gerade oder verzweigt ist und unsubstituiert oder ein- oder zweifach substituiert ist durch Phenyl, oder -OH, oder 1,3,4-Oxadiazol, worin1,3,4- Oxadiazol unsubstituiert oder einfach substituiert ist durch -NH₂, -NH(C₁-C₄)-Alkyl, -N-[(C₁-C₄)-Alkyl]₂, -NH-C(O)-(C₁-C₄)-Alkyl,- NH-C(O)-NH-(C₁-C₄)-Alkyl, -NH-C(O)-NH-(C₃-C₇)-Cyloalkyl, -NH-C(O)-NH-Aryl, worin Aryl ausgewählt ist aus der Gruppe Phenyl, 1-Naphthyl, 2-Naphthyl, 2-Biphenylyl, 3-Biphenylyl, 4-Biphenylyl, Anthryl und Fluorenyl, -NH-C(O)-NH-Phenyl, -NH-SO₂-Aryl, worin Aryl wie oben definiert ist ,-NH-SO₂-(C₁-C₄)-Alkyl, -OH oder -(C₁-C₄)-Alkyl, steht, worin
- R¹⁰: für -O-R¹¹, Phenyl, Pyrimidin, -OH, Morpholinyl, -N(R¹¹)₂ oder -NH₂ steht,
- R¹¹: für
1. -(C₁-C₄)-Alkyl,
2. R¹³ oder
3. -N(R¹³)₂, worin
R¹³ unabhängig voneinander
a) Wasserstoffatom,
b) -(C₁-C₆)-Alkyl,
c) -(C₁-C₄)-Alkyl-O-(C₁-C₄)-Alkyl,
d) -(C₁-C₆)-Alkyl-N-(C₁-C₆)ₙ-Alkyl, worin n die ganze Zahl Null,1 oder 2 bedeutet und Alkyl unsubstituiert oder ein-, zwei- oder dreifach unabhängig voneinander durch Halogen oder durch -COOH substituiert ist,
e) Halogen oder
f) -(C₀-C₄)-Alkyl, ein- oder zweifach substituiert durch Aryl, worin Aryl wie oben definiert ist, imidazolyl oder Morpholinyl, steht oder
- R⁸ und R⁹: bilden zusammen mit dem Stickstoff- und Kohlenstoffatom an das sie jeweils gebunden sind einen Ring der Formel IIa aus der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, Imidazolidin, Oxazol, Isoxazol, 2-Isoxazolidin, Isoxazolidin, Morpholin, Isothiazol, Thiazol, Tetrazol, 1,2,3,5-Oxathiadiazol-2-Oxide, Oxadiazolone, Isoxazolone, Triazolone, oxadiazolidindione, Triazole, welche durch F, CN, CF₃ oder COO-(C₁-C₄)-Alkyl substituert sind, 3-Hydroxypyrro-2,4-dione, 5-Oxo-1,2,4-Thiadiazole, 1,3,4-Oxadiazol, Isothiazolidin, Thiomorpholin, Indazol, Thiadiazol, Benzimidazol, Chinolin, Triazol, Phthalazin, Chinazolin, Chinoxalin, Purin, Pteridin, Indol, Tetrahydrochinolin, Tetrahydroisochinolin und Isochinolin, oder
- R⁹ und Z: bilden zusammen mit den Kohlenstoffatomen an das sie jeweils gebunden sind einen Ring der Formel IIc aus der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, Imidazolidin, Oxazol, Isoxazol, 2-Isoxazolidin, Isoxazolidin, Morpholin, Isothiazol, Thiazol, Isothiazolidin, Thiomorpholin, Indazol, Thiadiazol. Benzimidazol, Chinolin, Triazol, Phthalazin, Chinazolin, Chinoxalin, Purin, Pteridin, Indol, Tetrahydrochinolin, Tetrahydroisochinolin, Isochinolin, Tetrazol, 1,2,3,5-Oxathiadiazol-2-Oxide, Oxadiazolone, Isoxazolone, Triazolone, oxadiazolidindione, Triazole, welche durch F, CN, CF₃ oder COO-(C₁-C₄)-Alkyl substituert sind, 3-Hydroxypyrro-2,4-dione, 1,3,4-Oxadiazol und 5-Oxo-1,2,4-Thiadiazole und
die jeweils anderen Substituenten R¹, R², R³ und R⁴ unabhängig voneinander für
1. Wasserstoffatom,
2. Halogen,
3. (C₁-C₄)-Alkyl,
4. -CN,
5. -NO₂,
6. -O-(C₀-C₄)-Alkyl-Aryl, worin Aryl wie oben definiert ist,
7. -O-(C₁-C₄)-Alkyl,
8. -N-(C₀-C₄)-Alkyl-Aryl, worin Aryl wie oben definiert ist,
9. -N-(C₁-C₄)-Alkyl oder
10. -CF₃ stehen,

- R⁵: für
1. Wasserstoffatom,
2. -OH oder
3. =0 steht, und
- R⁶: für
1. Phenyl, ein oder zweifach substituiert durch
   1.1 -CN,
   1.2 -NO₂,
   1.3 -O-(C₁-C₄)-Alkyl,
   1.4 -NH₂ oder
   1.5 -(C₁-C₄)-Alkyl-NH₂
2. Heteroaryl mit 5 bis 14 Ringgliedern, worin Heteroaryl für einen Rest steht aus der Gruppe Pyrrol, Furan, Thiophen, Imidazol, Pyrazol, Oxazol, lsoxazol, Thiazol, Isothiazol, Tetrazol, 1,3,4-Oxadiazol, 1,2,3,5-Oxathiadiazol-2-Oxide, Triazolone, oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anelüerte Derivate dieser Heterocyclen, und worin Heteroaryl unsubstituiert oder ein- bis dreifach substituiert durch -N-R¹⁴, worin R¹⁴ -(C₁-C₆)-Alkyl, -(C₃-C₆)-Cycloalkyl oder Phenyl bedeutet, Halogen, -OH oder -(C₁-C₄)-Alkyl, oder
3. Heterocyclus mit 5 bis 12 Ringgliedern, worin Heterocyclus für einen monocyclischen oder bicyclischen 5-gliedrigen bis 12-gliedrigen heterocyclischen Ring steht, der teilweise gesättigt oder vollständig gesättigt aus der Gruppe Pyrrol, Furan, Thiophen, imidazol, Pyrazol, Oxazol, Isoxazol, Thiazol, isothiazol, Tetrazol, 1,3,4-Oxadiazot, 1,2,3,5-0xathiadiazot-2-Oxide, Triazolone, oxadiazolone, isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen und worin Heterocyclus unsubstituiert oder ein- bis dreifach substituiert durch -N-R¹⁴, worin R¹⁴ -(C₁-C₆)-Alkyl, -(C₃-C₆)-Cycloalkyl oder Phenyl bedeutet, Halogen, -OH oder -(C₁-C₄)-Alkyl, steht.

Die Erfindung betrifft ferner die Verwendung von mindestens einer Verbindung der Formel I 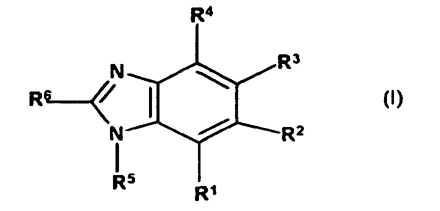 und/oder eine stereoisomere Form der Verbindung der Formel I und/oder ein physiologisch verträgliches Salz der Verbindung der Formel I,
zur Herstellung von Arzneimitteln zur Prophylaxe und Therapie von Erkrankungen aus der Reihe Asthma, Rheumatoide Arthritis, Osteoarthritis, Alzheimers Erkrankungen, Krebserkrankungen, Herzinfarkt, Herzinsuffizienz, akutes Koronarsyndrom, instabile Angina Pectoris, septischer Schock, akutes und chronisches Nierenversagen, Stroke oder Atherosklerose, wobei einer der Substituenten R¹, R², R³ und R⁴ für einen Rest der Formel II steht, 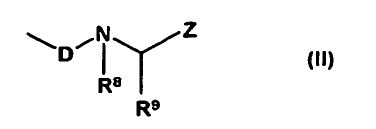 worin
- D: für -C(O)-. -S(O)- oder -S(O)₂- steht,
- R⁸: für Wasserstoffatom oder (C₁-C₄)-Alkyl steht,
- R⁹: für
1. charakteristischen Rest einer Aminosäure, der sich von einer natürlich vorkommenden α-Aminosäure aus der Gruppe Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin und Glutaminsäure ableitet, oder der sich von einer nicht natürlich vorkommenden Aminosäure aus der Gruppe 2-Aminoadipinsäure, 2-Aminobuttersäure, 2-Aminoisobutter-säure, 2,3-Diaminopropionsäure, 2,4-Diaminobuttersäure, 1,2,3,4-Tetra-hydroisochinotin-1-carbonsäure, 1,2,3,4-Tetrahydroisochinolin-3-carbon-säure, 2-Amino-pimelinsäure, Phenylglycin, 3-(2-Thienyl)-alanin, 3-(3-Thienyl)-alanin, Sarkosin, 2-(2-Thienyl)-glycin, 2-Amino-heptansäure, Pipecolinsäure, Hydroxylysin, N-Methylisoleucin, 6-N-Methyllysin, N-Methylvalin, Norvalin, Norleucin, Ornithin, allo-Isoleucin, 4-Hydroxy-prolin, allo-Hydroxylysin, allo-Threonin, 3-Hydroxyprolin, 3-(2-Naphtyl)-alanin, 3-(1-Naphtylalanin), Homophenylalanin, Homocystein, 2-Amino-3-phenylamino-ethylpropion-säure, Homocysteinsäure, Homotryptophan, Cysteinsäure, 3-(2-Pyridyl)-alanin, 3-(3-Pyridyl)-alanin, 3-(4-Pyridyl)-alanin, Phosphino-thricin, 4-Fluorphenylalanin, 3-Fluorphenyl-alanin, 2-Fluorphenylalanin, 4-Chlorphenylalanin, 4-Nitrophenylalanin, 4-Aminophenyfalanin, Cyclo-hexylalanin, Citrullin, 5-Fluortryptophan, 5-Methoxytryptophan, 2-Amino-3-phenylamino-propionsäure, Methionin-Sulfon, Methionin-Sulfoxid oder -NH-NR¹¹-CON(R¹¹)₂ ableitet,
2. Aryl, worin Aryl für einen aromatischen Kohlenstoffrest mit 6 bis 14 Kohlenstoffatomen im Ring steht aus der Gruppe Phenyl, 1-Naphthyl, 2-Naphthyl, 2-Biphenylyl, 3-Biphenylyl, 4-Biphenylyl, Anthryl oder Fluorenyl, worin Aryl unsubstituiert oder ein-, zwei- oder dreifach durch gleiche oder verschiedene Reste substituiert ist aus der Reihe (C₁-C₈)-Alkyl, (C₁-C₈)-Alkoxy, Halogen, Nitro, Amino, Trifluormethyl, Hydroxy, Hydroxy-methyl, 1-Hydroxyethyl, 2-Hydroxyethyl, Methylendioxy, Ethylendioxy, Formyl, Acetyl, Cyano, Hydroxycarbonyl, Aminocarbonyl, (C₁-C₄)-Alkoxy-carbonyl. Phenyl, Phenoxy, Benzyl, Benzyloxy oder Tetrazolyl,
3. Heteroaryl mit 5 bis 14 Ringgliedern, worin Heteroaryl für einen Rest steht aus der Gruppe Pyrrol, Furan, Thiophen, imidazol, Pyrazol, Oxazol, Isoxazol, Thiazol, Isothiazol, Tetrazot, 1,3,4-Oxadiazot, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen,
   worin Heteroary unsubstituiert oder ein-, zwei- oder dreifach durch gleiche oder verschiedene Reste substituiert ist aus der Reihe (C₁-C₈)-Alkyl, (C₁-C₈)-Alkoxy, Halogen, Nitro, -N(R¹¹)₂, Trifluormethyl, Hydroxy, Hydroxymethyl, 1-Hydroxyethyl, 2-Hydroxyethyl, Methylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy oder Tetrazolyl,
4. Heterocyclus mit 5 bis 12 Ringgliedern, worin Heterocyclus für einen monocyclischen oder bicyclischen 5-gliedrigen bis 12-gliedrigen heterocyclischen Ring steht, der teilweise gesättigt oder vollständig gesättigt ist aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen,
   worin Heterocyclus unsubstituiert oder ein-, zwei-, drei oder vierfach durch gleiche oder verschiedene Reste substituiert ist aus der Reihe (C₁-C₈)-Alkyl, (C₁-C₈)-Alkoxy, Phenyl-(C₁-C₄)-alkoxy, Hydroxy, Oxo, Halogen, Nitro, Amino oder Trifluormethyl, wobei die Stickstoffheterocyclen auch als N-Oxide vorliegen können oder als Quartärsalze,
5. (C₁-C₆)-Alkyl, worin Alkyl gerade oder verzweigt ist und unsubstituiert oder ein-, zwei- oder dreifach unabhängig voneinander substituiert ist durch
   5.1 Aryl, worin Aryl wie oben definiert ist und unsubstituiert oder wie oben substituiert ist,
   5.2 Heteroaryl mit 5 bis 14 Ringgliedern, worin Heteroaryl wie oben definiert ist und unsubstituiert oder wie oben substituiert ist,
   5.3 Heterocyclus mit 5 bis 12 Ringgliedern, worin Heterocyclus wie oben definiert ist und unsubstituiert oder wie oben substituiert ist
   5.4 ein Rest aus der Gruppe Azetidin, Pyrrolin, Pyrrolidin, Piperidin, Isothiazol, Diazepin, Thiomorpholin, Morpholin oder Azepin,
   5.5 -O-R¹¹,
   5.6 =O,
   5.7 Halogen,
   5.8 -CN,
   5.9 -CF₃,
   5.10 -S(O)ₓ-R¹¹, worin x die ganze Zahl Null, 1 oder 2 ist,
   5.11 -C(O)-O-R¹¹,
   5.12 -C(O)-N(R¹¹)₂,
   5.13 -N(R¹¹)₂,
   5.14 (C₃-C₆)-Cycloalkyl,
   5.15 Rest der Formel 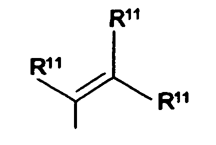 oder
   5.16 Rest der Formel 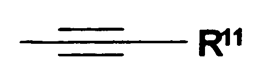 worin
- R¹¹: für
a) Wasserstoffatom,
b) (C₁-C₆)-Alkyl, worin Alkyl unsubstituiert oder ein-, zwei- oder dreifach substituiert ist durch
   1. Aryl, worin Aryl wie oben definiert ist und unsubstituiert oder wie oben substituiert ist,
   2. Heteroaryl mit 5 bis 14 Ringgliedern, worin Heteroaryl wie oben definiert ist,
   3. Halogen,
   4. -N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
   5. -O-(C₁-C₆)-Alkyl oder
   6. -COOH,
c) Aryl, worin Aryl wie oben definiert ist und unsubstituiert oder wie oben substituiert ist,
d) Heteroaryl mit 5 bis 14 Ringgliedern, worin Heteroaryl wie oben definiert ist,
e) Halogen oder
f) -N(R¹³)₂, worin
   R¹³ unabhängig voneinander für
   f) a) Wasserstoffatom,
   f) b) -(C₁-C₆)-Alkyl,
   f) c) -(C₁-C₄)-Alkyl-O-(C₁-C₄)-Alkyl,
   f) d) -(C₁-C₆)-Alkyl-N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
   f) e) Halogen oder
   f) f) -(C₀-C₄)-Alkyl, ein- oder zweifach substituiert durch Phenyl, 1-Naphthyl, 2-Naphthyl, 2-Biphenylyl, 3-Biphenylyl, 4-Biphenylyl, Anthryl, Fluorenyl, Imidazolyl oder Morpholinyl, bedeutet, steht, und
für den Fall des (R¹¹)₂ hat R¹¹ unabhängig voneinander die Bedeutung von a) bis f)
- Z: für
1. Aryl, worin Aryl wie oben definiert ist und unsubstituiert oder wie oben substituiert ist,
2. Heteroaryl mit 5 bis 14 Ringgliedern, worin Heteroaryl wie oben definiert ist und unsubstituiert oder wie oben substituiert ist,
3. Heterocyclus mit 5 bis 12 Ringgliedern, worin Heterocyclus wie oben definiert ist und unsubstituiert oder wie oben substituiert ist,
4. 1,3,4-Oxadiazol, worin1,3,4- Oxadiazol unsubstituiert oder einfach substituiert ist durch -NH-C(O)-(C₁-C₄)-Alkyl,- NH-C(O)-NH-(C₁-C₄)-Alkyl, -NH-C(O)-NH-(C₃-C₇)-Cyloalkyl, -NH-C(O)-NH-Aryl, worin Aryl wie oben definiert ist, -NH-C(0)-NH-Phenyl, -NH-SO₂-Aryl, worin Aryl wie oben definiert ist, oder -NH-SO₂-(C₁-C₄)-Alkyl,
5. -(C₁-C₆)-Alkyl, worin Alkyl gerade oder verzweigt ist und unsubstituiert oder ein- oder zweifach substituiert ist durch Phenyl oder -OH, oder
6. -C(O)-R¹⁰ steht, worin
   R¹⁰ für
   1. -O-R¹¹,
   2. -N(R¹¹)₂,
   3. Phenyl,
   4. Pyrimidinyl oder
   5. Morholinyl, steht oder
- R⁸ und R⁹: bilden zusammen mit dem Stickstoff- und Kohlenstoffatom an das sie jeweils gebunden sind einen heterocyclischen Ring der Formel IIa, 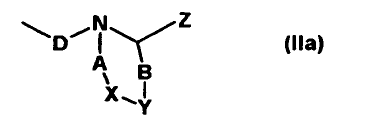 worin D und Z wie in Formel II definiert sind,
A für Stickstoffatom oder den Rest -CH₂- steht,
B für Sauerstoffatom, Schwefelatom, Stickstoffatom oder den Rest -CH₂- steht,
X für Sauerstoffatom, Schwefelatom, Stickstoffatom oder den Rest -CH₂- steht,
Y fehlt oder für Sauerstoffatom, Schwefelatom, Stickstoffatom oder den Rest -CH₂- steht, oder
X und Y zusammen einen Phenyl-, 1,2-Diazin-, 1,3-Diazin oder einen 1,4-Diazinrest bilden, wobei das durch N, A, X, Y, B und Kohlenstoffatom gebildete Ringsystem nicht mehr als ein Sauerstoffatom enthält, X nicht Sauerstoffatom, Schwefelatom oder Stickstoffatom darstellt, wenn A Stickstoffatom ist, nicht mehr als ein Schwefelatom enthält, 1, 2, 3 oder 4 Stickstoffatome enthält und wobei nicht gleichzeitig ein Sauerstoff- und Schwefelatom vorkommen,
wobei das durch N, A, X, Y, B und Kohlenstoffatom gebildete Ringsystem unsubstituiert ist oder ein-, zwei oder dreifach unabhängig voneinander substituiert ist durch (C₁-C₈)-Alkyl, worin Alkyl unsubstituiert oder ein- oder zweifach substituiert ist durch
1.1. -OH,
1.2. (C₁-C₈)-Alkoxy,
1.3. Halogen,
1.4. -NO₂,
1.5. -NH₂,
1.6. -CF₃,
1.6. -OH,
1.7. Methylendioxy,
1.8. -C(O)-CH₃,
1.9. -CH(O),
1.10. -CN,
1.11. -COOH,
1.12. -C(O)-NH₂,
1.13. (C₁-C₄)-Alkoxycarbonyl,
1.14. Phenyl,
1.15. Phenoxy,
1.16. Benzyl,
1.17. Benzyloxy oder
1.18. Tetrazolyl, oder
- R⁹ und Z: bilden zusammen mit den Kohlenstoffatomen an das sie jeweils gebunden sind einen heterocyclischen Ring der Formel IIc, 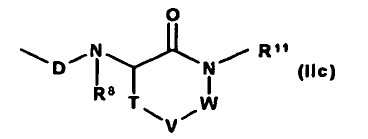 worin D, R⁸ und R¹¹ wie in Formel II definiert sind,
T für Sauerstoffatom, Schwefelatom, Stickstoffatom oder den Rest -CH₂- steht,
W für Sauerstoffatom, Schwefelatom, Stickstoffatom oder den Rest -CH₂- steht,
V fehlt oder für Sauerstoffatom, Schwefelatom, Stickstoffatom oder den Rest -CH₂- steht, oder
T und V oder V und W zusammen einen Phenyl-, 1,2-Diazin-, 1,3-Diazin oder einen 1,4-Diazinrest bilden,
wobei das durch N, T, V, W und zwei Kohlenstoffatome gebildete Ringsystem nicht mehr als ein Sauerstoffatom, nicht mehr als ein Schwefelatom und 1, 2, 3 oder 4 Stickstoffatome enthält, wobei nicht gleichzeitig ein Sauerstoff- und Schwefelatom vorkommen, und wobei das durch N, T, V, W und zwei Kohlenstoffatome gebildete Ringsystem unsubstituiert ist oder ein-, zwei oder dreifach unabhängig voneinander substituiert ist durch die oben unter 1.1. bis 1.18. definierten Substituenten, und
die jeweils anderen Substituenten R¹, R², R³ und R⁴ unabhängig voneinander für
1. Wasserstoffatom,
2. Halogen,
3. (C₁-C₄)-Alkyl,
4. Heteroaryl mit 5 bis 14 Ringgliedern, worin Heteroaryl wie oben definiert ist und unsubstituiert oder wie oben substituiert ist,
5. Heterocyclus mit 5 bis 12 Ringgliedern, worin Heterocyclus wie oben definiert ist und unsubstituiert oder wie oben substituiert ist,
6. (C₁-C₆)-Alkyl,
7. -CN,
8. -O-(C₀-C₄)-Alkyl-Aryl, worin Aryl wie oben definiert ist,
9. -O-(C₁-C₄)-Alkyl,
10. -OR¹¹,
11. -N(R¹¹)₂,
12. -S(O)ₓ-R¹¹, worin x die ganze Zahl Null, 1 oder 2 ist,
13. -NO₂ oder
14. -CF₃ stehen,

- R⁵: für
1. Wasserstoffatom,
2. -OH oder
3. =O steht, und
- R⁶: für
1. Aryl, worin Aryl wie oben definiert ist und unsubstituiert oder wie oben substituiert ist,
2. Phenyl, ein- oder zweifach substituiert durch
   2.1 -N(R¹¹)₂,
   2.2 -NH-C(O)-R¹¹,
   2.3 -S(O)ₓ-R¹¹, worin x die ganze Zahl Null, 1 oder 2 ist,
   2.4 -C(O)-R¹¹oder
   2.5 -(C₁-C₄)-Alkyl-NH₂ ,
3. Heteroaryl mit 5 bis 14 Ringgliedern, worin Heteroaryl wie oben definiert ist und unsubstituiert oder durch -N-(C₃-C₆)-Cycloalkyl oder wie oben substituiert ist oder
4. Heterocyclus mit 5 bis 12 Ringgliedern, worin Heterocyclus wie oben definiert ist und unsubstituiert oder durch -NH-(C₁-C₆)-Alkyl, -NH-Phenyl, -NH-(C₃-C₆)-Cycloalkyl oder ein-, zwei- oder dreifach wie oben substituiert ist, steht.

Die Erfindung betrifft ferner die genannte Verwendung wobei
einer der Substituenten R¹, R², R³ und R⁴ für einen Rest der Formel II steht, worin
- R⁸: für Wasserstoffatom steht,
- R⁹: für
1. charakteristischen Rest einer Aminosäure, der sich von einer natürlich vorkommenden α-Aminosäure aus der Gruppe Histidin, Tryptophan, Serin, Threonin,
Cystein, Methionin, Asparagin, Glutamin, Lysin, Arginin und Glutaminsäure ableitet oder
2. (C₁-C₆)-Alkyl, worin Alkyl gerade oder verzweigt ist und unsubstituiert oder ein- oder zweifach substituiert ist durch einen Rest aus der Gruppe Pyrrol, Pyrrol ein- oder zweifach substituiert durch -(C₁-C₄)-Alkyl, Pyrazol, Phenyl, Imidazol, Triazol, Thiophen, Thiazol, Oxazol, Isoxazol, Pyridin, Pyrimidin, Indol, Benzothiophen, Benzimidazol, Benzoxazol, Benzothiazol, Azetidin, Pyrrolin, Pyrrolidin, Piperidin, Isothiazol, Diazepin, Thiomorpholin, -CN, Morpholin, Azepin, 1,3,4-Oxadiazot, Pyrazin, -N(R¹³)-Phenyl, (C₃-C₆)-Cycloalkyl, -OR¹¹, -NH(R¹¹), worin R¹¹ jeweils wie in Anspruch 1 definiert ist, -S(O)ₓ-R¹², worin x für Null, 1 oder 2 und R¹² Naphthyl, Pyrimidinyl, Morpholinyl oder Phenyl bedeutet, welche unsubstituiert oder ein- oder zweifach substituiert sind durch -OH, (C₁-C₄)-Alkyl, -CF₃, Halogen, -O-(C₁-C₄)-Alkyl, -COOH, -C(O)-O-(C₁-C₄)-Alkyl, -NH₂ oder -NH-C(O)-(C₁-C₄)-Alkyl,
- Z: für -C(O)-R¹⁰, Tetrazol, (C₁-C₆)-Alkyl, worin Alkyl gerade oder verzweigt ist und unsubstituiert oder ein- oder zweifach substituiert ist durch Phenyl, oder -OH, oder 1,3,4-Oxadiazol, worin1,3,4- Oxadiazol unsubstituiert oder einfach substituiert ist durch -NH₂, -NH(C₁-C₄)-Alkyl, -N-[(C₁-C₄)-Alkyl]₂, -NH-C(O)-(C₁-C₄)-Alkyl,- NH-C(O)-NH-(C₁-C₄)-Alkyl, -NH-C(O)-NH-(C₃-C₇)-Cyloalkyl, -NH-C(O)-NH-Aryl, worin Aryl ausgewählt ist aus der Gruppe Phenyl, 1-Naphthyl, 2-Naphthyl, 2-Biphenylyl, 3-Biphenylyl, 4-Biphenylyl, Anthryl und Fluorenyl, -NH-C(O)-NH-Phenyl, -NH-SO₂-Aryl, worin Aryl wie oben definiert ist ,-NH-SO₂-(C₁-C₄)-Alkyl, -OH oder -(C₁-C₄)-Alkyl, steht, worin
- R¹⁰: für -O-R¹¹, Phenyl, Pyrimidin, -OH, Morpholinyl, -N(R¹¹)₂ oder -NH₂ steht,
- R¹¹: für
1. -(C₁-C₄)-Alkyl,
2. R¹³ oder
3. -N(R¹³)₂, worin
   R¹³ unabhängig voneinander
   a) Wasserstoffatom,
   b) -(C₁-C₆)-Alkyl,
   c) -(C₁-C₄)-Alkyl-O-(C₁-C₄)-Alkyl,
   d) -(C₁-C₆)-Alkyl-N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
   e) Halogen oder
   f) -(C₀-C₄)-Alkyl, ein- oder zweifach substituiert durch Aryl, worin Aryl wie oben definiert ist, Imidazolyl oder Morpholinyl, steht oder
- R⁸ und R⁹: bilden zusammen mit dem Stickstoff- und Kohlenstoffatom an das sie jeweils gebunden sind einen Ring der Formel IIa aus der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, Imidazolidin, Oxazol, Isoxazol, 2-Isoxazolidin, Isoxazolidin, Morpholin, Isothiazol, Thiazol, Tetrazol, 1,2,3,5-Oxathiadiazol-2-Oxide, Oxadiazolone, lsoxazolone, Triazolone, oxadiazolidindione, Triazole, welche durch F, CN, CF₃ oder COO-(C₁-C₄)-Alkyl substituert sind, 3-Hydroxypyrro-2,4-dione, 5-Oxo-1,2,4-Thiadiazole,1,3,4-Oxadiazol, Isothiazolidin, Thiomorpholin, Indazol. Thiadiazol, Benzimidazol, Chinolin, Triazol, Phthalazin, Chinazolin, Chinoxalin, Purin, Pteridin, Indol, Tetrahydrochinolin, Tetrahydroisochinolin und Isochinolin, oder
- R⁹ und Z: bilden zusammen mit den Kohlenstoffatomen an das sie jeweils gebunden sind einen Ring der Formel IIc aus der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, lmidazolidin, Oxazol, Isoxazol, 2-Isoxazolidin, Isoxazolidin, Morpholin, Isothiazol, Thiazol, Isothiazolidin, Thiomorpholin, Indazot, Thiadiazol, Benzimidazol, Chinolin, Triazol, Phthalazin, Chinazolin, Chinoxalin, Purin, Pteridin, Indol, Tetrahydrochinolin, Tetrahydroisochinolin, Isochinolin, Tetrazol, 1,2,3,5-Oxathiadiazol-2-Oxide, Oxadiazolone, Isoxazolone, Triazolone, Oxadiazolidindione, Triazole, welche durch F, CN, CF₃ oder COO-(C₁-C₄)-Alkyl substituert sind, 3-Hydroxypyrro-2,4-dione, 1,3,4-Oxadiazol und 5-Oxo-1,z,4-Thiadiazole und
die jeweils anderen Substituenten R¹, R², R³ und R⁴ unabhängig voneinander für
1. Wasserstoffatom,
2. Halogen,
3. (C₁-C₄)-Alkyl,
4. -CN,
5. -NO₂,
6. -O-(C₀-C₄)-Alkyl-Aryl, worin Aryl wie oben definiert ist,
7. -O-(C₁-C₄)-Alkyl,
8. -N-(C₀-C₄)-Alkyl-Aryl, worin Aryl wie oben definiert ist,
9. -N-(C₁-C₄)-Alkyl oder
10. -CF₃ stehen,

- R⁵: für
1. Wasserstoffatom,
2. -OH oder
3. =O steht, und
- R⁶: für
1. Phenyl, ein oder zweifach substituiert durch
   1.1 -CN,
   1.2 -NO₂,
   1.3 -O-(C₁-C₄)-Alkyl,
   1.4 -NH₂ oder
   1.5 -(C₁-C₄)-Alkyl-NH₂
2. Heteroaryl mit 5 bis 14 Ringgliedern, worin Heteroaryl für einen Rest steht aus der Gruppe Pyrrol, Furan, Thiophen, Imidazol, Pyrazol, Oxazol, Isoxazol, Thiazol, isothiazol, Tetrazol, 1,3,4-Oxadiazol, 1,2,3,5-Oxathiadiazol-2-Oxide, Triazolone, oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cydopenta-, cyclohexa- oder cyclohepta-anellierte Derivate dieser Heterocyclen, und worin Heteroaryl unsubstituiert oder ein- bis dreifach substituiert durch -N-R¹⁴, worin R¹⁴ -(C₁-C₆)-Alkyl, -(C₃-C₆)-Cycloalkyl oder Phenyl bedeutet, Halogen, -OH oder -(C₁-C₄)-Alkyl, oder
3. Heterocyclus mit 5 bis 12 Ringgliedern, worin Heterocyclus für einen monocydischen oder bicydischen 5-gliedrigen bis 12-gliedrigen heterocyclischen Ring steht, der teilweise gesättigt oder vollständig gesättigt aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen und worin Heterocyclus unsubstituiert oder ein- bis dreifach substituiert durch -N-R¹⁴, worin R¹⁴ -(C₁-C₆)-Alkyl, -(C₃-C₆)-Cycloalkyl oder Phenyl bedeutet, Halogen, -OH oder -(C₁-C₄)-Alkyl, steht.

Unter dem Begriff "Halogen" wird Fluor, Chlor, Brom oder Jod verstanden. Unter dem Begriff "(C₁-C₆)-Alkyl" werden Kohlenwasserstoffreste verstanden, deren Kohlenstoffkette gerade oder verzweigt ist und 1 bis 6 Kohlenstoffatome enthält. Unter dem Begriff "C₀-Alkyl" wird eine kovalente Bindung verstanden. Cyclische Alkylreste sind beispielsweise 3- bis 6-gliedrige Monocyclen wie Cyclopropyl, Cyclobutyl, Cyclopentyl oder Cyclohexyl.

Unter dem Begriff "R⁸ und R⁹ bilden zusammen mit dem Stickstoff- und Kohlenstoffatom an das sie jeweils gebunden sind einen heterocyclischen Ring der Formel IIa" werden Reste verstanden die sich von Pyrrol, Pyrrolin, Pyrrolidin, Imidazol, Pyrazol, Oxazol, Tetrazol, 1,2,3,5-Oxathiadiazol-2-Oxide, Triazolone, Oxadiazolone, Isoxazolone, 1,3,4-Oxadiazol, Oxadiazolidindione, Triazole, welche durch F, CN, CF₃ oder COO-(C₁-C₄)-Alkyl substituert sind, 3-Hydroxypyrro-2,4-dione, 5-Oxo-1,2,4-Thiadiazole, Isoxazol, Indol, Isoxazolin, Isoxazolidin, Morpholin, Thiazol, Isothiazol, Isothiazolin, Purin, Isothiazolidin, Thiomorpholin, Pyridin, Piperidin, Pyrazin, Piperazin, Pyrimidin, Pyridazin, Isoindol, Indazol, Benzimidazol, Phthalazin, Chinolin, Isochinolin, Chinoxalin, Chinazolin, Cinnolin, Pteridin, lmidazolidin, Carbolin und benz-annellierte Derivate dieser Heterocyclen ableiten.

Unter dem Begriff Aryl werden aromatische Kohlenstoffreste verstanden mit 6 bis 14 Kohlenstoffatomen im Ring. (C₆-C₁₄)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Arylreste, insbesondere Phenylreste, können einfach oder mehrfach, bevorzugt einfach, zweifach oder dreifach, durch gleiche oder verschiedene Reste substituiert sein, bevorzugt durch Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Hydroxy-(C₁-C₄)-alkyl wie Hydroxymethyl oder 1-Hydroxyethyl oder 2-Hydroxyethyl, Methylendioxy, Ethylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy, Tetrazolyl substituiert sein. Entsprechendes gilt beispielsweise für Reste wie Arylalkyl oder Arylcarbonyl. Arylalkylreste sind insbesondere Benzyl sowie 1- und 2-Naphthylmethyl, 2-, 3- und 4-Biphenylylmethyl und 9-Fluorenylmethyl. Substituierte Arylalkylreste sind beispielsweise durch einen oder mehrere (C₁-C₈)-Alkylreste, insbesondere (C₁-C₄)-Alkylreste, im Arylteil substituierte Benzylreste und Naphthylmethylreste, zum Beispiel 2-, 3- und 4-Methylbenzyl, 4-Isobutylbenzyl, 4-tert-Butylbenzyl, 4-Octylbenzyl, 3,5-Dimethylbenzyl, Pentamethylbenzyl, 2-, 3-, 4-, 5-, 6-, 7- und 8-Methyl-1-naphthylmethyl, 1-, 3-, 4-, 5-, 6-, 7- und 8-Methyl-2-naphthylmethyl, durch einen oder mehrere (C₁-C₈)-Alkoxyreste, insbesondere (C₁-C₄)-Alkoxyreste, im Arylteil substituierte Benzylreste und Naphthylmethylreste, zum Beispiel 4-Methoxybenzyl, 4-Neopentyloxybenzyl, 3,5-Dimethoxybenzyl, 3,4-Methylendioxybenzyl, 2,3,4-Trimethoxybenzyl, Nitrobenzylreste, zum Beispiel 2-, 3- und 4-Nitrobenzyl, Halobenzylreste, zum Beispiel 2-, 3- und 4-Chlor- und 2-, 3-, und 4-Fluorbenzyl, 3,4-Dichlorbenzyl, Pentafluorbenzyl, Trifluormethylbenzylreste, zum Beispiel 3- und 4-Trifluormethylbenzyl oder 3,5-Bis(trifluormethyl)benzyl.

In monosubstituierten Phenylresten kann sich der Substituent in der 2-Position, der 3-Position oder der 4-Position befinden. Zweifach substituiertes Phenyl kann in der 2,3-Position, der 2,4-Position, der 2,5-Position, der 2,6-Position, der 3,4-Position oder der 3,5-Position substituiert sein. In dreifach substituierten Phenylresten können sich die Substituenten in der 2,3,4-Position, der 2,3,5-Position, der 2,4,5-Position, der 2,4,6-Position, der 2,3,6-Position oder der 3,4,5-Position befinden.

Die Erläuterungen zu den Arylresten gelten entsprechend für zweiwertige Arylenreste, zum Beispiel für Phenylenreste, die beispielsweise als 1,4-Phenylen oder als 1,3-Phenylen vorliegen können.

Phenylen-(C₁-C₆)-alkyl ist insbesondere Phenylenmethyl (-C₆H₄-CH₂-) und Phenylenethyl, (C₁-C₆)-Alkylen-phenyl insbesondere Methylenphenyl (-CH₂-C₆H₄-). Phenylen-(C₂-C₆)-alkenyl ist insbesondere Phenylenethenyl und Phenylenpropenyl.

Der Begriff "Heteroaryl mit 5 bis 14 Ringgliedern" steht für einen Rest eines monocyclischen oder polycyclischen aromatischen Systems mit 5 bis 14 Ringgliedern, das 1, 2, 3, 4 oder 5 Heteroatome als Ringglieder enthält. Beispiele für Heteroatome sind N, O und S. Sind mehrere Heteroatome enthalten, können diese gleich oder verschieden sein. Heteroarylreste können ebenfalls einfach oder mehrfach, bevorzugt einfach, zweifach oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, -N(R¹¹)₂, Trifluormethyl, Hydroxy, Hydroxy-(C₁-C₄)-alkyl wie Hydroxymethyl oder 1-Hydroxyethyl oder 2-Hydroxyethyl, Methylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy, Tetrazolyl substituiert sein. Bevorzugt steht Heteroaryl mit 5 bis 14 Ringgliedern für einen monocyclischen oder bicyclischen aromatischen Rest, der 1, 2, 3 oder 4, insbesondere 1, 2 oder 3, gleiche oder verschiedene Heteroatome aus der Reihe N, O und S enthält und der durch 1, 2, 3 oder 4, insbesondere 1 bis 3, gleiche oder verschiedene Substituenten aus der Reihe (C₁-C₆)-Alkyl, (C₁-C₆)-Alkoxy, Fluor, Chlor, Nitro, -N(R¹¹)₂, Trifluormethyl, Hydroxy, Hydroxy-(C₁-C₄)-alkyl, (C₁-C₄)-Alkoxycarbonyl, Phenyl, Phenoxy, Benzyloxy und Benzyl substituiert sein kann. Besonders bevorzugt steht Heteroaryl für einen monocyclischen oder bicyclischen aromatischen Rest mit 5 bis 10 Ringgliedern, insbesondere für einen 5-gliedrigen oder 6-gliedrigen monocyclischen aromatischen Rest, der 1, 2 oder 3, insbesondere 1 oder 2, gleiche oder verschiedene Heteroatome aus der Reihe N, O und S enthält und durch 1 oder 2 gleiche oder verschiedene Substituenten aus der Reihe (C₁-C₄)-Alkyl, Halogen, Hydroxy, -N(R¹¹)₂, (C₁-C₄)-Alkoxy, Phenyl, Phenoxy, Benzyloxy und Benzyl substituiert sein kann.

Der Begriff "Heterocyclus mit 5 bis 12 Ringgliedern" steht für einen monocyclischen oder bicyclischen 5-gliedrigen bis 12-gliedrigen heterocyclischen Ring, der teilweise gesättigt oder vollständig gesättigt ist. Beispiele für Heteroatome sind N, O und S. Der Herterocyclus ist unsubstituiert oder an einem oder mehreren Kohlenstoffatomen oder an einem oder mehreren Heteroatomen durch gleiche oder verschiedene Substituenten substituiert. Diese Substituenten sind oben beim Rest Heteroaryl definiert worden. Insbesondere ist der heterocyclische Ring einfach oder mehrfach, zum Beispiel einfach, zweifach, dreifach oder vierfach, an Kohlenstoffatomen durch gleiche oder verschiedene Reste aus der Reihe (C₁-C₈)-Alkyl, zum Beispiel (C₁-C₄)-Alkyl, (C₁-C₈)-Alkoxy, zum Beispiel (C₁-C₄)-Alkoxy wie Methoxy, Phenyl-(C₁-C₄)-alkoxy, zum Beispiel Benzyloxy, Hydroxy, Oxo, Halogen, Nitro, Amino oder Trifluormethyl substituiert und/oder er ist an den Ring-Stickstoffatome/en im heterocyclischen Ring durch (C₁-C₈)-Alkyl, zum Beispiel (C₁-C₄)-Alkyl wie Methyl oder Ethyl, durch gegebenenfalls substituiertes Phenyl oder Phenyl-(C₁-C₄)-alkyl, zum Beispiel Benzyl, substituiert. Stickstoffheterocyclen können auch als N-Oxide vorliegen oder als Quartärsalze.

Beispiele für die Begriffe Heteroaryl mit 5 bis 14 Ringgliedem oder Heterocyclus mit 5 bis 12 Ringgliedem sind Reste, die sich von Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, welche durch F, CN, CF₃ oder COO-(C₁-C₄)-Alkyl substituert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-lmidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-lsochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Die allgemeine Strukturformel von α-Aminosäuren ist wie folgt:

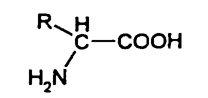

Die α-Aminosäuren unterscheiden sich untereinander durch den Rest R, der im Rahmen der vorliegenden Anmeldung als "charakteristischer Rest " einer Aminosäure bezeichnet wird. Für den Fall, daß R⁹ den charakteristischen Rest einer Aminosäure bedeutet, werden vorzugsweise die charakteristischen Reste der folgenden natürlich vorkommenden α-Aminosäuren Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure eingesetzt. Insbesondere bevorzugt sind Histidin, Tryptophan, Serin, Threonin, Cystein, Methionin, Asparagin, Glutamin, Lysin, Arginin, Glutaminsäure und Asparaginsäure. Ferner sind bevorzugte charakteristische Reste einer Aminosäure die eingesetzt werden als Rest R⁹ auch nicht natürlich vorkommenden Aminosäuren wie 2-Aminoadipinsäure, 2-Aminobuttersäure, 2-Aminoisobuttersäure, 2,3-Diamino-propionsäure, 2,4-Diaminobuttersäure, 1,2,3,4-Tetra-hydroisochinolin-1-carbonsäure, 1,2,3,4-Tetrahydroisochinolin-3-carbonsäure, 2-Amino-pimelinsäure, Phenylglycin, 3-(2-Thienyl)-alanin, 3-(3-Thienyl)-alanin, Sarkosin, 2-(2-Thienyl)-glycin, 2-Amino-heptansäure, Pipecolinsäure, Hydroxylysin, N-Methylisoleucin, 6-N-Methyllysin, N-Methylvalin, Norvalin, Norleucin, Ornithin, allo-Isoleucin, 4-Hydroxyprolin, allo-Hydroxylysin, allo-Threonin, 3-Hydroxyprolin, 3-(2-Naphtyl)-alanin, 3-(1-Naphtylalanin), Homophenylalanin, Homocystein, 2-Amino-3-phenylaminoethylpropionsäure, Homocysteinsäure, Homotryptophan, Cysteinsäure, 3-(2-Pyridyl)-alanin, 3-(3-Pyridyl)-alanin, 3-(4-Pyridyl)-alanin, Phosphinothricin, 4-Fluorphenylalanin, 3-Fluorphenylalanin, 4-Fluorphenylalanin, 3-Fluorphenylalanin, 3-Fluorphenylalanin, 2-Fluorphenylalanin, 4-Chlorphenylalanin, 4-Nitrophenylalanin, 4-Aminophenylalanin, Cyclo-hexylalanin, Citrullin, 5-Fluortryptophan, 5-Methoxytryptophan, 2-Amino-3-phenylaminopropionsäure, Methionin-Sulfon, Methionin-Sulfoxid oder -NH-NR¹¹-CON(R¹¹)₂, die gegebenenfalls auch substituiert sind. Bei den natürlich oder nicht natürlich vorkommenden Aminosäuren, die eine funktionelle Gruppe wie Amino, Hydroxy, Carboxy, Mercapto, Guanidyl, Imidazolyl oder Indolyl haben, kann diese Gruppe auch geschützt sein.

Als geeignete Schutzgruppe werden dafür vorzugsweise die in der Peptidchemie gebräuchlichen N-Schutzgruppen verwendet, beispielsweise Schutzgruppen vom Urethan-Typ, Benzyloxycarbonyl (Z), t-Butyloxycarbonyl (Boc), 9-Fluorenyloxycarbonyl (Fmoc), Allyloxycarbonyl (Aloc) oder vom Säureamid-Typ insbesondere Formyl, Acetyl oder Trifluoracetyl sowie vom Alkyl-Typ beispielsweise Benzyl. Für den Fall eines Imidazols-Restes in R⁹ dient beispielsweise das für die Sulfonamidbildung eingesetzte Sulfonsäurederivat der Formel IV als Schutzgruppe des Imidazol-Stickstoffs, die sich insbesondere in Gegenwart von Basen wie Natronlauge wieder abspalten läßt. Die Ausgangsstoffe der chemischen Umsetzungen sind bekannt oder lassen sich nach literaturbekannten Methoden leicht herstellen.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, daß man
a) eine Verbindung der Formel IV, 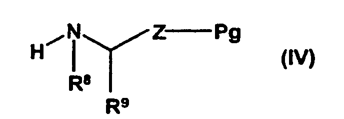 worin Pg ein geeignete Schutzgruppe (z.B. Methylester), eine Amidgruppe oder eine Hydroxy-Gruppe darstellt und Z, R⁸ und R⁹ wie in Formel II definiert sind, mit einem Säurechlorid oder einem aktivierten Ester der Verbindung der Formel III, 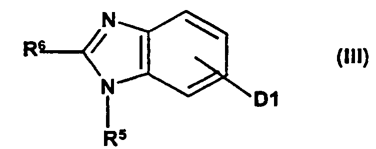 wobei D1 -COOH oder Sulfonylhalogen bedeutet und R⁵ und R⁶ wie in Formel I definiert sind, in Gegenwart einer Base oder gegebenenfalls eines wasserentziehenden Mittels in Lösung umsetzt und nach Abspaltung der Schutzgruppe in eine Verbindung der Formel I überführt, oder
b) eine Verbindung der Formel IVa, 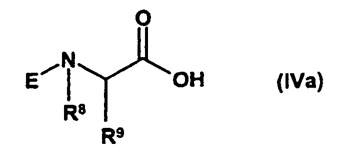 worin R⁸ und R⁹ wie in Formel II definiert sind und E eine N-Aminoschutzgruppe darstellt, mit ihrer Carboxylgruppe über eine Zwischenkette L an ein polymeres Harz der allgemeinen Formel PS ankoppelt, wobei eine Verbindung der Formel V 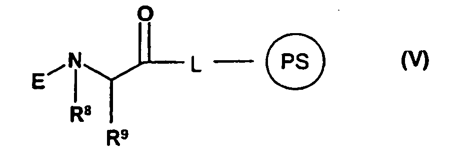 entsteht, die nach selektiver Abspaltung der Schutzgruppe E mit einer Verbindung der Formel III, wobei R⁵ und R⁶ wie in Formel I definiert sind, in Gegenwart einer Base oder gegebenenfalls eines wasserentziehenden Mittels zu 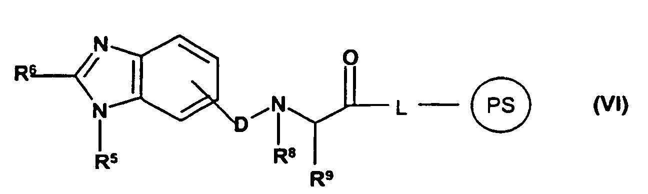 einer Verbindung der Formel VI umsetzt, und die Verbindung der Formel VI nach Abspaltung vom Trägermaterial in eine Verbindung der Formel I überführt, oder
c) eine Verbindung der Formel V , nach selektiver Abspaltung der Schutzgruppe E mit einer Verbindung der Formel VII, 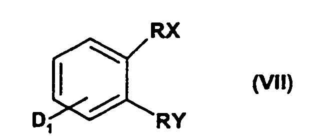 wobei D₁ für -COOH oder Sulfonylhalogen steht und RX für Halogen und RY für einen Rest -NO₂ oder -NH-E steht und E eine Schutzgruppe bedeutet, zu einer Verbindung der Formel VIII umsetzt 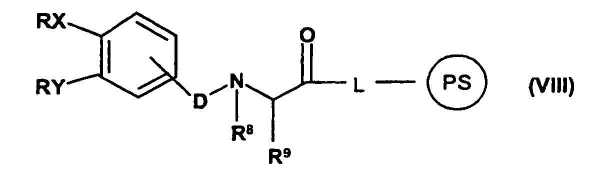 und anschließend die Verbindung der Formel VIII mit einer Verbindung der Formel IX

   NH₂-R⁶ (IX)

   worin R⁶ wie in der Verbindung der Formel I definiert ist, zu einer Zwischenverbindung der Formel VIa umsetzt, 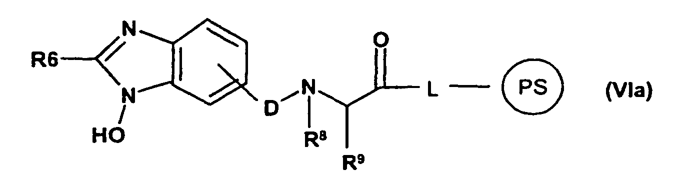 anschließend die Zwischenverbindung der Formel VIa entweder nach Abspaltung vom Trägermaterial in eine Verbindung der Formel I überführt oder beispielsweise mit Tributylphosphin, zu einer Verbindung der Formel VI reduziert und nach Abspaltung vom Trägermaterial in eine Verbindung der Formel I überführt, oder
d) eine Verbindung der Formel I in ein physiologisch verträgliches Salz überführt.

In der Verfahrensvariante a) werden die Säurefunktionen der Verbindungen der Formel IVa mit einer Schutzgruppe Pg versehen, diese selektive Carbonsäuren-Derivatisierung erfolgt nach Methoden wie sie in Houben-Weyl "Methoden der Org. Chemie", Band 15/1 beschrieben sind. In der Verfahrensvariante b) wird die Aminofunktionen der Ausgangsverbindungen der Formeln IVa mit einer Schutzgruppe E versehen, diese selektive Aminogruppen-Derivatisierung erfolgt nach Methoden wie sie in Houben-Weyl "Methoden der Org. Chemie", Band 15/1 beschrieben sind.

Als geeignete Schutzgruppe Pg wird dafür vorzugsweise die in der Peptidchemie gebräuchlichen Carboxy-Schutzgruppen verwendet, beispielsweise Schutzgruppen vom Alkylester-Typ, wie Methyl-, Ethyl-, tert.-Butyl-, iso-Propyl-, Benzyl-, Fluorenylmethyl-, Allyl-, Arylester-Typ, wie Phenyl-, Amid-Typ, wie Amid- oder Benzhydrylamin. Als geeignete Schutzgruppe E wird dafür vorzugsweise die in der Peptidchemie gebräuchlichen N-Schutzgruppen verwendet, beispielsweise Schutzgruppen vom Urethan-Typ, wie Benzyloxycarbonyl(Z), t-Butyloxycarbonyl (Boc), 9-Fluorenylmethoxycarbonyl (Fmoc) und Allyloxycarbonyl (Aloc) oder von Säureamid-Typ insbesondere Formyl, Acetyl oder Trifluoracetyl von Alkyl-Typ wie Benzyl.

Besonders geeignet hat sich dafür auch die (Trimethyl-silyl)ethoxycarbonyl (Teoc)Gruppe (P. Kociénski, Protecting Groups, Thieme Verlag 1994) bewährt.

Als Ausgangsprodukte zur Darstellung der Benzimidazolderivate der Formel III dienen bevorzugt 2,3- und 3,4-Diaminobenzoesäuren und Aryl- oder Heteroarylaldehyde, die in Gegenwart von Nitrobenzol als Lösungsmittel bei 145°C umgesetzt werden. Ferner werden die genannte Säuren mit Methyl- oder Ethylimidaten, die in einer Pinner-Reaktion aus den entsprechenden Arylnitrilen oder Heteroarylnitrilen hergestellt werden, umgesetzt.

Zur Kondensation der Verbindungen der Formel IV mit denen der Formel III verwendet man vorteilhafterweise die dem Fachmann an sich wohlbekannten Kupplungsmethoden der Peptidchemie (siehe z.B Houben-Weyl, Methoden der Organischen Chemie, Band 15/1 und 15/2, Georg Thieme Verlag, Stuttgart, 1974). Als Kondensationsmittel oder Kupplungsreagenzien kommen Verbindungen wie Carbonyldiimidazol, Carbodiimide wie Dicyclohexylcarbodümid oder Diisopropylcarbodiimid (DIC), das O-((Cyano(ethoxycarbonyl)-methylen)amino)-N,N,N',N'-tetramethyluronium-tetrafluoroborat (TOTU) oder Propanphosphonsäureanhydrid (PPA) in Frage.

Die Kondensationen können unter Standardbedingungen durchgeführt werden. Bei der Kondensation ist es in der Regel nötig, daß die vorhandenen, nicht reagierenden Aminogruppen durch reversible Schutzgruppen geschützt werden. Gleiches gilt für nicht an der Reaktion beteiligte Carboxylgruppen, die während der Kondensation bevorzugt als (C₁-C₆)-Alkylester, Benzylester oder tert-Butylester vorliegen. Ein Aminogruppen-Schutz erübrigt sich, wenn die Aminogruppen noch in Form von Vorstufen wie Nitrogruppen oder Cyanogruppen vorliegen und erst nach der Kondensation durch Hydrierung gebildet werden. Nach der Kondensation werden die vorhandenen Schutzgruppen in geeigneter Weise abgespalten. Beispielsweise können NO₂-Gruppen (Guanidinoschutz in Aminosäuren), Benzyloxycarbonylgruppen und Benzylgruppen in Benzylestern abhydriert werden. Die Schutzgruppen vom tert-Butyltyp werden sauer abgespalten, während der 9-Fluorenylmethyloxycarbonylrest durch sekundäre Amine entfernt wird.

Der in den Formeln V und VI mit PS bezeichnete polymere Träger ist ein quervernetztes Polystyrolharz mit einem als Zwischenkette L bezeichneten Linker. Dieser Linker trägt eine geeignete funktionelle Gruppe, beispielsweise Amin bekannt beispielsweise als Rink-Amid Harz, oder eine OH-Gruppe, bekannt beispielsweise als Wang-Harz oder Kaiser's Oxim-Harz). Alternativ können andere polymere Träger wie Glas, Baumwolle oder Cellulose mit verschiedenen Zwischenketten L eingesetzt werden.

Die mit L bezeichnete Zwischenkette ist kovalent an den polymeren Träger gebunden und erlaubt eine reversible, amidartige oder esterartige Bindung mit der Verbindung der Formel IVa, die während der weiteren Umsetzung an der gebundenen Verbindung der Formel lVa stabil bleibt; jedoch unter stark sauren Reaktionsbedingungen, z.B. Mischungen mit Trifluoressigsäure, die am Linker befindliche Gruppe wieder freisetzt.

Die Freisetzung der gewünschten Verbindung der allgemeinen Formel I vom Linker kann an verschiedenen Stellen in der Reaktionsfolge geschehen.

### A. Allgemeine Vorgehensweise zur Kopplung von geschützten Aminocarbonsäuren der Formel IVa an den festen Träger nach Verfahrensweise b):

Die Synthese wurde in Reaktoren mit je 15 ml Reaktionsvolumen durchgeführt. Jeder der Reaktoren wurde mit 0.179 g Rink-Amid-AM Harz (Fmoc-Rink-Amid AM/Nova-Biochem; Beladung 0,56 mmol/g; d.h. 0,1 mmol/Reaktor) befüllt. Zur Abspaltung der Fmoc-Schutzgruppe vom Harz wurde in jeden Reaktor eine 30%ige Piperidin / DMF-Lösung zudosiert und die Mischung 45 Minuten (Min) lang geschüttelt. Anschließend wurde filtriert und das Harz mit Dimethylformamid (DMF) 3 mal gewaschen.

Zur Kopplung der geschützten Aminosäure wurden zu dem so vorbereiteten Harz je eine 0,5 molare Lösung der entsprechenden Fmoc-Aminosäure (0,3 mmol in DMF); Lösung von HOBt (0,33 mmol in DMF) und eine Lösung von DIC (0,33 mmol in DMF) zudosiert und die Mischung 16 Stunden (h) bei 35°C geschüttelt. Anschließend wurde das Harz mehrmals mit DMF gewaschen.
Zur Überprüfung der Kopplung wurden einige Harzkügelchen entnommen und einem KAISER-Test unterworfen; in allen Fällen war der Test negativ.
Die Abspaltung der Fmoc-Schutzgruppe erfolgte, wie oben erwähnt, mit 30%iger Piperidin/DMF-Lösung.

Zur Kopplung der Benzimidazol-carbonsäuren wurden eine 0,1 molare Lösung der entsprechenden 4- oder 5- substituierten Säure (0,4 mmol in DMF); eine 0,5 molare Lösung des Kopplungsreagenzes TOTU (0,44 mmol in DMF) und eine 0,5 molare Lösung DIPEA (0,6 mmol in DMF) zudosiert und die Mischung 16 Stunden bei 40°C geschüttelt. Anschließend wurde mehrmals mit DMF gewaschen.
Zur Reaktionskontrolle wurden wiederum einige Harzkügelchen entnommen und einem KAISER-Test unterworfen.

Zur Abspaltung der gewünschten Substanzen vom festen Träger wurde das Harz mehrmals mit Dichlormethan gewaschen. Anschließend wurde die Abspaltlösung (50% Dichlormethan und 50% einer Mischung aus 95 % TFA, 2 % H₂O, 3 % Trüsopropylsilan) zudosiert und die Mischung 1 h bei Raumtemperatur geschüttelt. Die Mischung wurde filtriert und das Filtrat bis zur Trockne eingeengt. Der Rückstand wurde mit Diethylether ausgefällt und fitriert.

Die festen Rückstände enthielten die gewünschten Produkte meist in hoher Reinheit oder wurden beispielsweise mit präparativer Hochdruck-Flüssigkeits-Chromatographie an einer reversen Phase (Eluentien: A: H₂O/ 0,1 % TFA, B: Acetonitril/ 0, 1 % TFA) fraktioniert. Lyophilisation der erhaltenen Fraktionen lieferte die gewünschten Produkte.

Die Herstellung physiologisch verträglicher Salze aus zur Salzbildung befähigten Verbindungen der Formel I, einschließüch deren stereoisomeren Formen, erfolgt in an sich bekannter Weise. Die Carbonsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Omithin oder Arginin, stabile Alkali-, Erdalkali- oder gegebenenfalls substituierte Ammoniumsalze. Sofern die Verbindungen der Formel I basische Gruppen aufweist, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bemstein- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen,

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von IκB-Kinase beteiligt ist. Dazu gehören beispielsweise Asthma, Rheumatoide Arthritis (bei der Entzündung), Osteoarthritis, Alzheimers Erkrankungen, Krebserkrankungen (Potenzierung von Cytotoxica-Therapien), Herzinfarkt, Herzinsuffizienz, akutes Koronarsyndrom (instabile Angina Pectoris), septischer Schock, akutes und chronisches Nierenversagen, Stroke oder Atherosklerose.

Die efindungsgemäßen Arzneimittel werden im allgemeinen oral oder parenteral verabreicht. Die rektale, inhalative oder transdermale Applikation ist auch möglich.

Geeignete feste oder galenische Zubereitungsformen sind beispielsweise Granulate, Pulver, Dragees, Tabletten (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt von etwa 50 mg bis 300 mg und bei Injektionslösungen in Ampullenform bis zu etwa 300 mg, vorzugsweise von etwa 10 mg bis 100 mg, betragen.

Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt von etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) bestimmt. Temperaturangaben in Grad Celsius, RT bedeutet Raumtemperatur (22-26°C). Verwendete Abkürzungen sind entweder erläutert oder entsprechen den üblichen Konventionen.

Beispiele nach Verfahrensvariante b) gemäß allgemeiner Arbeitsvorschrift HPLC (RP 18; UV 210 nm): Gradient 0-15 Min. B = 5-70% (A = 100% H₂O/ 0.1 % Trifluoressigsäure; B = 100% Acetonitril/ 0, 1 % Trifluoressigsäure) Die in der folgenden Tabelle 1 genannten Beispiele sind analog zur Verfahrensvariante b) gemäß allgemeiner Arbeitsvorschrift hergestellt worden.

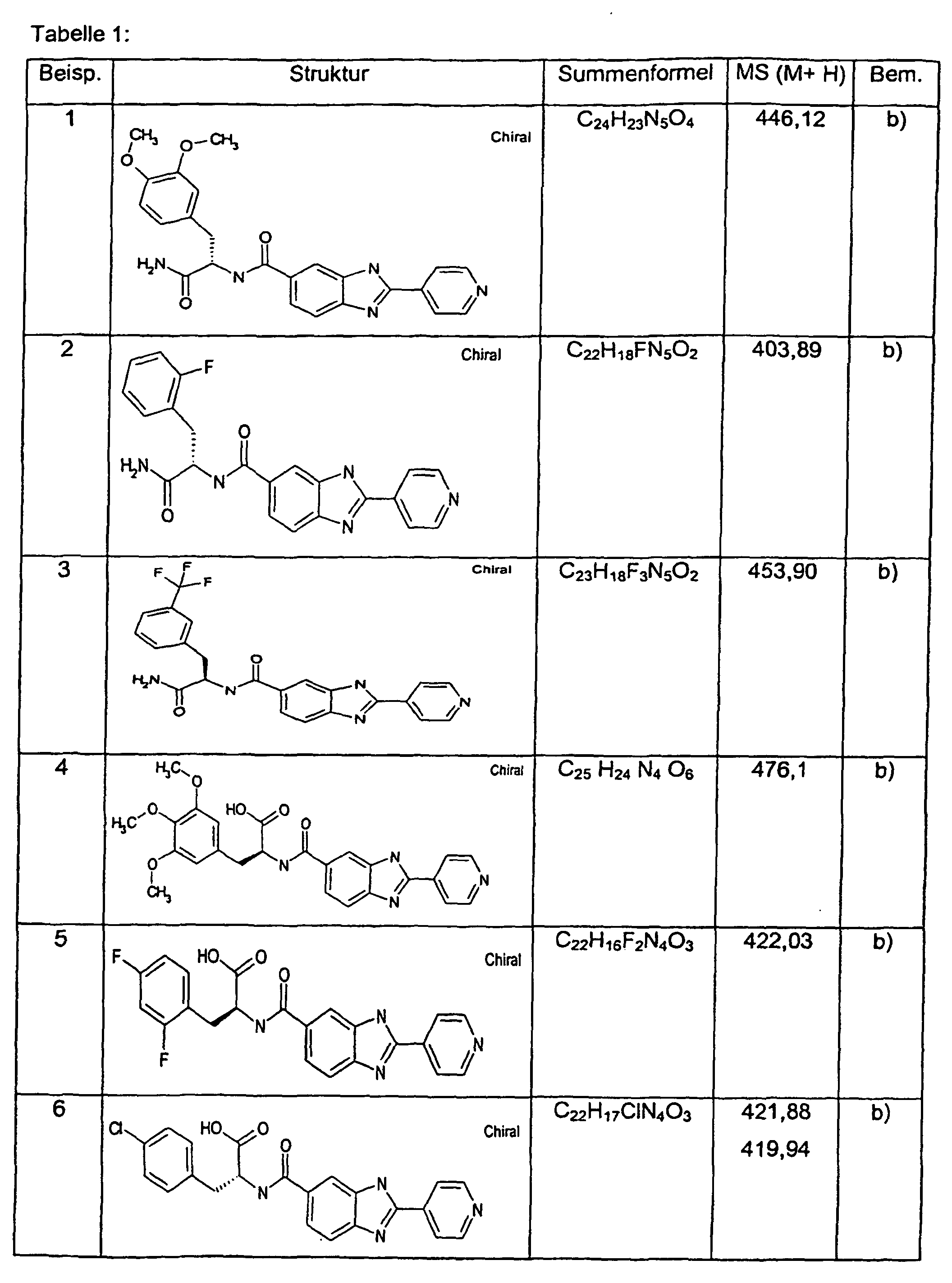

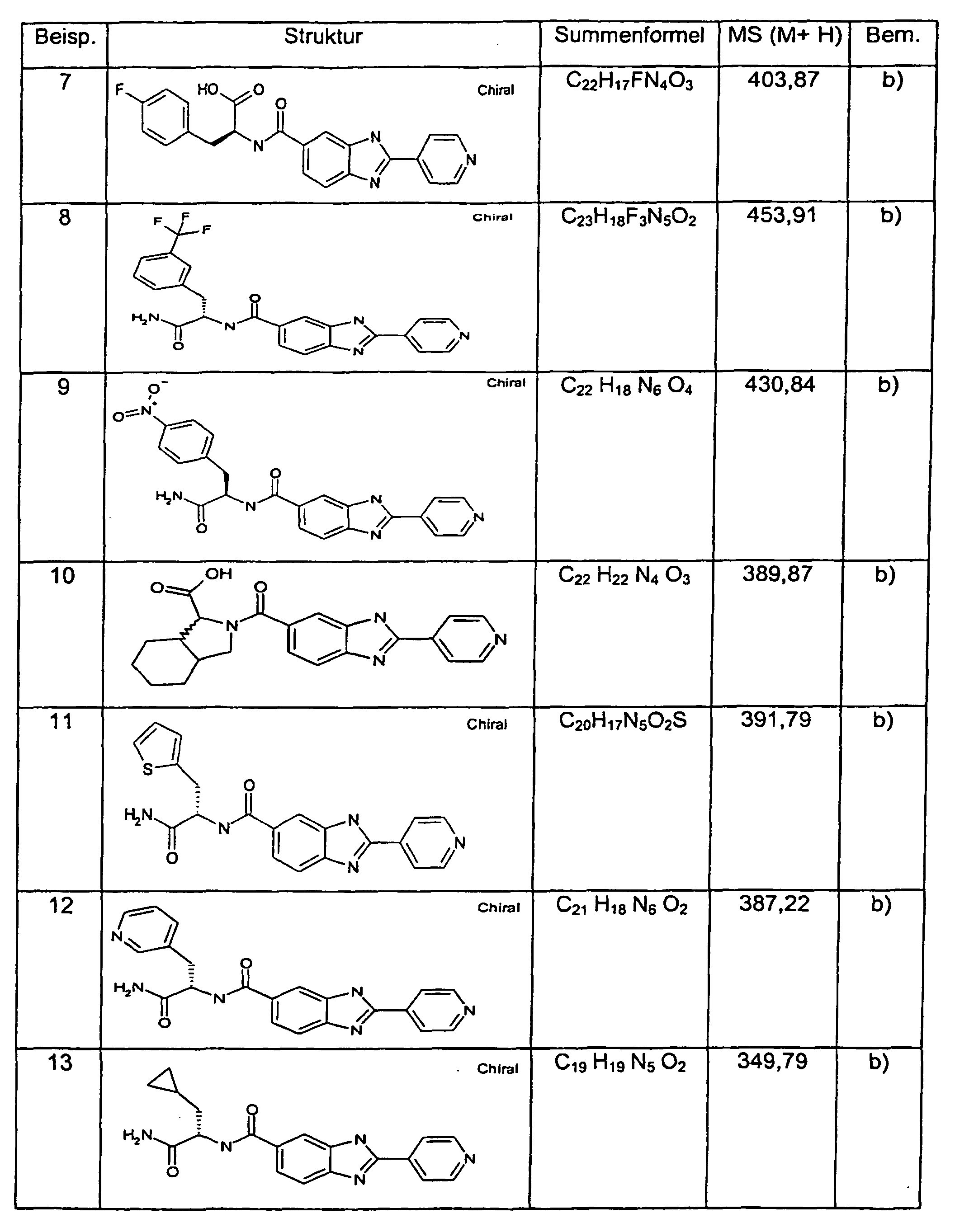

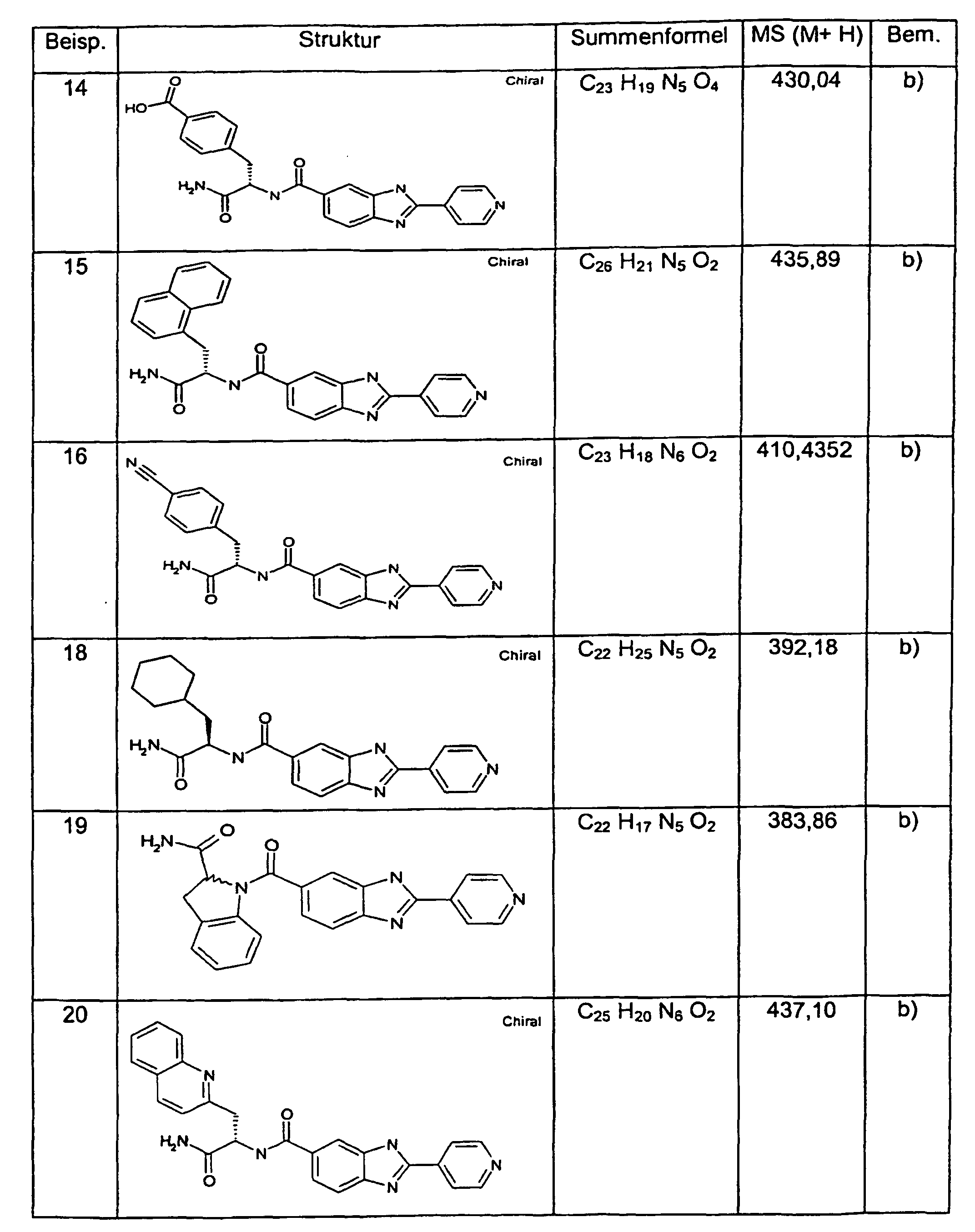

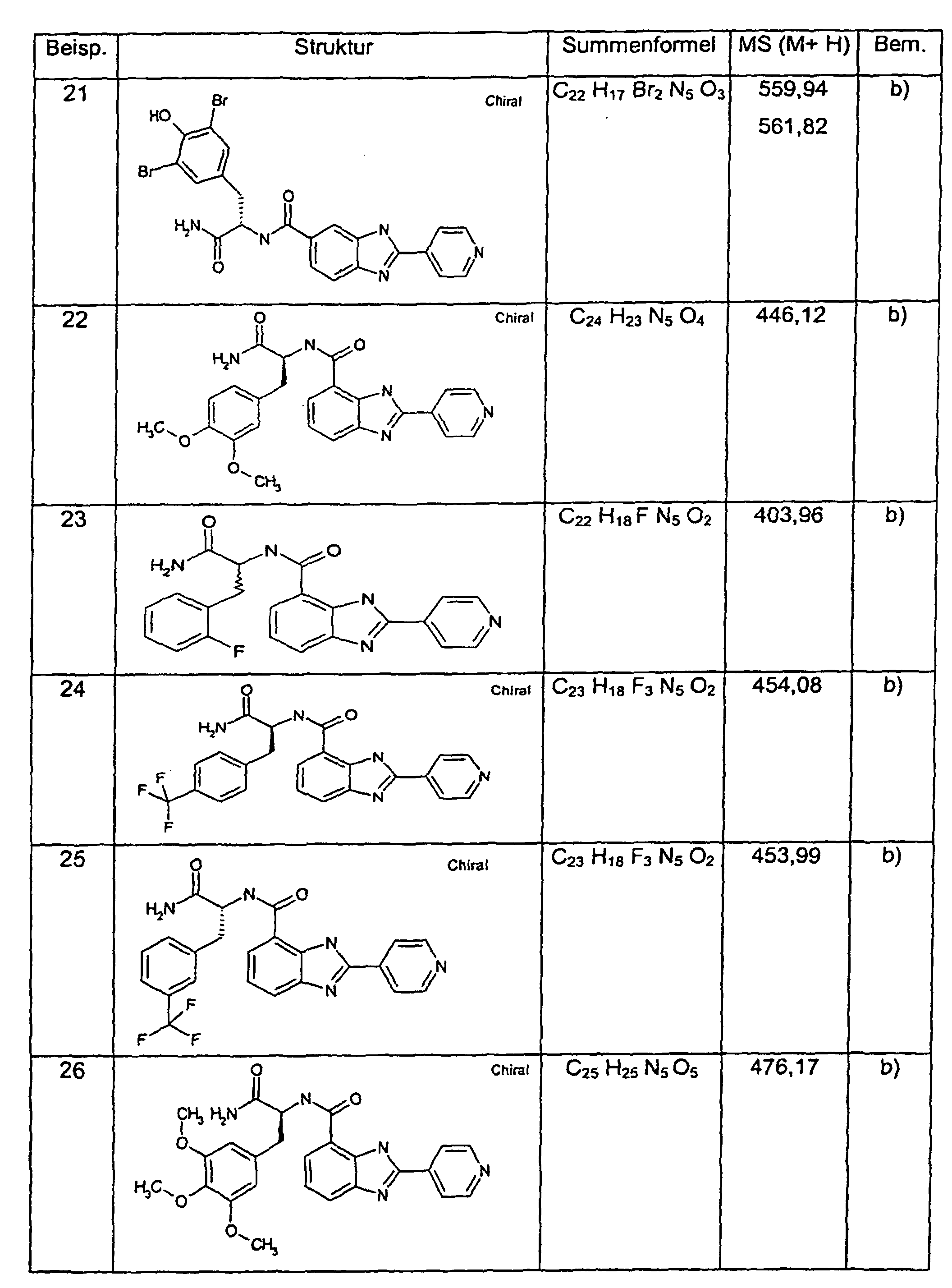

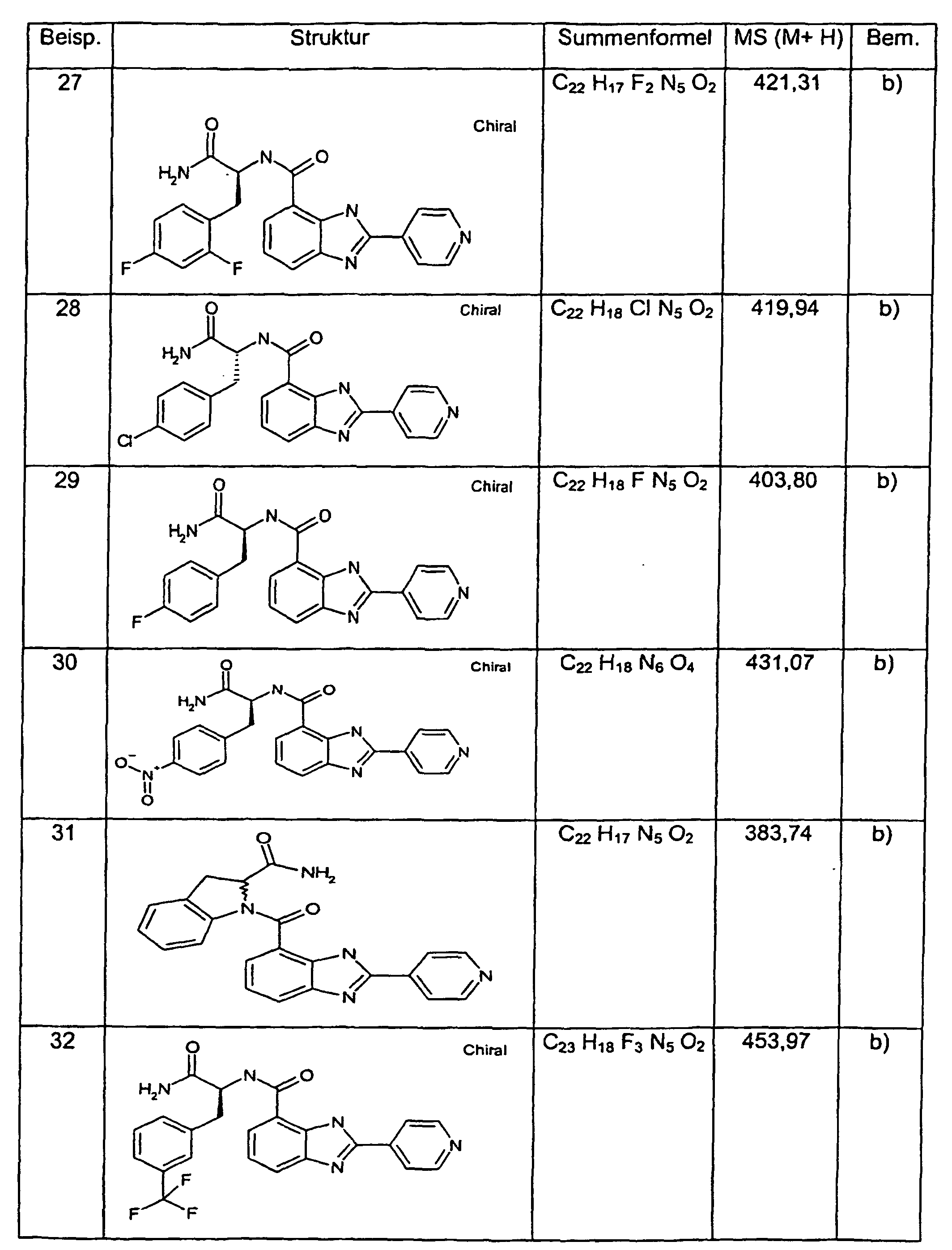

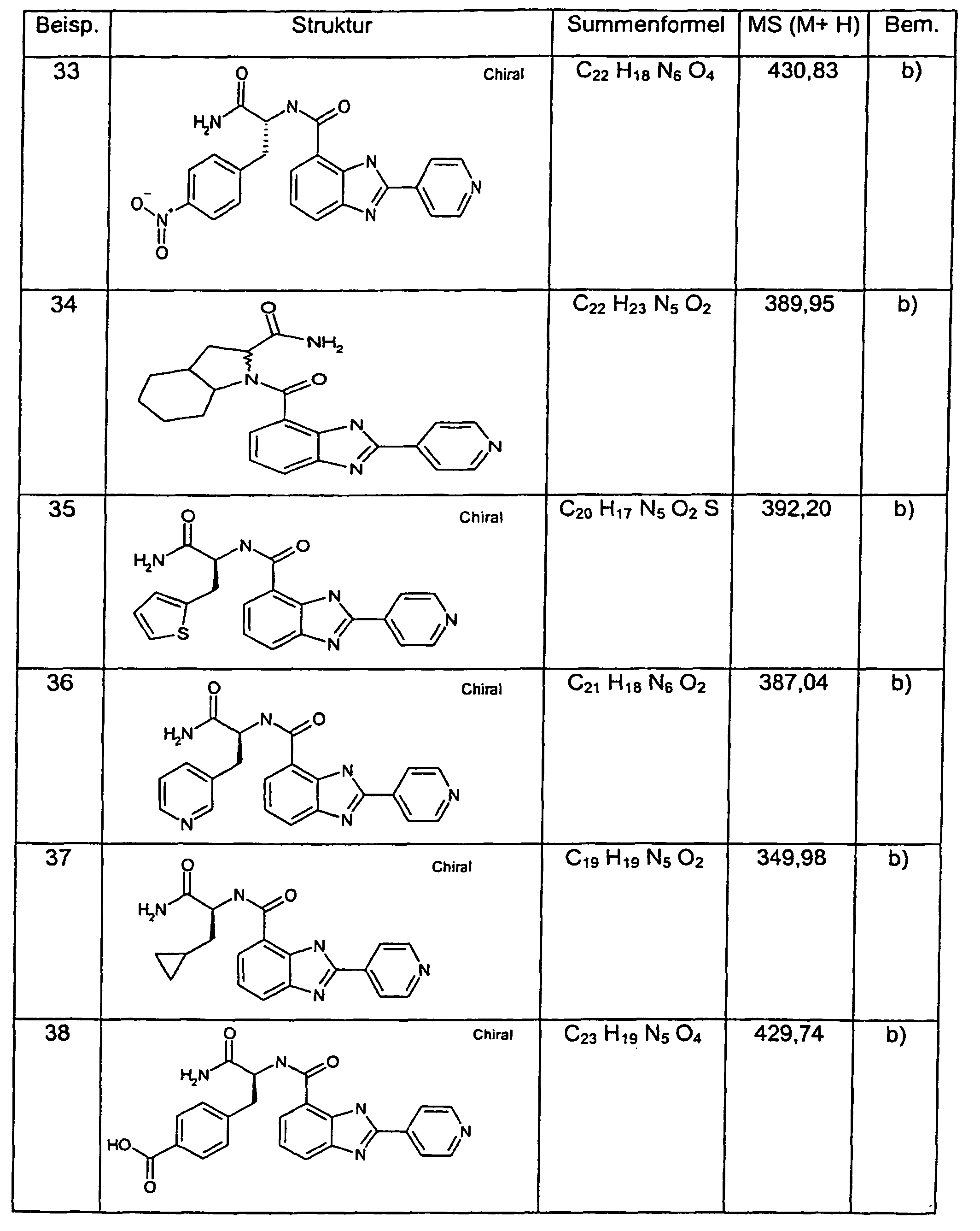

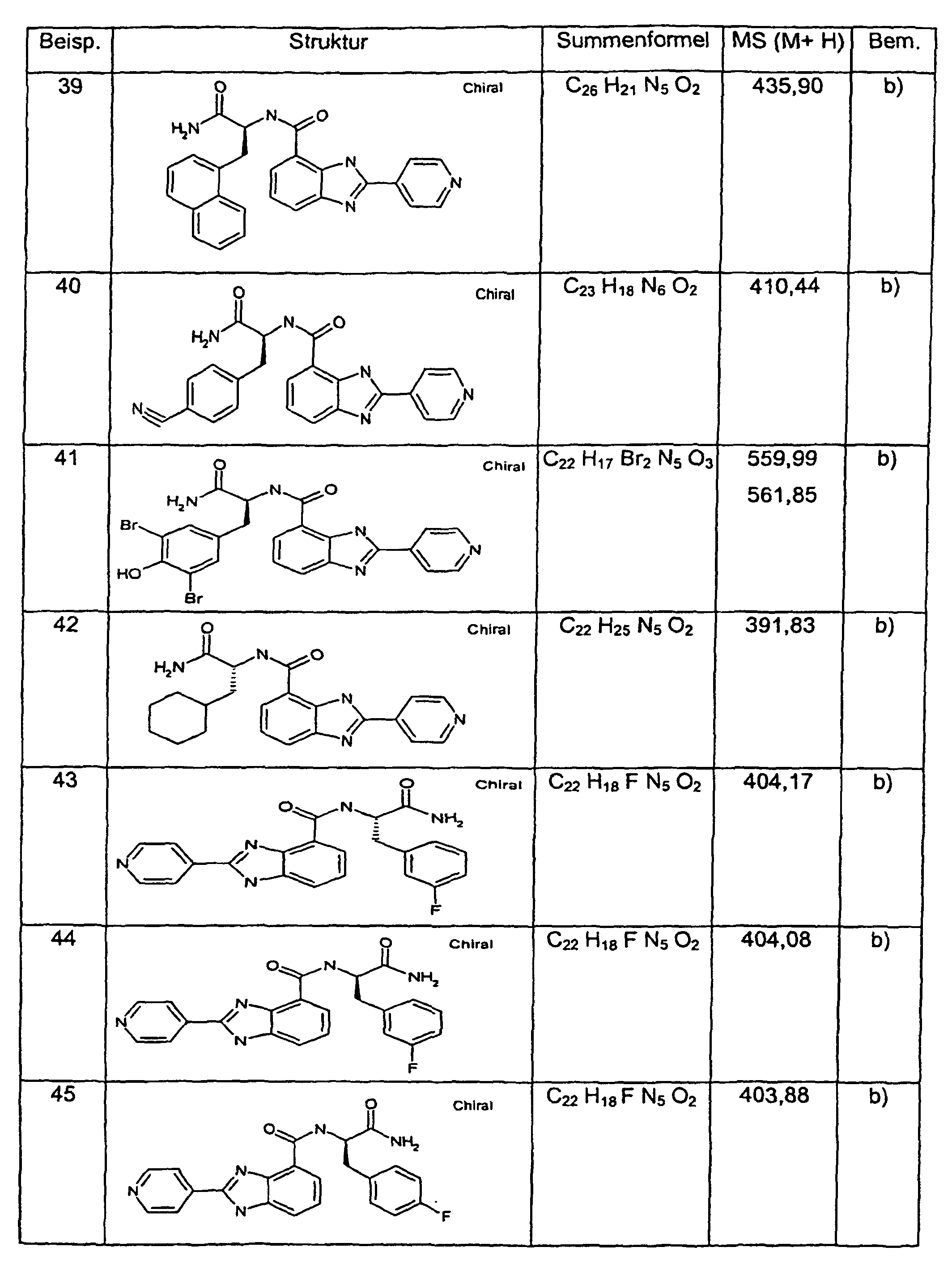

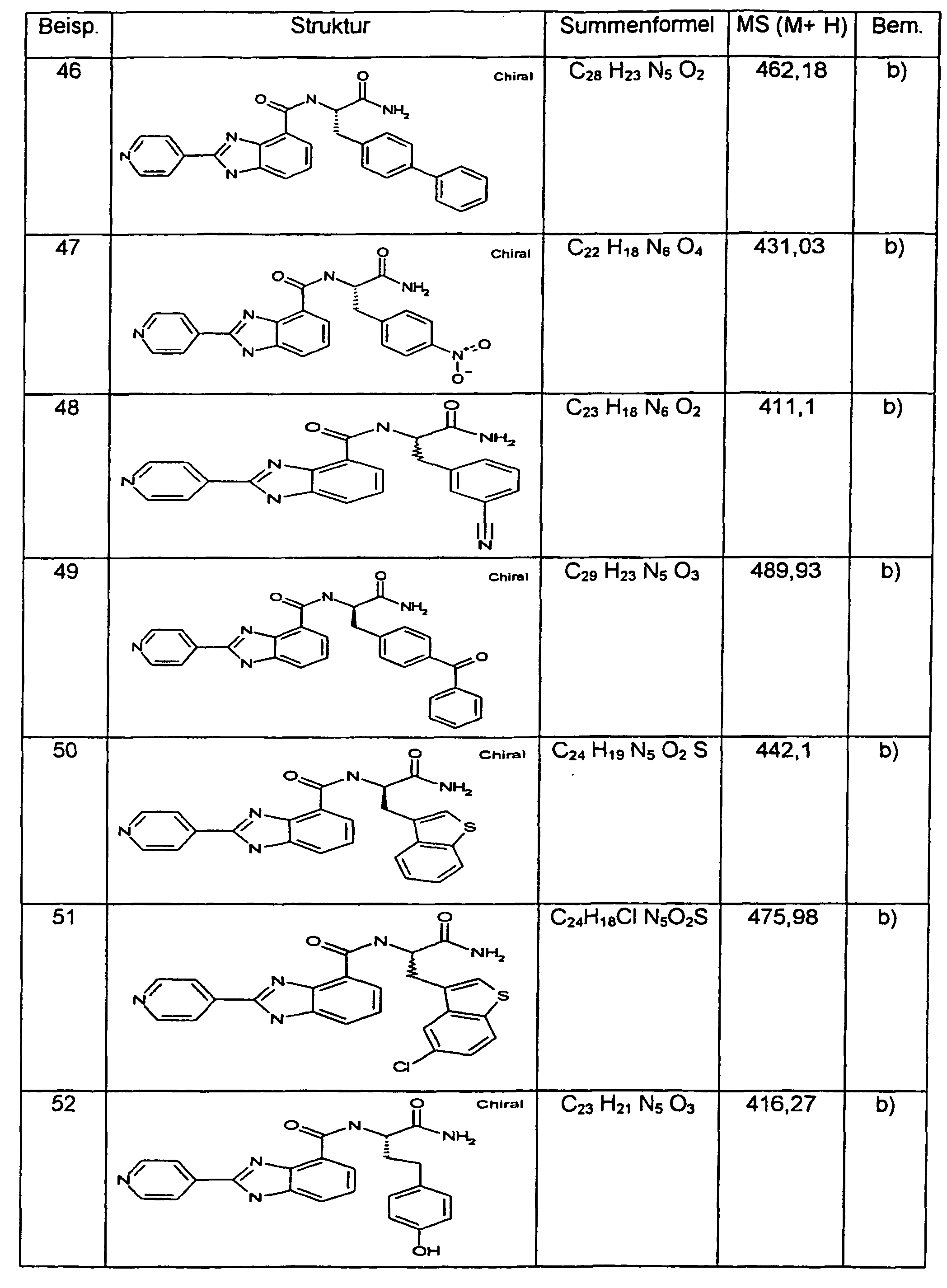

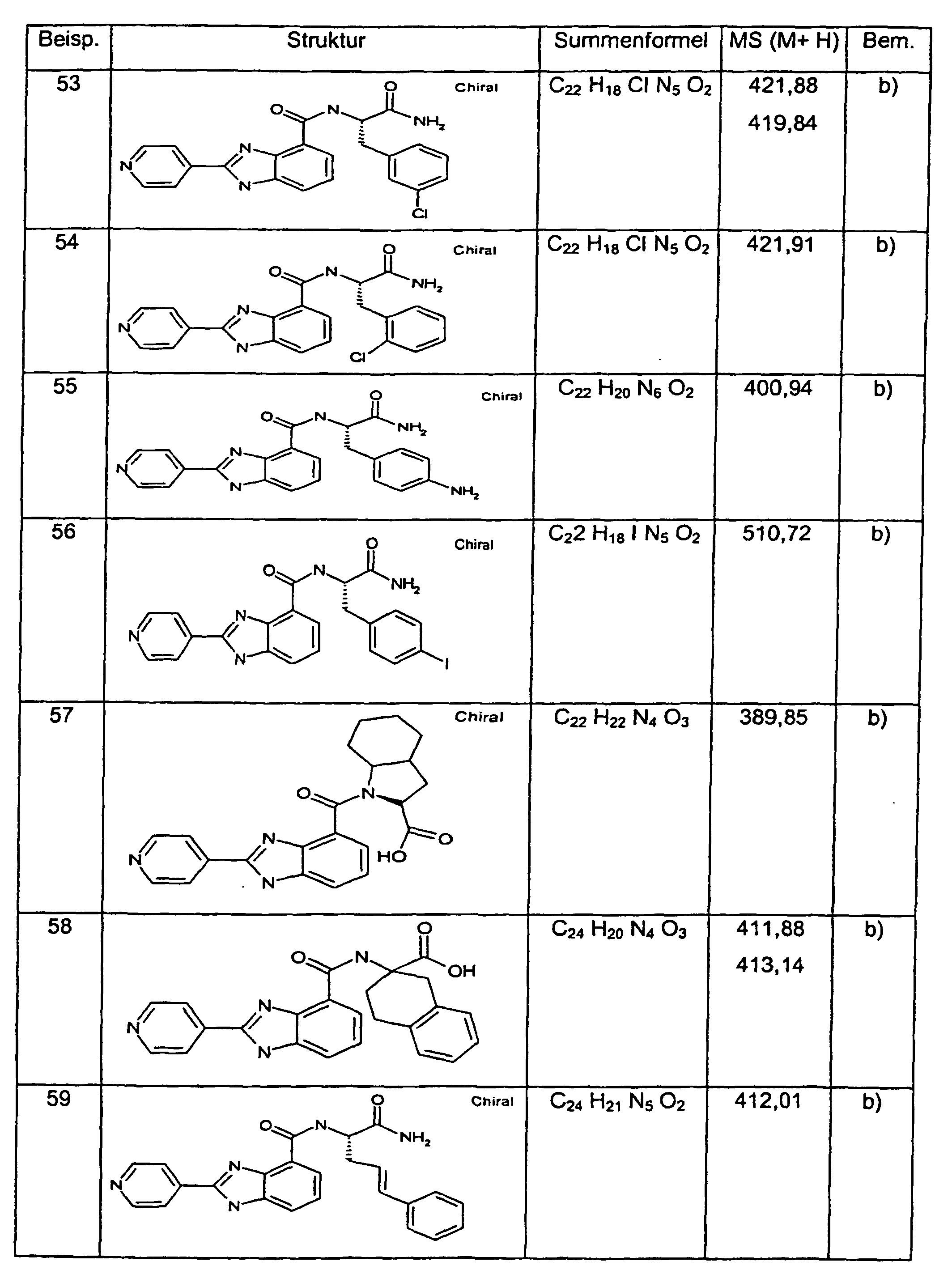

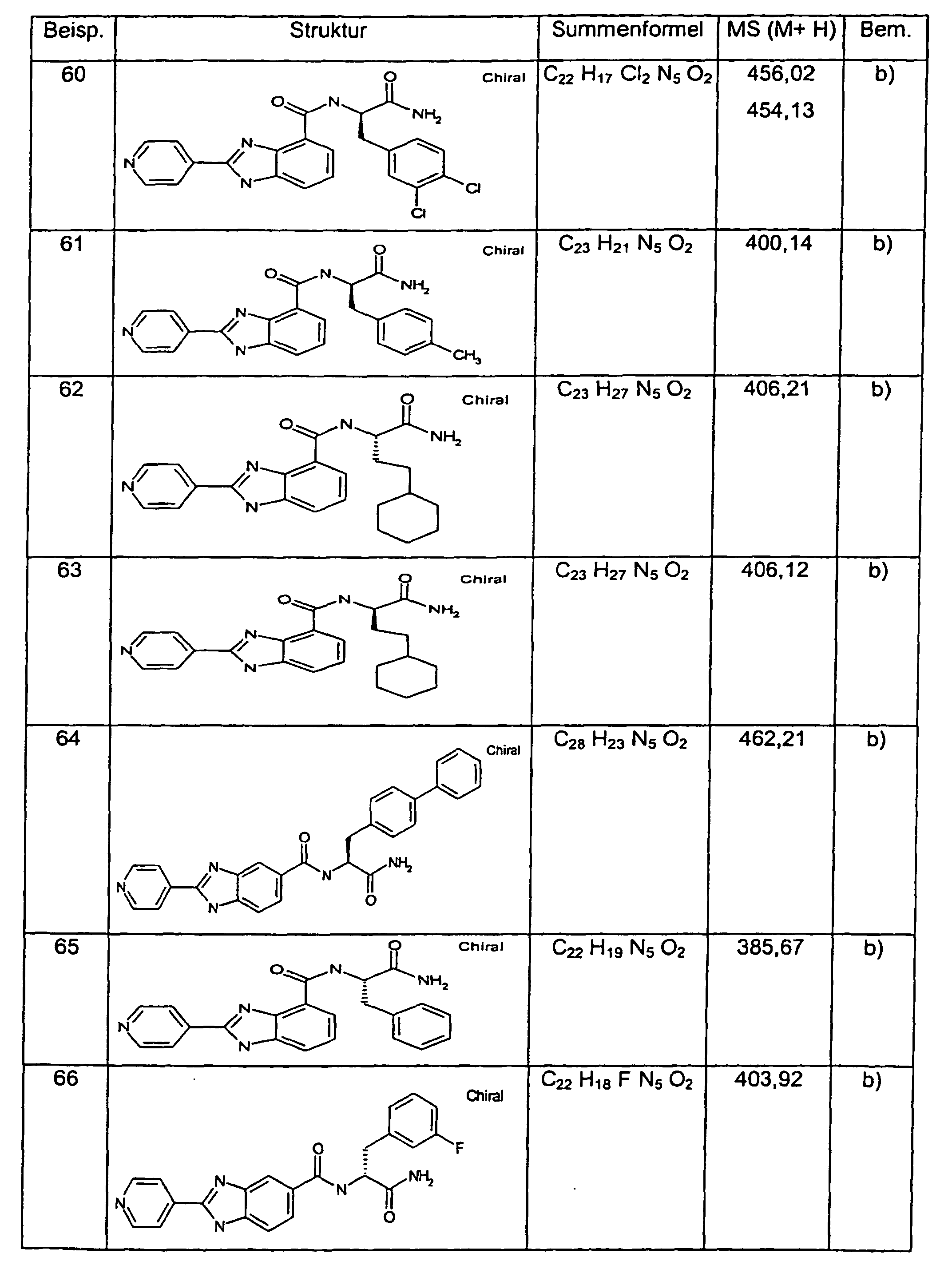

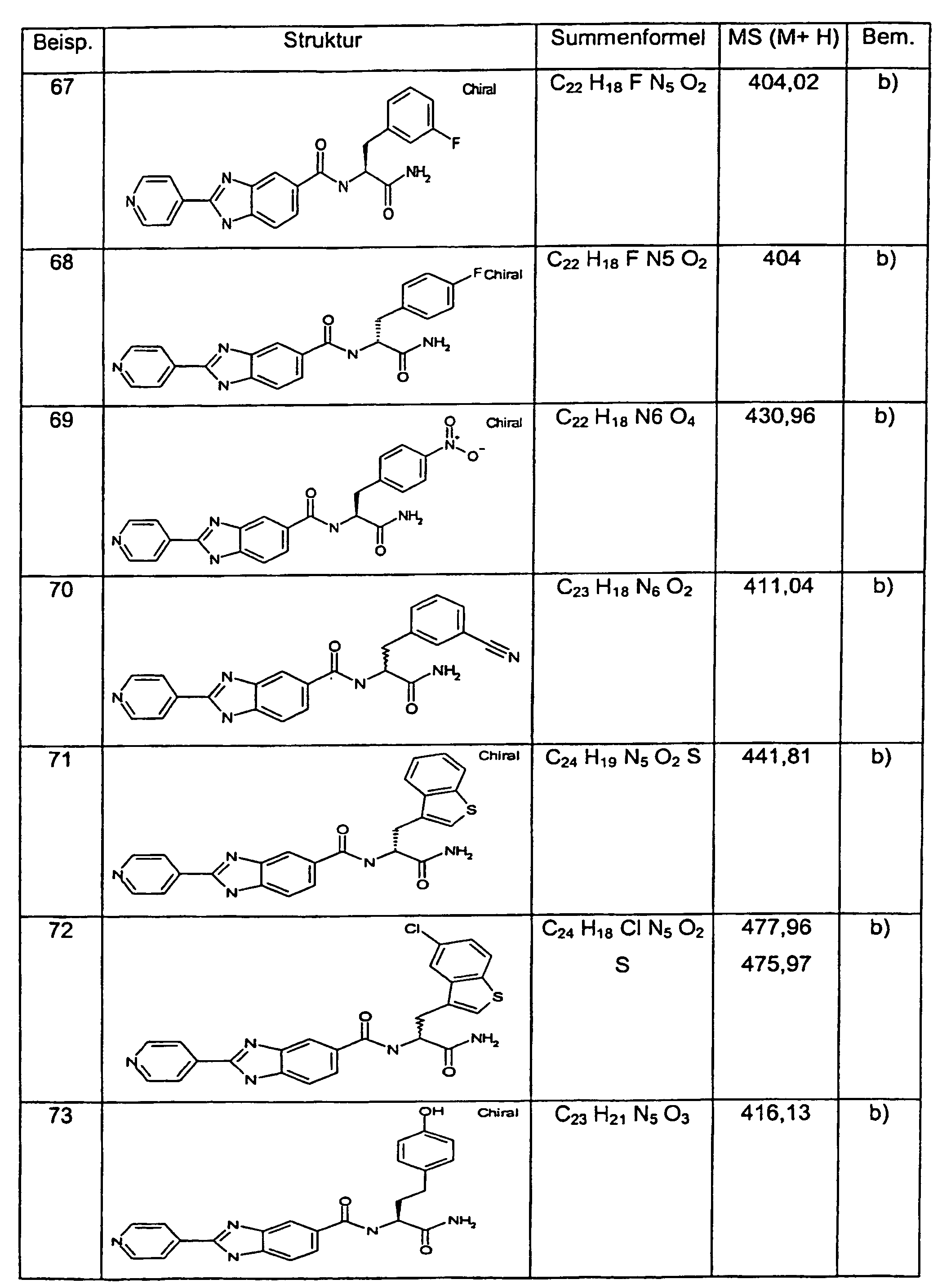

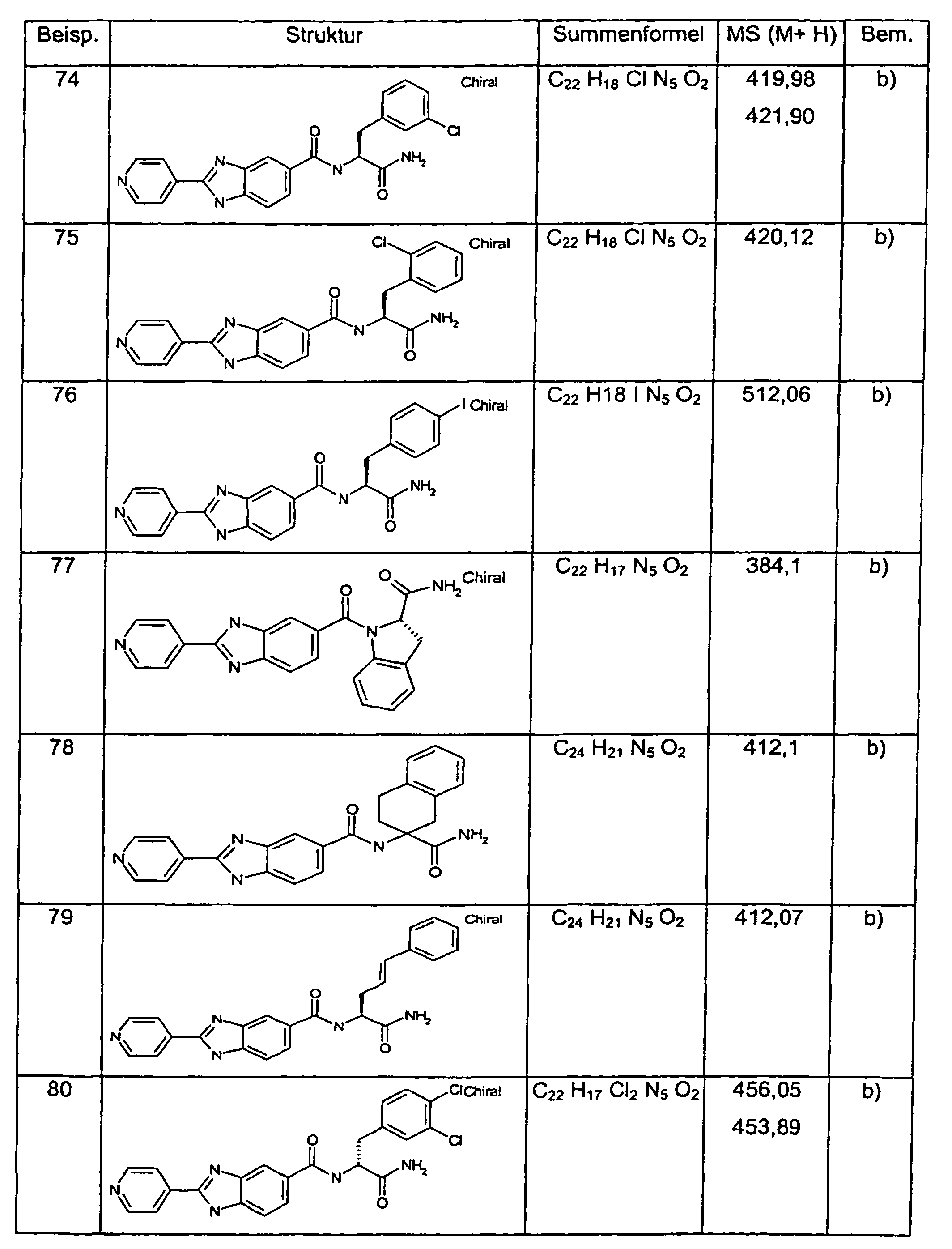

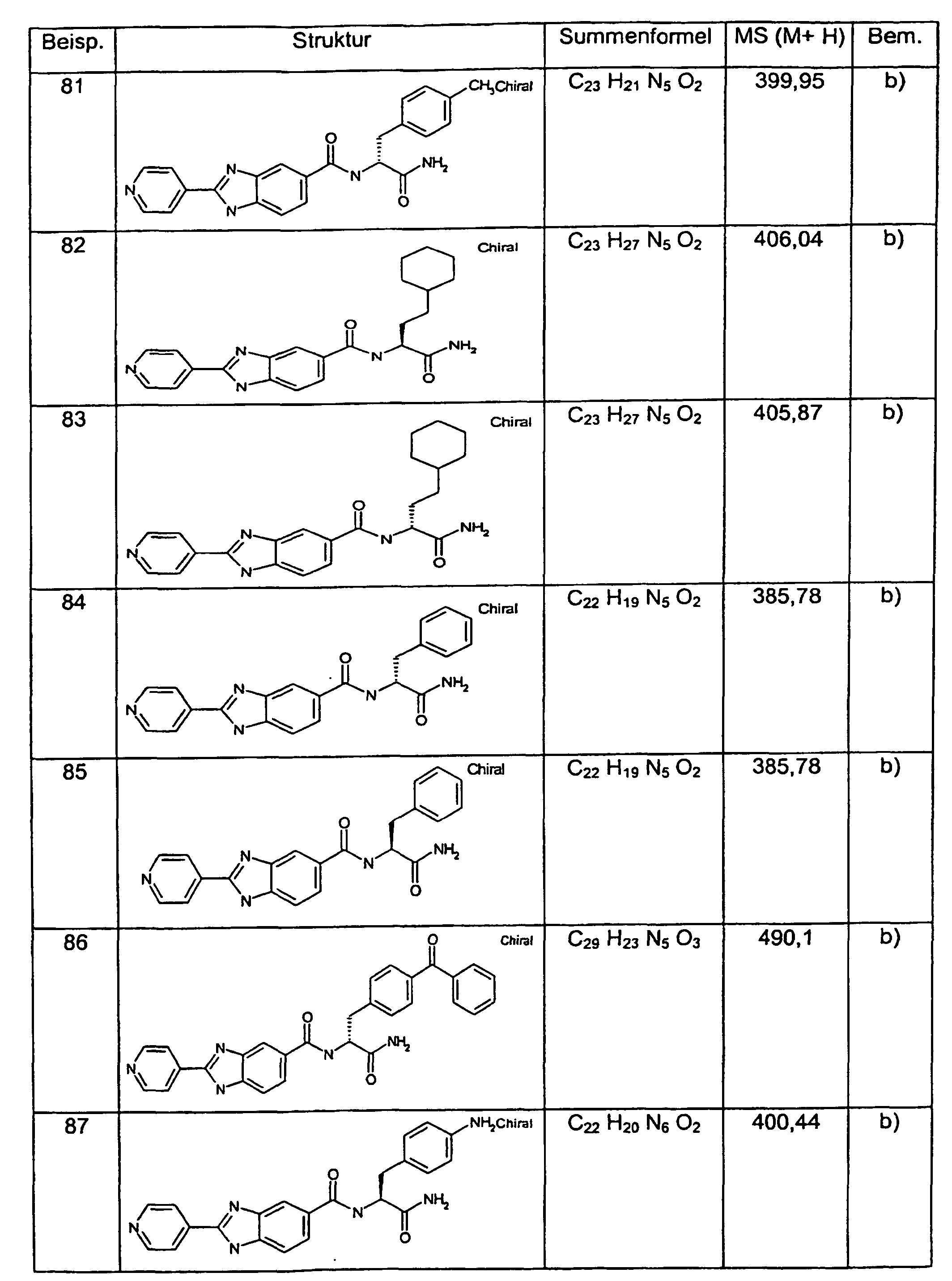

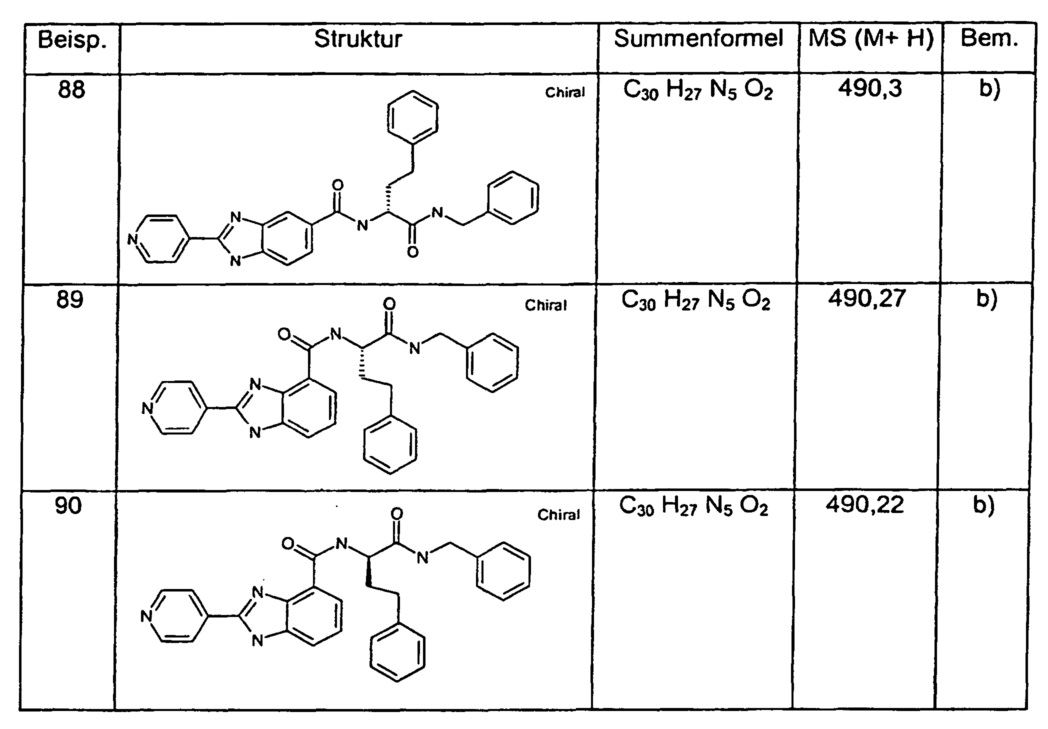

### Beispiel 91 (2-(Pyrid-4-yl)-1H-Benzoimidazol-4-Carbonyl)-(L)-Leucin-Methylester (1)

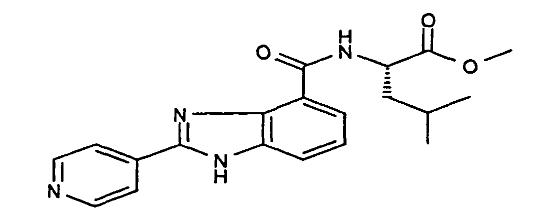

Ammonium-3-Nitro-Phthalamidsäure (1a). 100 g (518 mmol) 3-Nitrophthalsäureanhydrid wurden bei Raumtemperatur (RT) vorgelegt und unter Rühren schnell mit 170 ml konzentrierter Ammoniumhydroxidlösung versetzt. Es wurde 1 Stunde (h) bei RT nachgerührt. Der Niederschlag wurde abfiltriert und im Exsikkator getrocknet. Ausbeute: 95,6 g (88%).
2-Amino-3-Nitro-Benzoesäure (1b). 22 g (105,2 mmol) Ammonium-3-Nitro-Phthalamidsäure (1a) wurden unter Rühren mit 165 ml Natriumhypochlorit-Lösung versetzt. Nach 5 Minuten gab man eine Lösung von 8,8 g Natriumhydroxid in 22 ml Wasser zu und rührte anschließend 1 h bei 70°C. Die Suspension wurde unter Rühren in 500 ml Wasser gegossen. Die entstehende klare Lösung wurde mit konzentrierter HCl angesäuert. Der Niederschlag wurde abfiltriert und im Exsikkator getrocknet.
Ausbeute: 9,68 g (51 %).
2,3-Diamino-Benzoesäure (1c). 14 g (76,9 mMol) 2-Amino-3-Nitro-Benzoesäure (1 b) wurden in 500 ml Methanol gelöst, mit Pd/C versetzt und mit Wasserstoff hydriert. Nach 4 h filtrierte man den Katalysator ab und engte ein. Es wurde ein dunkelbrauner Feststoff
erhalten. Ausbeute: 11,67 g (99%).
(2-Pyridyl-4-yl)-1H-Benzoimidazol-4-carbonsäure (1d). 700 mg (4,6 mMol) 2,3-Diamino-Benzoesäure (1c) und 0,47 ml (4,95 mmol) 4-Pyridylaldehyd wurden in 40 ml Nitrobenzol gelöst und unter Rühren für 2 h auf 145°C erhitzt. Anschließend kühlte man ab und filtrierte den Niederschlag ab. Der Niederschlag wurde mit Essigester gewaschen und im Exsikkator getrocknet. Ausbeute: 800 mg (73%).
((2-Pyridyl-4-yl)-1H-Benzoimidazol-4-Carbonyl)-(L)-Leucin-Methylester (1). 120 mg (0,5 mMol) ((2-Pyridyl-4-yl)-1H-Benzoimidazol-4-carbonsäure (1d) und 84 mg (0,5 mMol) H-(L)-Leucin-Methylester wurden in 5 ml DMF gelöst. Man setzte 164 mg (0,5 mMol) TOTU (O-[(Cyano(ethoxycarbonyl)methyliden)amino-1,1,3,3,-tetramethyl]uroniumtetrafluoro-borat) und 0,086 ml Diisopropylethylamin zu und rührte 3 h bei RT. Der Niederschlag wurde abfiltriert und das Filtrat eingeengt. Der Rückstand wurde in Essigester gelöst, mit Wasser gewaschen, die organische Phase mit wasserfreiem Natriumsulfat getrocknet und eingeengt. Ausbeute: 180 mg (98%). (M+H)⁺ = 367,1 (Cl⁺)

### Beispiel 92 ((2-Pyridyl-4-yl)-1H-Benzimidazol-4-Carbonyl)-(L)-Valin-Amid (2)

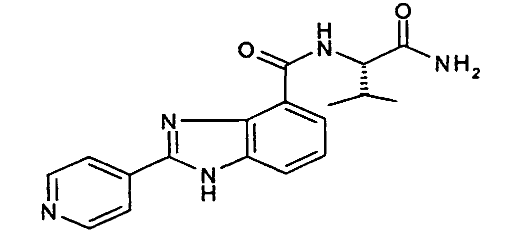

120 mg (0,5 mMol) ((2-Pyridyl-4-yl)-1H-Benzoimidazol-4-carbonsäure (1d) und 76,4 mg (0,5 mMo)) H-(L)-Valin-Amid wurden in 5 ml DMF gelöst. Man setzte 164 mg (0,5 mmol) TOTU (O-[(Cyano(ethoxycarbonyl)methyliden)amino-1,1,3,3,-tetramethyl]uroniumtetrafluorborat) und 0,086 ml Diisopropylethylamin zu und rührt 3 h bei RT. Der Niederschlag wurde abfiltriert und das Filtrat eingeengt. Der Rückstand wurde in Essigester gelöst, mit gesättigter Natriumchlorid-Lösung gewaschen, die organische Phase mit wasserfreiem Natriumsulfat getrocknet und eingeengt.
Ausbeute: 168 mg (99%). (M+H)⁺= 338,2 (Cl⁺).

### Beispiel 93 ((2-Pyridyl-4-yl)-1H-Benzimidazol-4-Carbonyl)-(S)-Histidin-Methylester (3)

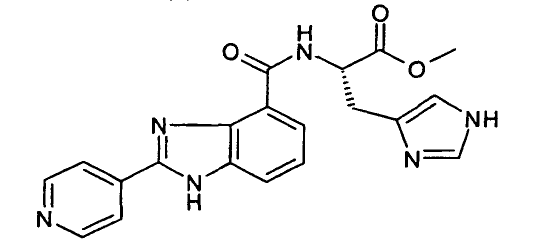

((2-Pyridyl-4-yl)-1H-Benzimidazol-4-Carbonyl)-(L)-Histidin(Trt)-Methylester (3 a). 120 mg (0,5 mMol) ((2-Pyridyl-4-yl)-1H-Benzimidazof-4-carbonsäure (1d) und 242 mg (0,5 mMol) H-(L)-Histidin(Trt)-Methylester wurden in 5 ml DMF gelöst. Man setzte 164 mg (0,5 mMol) TOTU und 0,172 ml Düsopropylethylamin zu und rührte 3 h bei RT. Die klare Lösung wurde eingeengt. Der Rückstand wurde in Essigester gelöst, mit Wasser gewaschen, die organische Phase mit wasserfreiem Natriumsulfat getrocknet und eingeengt. Ausbeute: 380 mg Rohprodukt. (M+H)⁺ = 633,3 (ES⁺).

### Beispiel 94 ((2-Pyridyl-4-yl)-1H-Benzimidazol-4-Carbonyl)-(L)-Methionin-Amid (4)

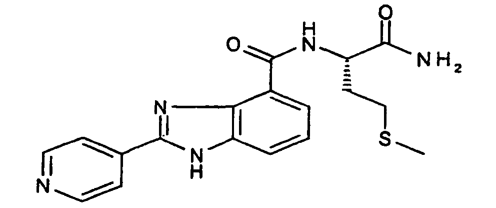

120 mg (0,5 mmol) ((2-Pyridyl-4-yl)-1H-Benzimidazol-4-carbonsäure (1d). und 74,2 mg (0,5 mmol) H-(L)-Methionin-Amid wurden in 5 ml DMF gelöst. Man setzte 164 mg (0,5 mmol) TOTU und 0,086 ml Diisopropylethylamin zu und rührte 3 Stunden bei RT. Die klare Lösung wurde eingeengt. Der Rückstand wurde in Essigester gelöst, mit gesättigter Natriumchlorid-Lösung gewaschen, die organische Phase mit wasserfreiem Natriumsulfat getrocknet und eingeengt. Ausbeute: 149 mg (81%) (M+H)⁺ = 370,2 (ES⁺).

Die in der folgenden Tabelle 2 genannten Beispiele sind analog zu den Beispielen 91 bis 94 hergestellt worden.

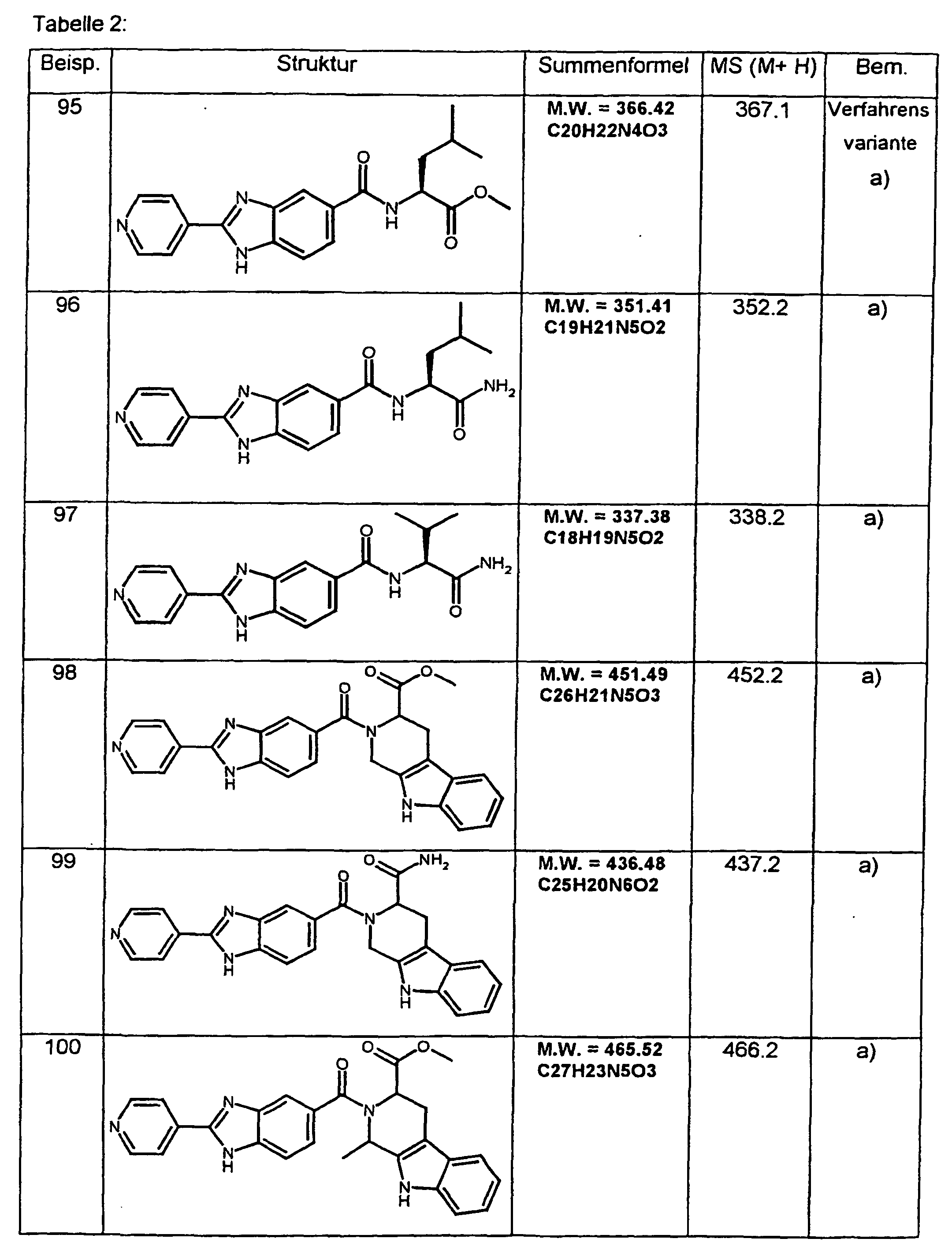

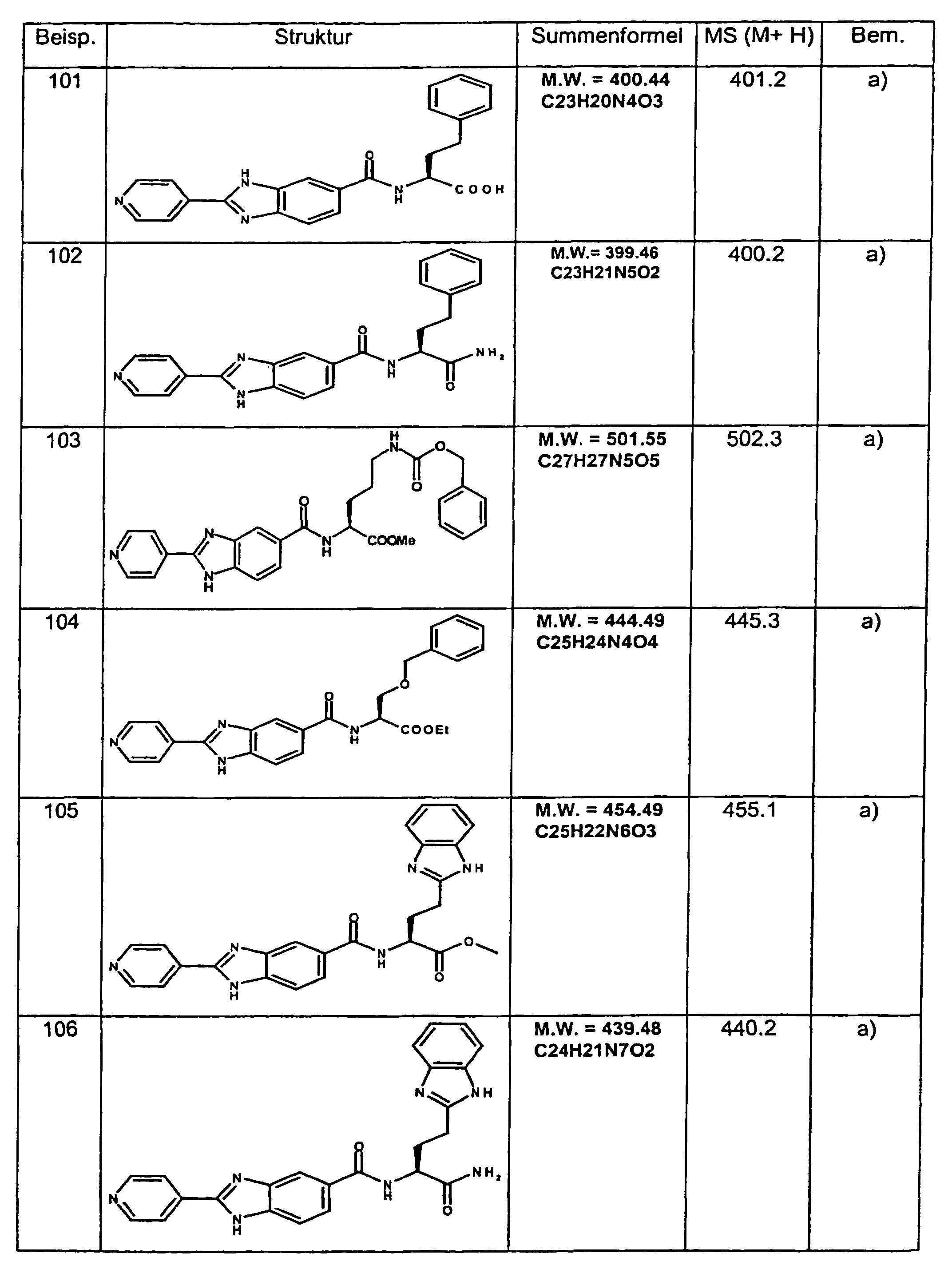

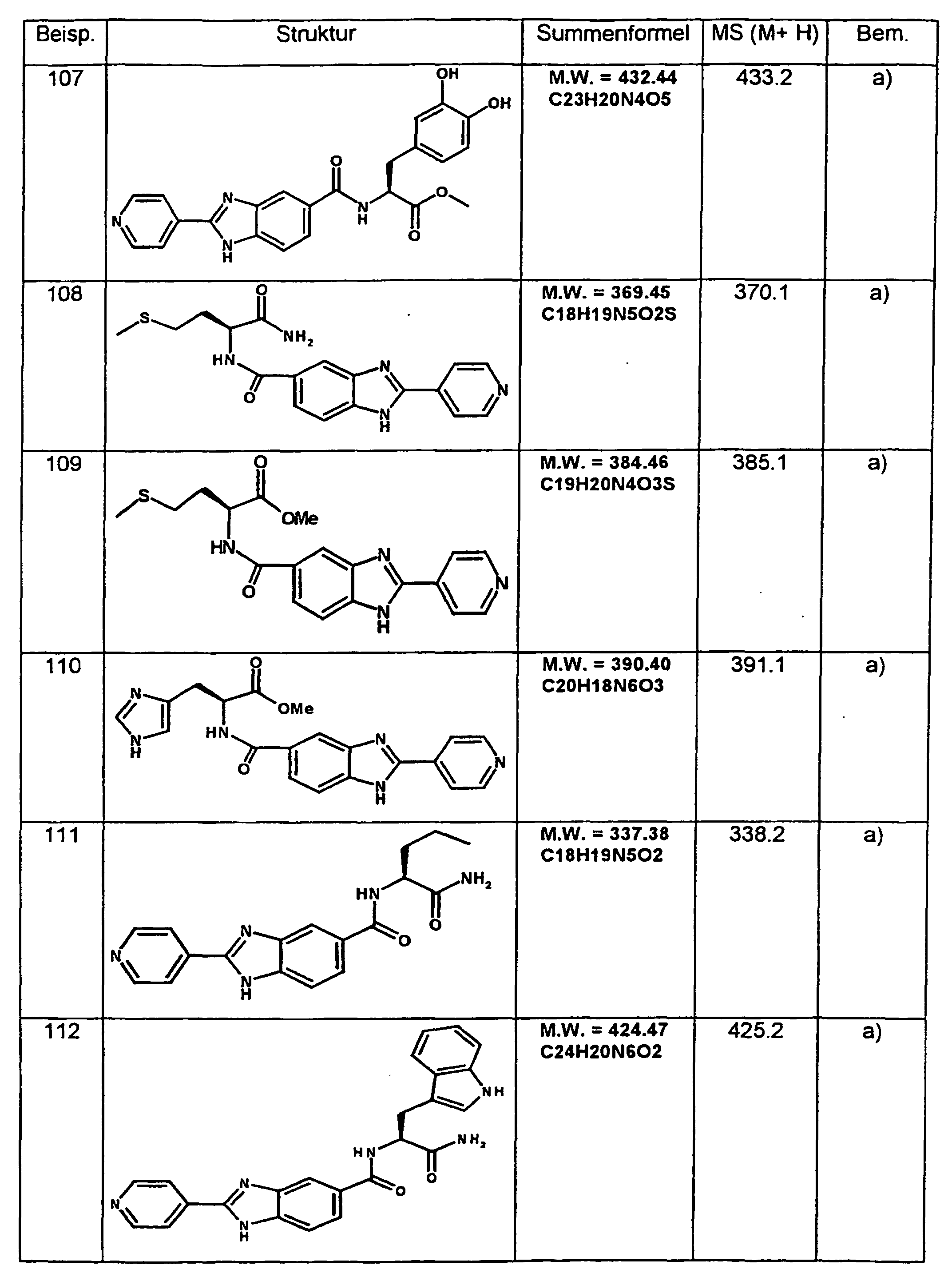

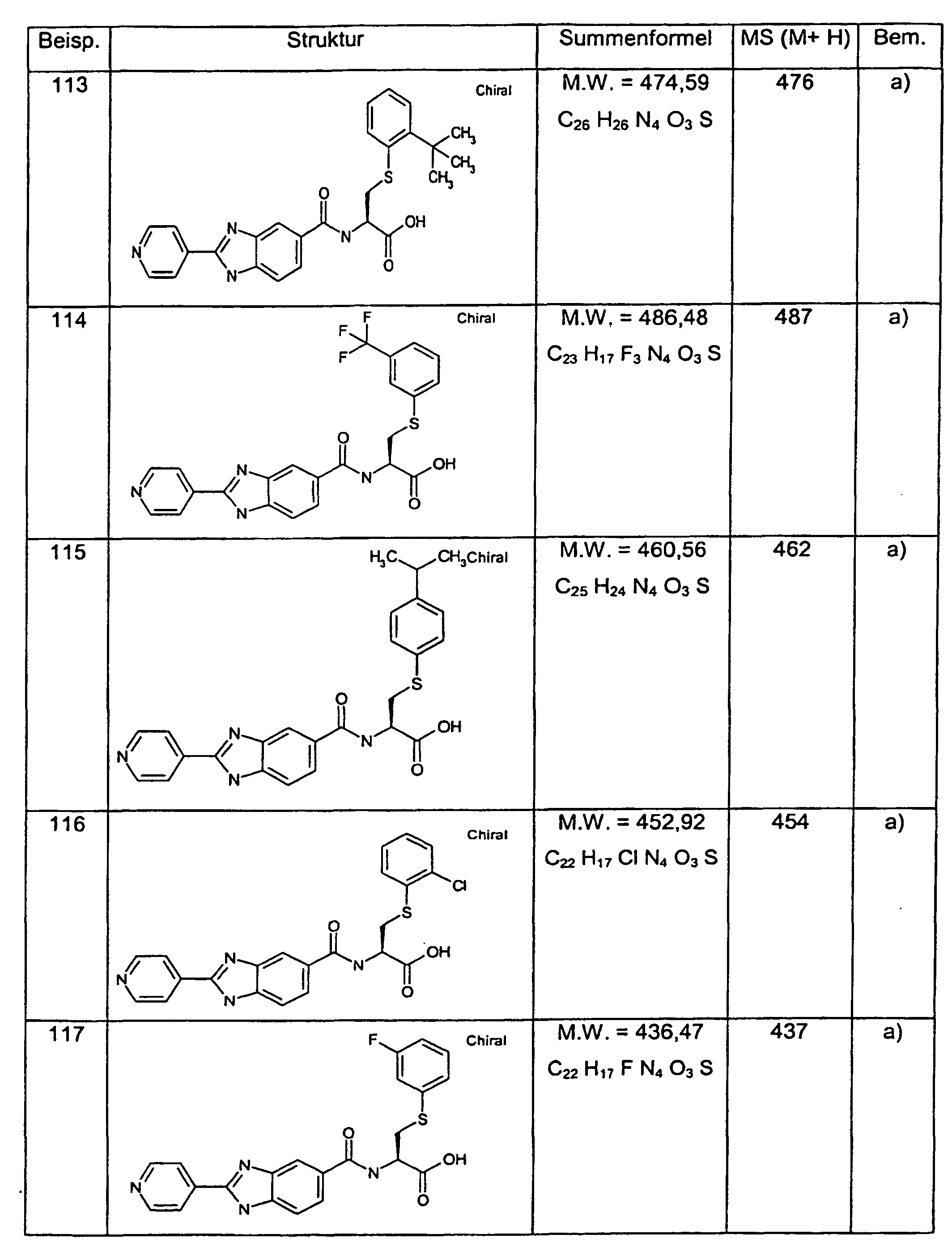

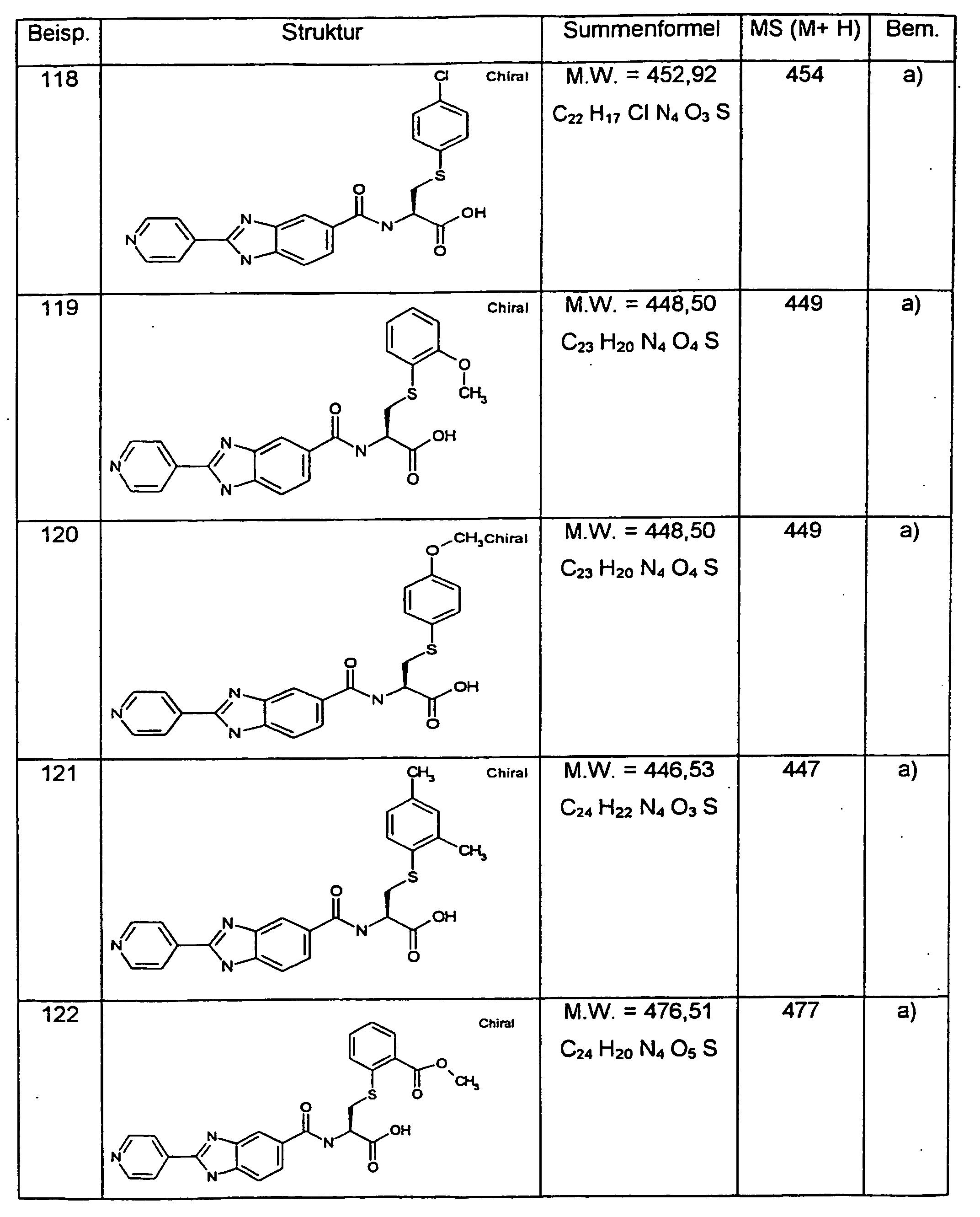

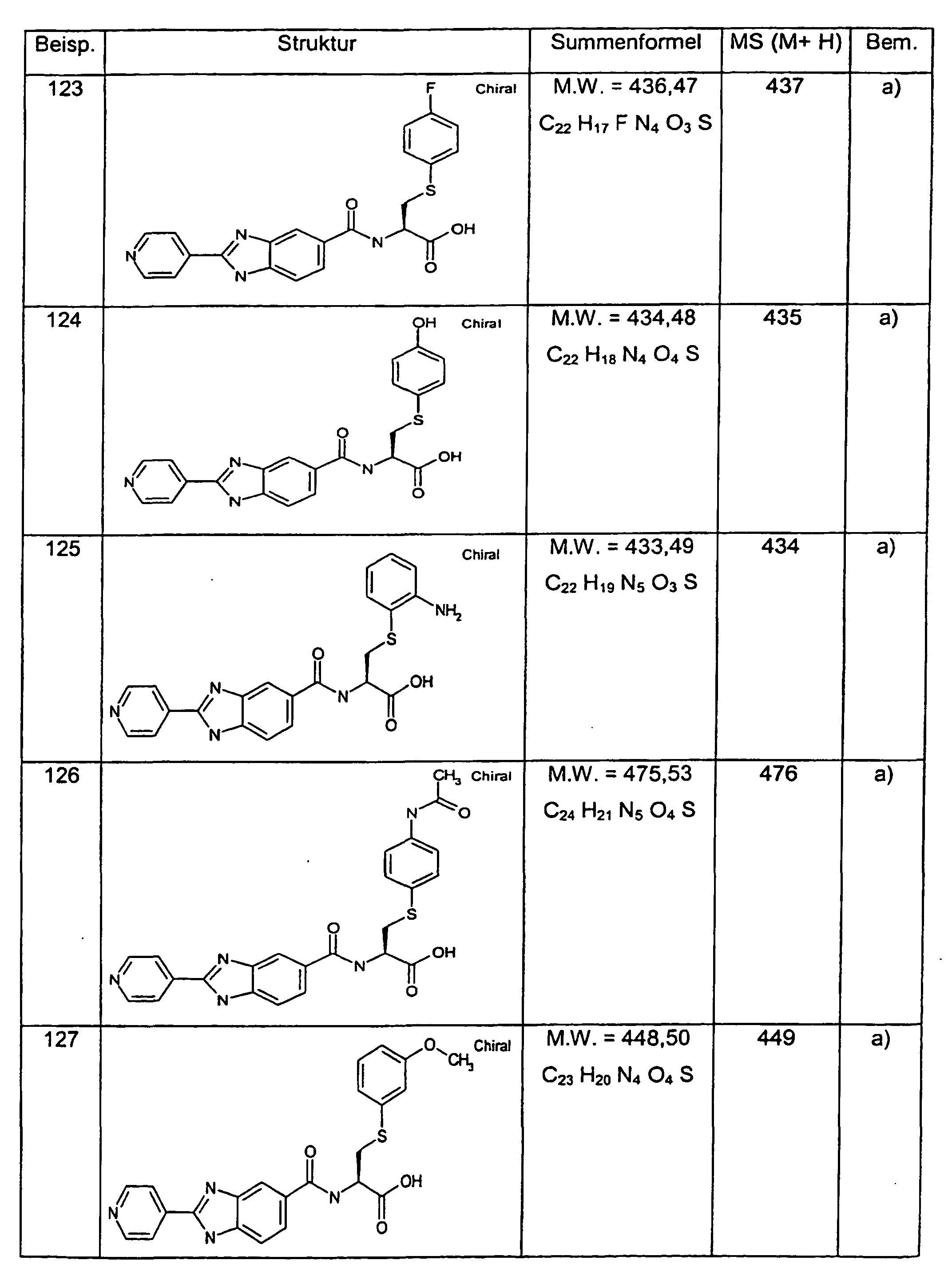

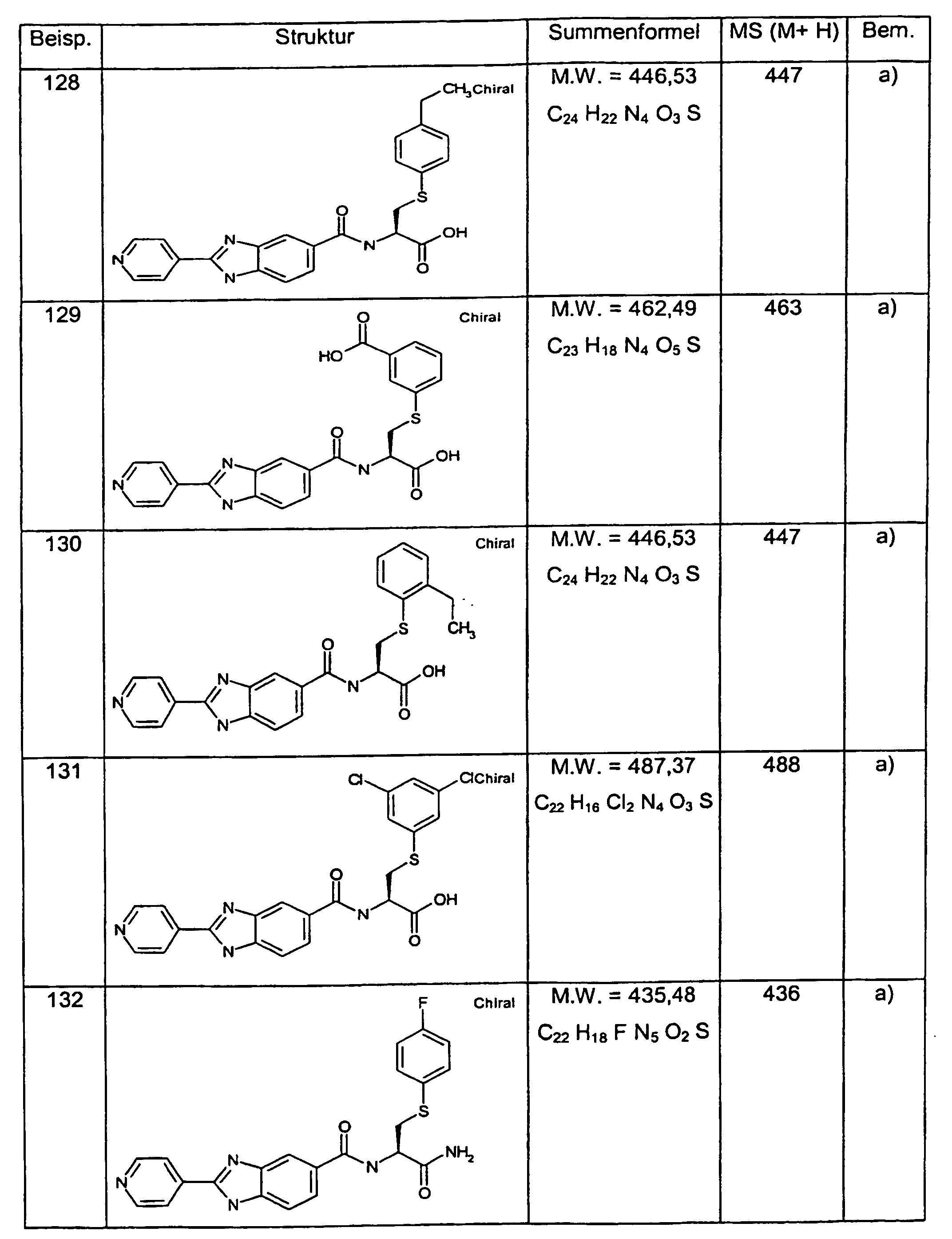

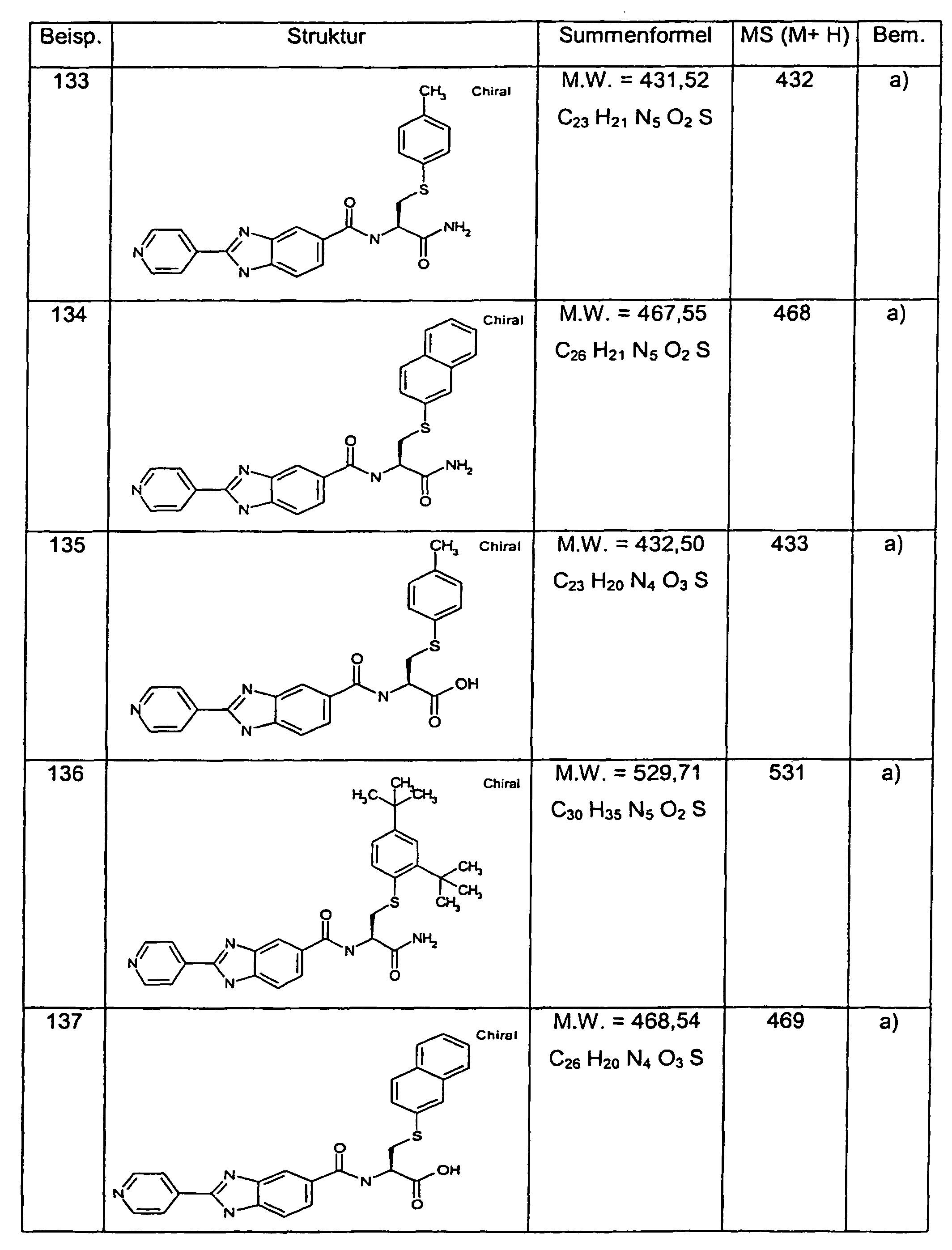

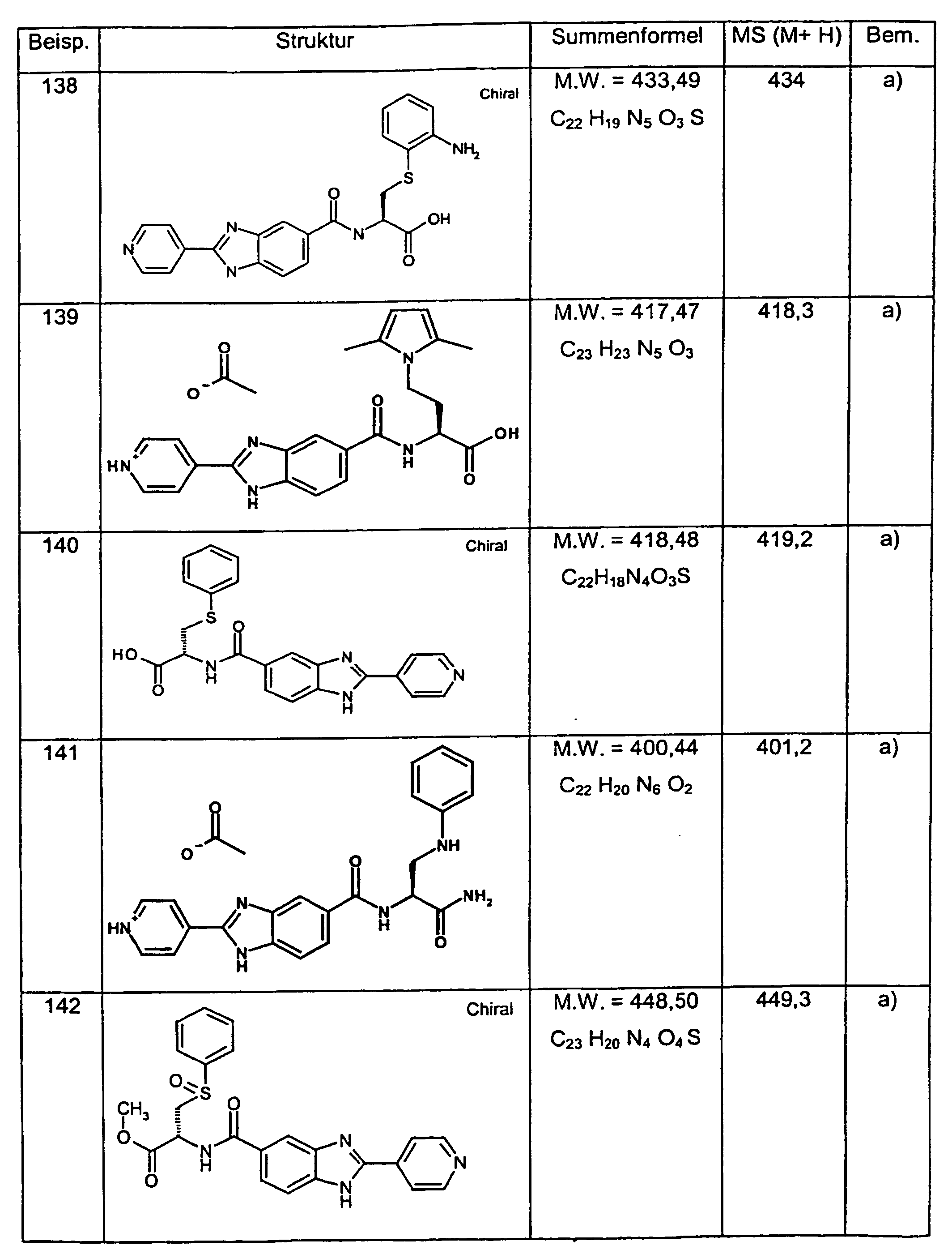

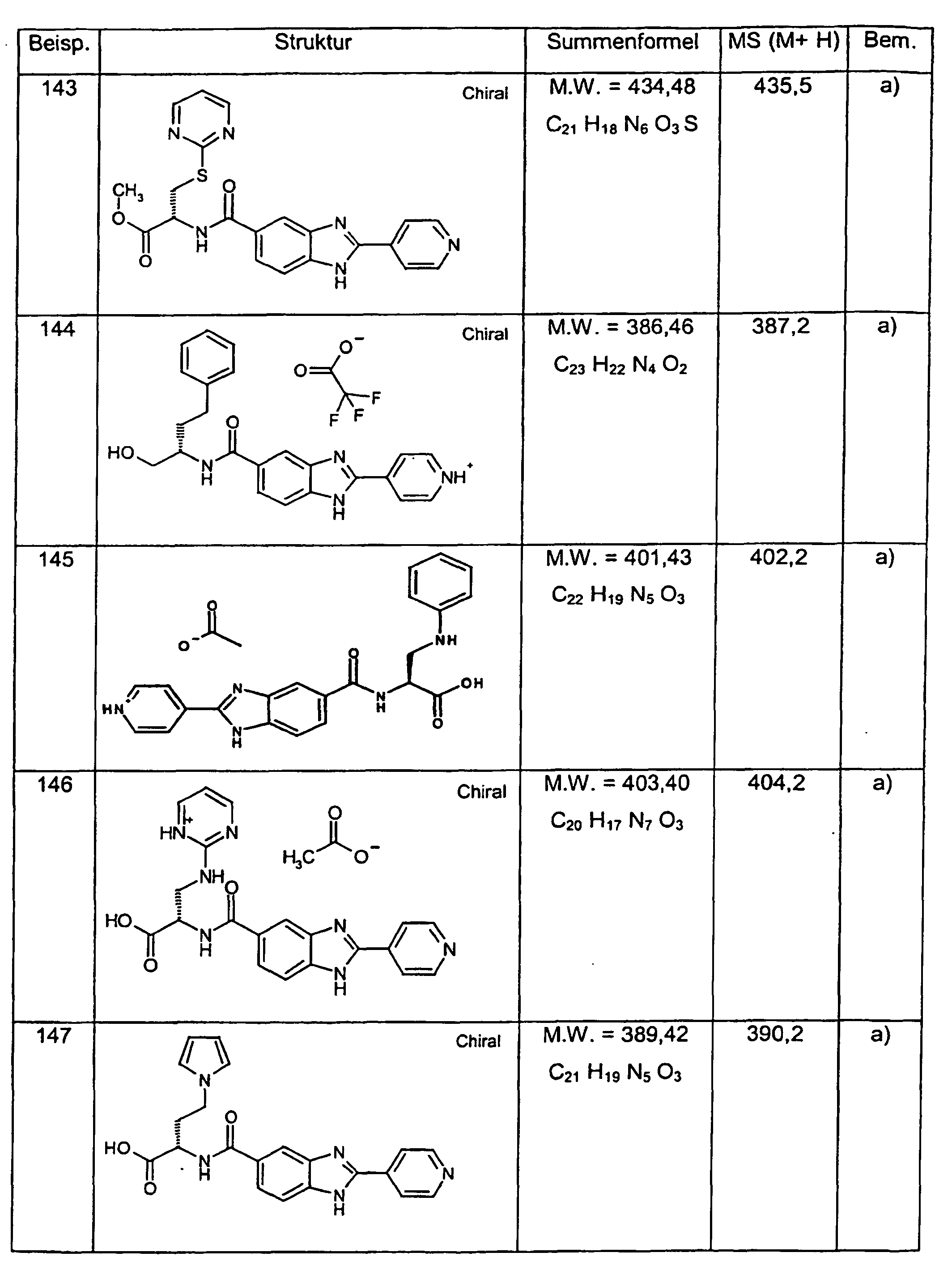

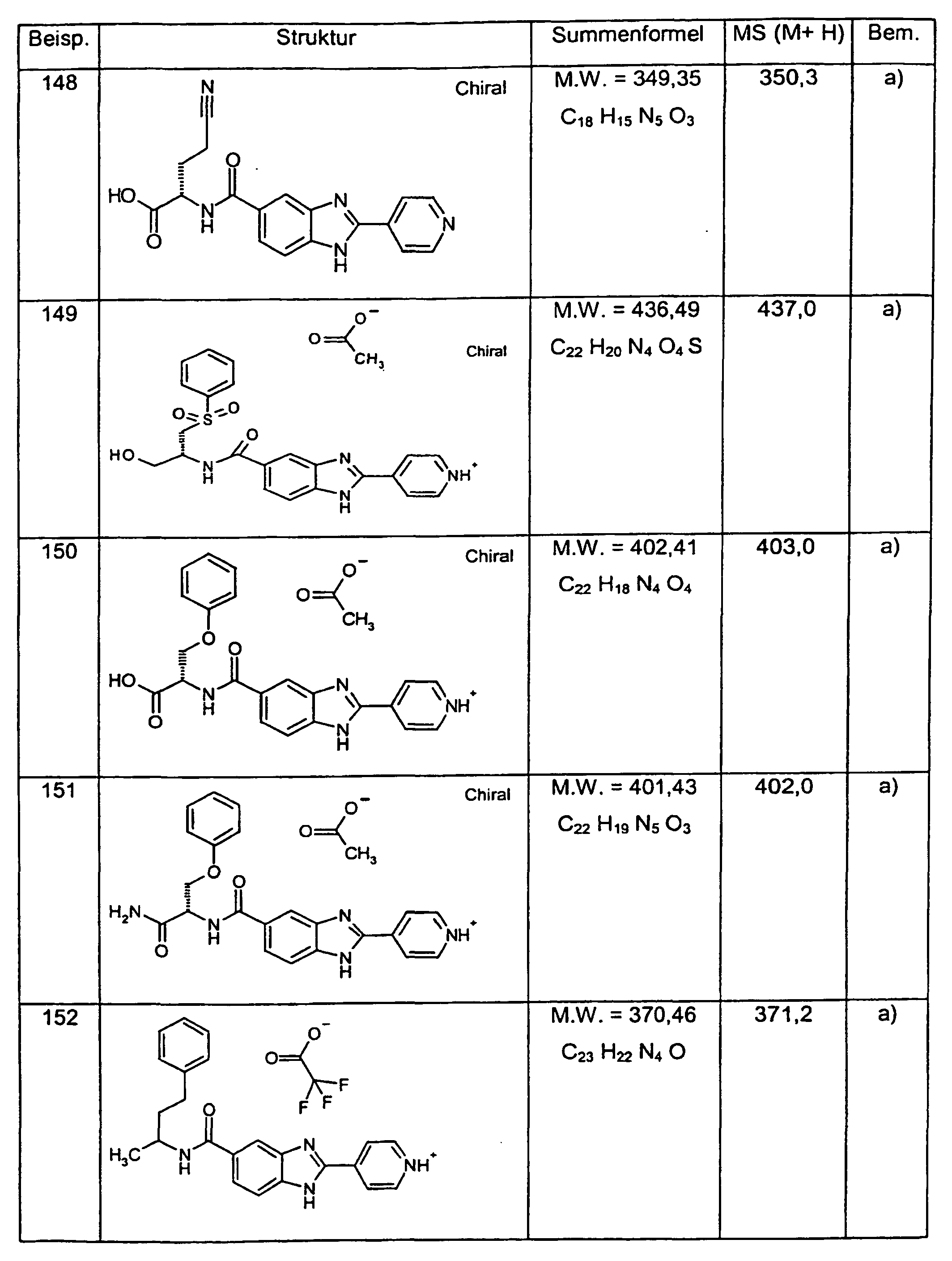

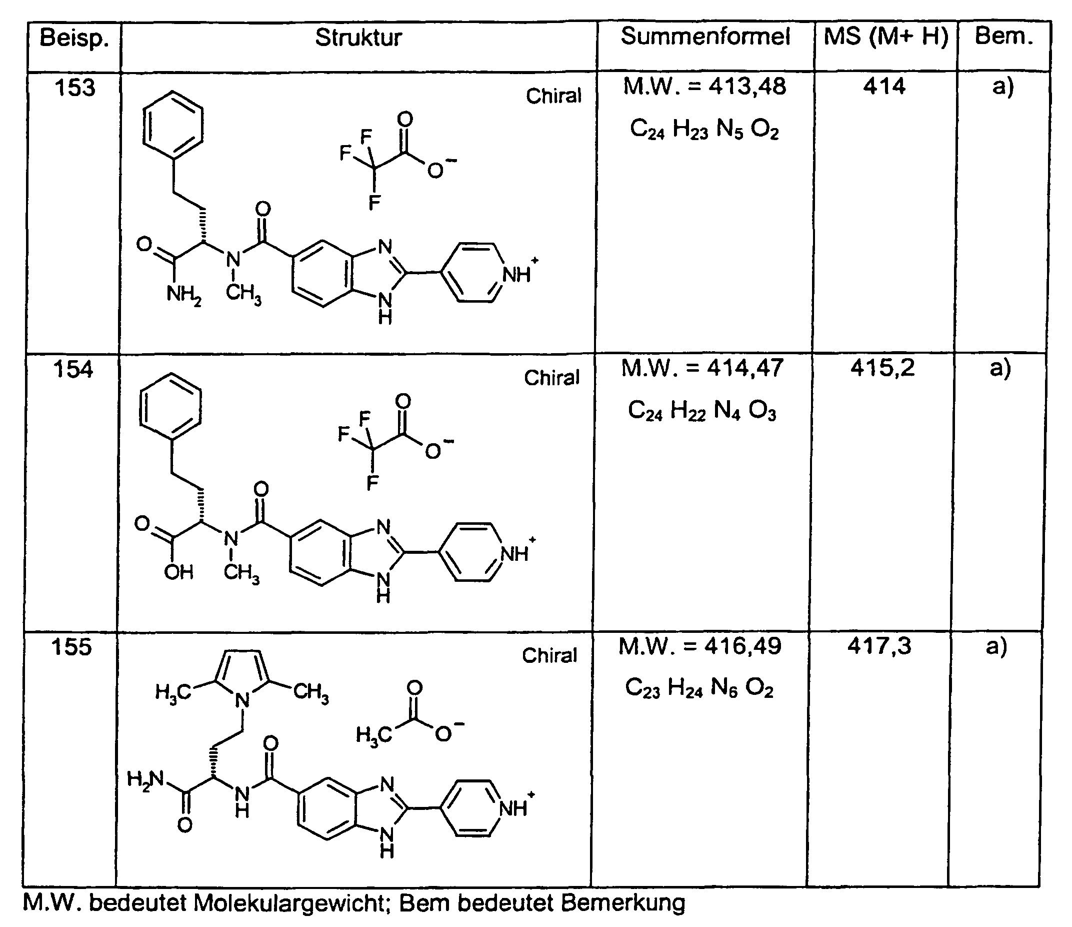

### Beispiel 156

Die nachfolgenden Verbindungen wurde nach Verfahrensvariante a) hergestellt:
a) Herstellung von 2-Fluor-lsonikotinsäure:
   5,00 g (45 mmol) 2-Fluor-4-Methylpyridin und 1,00 g (17 mmol) KOH wurde mit 50 ml Pyridin versetzt und unter Rückfluß erwärmt. Bei dieser Temperatur wurden portionsweise innerhalb von 30 Minuten 20,00 g (127 mmol) Kaliumpermanganat zugegeben und für weitere 1,5 Stunden unter Rückfluß erhitzt. Anschließend wurde im Eisbad abgekühlt, mit 100 ml Wasser versetzt und mit konzentrierter Salzsäure auf pH von 1 gebracht. Nach der Zugabe von 100 ml Essigester wurde von dem unlöslichen Rückstand abfiltriert und die wässrige Phase noch zweimal mit je 100 ml Essigester extrahiert. Die vereinigten Essigesterphasen wurden über Magnesiumsulfat getrennt und unter verminderten Druck eingeengt. Man erhielt 2,70 g 2-Fluor-Isonikotinsäure. Ausbeute: 42%
b) Herstellung von (2-Fluor-Pyridin-4-yl)-Methanol:
   12,60g (89 mmol) 2-Fluor-Isonikotinsäure wurden mit 13,3 ml (95mmol) Triethylamin in 300 ml Toluol vorgelegt und mit 9,08 ml (95 mmol) Chlorameisensäureethylester versetzt und 1 Stunde (h) bei Raumtemperatur (20 ° - 23 °C) gerührt. Anschließend wurde das Triethylammonium Chlorid abfiltriert und die Toluolphase unter verminderten Druck eingeengt. Der Rückstand wurde in 200 ml absoluten THF aufgenommen und auf -78 °C abgekühlt. Bei dieser Temperatur wurde eine Suspension von Lithiumaluminiumhydrid (3,55g, 95mmol) in THF zugetropft und weitere 30 Minuten gerührt. Danach ließ man die Reaktionsmischung auf Raumtemperatur kommen und goß auf 1l Eiswasser. Es folgten Extraktion mit 4 mal 300 ml Essigester, Trocknen der vereinigten Essigesterphase über Magnesiumsulfat und Evaporieren des Lösungsmittels lieferte das Rohprodukt, welches nach Reinigung über Mitteldruckchromatographie (CH₂Cl₂ : MeOH wie 9 : 1) 5,10 g (40 mmol) des gewünschten Produktes lieferte. Ausbeute 45%
c) Herstellung von 2-Fluor-Pyridin-4-Carbaldehyd
   Zu einer Lösung von 4,6 ml (54 mmol) Oxalylchlorid und 7,6 ml (106 mmol) Dimethylsulfoxid (DMSO) in 450 ml Dichlormethan wurde bei -78°C eine Lösung von 5 g (39 mmol) (2-Fluor-Pyridin-4-yl)-Methanol in Dichlormethan getropft und 15 Minuten nachgerührt. Anschließend wurden 24 ml (180 mmol) Triethylamin zugegeben und die Reaktionslösung langsam auf Raumtemperatur erwärmt. Es wurde auf 500 ml Wasser gegossen und je einmal mit 10%iger Zitronensäure (200 ml) und mit 10%iger Natriumcarbonatlösung gewaschen. Die Dichlormethanphase wurde über Magnesiumsulfat getrocknet und unter verminderten Druck eingeengt. Ausbeute 4,60 g (37 mmol) 94%.
d) Herstellung von 2-(2-Fluor-Pyridin-4-yl)-1H-Benzoimidazole-5-Carbonsäure:
   2,00 g (15 mmol) 2-Fluor-Pyridin-4-Carbaldehyd wurden mit 2,40g (15 mmol) 3,4-Diaminobenzoesäure in 100 ml Nitrobenzol suspendiert und 3 h bei 145°C gerührt. Anschließend wurde die Reaktionslösung auf 0°C gekühlt und die sich dabei langsam bildenden Kristalle abfiltriert. Es wurden 2,53 g (9,8 mmol) des gewünschten Benzimidazoles erhalten. Ausbeute 62%
e) Herstellung von 2-(2-Methylamino-Pyridin-4-yl)-1H-Benzoimidazol-5-Carbonsäure:
   100 mg (0,38 mmol) 2-(2-Fluor-Pyridin-4-yl)-1H-Benzoimidazole-5-Carbonsäure wurden in 5 ml Methanol gelöst. Anschließend wurde die Methanollösung mit gasförmigen Methylamin gesättigt und und die Reaktionsmischung in einem Autoklaven für 10 h unter Eigendruck bei 120 °C gerührt. Mitteldruckchromatographie (CH₂Cl₂ : MeOH = 2 : 1) lieferten 56 mg (0,21mmol) des Substitutionsproduktes. Ausbeute 55%
f) Herstellung von 2-(S)-{[2-(2-Methylamino-Pyridin-4-yl)-1H-Benzoimidazole-5-Carbonyl]-amino}-4-Pyrrol-1-yl-Buttersäure Trifluoracetate:
   50 mg (0,186 mmol) 2-(2-Fluor-Pyridin-4-yl)-1H-Benzoimidazole-5-Carbonsäure wurden in 3 ml DMF gelöst und auf 0°C gekühlt. Dazu gab man 100 µl (0,58 mmol) Diisopropylethlyamin und 64 mg (0,195 mmol) TOTU. Anschließend wurden 33 mg (0,196 mmol) 2-(S)-Amino-4-pyrrol-1-yl-buttersäure zugegeben und man ließ die Reaktionslösung auf Raumtemperatur kommen. Es wurden 18 h nachgerührt, anschließend auf 20 ml 10%iger Natriumhydrogencarbonatlösung gegossen und 3 mal mit n-Butanol (50 ml) extrahiert. Nach der Evaporierung des Butanols wurde der Rückstand über präparativer HPLC (Acetonitril, 0,1% ige Trifluoressigsäure) gereinigt. Man erhielt so 40 mg (0,075 mmol) des gekuppelten Produktes. Ausbeute 42%

Die in der folgenden Tabelle 3 genannten Beispiele wurden analog hergestellt:

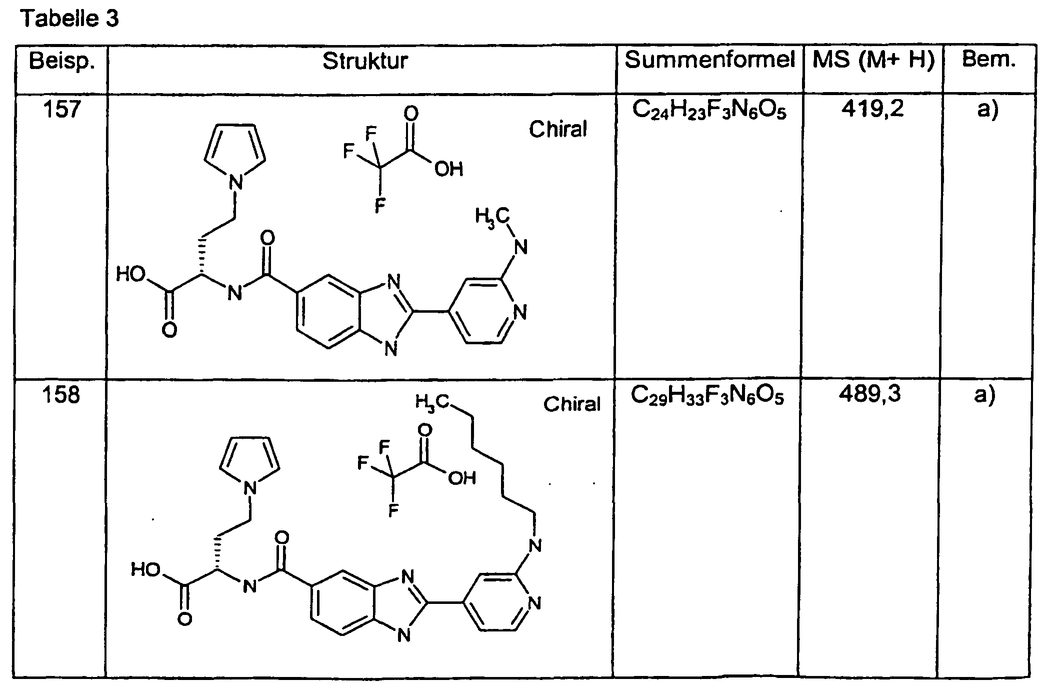

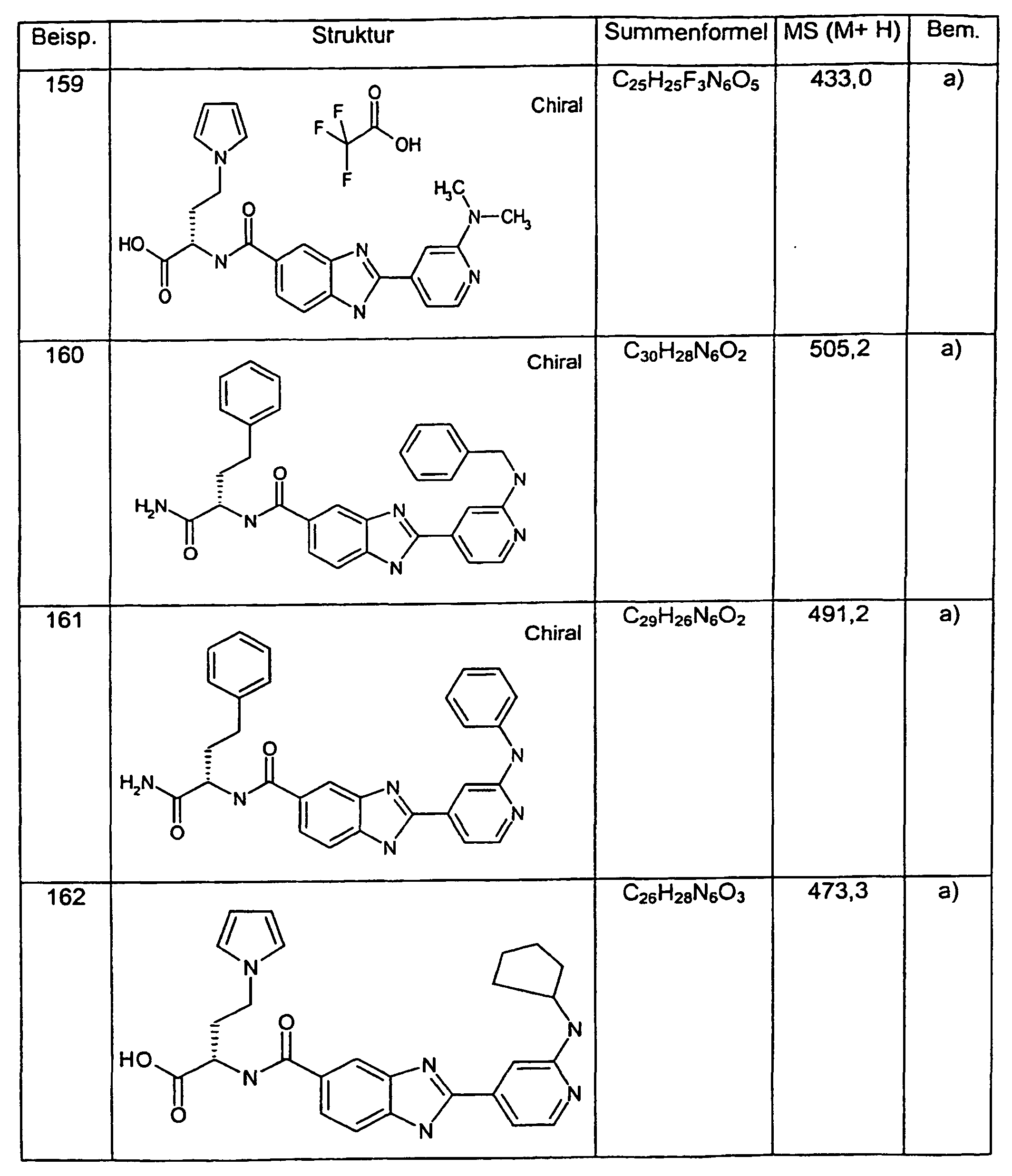

### Beispiel 163

Die nachfolgende Verbindung wurde nach Verfahrensvariante a) hergestellt: 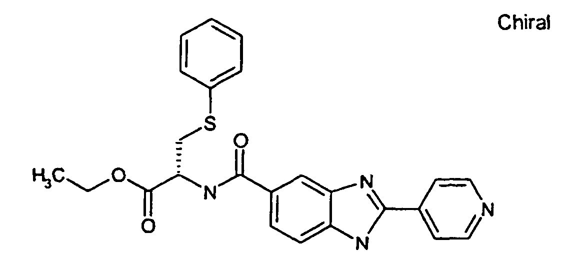
a) Herstellung von 2-(S)-Amino-3-Phenylsulfanyl-Propionsäureethylester
   Zu in 10 ml Methanol gelösten 1,00 g (5 mmol) 2-(S)-Amino-3-Phenylsulfanyl-Propionsäure wurden bei -10°C 1,7 ml (23mmol) Thionylchlorid zugetropft. Anschließend ließ man die Reaktionslösung auf Raumtemperatur kommen und gab 5 ml DMF zu. Es wurde dann 23 h auf 70°C erhitzt und nach Kühlung auf -10°C erneut 1 ml (13,5 mmol) Thionylchlorid zugegeben. Es wurde anschließend noch 14 h bei 70°C gerührt. Nach der Verdampfung der flüssigen Phase wurde der Rückstand in Wasser aufgenommen und mit konzentrierter wässriger Ammoniaklösung basisch gestellt und 3 mal mit Essigester (je 75 ml) extrahiert. Nach der Trocknung über Magnesiumsulfat und Verdampfung fiel das Produkt als Öl an welches ohne weitere Reinigung zur Kupplung mit der Carbonsäurekomponente genutzt wurde. Ausbeute 830 mg (3,7 mmol) 74%
b) Herstellung von 3-Phenylsulfanyl-2-(S)-[(2-Pyridin-4-yl-1H-Benzoimidazole-5-carbonyl)-amino]-propionsäureethylester
   Hierbei lieferte die Standard TOTU Kopplung mit 188 mg (0,83 mmol) 2-(S)-Amino-3-Phenylsulf anyl-Propionsäureethylester und 200 mg (0,83 mmol) 2-Pyridin-4-yl-1H-Benzoimidazole-5-Carbonsäure das gewünschte Produkt. Ausbeute 43% (160 mg, 0,36 mmol)

Die in der folgenden Tabelle 4 genannten Beispiele wurden analog hergestellt:

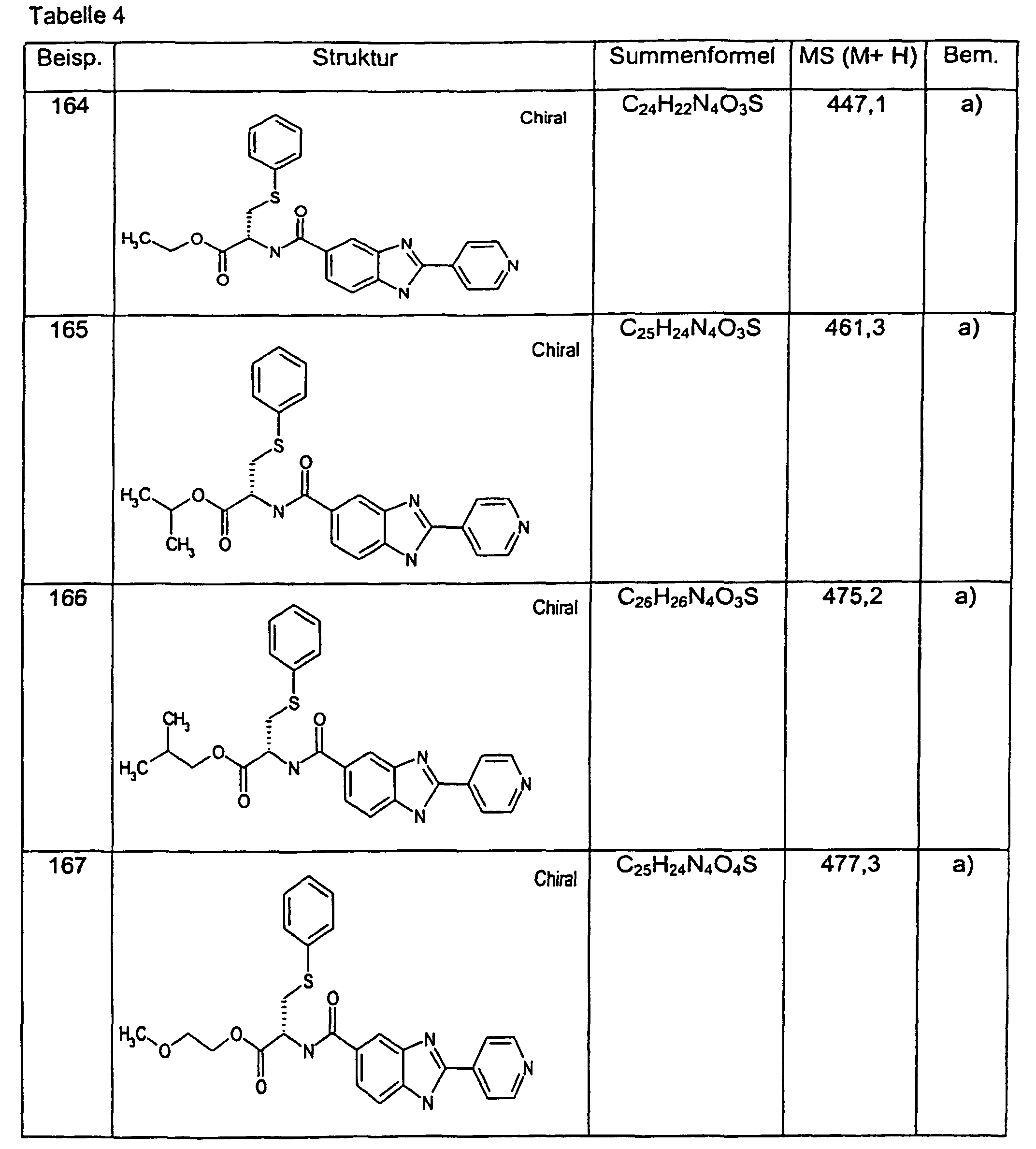

### Beispiel 168

Die nachfolgende Verbindung wurde nach Verfahrensvariante a) hergestellt: 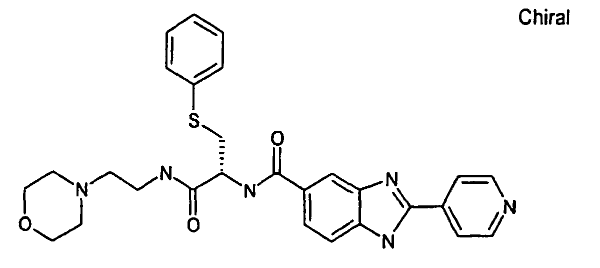
a) Herstellung von 2-Pyridin-4-yl-1H-Benzoimidazol-5-Carbonsäure [1-(2-morpholin-4-yl-Ethylcarbamoyl)-2-Phenylsulfanyl-Ethyl]-Amid
   100 mg (0,24mmol) 3-Phenylsulfanyl-2-[(2-Pyridin-4-yl-1H-Benzoimidazol-5-Carbonyl)-amino]-propionsäure wurde in 10 ml DMF gelöst. Dazu gab man bei 0°C 68µl (0,39 mmol) Disopropylethylamin und 248 mg (0,48 mmol) Benzotriazol-1-yloxy-tripyrrolidinphosphoniumhexafluorophosphate. Dann ließ man auf Raumtemperatur kommen und rührte 24 h nach. Das Lösungsmittel wurde im Hochvakuum bei Raumtemperatur entfernt und der Rückstand wurde mittels Mitteldruckchromatographie (CH₂Cl₂ : MeOH = 8 : 2) gereinigt. Ausbeute 73 mg (0,1376 mmol) 57%.
   Die in der folgenden Tabelle 5 genannten Beispiele wurden analog hergestellt:

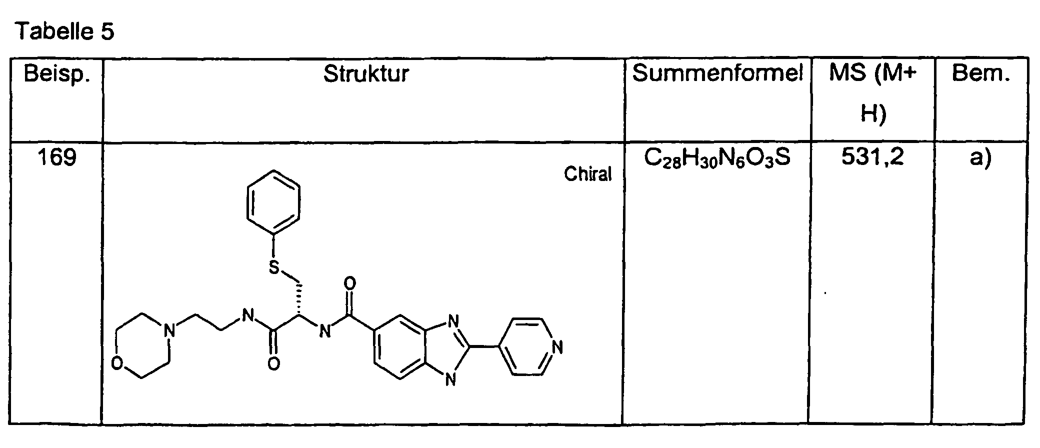

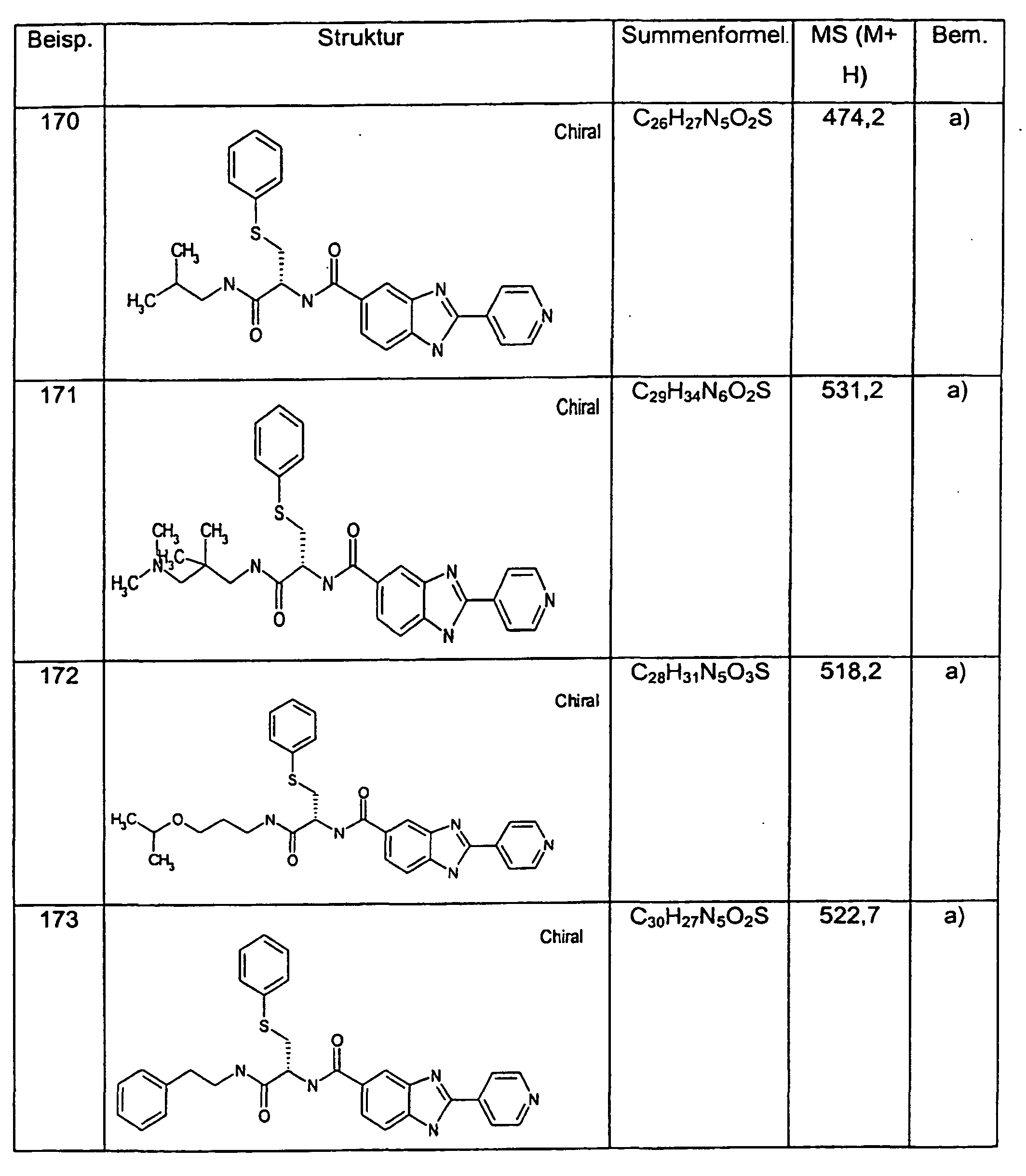

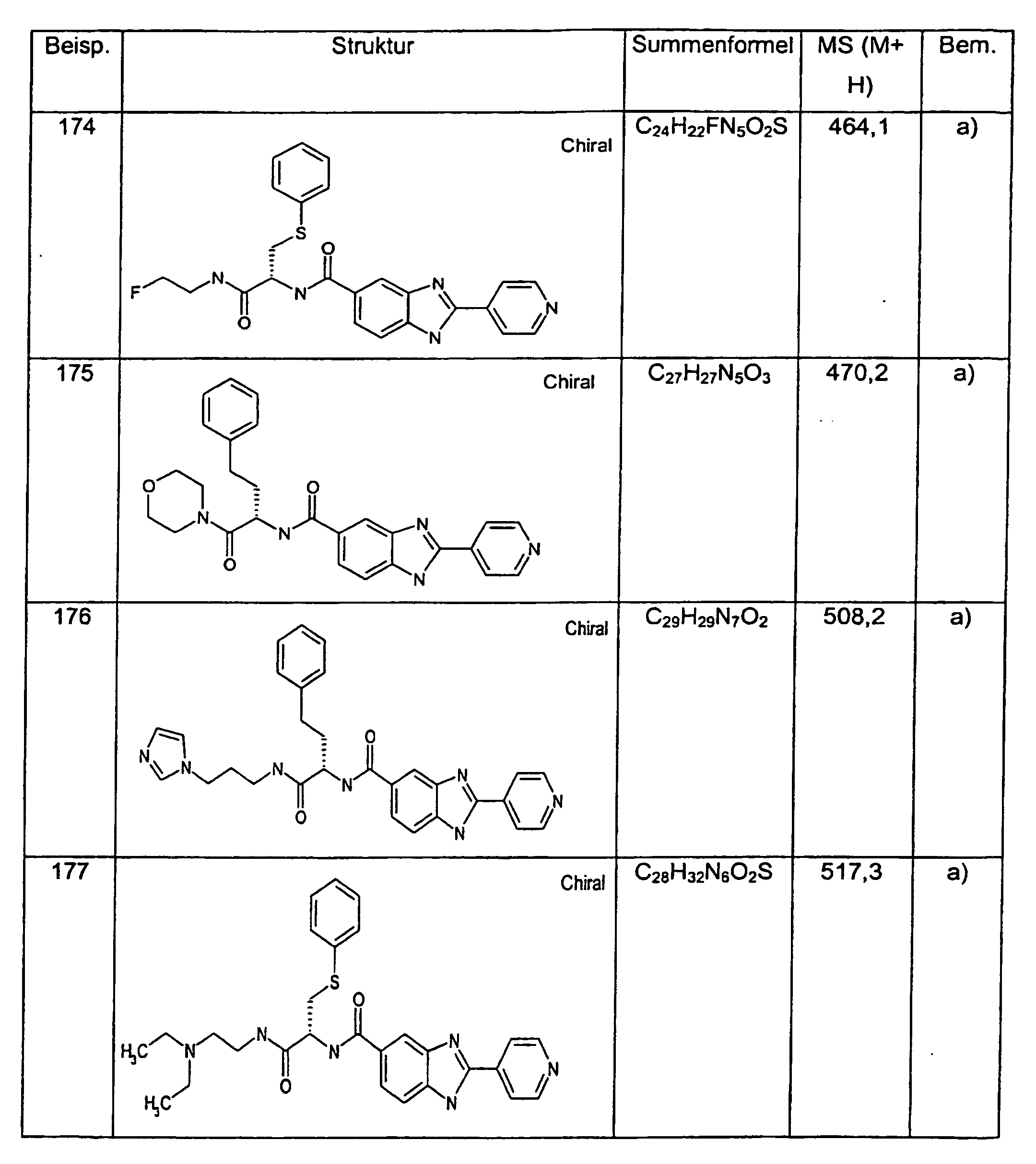

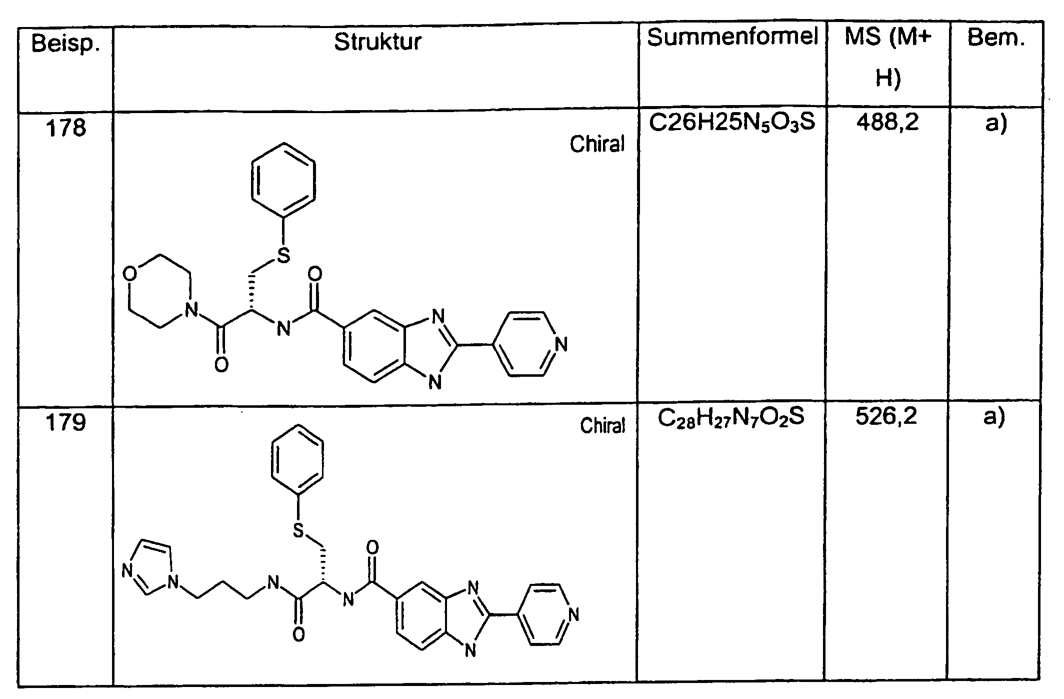

### Beispiel 180:

Die nachfolgende Verbindung wurde nach Verfahrensvariante a) hergestellt: 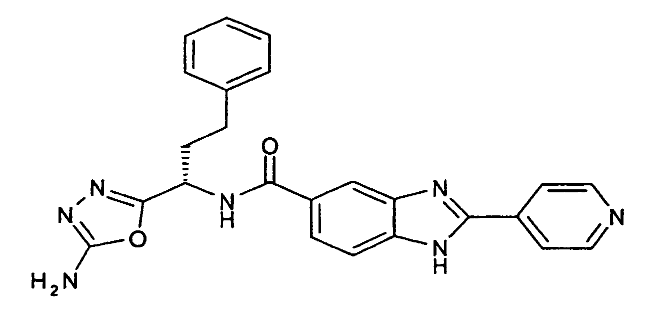
a) Herstellung von Z-Homophenylalaninhydrazid:
   5 g (16 mmol) Z-Homophenylalanin wurden bei Raumtemperatur in 100 ml Methyltert.Butyl-Ether gelöst, mit 3.3 g (16 mmol) N,N'-Dicycfohexylcarbodümid und 50 mg Dimethylaminopyridin versetzt und 2 h bei Raumtemperatur gerührt. Anschließend wurde die Reaktionsmischung über ein Faltenfilter filtriert, und das Filtrat wurde mit 1 M Kaliumhydrogensulfat-Lösung, gesättigter Natriumhydrogencarbonatlösung und Wasser gewaschen. Die organische Phase wurde über Magnesiumsulfat getrocknet, filtriert und unter verminderten Druck eingeengt. Der Rückstand wurde in 20 ml trockenem Ethanol gelöst, mit 0.78 ml (16 mmol) Hydrazinhydrat versetzt und 2h bei Raumtemperatur gerührt. Der Reaktionsverlauf wurde mittels Dünnschichtchromatographie (DC) verfolgt, und nach Ende der Reaktion wurde die Mischung unter verminderten Druck eingeengt. Der Rückstand wurde aus Essigsäureethylester/n-Heptan 1:1 umkristallisiert und man erhielt Z-Homophenylalaninhydrazid, welches so weiter umgesetzt wurde.
b) Herstellung von [1-(5-Amino-[1,3,4]oxadiazol-2-yl)-3-phenyl-propyl] carbaminsäure-benzylester:
   0,66 g Z-Homophenylalaninhydrazid wurden bei Raumtemperatur in 10 ml Wasser suspendiert, mit 200 mg Kaliumhydrogencarbonat versetzt und danach wurden 0,4 ml einer Bromcyan-Lösung (5 M in Acetonitril) zugetropft. Die Mischung wurde 3 h bei Raumtemperatur gerührt und danach mehrmals mit Essigsäureethylester extrahiert. Die vereinigten organischen Phasen wurden mit gesättigter Natriumchloridlösung gewaschen, über Magnesiumsulfat getrocknet, filtriert und unter verminderten Druck eingeengt. Der Rückstand wurde mit Methyl-tert.Butyl-Ether verrührt, abgesaugt und unter verminderten Druck getrocknet. Der so erhaltene [1-(5-Amino-[1,3,4]oxadiazol-2-yl)-3-phenyl-propyl]-carbaminsäure-benzylester wurde ohne weitere Reinigung in der nächsten Stufe eingesetzt.
c) Herstellung von 5-(1-Amino-3-phenyl-propyl)-[1,3,4]oxadiazol-2-ylamin:
   0,33 g [1-(5-Amino-[1,3,4]oxadiazol-2-yl)-3-phenyl-propyl]-carbaminsäure-benzylester wurden bei Raumtemperatur in 50 ml trockenem Methanol gelöst, unter Argon mit Hydrierkatalysator (10% Palladium auf Kohle) versetzt und 3 h bei Raumtemperatur hydriert. Die Reaktionsmischung wurde über Celite abfiltriert, das Filtrat wurde unter verminderten Druck eingeengt und der Rückstand im Hochvakuum getrocknet. Man erhielt 5-(1-Amino-3-phenyl-propyl)-[1,3,4]oxadiazol-2-ylamin, welches ohne weitere Reinigung in der nächsten Stufe eingesetzt wurde.
d) Herstellung von 2-Pyridin-4-yl-1H-benzimidazol-5-carbonsäure [1-(5-amino-[1,3,4]oxadiazot-2-yl)-3- phenyl-propyl]-amid:
   0,18 g 5-(1-Amino-3-phenyl-propyl)-[1,3,4]oxadiazol-2-ylamin wurden bei Raumtemperatur in 10 ml trockenem Dimethylformamid gelöst, mit 200 mg 2-Pyridin-4-yl-1H-benzimidazol-5-carbonsäure, 270 mg TOTU und 0,12 ml Diisopropylamin versetzt und 4 h bei Raumtemperatur gerührt. Die Reaktionsmischung wurde eingeengt, der Rückstand in Essigsäureethylester aufgenommen und nacheinander mit Wasser, gesättigter Natriumhydrogencarbonatlösung, Wasser und gesättigter Natriumchloridlösung gewaschen. Die organische Phase wurde über Magnesiumsulfat getrocknet, filtriert und eingeengt. Der Rückstand wurde mit Methyl-tert.Butyl-Ether verrührt, abfiltriert und im Hochvakuum getrocknet. Man erhielt 2-Pyridin-4-yl-1H-benzimidazol-5-carbonsäure [1-(5-amino-[1,3,4]oxadiazol-2-yl)-3-phenyl-propyl]-amid, welches bei 160 °C unter Zersetzung schmilzt.

### Beispiel 181:

Die nachfolgende Verbindung wurde nach Verfahrensvariante a) hergestellt: 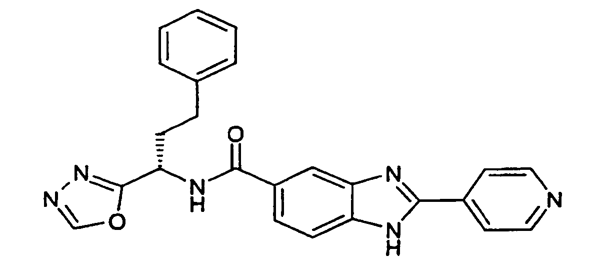
a) Z-Homophenylalaninhydrazid wurde wie in Beispiel 180 beschrieben hergestellt.
b) Herstellung von (1-[1,3,4]Oxadiazol-2-yl-3-phenyl-propyl)-carbaminsäure benzylester:
   1 g Z-Homophenylalaninhydrazid wurde bei Raumtemperatur in 8 ml Orthoameisensäureethylester suspendiert und 4 h unter Rückfluß erhitzt. Die abgekühlte Reaktionsmischung wurde mit Methyl-tert.Butyl-Ether versetzt, der Niederschlag wurde abfiltriert und das Filtrat unter vermindertem Druck eingeengt. Der Rückstand wurde über Kieselgel mit Essigsäureethylester/n-Heptan 1/1 chromatographiert und lieferte (1-[1,3,4]Oxadiazol-2-yl-3-phenyl-propyl)-carbaminsäure benzylester, welcher in der nächsten Stufe eingesetzt wurde.
c) Die Herstellung von 1-[1,3,4]Oxadiazol-2-yl-3-phenyl-propylamin erfolgt analog zur Herstellung von 5-(1-Amino-3-phenyl-propyl)-[1,3,4]oxadiazol-2-ylamin wie in Beispiel 180 beschrieben.
d) Herstellung von 2-Pyridin-4-yl-1H-benzimidazol-5-carbonsäure (1-[1,3,4]oxadiazol-2-yl-3-phenyl-propyl)-amid:
   220 mg 1-[1,3,4]Oxadiazol-2-yl-3-phenyl-propylamin wurden bei Raumtemperatur in 10 ml trockenem Dimethylformamid gelöst, mit 260 mg 2-Pyridin-4-yl-1H-benzimidazol-5-carbonsäure, 350 mg TOTU und 0,15 ml Diisopropylamin versetzt und 4 h bei Raumtemperatur gerührt. Die Reaktionsmischung wurde eingeengt, der Rückstand in Essigsäureethylester aufgenommen und nacheinander mit Wasser, gesättigter Natriumhydrogencarbonatlösung, Wasser und gesättigter Natriumchloridlösung gewaschen. Die organische Phase wurde über Magnesiumsulfat getrocknet, filtriert und eingeengt. Der Rückstand wurde über Kieselgel mit Dichlormethan/Methanol 8/1 chromatographiert und lieferte 2-Pyridin-4-yl-1H-benzimidazol-5-carbonsäure (1-[1,3,4]oxadiazol-2-yl-3-phenyl-propyl)-amid, welches bei 103 °C unter Zersetzung schmilzt.

### Beispiel 182

Die nachfolgende Verbindung wurde nach Verfahrensvariante a) hergestellt: 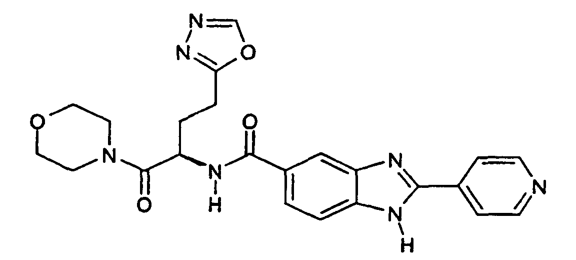
a) Herstellung von L-N-Benzyloxycarbonyl-4-([1,3,4]oxadiazol-2-yl)-2-aminobutansäure
   1 g Z-Glutaminsäure-y-hydrazid wurde mit 30 mg para-Toluolsulfonsäure in 20 ml Orthoameisensäuretrimethylester suspendiert und bei Raumtemperatur gerührt. Die Suspension klärte sich innerhalb von 30 Minuten, die dadurch entstandene Lösung wurde filtriert und mit 100 ml Wasser verdünnt. Nach Zugabe von 20 ml 2N Salzsäure wurde 5 mal mit Ethylacetat extrahiert und anschließend wurden die vereinigten organischen Phasen über Natriumsulfat getrocknet. Nach Filtration engte man die Lösung unter vermindertem Druck ein und erhielt eine zähe opake Masse.
b) Herstellung von L-*N*-Benzyloxycarbonyl-4-([1,3,4]oxadiazol-2-yl)-2-aminobutansäuremorpholid
   300 mg L-*N*-Benzyloxycarbonyl-4-([1,3,4]oxadiazol-2-yl)-2-amino-butansäure und 200 mg EDC-Hydrochlorid wurden in 20 ml Dichlormethan gelöst und dann mit 2 ml Morpholin versetzt. Nach zweitägigem Rühren bei Raumtemperatur wurde die Lösung 3 mal mit gesättigter Natriumhydrogencarbonat-Lösung, 3 mal mit wäßriger Citronensäure-Lösung und einmal mit gesättigter Natriumhydrogencarbonat-Lösung ausgeschüttelt. Die organische Phase wurde über Natriumsulfat getrocknet und nach Filtration unter vermindertem Druck eingeengt. Man erhielt einen geblichen opaken Rückstand.
c) Herstellung von L-4-([1,3,4]Oxadiazol-2-yl)-2-amino-butansäurernorpholid
   70 mg L-*N*-Benzyloxycarbonyl-4-([1,3,4]oxadiazol-2-yl)-2 aminobutansäuremorpholid wurden in 20 ml Methanol gelöst und mit 1 Spatelspitze Palladium auf Aktivkohle (5%) versetzt und die Suspension unter Wasserstoffatmosphäre gerührt. Nach drei Stunden wurde der Katalysator über Celite abfiltriert und das Filtrat nach Filtration durch ein 0,45 pm-Filter unter vermindertem Druck eingeengt.
d) Herstellung von 2-Pyridin-4-yl-1H-benzimidazol-5-carbonsäure[1-(morpholin-4-carbonyl)-3-[1,3,4]oxadiazol-2-yl-propyl]-amid
   43 mg 2-Pyridin-4-yl-1H-benzimidazol-5-carbonsäure, 75 mg HATU und 51 mg Diisopropylethylamin wurden in 1ml *N*,*N*-Dimethylformamid gelöst und nach Rühren für 10 min. mit 40 mg L-4-([1,3,4]oxadiazol-2-yl)-2-amino-butansäuremorpholid in 0,4 ml *N*,*N*-Dimethylformamid versetzt. Nach 7 h Rühren bei Raumtemperatur wurden 200 mg Aminomethylpolystyrol (1,37 mmol/g) und 20 ml *N*,*N*-Dimethylformamid zugesetzt. Nach 1 h filtrierte man und destilliert das *N*,*N*-Dimethylformamid unter vermindertem Druck ab. Der Rückstand wurde mit kaltem Acetonitril digeriert. Der unlösliche Rückstand wurde verworfen und die Acetonitril-Lösung einer Gradientenfiltration über RP18-Kieselgel mit Wasser/Acetonitril-Gemischen unterzogen. Man isolierte einen glasigen gelblichen Feststoff.

### Beispiel 183 3-Phenoxy-2-[(2-pyridin-4-yl-1H-benzoimidazol-5-carbonyl)-amino]-propionsäure Hydrogenacetat

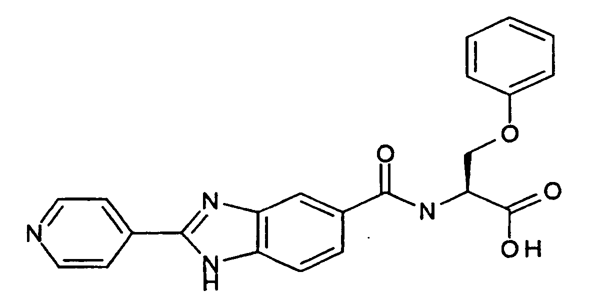

a) 2-Pyridin-4-yl-1H-benzoimidazol-5-carbonsäure (im folgenden Verbindung I genannt) 15,2 g (0,1 mol) 3,4-Diaminobenzoesäure wurden in 1 l Nitrobenzol suspendiert und mit 11,2 g (0,104 mol) Pyridin-4-aldehyd versetzt. Dann erhitzte man das Gemisch unter heftigem Rühren 8 h auf 145 °C bis 155 °C. Nach Abkühlung der Lösung wurde das ausgefallene Produkt abgesaugt und gründlich mit Ethylacetat und Dichlormethan gewaschen. Zur Reinigung erhitzte man das Rohprodukt in einem Gemisch aus 300 ml Methanol, 100 ml Dichlormethan und 10 ml DMF zum Sieden. Nach Abkühlung filtrierte man das ungelöste Produkt ab, und wusch mit Dichlormethan. Das erhaltene Material wurde in etwa 200 ml DMSO aufgenommen, danach erhitzte man die Mischung bis eine homogene Lösung entstand - kühlte auf etwa 50 °C ab und versetzte mit 200 ml Methanol- das Produkt kristallisierte nach etwa 30 min. bei RT. Man filtrierte den Niederschlag ab und wusch gründlich mit Methanol. Ausbeute: 19,4 g .
b) (S) 2-Amino-3-phenoxypropionsäure Hydrochlorid (M.W. 217,6)
   2,8 g Trt-Ser-OMe (Bachem), 0,75 g Phenol und 2,25 g Triphenylphosphin wurden zusammen in 60 ml THF, absolut, gelöst und bei 0 °C innerhalb von 30 min tropfenweise mit 1,49 g Azodicarbonsäurediethylester versetzt. Das Reaktionsgemisch wurde 30 min bei 0 °C nachgerührt, auf RT erwärmt (etwa 1 h). Zur Aufarbeitung entfernte man das Lösungsmittel unter vermindertem Druck und reinigte das Rohprodukt durch Chromatographie an Kieselgel (Heptan : EE = 1,5 : 1). Der so erhaltene (S)-2-Tritylamino-3-phenoxy-propionsäuremethylester kristallisierte langsam in langen Nadeln, und wurde zur Abspaltung der Schutzgruppen 1 h in 5 M HCl unter Rückfluß erhitzt. Die Reaktionslösung wurde unter verminderten Druck durch mehrmaliges Koevaporieren mit Toluol zur Trockne eingedampft und der Rückstand aus wenig tert.-Butanol umkristallisiert.
   Verfahrensschritt c)
   239 mg 2-Pyridin-4-yl-1H-benzoimidazol-5-carbonsäure aus a) wurden in 10 ml DMF suspendiert und nacheinander mit 328 mg TOTU und 0,17 ml Ethyldiisopropylamin versetzt. Man rührte 45 min. bei RT und gab zu der entstandenen klaren Lösung 217,6 mg (S) 2-Amino-3-phenoxypropionsäure Hydrochlorid hergestellt wie unter b), sowie 0,34 ml Ethyldiisopropylamin hinzu. Nach 4 h Rühren engte man unter vermindertem Druck ein und isolierte die Titelverbindung durch Flash-Chromatographie an Kieselgel (DCM:MeOH:AcOH:Wasser = 70:10:1:1). Die erhaltene Titelverbindung zeigte M.W. = 402,41; Molmasse 402; Summenfonnel C₂₂H₁₈N₄O₄

### Beispiel 184 3-Phenylamino-2-[(2-pyridin-4-yl-1H-benzoimidazol-5-carbonyl)-amino]-propionsäure Hydrogenacetat

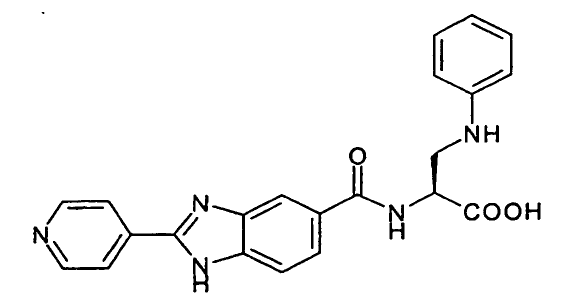

a) L-2-Amino-3-phenylaminopropionsäure
   2,74 g Triphenylphosphin wurden unter Erwärmung in 30 ml Acetonitril gelöst und unter Ausschluß von Feuchtigkeit auf -35 °C bis -45 °C gekühlt (dabei fiel das Phosphin feinverteilt an) und dann wurde bei dieser Temperatur innerhalb von 40 min. tropfenweise 1,82 ml Azodicarbonsäure-diethylester hinzugegeben. Man rührte 15 min. bei -35 °C nach. Zu diesem Gemisch tropfte man eine Lösung aus 2,5 g N-Benzyloxycarbonyl-L-serin in 5 ml Acetonitril und 2 ml THF, dabei ließ man die Temperatur nicht über -35 °C steigen. Anschließend ließ man 1 h bei -35 °C nachreagieren und erwärmte auf RT. Die Reaktionslösung wurde unter vermindertem Druck vom Lösungsmittel befreit und das Rohprodukt sofort mit Mitteldruckchromatographie an Kieselgel gereingt. (DCM/AcCN : 20/1) Nach Entfernen des Lösungsmittels erhielt man 1,4 g sauberes N-Benzyloxycarbonyl-L-serin-β-lacton (siehe auch Org. Synth. 1991 (70) 1ff.) in feinen Nadeln. 1,2 g des Lactons wurden in 10 ml Acetonitril gelöst und mit 0,51 g Anilin 2 h unter Rückfluß erhitzt. Nach Entfernung des Lösungsmittels isolierte man das Produkt durch Chromatographie an Kieselgel (DCM / MeOH / AcOH = 100 / 5 / 1 ). Man erhielt so 1,1 g (68%) L-2-Benzyloxycarbonylamino-3-phenylaminopropionsäure.
   Zur Abspaltung der Schutzgruppe löste man das Z-geschützte Derivat in Methanol, fügte unter Inertgas 80 mg Katalysator (10% Pd-C) zu, und leitete bis zur vollständigen Abspaltung der Z-Schutzgruppe Wasserstoff ein. Nach Abfiltrieren des Katalysators und Eindampfen des Filtrates erhielt man 0,58 g L-2-Amino-3-phenylamino-propionsäure (92%) als gelbliche weiche Nadeln
Verfahrensschritt b)
   239 mg 2-Pyridin-4-yl-1H-benzoimidazol-5-carbonsäure hergestellt wie in Beispiel 183 wurden in 10 ml DMF suspendiert und nacheinander mit 328 mg TOTU und 0,17 ml Ethyldiisopropylamin versetzt. Man rührte 45 min bei RT und gab zu der entstandenen klaren Lösung 180,2 mg (S) 2-Amino-3-phenylaminopropionsäure hergestellt gemäß a) sowie 0,34 ml Ethyldiisopropylamin. Nach 4 h Rühren engte man unter vermindertem Druck ein und isolierte die Titelverbindung durch Flash-Chromatographie an Kieselgel (DCM:MeOH:AcOH:Wasser = 70:10:1:1). Die erhaltene Titelverbindung zeigte M.W. = 401,43; Summenformel C₂₂H₁₉N₅O₃

### Beispiel 185

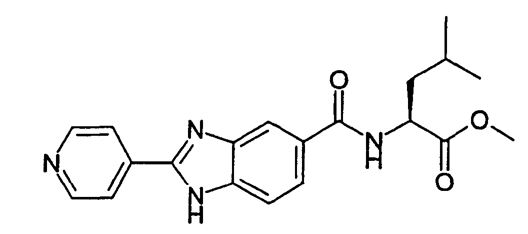

239,2 mg (1mmol) der Verbindung I hergestellt wie in Beispiel 183 wurden in etwa 8 ml DMF nacheinander mit 182,7 mg (1mmol) H-Leu-OMe HCI, 328 mg (1mmol) TOTU und 0,34 ml (2 mmol) Ethyldiisopropylamin versetzt. Nach 6 h bei RT wurde das Lösungsmittel unter vermindertem Druck abdestilliert, der Rückstand in EE aufgenommen und je 3 mal mit Wasser und gesättigter NaCI-Lösung gewaschen. Die organische Phase wurde getrocknet und unter vermindertem Druck zur Trockne eingedampft. Der Rückstand wurde durch Flash-Chromatographie an Kieselgel (DCM / MeOH : 15/1) gereinigt. Ausbeute: etwa 200 mg.

### Beispiel 186

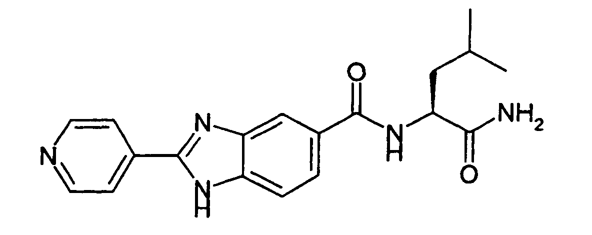

239,2 mg (1mmol) der Verbindung I hergestellt wie in Beispiel 183 wurden in etwa 8 ml DMF nacheinander mit 166,6 mg (1mmol) H-Leu-NH₂ HCl, 328 mg (1mmol) TOTU und 0,34 ml (2 mmol) Ethyldiisopropylamin versetzt. Nach 6 h bei RT wurde das Lösungsmittel unter verminderten Druck abdestilliert, der Rückstand in EE aufgenommen und 1 mal mit Wasser gewaschen. Die wässrige Phase wurde dann mit NaCl gesättigt und mit EE/THF=1/1 zweimal extrahiert. Die vereinigten organischen Phasen wurden 1 mal mit gesättigter NaCl-Lösung gewaschen, getrocknet und unter vermindertem Druck zur Trockne eingedampft. Der Rückstand wurde mit Dichlormethan ausgefällt und abfiltriert. Zur Reinigung wurde das Rohprodukt mit DCM/EE=1/1 ausgekocht, abfiltriert und gründlich mit DCM / Ether gewaschen. Ausbeute: etwa 200 mg

### Beispiel 187

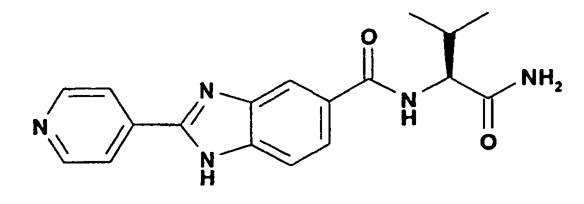

239.2 mg (1 mmol) der Verbindung I hergestellt wie in Beispiel 183 wurden in etwa 8 ml DMF nacheinander mit 152,6 mg (1 mmol) H-Val-NH₂ ·HCl, 328 mg (1 mmol) TOTU und 0,34 ml (2 mmol) Ethyl-düsopropylamin versetzt. Nach 6 h bei RT wurde das Lösungsmittel unter vermindertem n Druck abdestilliert, der Rückstand in EE aufgenommen und 1 mal mit Wasser gewaschen. Die wässrige Phase wurde dann mit NaCI gesättigt und mit EE/THF=1/1 zweimal extrahiert. Die vereinigten organischen Phasen wurden 1 mal mit gesättigter NaCI-Lösung gewaschen, getrocknet und unter vermindertem Druck zur Trockene eingedampft. Der Rückstand wurde mit Dichlormethan ausgefällt und abfiltriert. Zur Reinigung wurde das Rohprodukt mit DCM/EE=1/1 ausgekocht, abfiltriert und gründlich mit DCM / Ether gewaschen. Ausbeute: etwa 200 mg

### Beispiel 188

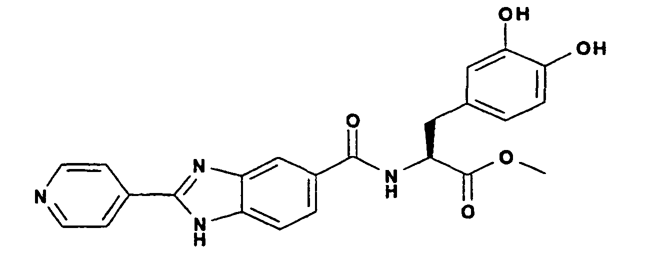

239,2 mg (1 mmol) der Verbindung I hergestellt wie in Beispiel 183 wurden in etwa 8 ml DMF nacheinander mit 247,7 mg (1 mmol) H-Dopa-OMe ·HCl, 328 mg (1 mmol) TOTU und 0,34 ml (2 mmol) Ethyldüso-propylamin versetzt. Nach 6 h bei RT wurde das Lösungsmittel unter vermindertem Druck abdestilliert, der Rückstand in EE aufgenommen und je 3 mal mit Wasser und gesättigter NaCl-Lösung gewaschen. Die organische Phase wurde getrocknet und unter vermindertem Druck zur Trockne eingedampft. Der Rückstand wurde mit Dichlormethan ausgefällt und abfiltriert. Zur Reinigung wurde das Rohprodukt mit DCM/EE=1/1 ausgekocht, abfiltriert und gründlich mit DCM / Ether gewaschen. Ausbeute: etwa 200 mg

### Beispiel 189

Die nachfolgende Verbindung wurde nach Verfahrensvariante c) hergestellt:

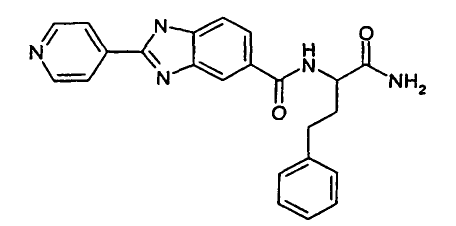

2,0 g von Polystyrene-AM RAM, 160-200 micron (0,64 mmol/g; Rapp Polymere) - Harz wurde in eine Plastikspritze gegeben, 20 min in DMF quellen gelassen und dann mit einer Lösung von DMF/Piperidin (1:1) 20 min lang behandelt. Nach Waschen mit DMF, DCM, und noch mal DMF wurde das Harz so im nächsten Syntheseschritt verwendet.
Verfahrensschritt a)
   Zu einer Lösung von Fmoc-homoPheOH (0.71 g, 3.84 mmol) und HOBt-Hydrat (0.59g, 3.84 mmol) in DMF wurde DIC (0.59mL, 3.84 mmol) gegeben. Die entstandene Lösung wurde in die obengenannte Spritze eingezogen und die Mischung 16 Stunden (h) bei RT geschüttelt. Das Harz wurde mit DMF (10x15mL), DCM (4x15mL) und DMF (2x15mL) gewaschen, und dann bei 4°C aufbewahrt. Zur Reaktionskontrolle wurde ein KAISER-Test an einigen Harzkügelchen durchgeführt.
Verfahrensschritt b)
   Das Harz wurde, wie oben beschrieben , entschützt und gewaschen. Zu einer Lösung von 4-Fluor-3-nitro-benzoesäure (0,71g, 3,84 mmol) und HOBt Hydrat (0,59g, 3,84 mmol) in DMF (etwa 15 mL) wurde DIC (0,59mL, 3,84 mmol) gegeben. Diese Lösung wurde in die Spritze mit dem vorbereiteten Harz eingezogen und die Mischung 16 h bei RT geschüttelt. Das Harz wurde mit DMF (10x15mL) gewaschen, und bei 4°C aufbewahrt. Zur Reaktionskontrolle wurde ein KAISER-Test an einigen Harzkügelchen durchgeführt.
Verfahrensschritt c)
   Eine Lösung von 4-(Aminomethyl)pyridin (1,4ml, 12,8mmol) in DMF (10mL) wurde zu dem vorbereiteten Harz gegeben und die Mischung 2 Tage bei RT schütteln gelassen. Das Harz wurde mit DMF (8x15mL), DCM (4x15mL) und DMF (2x15 mL) gewaschen. Anmerkung: Es wurde später gefunden, daß einfaches Erhitzen der Harzmischung in DMA (Dimethylacetamid, anstelle von DMF) für 16 h das gewünschte Hydroxybenzimidazol ergab; so konnte die Synthese beschleunigt werden.
Verfahrensschritt d)
   Eine Lösung des Harzes in DMA wurde in einen verschließbaren Glasreaktor gefüllt und die Reaktionsmischung 16 h lang bei leichtem Bewegen auf 125°C erhitzt. Die erfolgte Cyclisierung konnte durch GC/MS (nach Abspaltung eines Aliquots der Substanz vom Harz) bestätigt werden. Nach Waschen mit DMA(5x15mL) wurde das Harz so im Syntheseschritt e) verwendet.
Verfahrensschritt e)
   Zu einer Lösung des Harzes (0,5g) aus Verfahrensschritt d) in DMA (5,0 mL) wurde Tributylphosphin (0,6 mL) gegeben und die Mischung 6 h lang bei 150 °C leicht rühren gelassen. Das Harz wurde dann mit DMF(20x10mL), MeOH (10x10mL) und DCM (10x10mL) gewaschen.
Verfahrensschritt f) Abspaltung und Aufreinigung
   Das in Verfahrensschritt e) erhaltene Harz wurde mit TFA/H₂O (95/5) 3 h lang bei RT behandelt. TFA/H₂O wurde unter vermindertem Druck entfernt und ein brauner glasiger Feststoff wurde als Rohprodukt erhalten. Das Rohprodukt wurde an Kieselgel chromatographiert (Flash-Chromatographie; Laufmittel: 95/5 DCM/2,0M NH₃ in MeOH, dann 92/8 DCM/2.0M NH₃ in MeOH. Die gewünschten Fraktionen wurden gesammelt und die Lösungsmittel unter vermindertem Druck entfernt. Das Produkt wurde als weißer Feststoff erhalten. MS (ES; M+H⁺ = 400) 1H-NMR entsprach der obengenannten Strukturformel.

### Beispiel 190

Die nachfolgende Verbindung wurde nach Verfahrensvariante c) hergestellt: 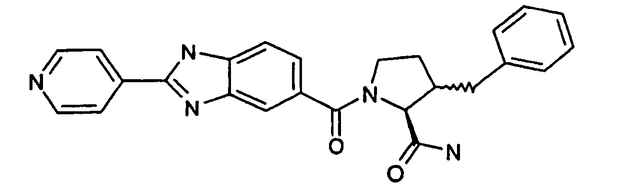
A) Synthese von 3(R/S)-Vinyl-2(S)-azido-3-phenylpropionsäure
   a) Zu einer Lösung von 3-Vinyl-4-phenylbuttersäureethylester (0,129 Mol) in wäßrigem THF (120 mL/ 20mL H₂O) wurde Lithiumhydroxid (Monohydrat, 21 g; 645 mmol) gegeben. Die Reaktionsmischung wurde über Nacht gerührt und anschließend unter vermindertem Druck konzentriert. Der Rückstand wurde zwischen AcOEt und wäßriger HCl (1M) verteilt, die Phasen getrennt, und die wäßrige Phase 2 mal mit AcOEt gewaschen. Die vereinigten organischen Phasen wurden mit gesättigter Kochsalzlösung gewaschen, über MgSO₄ getrocknet, filtriert und unter vermindertem Druck evaporiert. Es wurden 15,6 g (Ausbeute 62%) Säure erhalten, die so in der folgenden Reaktion eingesetzt wurde.
   b) Zu einer gekühlten (-78 °C) Lösung der oben genannte Säure (1,74 g, 9,16 mmol) in wasserfreiem THF (10 mL) wurde Triethylamin (1.27 mL) und 15 Minuten später Pivaloylchlorid (1,18 mL) gegeben. Die Mischung wurde 1 h bei 0° C gerührt und auf -78° C abgekühlt. In einem separaten Kolben wurde zu einer gekühlten (-78 °C) Lösung von S-Phenyl-oxazolidinon (1,64 g) in THF (36 mL) n-Butyl-Lithium (5,7 mL, 1,6 M in Hexan) langsam zugegeben. Die Lösung wurde 1h bei -78 °C gerührt, auf 0 °C erwärmt und via Cannula zu der obigen Lösung zugetropft. Nach beendeter Zugabe wurde die Lösung über Nacht bei Raumtemperatur gerührt. Gesättigte NH₄Cl Lösung (20 mL) wurde zugegeben und die Lösung unter vermindertem Druck auf 1/3 des Volumens reduziert. Die wäßrige Lösung wurde 3 mal mit Dichlormethan extrahiert, die kombinierten organischen Phasen mit wäßriger Natronlauge gewaschen, über MgSO4 getrocknet, filtriert und evaporiert. Der Rückstand wurde mittels Flash-Chromatographie (Kieselgel, 5-20% AcOEt/ Hexan) gereingt. Es wurden 2,06 g (67% Ausbeute) Imid als Gemisch der beiden Diastereomeren erhalten.
   c) Zu einer gekühlten (-78 °C) Lösung des Imids (1,88 g, 5,61 mmol) in wasserfreiem THF (15 mL) wurde 1,1,1,3,3,3-Hexamethyldisilazan Kaliumsalz (14,6 mL, 0,5molar Lösung in Toluol) tropfenweise zugegeben. Nach 40 min. wurde eine gekühlte (-78 °C) Lösung von Trimethylsilyl azid (2,51 g) in THF zugegeben. Nach weiteren 35 min. wurde Essigsäure (1,47 mL) zugegeben und die Lösung bei Raumtemperatur über Nacht rühren gelassen Die Reaktionsmischung wurde zwischen Dichlormethan und gesättigter Kochsalzlösung verteilt, die organische Phase über MgSO₄ getrocknet, filtriert und evaporiert. Der Rückstand wurde über Kieselgel Flash-chromatographiert (Eluentien: a) DCM/MeOH/wäßr. Ammoniak = 95/5/1; b) DCM; Anfangsverhältnis a: b = 1: 10, bis reines DCM). Es wurden 2.5 g (95% Ausbeute) Azidoprodukt erhalten.
   d) Zu einer gekühlten Lösung (0 °C) der obigen Azidoverbindung (2,5 g) in THF (20 mL), H₂O (4 mL) und Lithiumhydroxid Mononohydrat (558 mg) wurde 3 mL Wasserstoffperoxid (30%) gegeben. Die Mischung wurde 2 h bei Raumtemperatur gerührt und dann wurden 19 mL einer 10%igen Lösung Natriumsulfat zugegeben. Die Lösung wurde unter vermindertem Druck auf 1/10 des ursprünglichen Volumens reduziert, der erhaltene Rückstand 3 mal mit Ethylacetat extrahiert, die vereinigten organischen Phasen über MgSO₄ getrocknet, filtriert und unter vermindertem Druck bis zur Trockne eingeengt. Der Rückstand wurde über Kieselgel aufgereinigt (Flash-Chromatographie, Eluentien: 1-5% MeOH in 1 %iger wäßriger Ammoniaklösung / 99% DCM). Es wurden 912 mg der gewünschten Säure (74% Ausbeute) erhalten.
B) Synthese von 2-Pyridin-4yl-1H-benzoimidazole-5-carbonyl-3-R,S-benzyl-S-prolinamid 0,4 g von Polystyrol-Knorr Harz (0,61 mmol/g) - Harz wurde in eine Plastikspritze gegeben, 20 min in DMF quellen gelassen und dann mit einer Lösung von DMF/Piperidin (1:1) 20 min lang behandelt. Nach Waschen mit DMF, DCM, und noch mal DMF wurde das Harz so im nächsten Syntheseschritt verwendet.
   Verfahrensschritt a)
      Zu dem obigen Harz wurde eine Lösung der 3(R/S)-Vinyl-2(S)-azido-3-phenylpropionsäure (siehe auch Schritte a) - d); 0,28 mmol), PyBOP (0,28 mmol) und DIPEA (0,32 mmol) gegeben. Die entstandene Mischung wurde 16 h bei RT geschüttelt. Das Harz wurde mit MeOH (3 mal 15 mL) und DCM (4 mal 15 mL) gewaschen und unter vermindertem Druck getrocknet. Zur Reaktionskontrolle wurde ein KAISER-Test an einigen Harzkügelchen durchgeführt.
   Verfahrensschritt b)
      Cyclohexen (23 mmol) wurde zu einer 2 M Lösung von Dicyclohexylboran/Dimethylsulfid Komplex (11,6 mmol) in wasserfreiem Diethylether unter einer Inertgasatmosphäre gegeben. Nach einer Stunde war ein weißer Feststoff gebildet. Das Lösungsmittel wurde unter vermindertem Druck entfernt und obiges Harz und 10 mL DCM dazugegeben. Die heterogene Mischung wurde 2,5 h leicht gerührt bis die Gasentwicklung beendet war. Das Harz wurde mit MeOH gewaschen und unter vermindertem Druck getrocknet. Anschließend wurde es mit einer 50/50 (Vol/Vol) Mischung aus Ethanolamin und DMF 1 h lang gerührt und dann mit MeOH und DCM (je 3 mal) gewaschen, anschließend getrocknet.
   Verfahrensschritt c)
      Zu einer Lösung von 4-Fluor-3-nitro-benzoesäure (0,69 mmol) und HOAt (0,69 mmol) in DMF (etwa 5 mL) wurde DIC (0,69 mmol) gegeben. Diese Lösung wurde in die Spritze mit dem vorbereiteten Harz eingezogen und die Mischung wurde 16 h bei Raumtemperatur (RT) geschüttelt. Das Harz wurde mit DMF (10x15mL) gewaschen und unter Vakuum getrocknet. Zur Reaktionskontrolle wurde ein KAISER-Test an einigen Harzkügelchen durchgeführt.
   Verfahrensschritt d)
      Eine Lösung von 4-(Aminomethyl)pyridin (4,6 mmol) in DMF (4 mL) wurde zu dem vorbereiteten Harz gegeben und die Mischung wurde 32 Tage bei RT geschüttelt. Das Harz wurde mit MeOH und DCM (je 3x15mL) gewaschen und getrocknet.
   Verfahrensschritt e)
      Eine Lösung des Harzes in DMA wurde in einen verschließbaren Glasreaktor gefüllt und die Reaktionsmischung wurde 16 h bei leichtem Bewegen auf 125 °C erhitzt. Die erfolgte Cyclisierung konnte durch GC/MS (nach Abspaltung eines Aliquots der Substanz vom Harz) bestätigt werden. Das Harz wurde mit MeOH und DCM (je 3x15mL) gewaschen und getrocknet.
   Verfahrensschritt f)
      Zu einer Lösung des Harzes (0,0.21 mmol) aus Verfahrensschritt e) in DMA (3,0 mL) wurde Tributylphosphin (0,5 mL) gegeben und die Mischung 5 h lang bei 125 °C leicht rühren gelassen. Das Harz wurde dann mit DMF(2 mal 10mL), MeOH (2 mal 10mL) und DCM (3 mal 10mL) gewaschen und unter vermindertem Druck getrocknet.
   Verfahrensschritt g) Abspaltung und Reinigung
      Das in Verfahrensschritt f) erhaltene Harz wurde mit TFA/H₂O (97/3) 1 h lang bei RT behandelt. TFA/H₂O wurde unter vermindertem Druck entfernt und ein brauner glasiger Feststoff wurde als Rohprodukt erhalten. Das Rohprodukt wurde an Kieselgel chromatographiert (Flash-Chromatographie; Laufmittel: 95/5 DCM/2,OM NH₃ in MeOH, dann 92/8 DCM/2.0M NH₃ in MeOH. Die gewünschten Fraktionen wurden gesammelt und die Lösungsmittel unter vermindertem Druck entfemt. Das Produkt wurde als weißer Feststoff erhalten.
      MS (ES; M+H⁺ = 426) 1H-NMR entsprach der obengenannten Strukturformel

### Beispiel 191

Die nachfolgende Verbindung wurde nach Verfahrensvariante c) hergestellt:

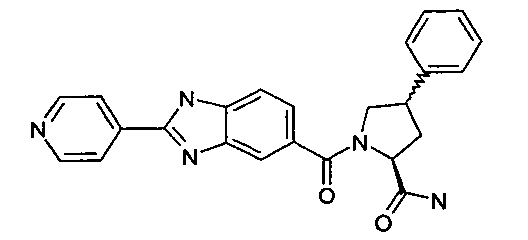

1,5 g von Polystyrol-Knorr Harz (0,64 mmol/g) wurde in eine Plastikspritze gegeben, 20 min. in DMF quellen gelassen und dann mit einer Lösung von DMF/Piperidin (1:1) 20 min. lang behandelt. Nach Waschen mit DMF, DCM, und noch mal DMF wurde das Harz so im nächsten Syntheseschritt verwendet.
Verfahrensschritt a)
   Eine Lösung von Fmoc-3R,S-Phenyl-S-Prolin (1,5 mmol), PyBOP (1,5 mmol) und DIPEA (2,1 mmol) in DCM wurde zu dem Harz gegeben. Die entstandene Mischung wurde 16 Stunden (h) bei RT geschüttelt. Das Harz wurde mit DCM (4 mal 15mL), MeOH (2 mal 15mL) und DCM gewaschen und unter vermindertem Druck getrocknet. Zur Reaktionskontrolle wurde ein KAISER-Test an einigen Harzkügelchen durchgeführt.
Verfahrensschritt b)
   Das Harz wurde, wie in Beispiel 190 unter Verfahren B beschrieben, zur gewünschten Verbindung weiter umgesetzt.
   MS (ES; M+H⁺ = 412) 1H-NMR entsprach der obengenannten Strukturformel.

### Beispiel 192:

Die nachfolgende Verbindung wurde nach Verfahrensvariante a) hergestellt: 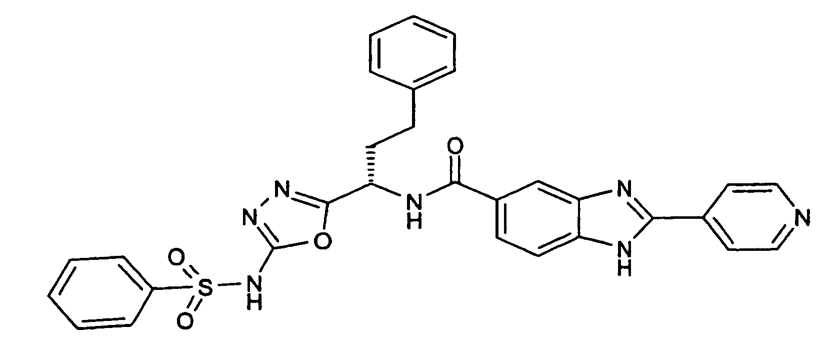
a) Herstellung von Z-Homophenylalaninhydrazid:
   Z-Homophenylalaninhydrazid wurde wie in Beispiel 180a beschrieben hergestellt.
b) Herstellung von [1-(5-Amino-[1,3,4]oxadiazol-2-yl)-3-phenyl-propyl] carbaminsäure-benzylester:
   [1-(5-Amino-(1,3,4joxadiazol-2-yl)-3-phenyl-propyl] carbaminsäure-benzylester wurde wie in Beispiel 180b beschrieben hergestellt.
c) Herstellung von [1-(5-Benzolsulfonylamino-[1,3,4]oxadiazol-2-yl)-3-phenylpropyl]-carbaminsäure-benzylester:
   0,35 g der Verbindung gemäß Beispiel 192b wurden bei Raumtemperatur in 5 ml Pyridin gelöst, mit 0,13 ml Benzolsulfonylchlorid versetzt und 4 h bei 80°C gerührt. Man fügte nochmals 0,13 ml Benzolsulfonylchlorid zu und rührte weitere 2 h bei 80°C. Die Reaktionsmischung wurde im Vakuum eingeengt, und der Rückstand wurde in Essigsäureethylester aufgenommen, zweimal mit Wasser und einmal mit gesättigter Kochsalzlösung gewaschen, über Magnesiumsulfat getrocknet, filtriert und im Vakuum eingeengt. Man erhielt [1-(5-Benzolsulfonylamino-[1,3,4]oxadiazol-2-yl)-3-phenyl-propyl]carbaminsäure-benzylester, welcher ohne weitere Reinigung weiter umgesetzt wurde.
d) Herstellung von N-[5-(1-Amino-3-phenyl-propyl)-[1,3,4]oxadiazol-2-yl]-benzolsulfonamid:
   0,18 g der Verbindung 192c wurden bei Raumtemperatur in 30 ml trockenem Methanol gelöst, unter Argon mit Pd/C-Katalysator versetzt und 4h bei Raumtemperatur hydriert. Nach Filtration, Waschen des Rückstandes mit Methanol und Einengen des Filtrates erhielt man N-[5-(1-Amino-3-phenyl-propyl)-[1,3,4]oxadiazol-2-yl]-benzolsulfonamid, welches in die nächste Stufe eingesetzt wurde.
e) Herstellung von 2-Pyridin-4-yl-1H-benzimidazole-5-carbonsäure[1-(5-benzolsulfonylamino-[1,3,4]oxadiazol-2-yl)-3-phenyl-propyl]-amid:
   70 mg der Verbindung gemäß Beispiel 192d wurden bei Raumtemperatur in 5 ml trockenem DMF gelöst, mit 30µl Diisopropylamin, 48 mg 2-Pyridin-4-yl-1H-benzimidazol-5-carbonsäure und 66 mg TOTU versetzt. Nach 4 h Rühren bei Raumtemperatur war die Reaktion beendet und die Reaktionsmischung wurde eingeengt. Der Rückstand wurde mit Essigsäureethylester und Wasser behandelt. Die Lösemittel wurden dekantiert; der ölige Rückstand wurde mit warmem Aceton behandelt, abgekühlt und das kristalline Produkt wurde auf einem Filter gesammelt, mit Aceton gewaschen und getrocknet. Man erhielt 2-Pyridin-4-yl-1H-benzimidazole-5-carbonsäure [1-(5-benzolsulfonylamino-[1,3,4]oxadiazol-2-yl)-3-phenyl-propyl]-amid, welches ab 220°C unter Zersetzung schmolz.

### Beispiel 193:

Die nachfolgende Verbindung wurde nach Verfahrensvariante a) hergestellt: 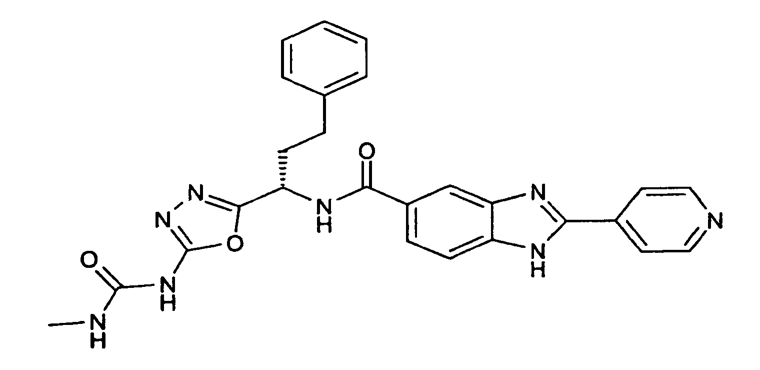
a) Herstellung von Z-Homophenylalaninhydrazid:
   Z-Homophenylalaninhydrazid wurde wie in Beispiel 180a beschrieben hergestellt.
b) Herstellung von [1-(5-Amino-[1,3,4]oxadiazol-2-yl)-3-phenyl-propyl] carbaminsäure-benzylester:
   [1-(5-Amino-[1,3,4]oxadiazol-2-yl)-3-phenyl-propyl] carbaminsäure-benzylester wurde wie in Beispiel 180b beschrieben hergestellt.
c) Herstellung von {1-[5-(3-Methyl-ureido)-[1,3,4]oxadiazol-2-yl]-3-phenylpropyl}-carbaminsäure benzylester:
   350 mg [1-(5-Amino-[1,3,4]oxadiazol-2-yl)-3-phenyl-propyl] carbaminsäure-benzylester wurden in 5 ml trockenem Dimethylsulfoxid gelöst, mit 140 mg Kaliumcarbonat und 140 mg Methylisocyanat versetzt und 16 h bei 80°C gerührt. Die Reaktionsmischung wurde abgekühlt, mit Essigsäureethylester versetzt, zweimal mit Wasser und einmal mit gesättigter Kochsalzlösung gewaschen, über Magnesiumsulfat getrocknet, filtriert und im Vakuum eingeengt. Man erhielt {1-[5-(3-Methyl-ureido)-[1,3,4]oxadiazol-2-yl]-3-phenylpropyl}-carbaminsäure benzylester, welcher ohne weitere Reinigung in die nächste Stufe (Hydrogenolyse) eingesetzt wurde .
d) Herstellung von 1-[5-(1-Amino-3-phenyl-propyl)-[1,3,4]oxadiazol-2-yl]-3-methyl-harnstoff
   120 mg der vorherigen Verbindung wurden bei RT in 20 ml trockenem Methanol gelöst, unter Argon mit Pd/C-Katalysator versetzt und 4 h bei Raumtemperatur hydriert. Die Reaktionsmischung wurde filtriert, der Rückstand mit Methanol gewaschen und das Filtrat wurde im Vakuum eingeengt. Der 1-[5-(1-Amino-3-phenyl-propyl)-[1,3,4]oxadiazol-2-yl]-3-methyl-harnstoff wurde ohne weitere Reinigung in die nächste Stufe eingesetzt.
e) Herstellung von 2-Pyridin-4-yl-1H-benzimidazol-5-carbonsäure {1-(5-(3-methylureido)-[1,3,4]oxadiazol-2-yl]-3-phenyl-propyl}-amid:
   40 mg der vorherigen Verbindung wurden bei RT in 3 ml trockenem DMF gelöst und nacheinander mit 33 mg 2-Pyridin-4-yl-1H-benzimidazol-5-carbonsäure, 20 µl Diisopropylamin und 40 mg TOTU versetzt und 4 h bei Raumtemperatur gerührt. Die Reaktionsmischung wurde im Vakuum eingeengt; der Rückstand wurde in Essigsäureethylester/Tetrahydrofuran 1/1 aufgenommen, mit Wasser, gesättigter Natriumhydrogencarbonatlösung und gesättigter Kochsalzlösung gewaschen, über Magnesiumsulfat getrocknet, filtriert und im Vakuum eingeengt. Man erhielt 2-Pyridin-4-yl-1H-benzimidazol-5-carbonsäure {1-[5-(3-methyl-ureido)-[1,3,4]oxadiazol-2-yl]-3-phenylpropyl}-amid, welches im Massenspektrum m/z = 497.3 (=MH⁺) aufweist.

### Beispiel 194:

Die nachfolgende Verbindung wurde nach Verfahrensvariante a) hergestellt:

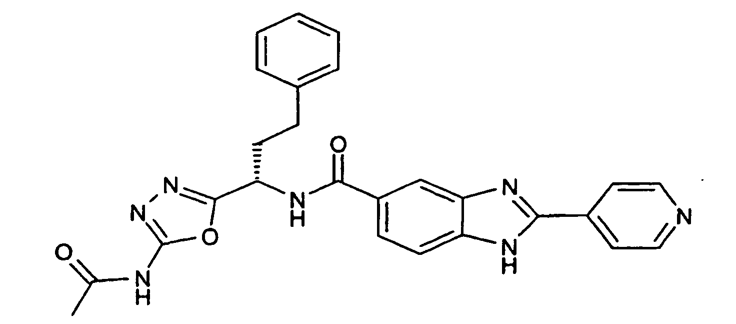

2-Pyridin-4-yl-1H-benzimidazol-5-carbonsäure [1-(5-acetylamino-[1,3,4]oxadiazol-2-yl)-3-phenyl-propyl]-amid wurde in prinzipiell analoger Weise, aber mit dem Unterschied, dass statt mit Methylisocyanat mit Acetylchlorid umgesetzt wurde, hergestellt. Die Verbindung weist einen Schmelzpunkt von 183-186°C unter Zersetzung auf.

### Beispiel 195:

Die nachfolgende Verbindung wurde nach Verfahrensvariante a) hergestellt: 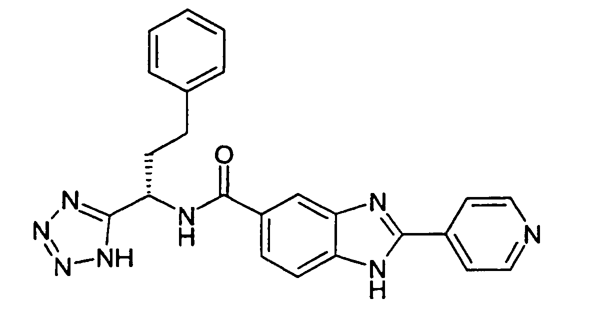
a) Herstellung von (1-Cyano-3-phenyl-propyl)-carbaminsäure benzylester:
   2,78 g (1-Carbamoyl-3-phenyl-propyl)-carbaminsäure benzylester, hergestellt aus L-Homophenylalaninamid Hydrochlorid und N-Cbz-succinimid, wurden in 30 ml trockenem Pyridin gelöst und mit 6 ml Acetanhydrid versetzt. Die Reaktionsmischung wurde 24 h bei 75°C gerührt. Die abgekühlte Lösung wurde im Vakuum eingeengt, mit 100 ml Essigsäureethylester versetzt und danach je dreimal mit 50 ml 5%iger Zitronensäurelösung und gesättigter Kochsalzlösung ausgeschüttelt. Der organische Extrakt wurde über Magnesiumsulfat getrocknet, filtriert, im Vakuum eingeengt und über Kieselgel mit n-Heptan/Essigsäureethylester 1/1 chromatographiert. Man erhielt (1-Cyano-3-phenyl-propyl)-carbaminsäure benzylester, welcher ohne weitere Reinigung in die nächste Stufe eingesetzt wurde.
b) Herstellung von [3-Phenyl-1-(1H-tetrazol-5-yl)-propyl]-carbaminsäure benzyl ester:
   0,15 g der Verbindung des Beispiels 195a wurden mit 0,115 g Trimethylzinnazid in 5 ml trockenem Toluol suspendiert und 6 h unter Rückfluß gerührt. Die abgekühlte Reaktionsmischung wurde mit etherischer Salzsäure angesäuert und über Nacht im Kühlschrank stehen gelassen. Andemtags wurde die Mischung im Vakuum eingeengt und über Kieselgel mit Dichlormethan/Methanol 9/1 chromatographiert. Der so erhaltene [3-Phenyl-1-(1H-tetrazol-5-yl)-propyl]-carbaminsäure benzylester wurde ohne weitere Reinigung in die nächste Stufe eingesetzt.
c) Herstellung von 3-Phenyl-1-(1H-tetrazol-5-yl)-propylamin:
   337 mg der Verbindung des Beispiels 195b wurden in 2 ml Acetonitril gelöst, mit 0,477 ml Triethylsilan, einem Tropfen Triethylamin und einer Spatelspitze Palladium-II-chlorid versetzt und 3 h unter Rückfluß gerührt. Die abgekühlte Lösung wurde filtriert, im Vakuum eingeengt und der Rückstang im Hochvakuum getrocknet. Das so gewonnene 3-Phenyl-1-(1H-tetrazol-5-yl)-propylamin wurde weiter umgesetzt.
d) Herstellung von 2-Pyridin-4-yl-1H-benzimidazol-5-carbonsäure [3-phenyl-1-(1H-tetrazol-5-yl)- propyl]-amid:
   0,9 mmol der Verbindung des Beispiels 195c wurden in 5 ml trockenem DMF gelöst und mit 0,9 mmol 2-Pyridin-4-yl-1H-benzimidazol-5-carbonsäure, 0,365 ml Diisopropylamin und 415 mg TOTU versetzt, 20 h bei Raumtemperatur und weitere 4 h bei 50°C gerührt. Die Reaktionsmischung wurde im Vakuum eingeengt; der Rückstand wurde in Essigsäure-ethylester aufgenommen, mit Wasser, gesättigter Natriumhydrogencarbonatlösung und gesättigter Kochsalzlösung gewaschen, über Magnesiumsulfat getrocknet, filtriert und im Vakuum eingeengt. Das so erhaltene Rohprodukt wurde über Kieselgel mit Dichlormethan/Methanol/Wasser/Eisessig 60/10/1/1 chromatographiert. Man erhielt 2-Pyridin-4-yl-1H-benzimidazol-5-carbonsäure [3-phenyl-1-(1H-tetrazol-5-yl)-propyl]-amid, welches sich ab 87°C zersetzte und einen Molpeak bei m/z=425,2 (MH⁺) aufwies.

### Beispiel 196 3-Phenylaminoethyl-2-[(2-pyridin-4-yl-1H-benzoimidazol-5-carbonyl)-amino]-propionsäure Trifluoracetat

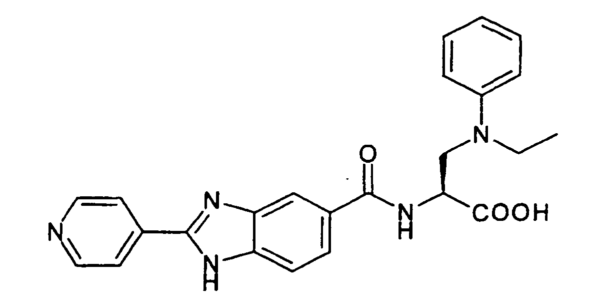

a) L-2-Amino-3-phenylaminoethylpropionsäure
   54,8 g (0,209 mol) Triphenylphosphin wurden in 600 ml Acetonitril suspendiert und unter Ausschluss von Feuchtigkeit auf -35 °C bis -45 °C gekühlt. Anschließend wurde bei dieser Temperatur innerhalb von 50 min tropfenweise 36,4 g (0,209 mol) Azodicarbonsäurediethylester hinzugegeben. Man rührte 15 min bei -35 °C nach. Zu diesem Gemisch tropfte man eine Lösung aus 50 g (0,209 mol) N-Benzyloxycarbonyl-L-serin in 500 ml Acetonitril, dabei ließ man die Temperatur nicht über -35 °C steigen. Anschließend ließ man 12 h bei 5°C nachreagieren und erwärmte auf RT. Die Reaktionslösung wurde unter vermindertem Druck vom Lösungsmittel befreit und das Rohprodukt mit Mitteldruckchromatographie an Kieselgel gereinigt. (DCM/AcCN : 25/1) Nach Entfernen des Lösungsmittels erhielt man 20,8 g (Ausbeute 45%) sauberes N-Benzyloxy-carbonyl-L-serin-β-lacton (siehe auch Org. Synth. 1991 (70) 1ff.) in feinen Nadeln. Summenformel: C₁₁H₁₁NO₄; M.W. = 221,2 : MS (M+H) 222,1
   Zu 7,3 ml (57,36 mmol) N-Ethylanilin in 250 ml Acetonitril wurden unter Argon Schutzatmosphäre 15,5 ml (63,51 mmol) N,O-Bis-(Trimethylsilyl)acetamid gegeben und 3 h bei 50 °C gerührt. Anschließend wurde bei 20 °C eine Lösung des obigen Lactons (10,7 g, 48,37 mmol) gelöst in 250 ml Acetonitril zugegeben und 17 h unter Rückfluss erhitzt. Nach Entfernung des Lösungsmittels wurde der Rückstand mit gesättigter Natriumcarbonat Lösung versetzt wobei der pH-Wert der Lösung 9 nicht überschritt. Die wässrige Suspension wurde mit wenig Diethylether gewaschen und anschließend mit konzentrierter Salzäure auf einen pH-Wert von 6 bis 7 eingestellt und mit NaHPO₄-Puffer auf einen pH-Wert von 5 eingestellt. Die wässrige Lösung wurde mehrmals mit Essigester extrahiert. Nach Evaporierung der Lösungsmittel erhielt man das gewünschte Produkt in einer Ausbeute von 45% (7,4 g). Summenformel: C₁₉H₂₂N₂O₄ ; M.W. = 342,4; MS (M+H) 343,2

Zu 75 ml Methanol wurden bei -10°C 6,5 ml (89,1 mmol) Thionylchlorid getropft und 30 min gerührt. Danach wurde in 75 ml Methanol gelöste L-2-Aminoethyl-3-phenylaminopropionsäure 8,6 g (25,12 mmol) zugegeben, 30 Minuten bei -10 °C und weitere 3 h bei RT gerührt. Nach Verdampfen des Lösungsmittel wurde der Rückstand in Essigester aufgenommen und mit Natriumcarbonat Lösung gewaschen. Nach Verdampfen des Lösungsmittels und Reinigung mittels Flash-Chromatographie (n-Heptan/ Essigester 7:3) wurde 4,43 g (50% Ausbeute) von L-2-Aminoethyl-3-phenylaminopropionsäuremethylester erhalten. Summenformel: C₂₀H₂₄N₂O₄ ; M.W. = 356,4 ; MS (M+H) 357,3

Zur Abspaltung der Schutzgruppe löste man 4,4 g (12,35 mmoi) des Z-geschützte Derivates in 500 ml Methanol, fügte unter Inertgas 100 mg Katalysator (10% Pd(OH)₂-C) zu, und leitete bis zur vollständigen Abspaltung der Z-Schutzgruppe Wasserstoff ein. Nach Abfiltrieren des Katalysators und Eindampfen des Filtrates erhielt man 2,8 g L-2-Aminoethyl-3-phenylamino-propionsäure (quant.). Summenformel: C₁₂H₁₈N₂O₂: M.W. = 222,3; MS (M+H) 223,1
Verfahrensschritt b)
   2,4 g (10,03 mmol) 2-Pyridin-4-yl-1H-benzoimidazol-5-carbonsäure hergestellt wie in Beispiel 183 wurden in 350 ml DMF suspendiert und nacheinander mit 4,2 g (12,80 mmol) TOTU und 2,3 ml (13,52 mmol) Ethyldiisopropylamin versetzt. Man rührte 10 min bei RT und gab zu der entstandenen klaren Lösung 2,8 g (12,60 mmol) (S) 2-Amino-3-phenylaminethylopropionsäuremethylester hergestellt gemäß a) hinzu. Nach 2 h Rühren engte man unter vermindertem Druck ein und isolierte den Methylester der Titelverbindung durch Flash-Chromatographie an Kieselgel (DCM:MeOH= 9:1). Ausbeute: 4,36 g (98%), Summenformel: C₂₅H₂₅N₅O₃; M.W. = 443,5.; MS (M+H) 444,3
   4,3 g (9,7 mmol) des so erhaltenen Methylesters wurde in 400 ml Methanol durch die Zugabe von 200 ml 1M Natromlauge bei RT 2 h hydrolysiert. Nach Verdampfen des Methanols wurde die erhaltene Suspension mit NaH₂PO₄- Lösung auf pH = 5 gestellt. Dabei fällt das Produkt aus der Lösung aus. Reinigung mittels Flash-Chromatographie an Kieselgel (DCM:MeOH= 4:1) und preparative HPLC (Acetonitril/0,1% TFA) lieferte 2,92 g (Ausbeute 70%) von 3-Phenylaminoethyl-2-[(2-pyridin-4-yl-1H-benzoimidazol-5-carbonyl)-amino]-propionsäure Trifluoracetat.
   Summenformel: C₂₄H₂₃N₅O₃;M.W. = 429,5 ; MS (M+H) 430,3; Schmelzpunkt: etwa 258°C (unter Zersetzung).

### Pharmakologische Beispiele

### IκB-Kinase Filtertest:

Die Aktivität der IκB-Kinase wurde mit dem "SignaTECT™ Protein Kinase Assay System" (Promega Katalog 1998, S.330; analog zur SignaTECT™ DNA-Dependent Protein Kinase Vorschrift) bestimmt. Die Kinase wurde gemäß Z. J. Chen (Cell 1996, Vol. 84, S 853-862) aus HeLa-Zellextrakten gereinigt und mit dem Substratpeptid (Biotin-(CH₂)₆-DRHDSGLDSMKD-CONH₂) (20 µM) inkubiert. Der Reaktionspuffer enthielt 50 mM HEPES, pH 7,5, 10 mM MgCl₂, 5 mM Dithiothreitol (DTT), 10 mM β-Glycerophosphat, 10 mM 4-Nitrophenylphosphat, 1 µM Microcystin-LR und 50 µM ATP (enthaltend 1 µCi γ-³³P-ATP).
Methode PKA, PKC, CK II
cAMP-abhängige Proteinkinase (PKA), Proteinkinase C (PKC) und Caseinkinase II (CK II) wurden mit den entsprechenden Testkits von Upstate Biotechnology gemäß der Vorschrift des Hersteller bei einer ATP-Konzentration von 50 µM bestimmt. Abweichend wurden keine Phosphocellulosefilter, sondern MultiScreen-Platten (Millipore; Phosphocellulose MS-PH, Kat. MAPHNOB10) mit dem entsprechenden Absaugsystem verwendet. Die Platten oder Membranen (IκB-Kinase) wurden anschließend in einem Wallac MicroBeta Szintillationszähler vermessen. Es wurde jeweils 100 µM der Testsubstanz eingesetzt.

Jede Substanz wurde in Doppelbestimmung getestet. Von den Mittelwerten (Enzym mit und ohne Substanzen) wird der Mittelwert des Blanks (ohne Enzym) subtrahiert und die % Inhibition errechnet. IC₅₀-Berechnungen wurden mit dem Softwarepaket GraFit 3.0 durchgeführt. Die nachfolgende Tabelle 6 zeigt die Ergebnisse.

**Tabelle 6:**

| Kinaseinhibition bei einer Substanzkonzentration von 100 µM oder IC₅₀ in µM | | | | |
|---|---|---|---|---|
| Beispiel Nummer | IκB-Kinase IC₅₀ | PKA %-Hemmung | PKC %-Hemmung | CK II %-Hemmung |
| 5 | 25 | 0 | 16 | 19 |
| 6 | 72 | 0 | 46 | 14 |
| 7 | 22 | 0 | 15 | 10 |
| 8 | 4 | 0 | 9 | 7 |
| 11 | 7 | 0 | 16 | 0 |
| 12 | 42 | 0 | 16 | 0 |
| 14 | 7 | 0 | 0 | 0 |
| 16 | 9 | 0 | 11 | 4 |
| 20 | 1 | 36 | 63 | 70 |
| 21 | 34 | 26 | 31 | 60 |
| 35 | 16 | n.b. | n.b. | n.b. |
| 37 | 36 | n.b. | n.b. | n.b. |
| 108 | 1 | 0 | 13 | 92 |
| 113 | 25 | 24% | 7% | 17% |
| 180 | 0,43 | n.b. | n.b. | n.b. |
| 181 | 0,62 | n.b. | n.b. | n.b. |
| 192 | 0,12 | n.b. | n.b. | n.b. |
| 193 | 0,36 | n.b. | n.b. | n.b. |
| 196 | 0,07 | 31 | 40 | 50 |
| n.b. bedeutet nicht bestimmt | | | | |

## Patentansprüche

1. Verbindung der Formel I 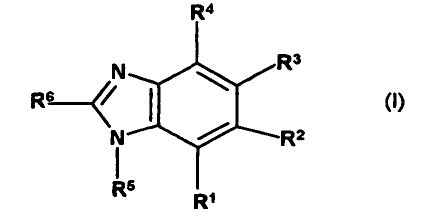 und/oder eine stereoisomere Form der Verbindung der Formel I und/oder ein physiologisch verträgliches Salz der Verbindung der Formel I, wobei
einer der Substituenten R¹, R², R³ und R⁴ für einen Rest der Formel II steht, 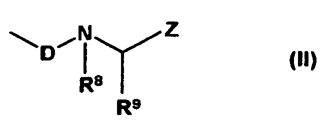 worin
D für -C(O)-, -S(O)- oder -S(O)₂- steht,
R⁸ für Wasserstoffatom oder (C₁-C₄)-Alkyl steht,
R⁹ für
1. charakteristischen Rest einer Aminosäure, der sich von einer natürlich vorkommenden α-Aminosäure aus der Gruppe Alanin, Valin, Leucin, lsoleucin, Phenylalanin, Tyrosin, Tryptophan, Serin, Threonin, Cystein, Methionin, Asparagin, Glutamin, Lysin, Histidin, Arginin und Glutaminsäure ableitet,
2. charakteristischen Rest einer Aminosäure, der sich von einer nicht natürlich vorkommenden Aminosäure aus der Gruppe 2-Aminoadipinsäure, 2-Aminobuttersäure, 2-Aminoisobuttersäure , 2,3-Diamino-propionsäure, 2,4-Diaminobuttersäure,1,2,3,4-Tetra-hydroisochinolin-1-carbonsäure,1,2,3,4-Tetrahydroisochinolin-3-carbonsäure, 2-Amino-pimelinsäure, Phenylglycin, 3-(2-Thienyl)-alanin, 3-(3-Thienyl)-alanin, Sarkosin, 2-(2-Thienyl)-glycin, 2-Amino-heptansäure, Pipecolinsäure, Hydroxylysin, N-Methylisoleucin, 6-N-Methyllysin, N-Methylvalin, Norvalin, Norleucin, ornithin, allo-Isoleucin, 4-Hydroxy-prolin, allo-Hydroxylysin, allo-Threonin, 3-Hydroxyprolin, 3-(2-Naphtyl)-alanin, 3-(1-Naphtylalanin), Homophenylalanin, Homocystein, 2-Amino-3-phenylaminoethylpropion-säure, Homocysteinsäure, Homotryptophan, Cysteinsäure, 3-(2-Pyridyl)-alanin, 3-(3-Pyridyl)-alanin, 3-(4-Pyridyl)-alanin, Phosphinothricin, 4-Fluorphenylalanin, 3-Fluorphenylalanin, 2-Fluor-phenylalanin, 4-Chlorphenylalanin, 4-Nitrophenylalanin, 4-Aminophenylalanin, Cyclo-hexylalanin, Citruilin, 5-Fluortryptophan, 5-Methoxytryptophan, 2-Amino-3-phenylamino-propionsäure, Methionin-Sulfon, Methionin-Sulfoxid oder -NH-NR¹¹-CON(R¹¹)₂ ableitet,
3. -(C₁-C₆)-Alkyl, worin Alkyl gerade oder verzweigt ist und ein-, zwei- oder dreifach unabhängig voneinander substituiert ist durch
3.1 Aryl, worin Aryl für einen aromatischen Kohlenstoffrest mit 6 bis 14 Kohlenstoffatomen im Ring steht aus der Gruppe Phenyl, 1-Naphthyl, 2-Naphthyl, 2-Biphenylyl, 3-Biphenylyl, 4-Biphenylyl, Anthryl oder Fluorenyl,
worin Aryl unsubstituiert oder ein-, zwei- oder dreifach durch gleiche oder verschiedene Reste substituiert ist aus der Reihe (C₁-C₈)-Alkyl, (C₁-C₈)-Alkoxy, Halogen, Nitro, Amino, Trifluormethyl, Hydroxy, Hydroxymethyl, 1-Hydroxyethyl, 2-Hydroxyethyl, Methylendioxy, Ethylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy oder Tetrazolyl,
3.2 Heteroaryl mit 5 bis 14 Ringgliedern, worin Heteroaryl für einen Rest steht aus der Gruppe Pyrrol. Furan, Thiophen, Imidazol, Pyrazol, oxazol, Isoxazol, Thiazol, Isothiazol, Tetrazol, 1,3,4-Oxadiazol, 1,2,3,5-oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen,
worin Heteroary unsubstituiert oder ein-, zwei- oder dreifach durch gleiche oder verschiedene Reste substituiert ist aus der Reihe (C₁-C₈)-Alkyl, (C₁-C₈)-Alkoxy, Halogen, Nitro, -N(R¹¹)₂, Trifluormethyl, Hydroxy, Hydroxymethyl, 1-Hydroxyethyl, 2-Hydroxyethyl, Methylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy oder Tetrazolyl,
3.3 ein Rest aus der Gruppe Azetidin, Pyrrolin, Pyrrolidin, Piperidin, isothiazol, Diazepin, Thiomorpholin, Morpholin oder Azepin,
3.4 -O-R¹¹,
3.5 =O,
3.6 Halogen,
3.7 -CN,
3.8 -CF₃,
3.9 -S(O)ₓ-R¹¹, worin x die ganze Zahl Null, 1 oder 2 ist,
3.10 -C(O)-N(R¹¹)₂,
3.11 -N(R¹¹)₂,
3.12 (C₃-C₆)-Cycloalkyl,
3.13 Rest der Formel 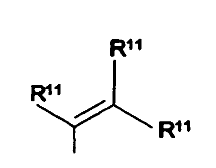 oder
3.14 Rest der Formel 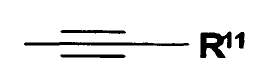
worin
R¹¹ für
a) Wasserstoffatom,
b) (C₁-C₆)-Alkyl, worin Alkyl unsubstituiert oder ein-, zwei- oder dreifach substituiert ist durch
1. Aryl, worin Aryl wie oben definiert ist und unsubstituiert oder wie oben substituiert ist,
2. Heteroaryl mit 5 bis 14 Ringgliedern, worin Heteroaryl wie oben definiert ist,
3. Halogen,
4. -N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
5. -O-(C₁-C₆)-Alkyl oder
6. -COOH,
c) Aryl, worin Aryl wie oben definiert ist und unsubstituiert oder wie oben substituiert ist,
d) Heteroaryl mit 5 bis 14 Ringgliedern, worin Heteroaryl wie oben definiert ist,
e) Halogen oder
f) -N(R¹³)₂, worin
R¹³ unabhängig voneinander für
f) a) Wasserstoffatom,
f) b) -(C₁-C₆)-Alkyl,
f) c) -(C₁-C₄)-Alkyl-O-(C₁-C₄)-Alkyl,
f) d) -(C₁-C₆)-Alkyl-N-(C₁-C₆)ₙ-,Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
f) e) Halogen oder
f) f) -(C₀-C₄)-Alkyl, ein- oder zweifach substituiert durch Phenyl, 1-Naphthyl, 2-Naphthyl, 2-Biphenylyl, 3-Biphenylyl, 4-Biphenylyl, Anthryl, Fluorenyl, Imidazolyl oder Morpholinyl, bedeutet, steht, und
für den Fall des (R¹¹)₂ hat R¹¹ unabhängig voneinander die Bedeutung von a) bis f)
Z für
1. Aryl, worin Aryl wie oben definiert ist und unsubstituiert oder wie oben substituiert ist,
2. Heteroaryl mit 5 bis 14 Ringgliedern, worin Heteroaryl wie oben definiert ist und unsubstituiert oder wie oben substituiert ist,
3. Heterocyclus mit 5 bis 12 Ringgliedern, worin Heterocyclus für einen monocyclischen oder bicyclischen 5-gliedrigen bis 12-gliedrigen heterocyclischen Ring steht, der teilweise gesättigt oder vollständig gesättigt ist aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazote, Pyridin, Pyrazin, Pyrimidin, Indol, Isoindol. Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen,
worin Heterocyclus unsubstituiert oder ein-, zwei-, drei oder vierfach durch gleiche oder verschiedene Reste substituiert ist aus der Reihe (C₁-C₈)-Alkyl, (C₁-C₈)-Alkoxy, Phenyl-(C₁-C₄)-alkoxy, Hydroxy, Oxo, Halogen, Nitro, Amino oder Trifluormethyl, wobei die Stickstoffheterocyclen auch als N-Oxide vorliegen können oder als Quartärsalze,
4. 1,3,4-Oxadiazol, worin1,3,4- Oxadiazol unsubstituiert oder einfach substituiert ist durch -NH-C(O)-(C₁-C₄)-Alkyl,- NH-C(O)-NH-(C₁-C₄)-Alkyl, -NH-C(O)-NH-(C₃-C₇)-Cyloalkyl, -NH-C(O)-NH-Aryl, worin Aryl wie oben definiert ist, -NH-C(O)-NH-Phenyl, -NH-SO₂-Aryl, worin Aryl wie oben definiert ist, oder -NH-SO₂-(C₁-C₄)-Alkyl,
5. -(C₁-C₆)-Alkyl, worin Alkyl gerade oder verzweigt ist und unsubstituiert oder ein- oder zweifach substituiert ist durch Phenyl oder -OH, oder
6. -C(O)-R¹⁰ steht, worin
R¹⁰ für
1. -O-R¹¹,
2. -N(R¹¹)₂,
3. Phenyl,
4. Pyrimidinyl oder
5. Morholinyl, steht oder
R⁸ und R⁹ bilden zusammen mit dem Stickstoff- und Kohlenstoffatom an das sie jeweils gebunden sind einen heterocyclischen Ring der Formel IIa, 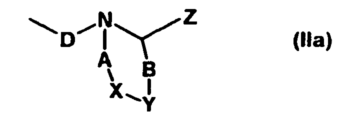 worin D und Z wie in Formel II definiert sind,
A für Stickstoffatom oder den Rest -CH₂- steht,
B für Sauerstoffatom, Schwefelatom, Stickstoffatom oder den Rest -CH₂- steht,
X für Sauerstoffatom, Schwefelatom, Stickstoffatom oder den Rest -CH₂- steht,
Y fehlt oder für Sauerstoffatom, Schwefelatom, Stickstoffatom oder den Rest -CH₂- steht, oder
X und Y zusammen einen Phenyl-, 1,2-Dtazin-, 1,3-Diazin oder einen 1,4-Diazinrest bilden,
wobei das durch N, A, X, Y, B und Kohlenstoffatom gebildete Ringsystem nicht mehr als ein Sauerstoffatom enthält, X nicht Sauerstoffatom, Schwefelatom oder Stickstoffatom darstellt, wenn A Stickstoffatom ist, nicht mehr als ein Schwefelatom enthält,1, 2, 3 oder 4 Stickstoffatome enthält und wobei nicht gleichzeitig ein Sauerstoff- und Schwefelatom vorkommen,
wobei das durch N, A, X, Y, B und Kohlenstoffatom gebildete Ringsystem unsubstituiert ist oder ein-, zwei oder dreifach unabhängig voneinander substituiert ist durch (C₁-C₈)-Alkyl, worin Alkyl unsubstituiert oder ein- oder zweifach substituiert ist durch
1.1. -OH,
1.2. (C₁-C₈)-Alkoxy,
1.3. Halogen,
1.4. -NO₂,
1.5. -NH₂,
1.6. -CF₃,
1.6. -OH,
1.7. Methylendioxy,
1.8. -C(O)-CH₃,
1.9. -CH(O),
1.10. -CN,
1.11. -COOH,
1.12. -C(O)-NH₂,
1.13. (C₁-C₄)-Alkoxycarbonyl,
1.14. Phenyl,
1.15. Phenoxy,
1.16. Benzyl,
1.17. Benzyloxy oder
1.18. Tetrazolyl, oder
R⁹ und Z bilden zusammen mit den Kohlenstoffatomen an das sie jeweils gebunden sind einen heterocyclischen Ring der Formel IIc, 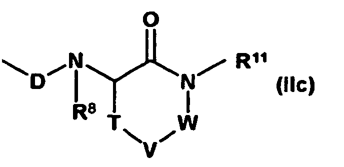 worinD, R⁸ und R¹¹ wie in Formel II definiert sind,
T für Sauerstoffatom, Schwefelatom, Stickstoffatom oder den Rest -CH₂- steht,
W für Sauerstoffatom, Schwefelatom, Stickstoffatom oder den Rest -CH₂- steht,
V fehlt oder für Sauerstoffatom, Schwefelatom, Stickstoffatom oder den Rest -CH₂- steht, oder
T und V oder V und W zusammen einen Phenyl-, 1,2-Diazin-, 1,3-Diazin oder einen 1,4-Diazinrest bilden,
wobei das durch N, T, V, W und zwei Kohlenstoffatome gebildete Ringsystem nicht mehr als ein Sauerstoffatom, nicht mehr als ein Schwefelatom und 1, 2, 3 oder 4 Stickstoffatome enthält, wobei nicht gleichzeitig ein Sauerstoff- und Schwefelatom vorkommen, und wobei das durch N, T, V, W und zwei Kohlenstoffatome gebildete Ringsystem unsubstituiert ist oder ein-, zwei oder dreifach unabhängig voneinander substituiert ist durch die oben unter 1.1. bis 1.18. definierten Substituenten, und
die jeweils anderen Substituenten R¹, R², R³ und R⁴ unabhängig voneinander für
1. Wasserstoffatom,
2. Halogen,
3. (C₁-C₄)-Alkyl,
4. Heteroaryl mit 5 bis 14 Ringgliedern, worin Heteroaryl wie oben definiert ist und unsubstituiert oder wie oben substituiert ist,
5. Heterocyclus mit 5 bis 12 Ringgliedern, worin Heterocyclus wie oben definiert ist und unsubstituiert oder wie oben substituiert ist,
6. (C₁-C₆)-Alkyl,
7. -CN,
8. -O-(C₀-C₄)-Alkyl-Aryl, worin Aryl wie oben definiert ist,
9. -O-(C₁-C₄)-Alkyl,
10. -O-R¹¹,
11. -N(R¹¹)₂,
12. -S(O)ₓ-R¹¹, worin x die ganze Zahl Null, 1 oder 2 ist,
13. -NO₂ oder
14. -CF₃ stehen,
R⁵ für
1. Wasserstoffatom,
2. -OH oder
3. =0 steht, und
R⁶ für
1. Aryl, worin Aryl wie oben definiert ist und unsubstituiert oder wie oben substituiert ist,
2. Phenyl, ein- oder zweifach substituiert durch
2.1 -N(R¹¹)₂,
2.2 -NH-C(O)-R¹¹,
2.3 -S(O)ₓ-R¹¹, worin x die ganze Zahl Null, 1 oder 2 ist,
2.4 -C(O)-R¹¹oder
2.5 -(C₁-C₄)-Alkyl-NH₂ ,
3. Heteroaryl mit 5 bis 14 Ringgliedern, worin Heteroaryl wie oben definiert ist und unsubstituiert oder durch -N-(C₃-C₆)-Cycloalkyl oder wie oben substituiert ist oder
4. Heterocyclus mit 5 bis 12 Ringgliedern, worin Heterocyclus wie oben definiert ist und unsubstituiert oder durch -NH-(C₁-C₆)-Alkyl, -NH-Phenyl, -NH-(C₃-C₆)-Cycloalkyl oder ein-, zwei- oder dreifach wie oben substituiert ist, steht.

2. Verbindung der Formel I gemäß Anspruch 1, **dadurch gekennzeichnet, dass** einer der Substituenten R¹, R², R³ und R⁴ für einen Rest der Formel II steht, worin
R⁸ für Wasserstoffatom steht,
R⁹ für
1. charakteristischen Rest einer Aminosäure, der sich von einer natürlich vorkommenden α-Aminosäure aus der Gruppe Histidin, Tryptophan, Serin, Threonin, Cystein, Methionin, Asparagin, Glutamin, Lysin, Arginin und Glutaminsäure ableitet oder
2. (C₁-C₆)-Alkyl, worin Alkyl gerade oder verzweigt ist und unsubstituiert oder ein- oder zweifach substituiert ist durch einen Rest aus der Gruppe Pyrrol, Pyrrol ein- oder zweifach substituiert durch -(C₁-C₄)-Alkyl, Pyrazol, Phenyl, Imidazol, Triazol, Thiophen, Thiazol, Oxazol, Isoxazol, Pyridin, Pyrimidin, Indol, Benzothiophen, Benzimidazol, Benzoxazol, Benzothiazol, Azetidin, Pyrrolin, Pyrrolidin, Piperidin, isothiazol, Diazepin, Thiomorpholin, -CN, Morpholin, Azepin, 1,3,4-Oxadiazol, Pyrazin, -N(R¹³)-Phenyl, (C₃-C₆)-Cycloalkyl, -OR¹¹, -NH(R¹¹), worin R¹¹ jeweils wie in Anspruch 1 definiert ist, -S(O)ₓ-R¹², worin x für Null, 1 oder 2 und R¹² Naphthyl, Pyrimidinyl, Morpholinyl oder Phenyl bedeutet, welche unsubstituiert oder ein- oder zweifach substituiert sind durch -OH, (C₁-C₄)-Alkyl, -CF₃, Halogen, -O-(C₁-C₄)-Alkyl, -COOH, -C(O)-O-(C₁-C₄)-Alkyl, -NH₂ oder -NH-C(O)-(C₁-C₄)-Alkyl,
Z für -C(O)-R¹⁰, Tetrazol, (C₁-C₆)-Alkyl, worin Alkyl gerade oder verzweigt ist und unsubstituiert oder ein- oder zweifach substituiert ist durch Phenyl, oder -OH, oder 1,3,4-Oxadiazol, worin1,3,4- Oxadiazol unsubstituiert oder einfach substituiert ist durch -NH₂, -NH(C₁-C₄)-Alkyl, -N-[(C₁-C₄)-Alkyl]₂, -NH-C(O)-(C₁-C₄)-Alkyl,- NH-C(O)-NH-(C₁-C₄)-Alkyl, -NH-C(O)-NH-(C₃-C₇)-Cyloalkyl, -NH-C(O)-NH-Aryl, worin Aryl ausgewählt ist aus der Gruppe Phenyl, 1-Naphthyl, 2-Naphthyl, 2-Biphenylyl, 3-Biphenylyl, 4-Biphenylyl, Anthryl und Fluorenyl, -NH-C(O)-NH-Phenyl, -NH-SO₂-Aryl, worin Aryl wie oben definiert ist ,-NH-SO₂-(C₁-C₄)-Alkyl, -OH oder -(C₁-C₄)-Alkyl, steht, worin
R¹⁰ für -O-R¹¹, Phenyl, Pyrimidin, -OH, Morpholinyl, -N(R¹¹)₂ oder -NH₂ steht,
R¹¹ für
1. -(C₁-C₄)-Alkyl,
2. R¹³ oder
3. -N(R¹³)₂, worin
R¹³ unabhängig voneinander
a) Wasserstoffatom,
b) -(C₁-C₆)-Alkyl,
c) -(C₁-C₄)-Alkyl-O-(C₁-C₄)-Alkyl,
d) -(C₁-C₆)-Alkyl-N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
e) Halogen oder
f) -(C₀-C₄)-Alkyl, ein- oder zweifach substituiert durch Aryl, worin Aryl wie oben definiert ist, lmidazolyl oder Morpholinyl, steht oder
R⁸ und R⁹ bilden zusammen mit dem Stickstoff- und Kohlenstoffatom an das sie jeweils gebunden sind einen Ring der Formel Ila aus der Gruppe Pyrrol, Pyrrolin, Pyrrolidin, Pyridin, Piperidin, Piperylen, Pyridazin, Pyrimidin, Pyrazin, Piperazin, Pyrazol, imidazol, Pyrazolin, Imidazolin, Pyrazolidin, imidazolidin, Oxazol, Isoxazol, 2-lsoxazolidin, Isoxazolidin, Morpholin, Isothiazol, Thiazol, Tetrazol, 1,2,3,5-Oxathiadiazol-2-Oxide, Oxadiazolone, lsoxazolone, Triazolone, oxadiazolidindione, Triazole, welche durch F, CN, CF₃ oder COO-(C₁-C₄)-Alkyl substituert sind, 3-Hydroxypyrro-2,4-dione, 5-Oxo-1,2,4-Thiadiazole, 1,3,4-Oxadiazol, Isothiazolidin, Thiomorpholin, Indazol, Thiadiazol, Benzimidazol, Chinolin, Triazol, Phthalazin, Chinazolin, Chinoxalin, Purin, Pteridin, Indol, Tetrahydrochinolin, Tetrahydroisochinolin und Isochinolin, oder
R⁹ und Z bilden zusammen mit den Kohlenstoffatomen an das sie jeweils gebunden sind einen Ring der Formel IIc aus der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, Imidazolidin, Oxazol, Isoxazol, 2-Isoxazolidin, Isoxazolidin, Morpholin, Isothiazol, Thiazol, Isothiazolidin, Thiomorpholin, Indazol, Thiadiazol, Benzimidazol, Chinolin, Triazol, Phthalazin, Chinazolin, Chinoxalin, Purin, Pteridin, Indol, Tetrahydrochinolin, Tetrahydroisochinolin, Isochinolin, Tetrazol, 1,2,3,5-Oxathiadiazol-2-Oxide, Oxadiazolone, Isoxazolone, Triazolone, Oxadiazolidindione, Triazole, welche durch F, CN, CF₃ oder COO-(C₁-C₄)-Alkyl substituert sind, 3-Hydroxypyrro-2,4-dione, 1,3,4-Oxadiazol und 5-Oxo-1,2,4-Thiadiazole und
die jeweils anderen Substituenten R¹, R², R³ und R⁴ unabhängig voneinander für
1. Wasserstoffatom,
2. Halogen,
3. (C₁-C₄)-Alkyl,
4. -CN,
5. -NO₂,
6. -O-(C₀-C₄)-Alkyl-Aryl, worin Aryl wie oben definiert ist,
7. -O-(C₁-C₄)-Alkyl,
8. -N-(C₀-C₄)-Alkyl-Aryl, worin Aryl wie oben definiert ist,
9. -N-(C₁-C₄)-Alkyl oder
10. -CF₃ stehen,
R⁵ für
1. Wasserstoffatom,
2. -OH oder
3. =0 steht, und
R⁶ für
1. Phenyl, ein oder zweifach substituiert durch
1.1 -CN,
1.2 -NO₂,
1.3 -O-(C₁-C₄)-Alkyl,
1.4 -NH₂ oder
1.5 -(C₁-C₄)-Alkyl-NH₂
2. Heteroaryl mit 5 bis 14 Ringgliedern, worin Heteroaryl für einen Rest steht aus der Gruppe Pyrrol, Furan, Thiophen, Imidazol, Pyrazol, Oxazol, Isoxazol, Thiazol, isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen, und worin Heteroaryl unsubstituiert oder ein- bis dreifach substituiert durch -N-R¹⁴, worin R¹⁴ -(C₁-C₆)-Alkyl, -(C₃-C₆)-Cycloalkyl oder Phenyl bedeutet, Halogen, -OH oder -(C₁-C₄)-Alkyl, oder
3. Heterocyclus mit 5 bis 12 Ringgliedern, worin Heterocyclus für einen monocyclischen oder bicyclischen 5-gliedrigen bis 12-gliedrigen heterocyclischen Ring steht, der teilweise gesättigt oder vollständig gesättigt aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen und worin Heterocyclus unsubstituiert oder ein- bis dreifach substituiert durch -N-R¹⁴, worin R¹⁴ -(C₁-C₆)-Alkyl, -(C₃-C₆)-Cycloalkyl oder Phenyl bedeutet, Halogen, -OH oder -(C₁-C₄)-Alkyl, steht.

3. Verfahren zur Herstellung der Verbindung der Formel I gemäß einem oder mehreren der Ansprüche 1 und 2, **dadurch gekennzeichnet, dass** man
a) eine Verbindung der Formel IV, 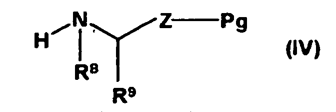 worin Pg ein geeignete Schutzgruppe (z.B. Methylester), eine Amidgruppe oder eine Hydroxy-Gruppe darstellt und Z, R⁸ und R⁹ wie in Formel II definiert sind, mit einem Säurechlorid oder einem aktivierten Ester der Verbindung der Formel III, 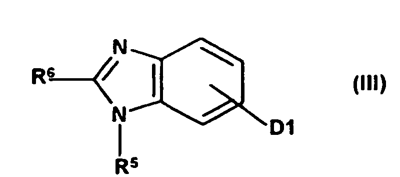 wobei D1 -COOH oder Sulfonylhalogen bedeutet und R⁵ und R⁶ wie in Formel I definiert sind, in Gegenwart einer Base oder gegebenenfalls eines wasserentziehenden Mittels in Lösung umsetzt und nach Abspaltung der Schutzgruppe in eine Verbindung der Formel I überführt, oder
b) eine Verbindung der Formel IVa, 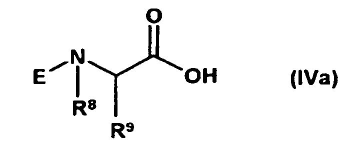 worin R⁸ und R⁹ wie in Formel II definiert sind und E eine N-Aminoschutzgruppe darstellt, mit ihrer Carboxylgruppe über eine Zwischenkette L an ein polymeres Harz der allgemeinen Formel PS ankoppelt, wobei eine Verbindung der Formel V 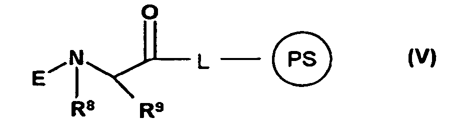 entsteht, die nach selektiver Abspaltung der Schutzgruppe E mit einer Verbindung der Formel III, wobei R⁵ und R⁶ wie in Formel I definiert sind, in Gegenwart einer Base oder gegebenenfalls eines wasserentziehenden Mittels zu einer Verbindung der Formel VI 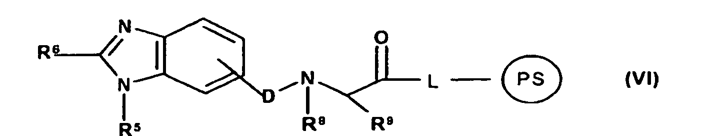 umsetzt, und die Verbindung der Formel VI nach Abspaltung vom Trägermaterial in eine Verbindung der Formel I überführt, oder
c) eine Verbindung der Formel V , nach selektiver Abspaltung der Schutzgruppe E mit einer Verbindung der Formel VII, 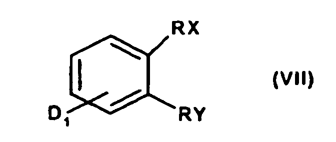 wobei D₁ für -COOH oder Sulfonylhalogen steht und RX für Halogen und RY für einen Rest -NO₂ oder -NH-E steht und E eine Schutzgruppe bedeutet, zu einer Verbindung der Formel VIII umsetzt 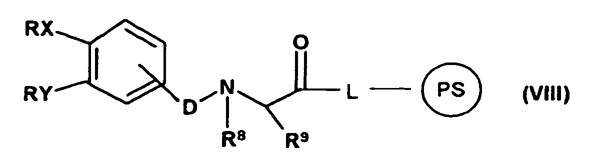 und anschließend die Verbindung der Formel VIII mit einer Verbindung der Formel IX
NH₂-R⁶ (IX)
worin R⁶ wie in der Verbindung der Formel I definiert ist,
zu einer Zwischenverbindung der Formel Vla umsetzt, 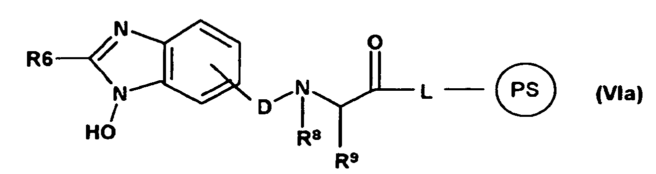 anschließend die Zwischenverbindung der Formel VIa entweder nach Abspaltung vom Trägermaterial in eine Verbindung der Formel I überführt oder beispielsweise mit Tributylphosphin, zu einer Verbindung der Formel VI reduziert und nach Abspaltung vom Trägermaterial in eine Verbindung der Formel I überführt, oder
d) eine Verbindung der Formel I in ein physiologisch verträgliches Salz überführt.

4. Arzneimittel, **gekennzeichnet durch** einen wirksamen Gehalt an mindestens einer Verbindung der Formel I gemäß einem oder mehreren der Ansprüche 1 und 2 zusammen mit einem pharmazeutisch geeigneten und physiologisch verträglichen Trägerstoff, Zusatzstoff und/oder anderen Wirk- und Hilfsstoffen.

5. Verwendung von mindestens einer Verbindung der Formel I 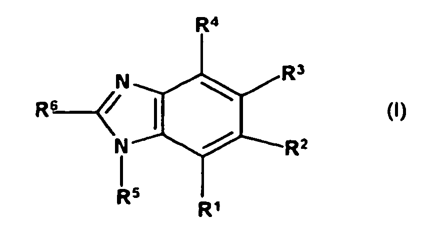 und/oder eine stereoisomere Form der Verbindung der Formel I und/oder ein physiologisch verträgliches Salz der Verbindung der Formel I,
zur Herstellung von Arzneimitteln zur Prophylaxe und Therapie von Erkrankungen aus der Reihe Asthma, Rheumatoide Arthritis, Osteoarthritis, Alzheimers Erkrankungen, Krebserkrankungen, Herzinfarkt, Herzinsuffizienz, akutes Koronarsyndrom, instabile Angina Pectoris, septischer Schock, akutes und chronisches Nierenversagen, Stroke oder Atherosklerose, wobei
einer der Substituenten R¹, R², R³ und R⁴ für einen Rest der Formel II steht, 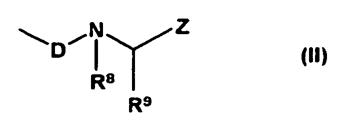 worin
D für -C(O)-, -S(O)- oder -S(O)₂- steht,
R⁸ für Wasserstoffatom oder (C₁-C₄)-Alkyl steht,
R⁹ für
1. charakteristischen Rest einer Aminosäure, der sich von einer natürlich vorkommenden α-Aminosäure aus der Gruppe Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin und Glutaminsäure ableitet, oder
der sich von einer nicht natürlich vorkommenden Aminosäure aus der Gruppe 2-Aminoadipinsäure, 2-Aminobuttersäure, 2-Aminoisobuttersäure, 2,3-Diamino-propionsäure, 2,4-Diaminobuttersäure, 1,2,3,4-Tetrahydroisochinolin-1-carbonsäure, 1,2,3,4-Tetrahydroisochinolin-3-carbonsäure, 2-Amino-pimelinsäure, Phenylglycin, 3-(2-Thienyl)-alanin, 3-(3-Thienyl)-alanin, Sarkosin, 2-(2-Thienyl)-glycin, 2-Amino-heptansäure, Pipecolinsäure, Hydroxylysin, N-Methylisoleucin, 6-N-Methyllysin, N-Methylvalin, Norvalin, Norleucin, Ornithin, allo-Isoleucin, 4-Hydroxy-prolin, allo-Hydroxylysin, allo-Threonin, 3-Hydroxyprolin, 3-(2-Naphtyl)-alanin, 3-(1-Naphtylalanin), Homophenylalanin, Homocystein, 2-Amino-3-phenylaminoethylpropion-säure, Homocysteinsäure, Homotryptophan, Cysteinsäure, 3-(2-Pyridyl)-alanin, 3-(3-Pyridyl)-alanin, 3-(4-Pyridyl)-alanin, Phosphinothricin, 4-Fluorphenylalanin, 3-Fluorphenyl-alanin, 2-Fluor-phenylalanin, 4-Chlorphenylalanin, 4-Nitrophenylalanin, 4-Aminophenylalanin, Cyclohexylalanin, Citrullin, 5-Fluortryptophan, 5-Methoxytryptophan, 2-Amino-3-phenytamino-propionsäure, Methionin-Sulfon, Methionin-Sulfoxid oder -NH-NR¹¹-CON(R¹¹)₂ ableitet,
2. Aryl, worin Aryl für einen aromatischen Kohlenstoffrest mit 6 bis 14 Kohlenstoffatomen im Ring steht aus der Gruppe Phenyl, 1-Naphthyl, 2-Naphthyl, 2-Biphenylyl, 3-Biphenylyl, 4-Biphenylyl, Anthryl oder Fluorenyl,
worin Aryl unsubstituiert oder ein-, zwei- oder dreifach durch gleiche oder verschiedene Reste substituiert ist aus der Reihe (C₁-C₈)-Alkyl, (C₁-C₈)-Alkoxy, Halogen, Nitro, Amino, Trifluormethyl, Hydroxy, Hydroxymethyl, 1-Hydroxyethyl, 2-Hydroxyethyl, Methylendioxy, Ethylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy oder Tetrazolyl,
3. Heteroaryl mit 5 bis 14 Ringgliedern, worin Heteroaryl für einen Rest steht aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen,
worin Heteroary unsubstituiert oder ein-, zwei- oder dreifach durch gleiche oder verschiedene Reste substituiert ist aus der Reihe (C₁-C₈)-Alkyl, (C₁-C₈)-Alkoxy, Halogen, Nitro, -N(R¹¹)₂, Trifluormethyl, Hydroxy, Hydroxymethyl, 1-Hydroxyethyl, 2-Hydroxyethyl, Methylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy oder Tetrazolyl,
4. Heterocyclus mit 5 bis 12 Ringgliedern, worin Heterocyclus für einen monocyclischen oder bicyclischen 5-gliedrigen bis 12-gliedrigen heterocyclischen Ring steht, der teilweise gesättigt oder vollständig gesättigt ist aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen,
worin Heterocyclus unsubstituiert oder ein-, zwei-, drei oder vierfach durch gleiche oder verschiedene Reste substituiert ist aus der Reihe (C₁-C₈)-Alkyl, (C₁-C₈)-Alkoxy, Phenyl-(C₁-C₄)-alkoxy, Hydroxy, Oxo, Halogen, Nitro, Amino oder Trifluormethyl, wobei die Stickstoffheterocyclen auch als N-Oxide vorliegen können oder als Quartärsalze,
5. (C₁-C₆)-Alkyl, worin Alkyl gerade oder verzweigt ist und unsubstituiert oder ein-, zwei- oder dreifach unabhängig voneinander substituiert ist durch
5.1 Aryl, worin Aryl wie oben definiert ist und unsubstituiert oder wie oben substituiert ist,
5.2 Heteroaryl mit 5 bis 14 Ringgliedern, worin Heteroaryl wie oben definiert ist und unsubstituiert oder wie oben substituiert ist,
5.3 Heterocyclus mit 5 bis 12 Ringgliedern, worin Heterocyclus wie oben definiert ist und unsubstituiert oder wie oben substituiert ist
5.4 ein Rest aus der Gruppe Azetidin, Pyrrolin, Pyrrolidin, Piperidin, isothiazol, Diazepin, Thiomorpholin, Morpholin oder Azepin,
5.5 -O-R¹¹,
5.6 =O,
5.7 Halogen,
5.8 -CN,
5.9 -CF₃,
5.10 -S(O)ₓ-R¹¹, worin x die ganze Zahl Null, oder 2 ist,
5.12 -C(O)-N(R¹¹)₂,
5.13 -N(R¹¹)₂,
5.14 (C₃-C₆)-Cycloalkyl,
5.15 Rest der Formel 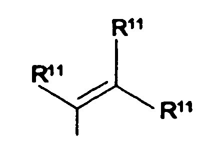 oder
5.16 Rest der Formel 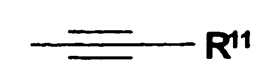
worin
R¹¹ für
a) Wasserstoffatom,
b) (C₁-C₆)-Alkyl, worin Alkyl unsubstituiert oder ein-, zwei- oder dreifach substituiert ist durch
1. Aryl, worin Aryl wie oben definiert ist und unsubstituiert oder wie oben substituiert ist,
2. Heteroaryl mit 5 bis 14 Ringgliedern, worin Heteroaryl wie oben definiert ist,
3. Halogen,
4. -N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
5. -O-(C₁-C₆)-Alkyl oder
6. -COOH,
c) Aryl, worin Aryl wie oben definiert ist und unsubstituiert oder wie oben substituiert ist,
d) Heteroaryl mit 5 bis 14 Ringgliedern, worin Heteroaryl wie oben definiert ist,
e) Halogen oder
f) -N(R¹³)₂, worin
R¹³ unabhängig voneinander für
f) a) Wasserstoffatom,
f) b) -(C₁-C₆)-Alkyl,
f) c) -(C₁-C₄)-Alkyl-O-(C₁-C₄)-Alkyl,
f) d) -(C₁-C₆)-Alkyl-N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
f) e) Halogen oder
f) f) -(C₀-C₄)-Alkyl, ein- oder zweifach substituiert durch Phenyl, 1-Naphthyl, 2-Naphthyl, 2-Biphenylyl, 3-Biphenylyl, 4-Biphenylyl, Anthryl, Fluorenyl, Imidazolyl oder Morpholinyl, bedeutet, steht, und
für den Fall des (R¹¹)₂ hat R¹¹ unabhängig voneinander die Bedeutung von a) bis f)
Z für
1. Aryl, worin Aryl wie oben definiert ist und unsubstituiert oder wie oben substituiert ist,
2. Heteroaryl mit 5 bis 14 Ringgliedern, worin Heteroaryl wie oben definiert ist und unsubstituiert oder wie oben substituiert ist,
3. Heterocyclus mit 5 bis 12 Ringgliedern, worin Heterocyclus wie oben definiert ist und unsubstituiert oder wie oben substituiert ist,
4. 1,3,4-Oxadiazol, worin1,3,4- Oxadiazol unsubstituiert oder einfach substituiert ist durch -NH-C(O)-(C₁-C₄)-Alkyl,- NH-C(O)-NH-(C₁-C₄)-Alkyl, -NH-C(O)-NH-(C₃-C₇)-Cyloalkyl, -NH-C(O)-NH-Aryl, worin Aryl wie oben definiert ist, -NH-C(O)-NH-Phenyl, -NH-SO₂-Aryl, worin Aryl wie oben definiert ist, oder -NH-SO₂-(C₁-C₄)-Alkyl,
5. -(C₁-C₆)-Alkyl, worin Alkyl gerade oder verzweigt ist und unsubstituiert oder ein- oder zweifach substituiert ist durch Phenyl oder -OH, oder
6. -C(O)-R¹⁰ steht, worin
R¹⁰ für
1.-O-R¹¹,
2. -N(R¹¹)₂,
3. Phenyl,
4. Pyrimidinyl oder
5. Morholinyl, steht oder
R⁸ und R⁹ bilden zusammen mit dem Stickstoff- und Kohlenstoffatom an das sie jeweils gebunden sind einen heterocyclischen Ring der Formel IIa, 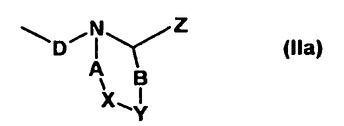 worin D und Z wie in Formel II definiert sind,
A für Stickstoffatom oder den Rest -CH₂- steht,
B für Sauerstoffatom, Schwefelatom, Stickstoffatom oder den Rest -CH₂- steht,
X für Sauerstoffatom, Schwefelatom, Stickstoffatom oder den Rest -CH₂- steht,
Y fehlt oder für Sauerstoffatom, Schwefelatom, Stickstoffatom oder den Rest -CH₂- steht, oder
X und Y zusammen einen Phenyl-, 1,2-Diazin-,1,3-Diazin oder einen 1,4-Diazinrest bilden,
wobei das durch N, A, X, Y, B und Kohlenstoffatom gebildete Ringsystem nicht mehr als ein Sauerstoffatom enthält, X nicht Sauerstoffatom, Schwefelatom oder Stickstoffatom darstellt, wenn A Stickstoffatom ist, nicht mehr als ein Schwefelatom enthält, 1, 2, 3 oder 4 Stickstoffatome enthält und wobei nicht gleichzeitig ein Sauerstoff- und Schwefelatom vorkommen,
wobei das durch N, A, X, Y, B und Kohlenstoffatom gebildete Ringsystem unsubstituiert ist oder ein-, zwei oder dreifach unabhängig voneinander substituiert ist durch (C₁-C₈)-Alkyl, worin Alkyl unsubstituiert oder ein- oder zweifach substituiert ist durch
1.1. -OH,
1.2. (C₁-C₈)-Alkoxy,
1.3. Halogen,
1.4. -NO₂,
1.5. -NH₂,
1.6. -CF₃,
1.6. -OH,
1.7. Methylendioxy,
1.8. -C(O)-CH₃,
1.9. -CH(O),
1.10. -CN,
1.11. -COOH,
1.12. -C(O)-NH₂,
1.13. (C₁-C₄)-Alkoxycarbonyl,
1.14. Phenyl,
1.15. Phenoxy,
1.16. Benzyl,
1.17. Benzyloxy oder
1.18. Tetrazolyl, oder
R⁹ und Z bilden zusammen mit den Kohlenstoffatomen an das sie jeweils gebunden sind einen heterocyclischen Ring der Formel IIc, 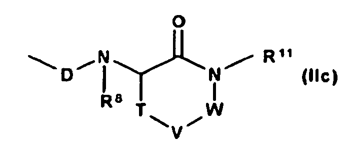 worin D, R⁸ und R¹¹ wie in Formel II definiert sind,
T für Sauerstoffatom, Schwefelatom, Stickstoffatom oder den Rest -CH₂- steht,
W für Sauerstoffatom, Schwefelatom, Stickstoffatom oder den Rest -CH₂- steht,
V fehlt oder für Sauerstoffatom, Schwefelatom, Stickstoffatom oder den Rest -CH₂- steht, oder
T und V oder V und W zusammen einen Phenyl-, 1,2-Diazin-, 1,3-Diazin oder einen 1,4-Diazinrest bilden,
wobei das durch N, T, V, W und zwei Kohlenstoffatome gebildete Ringsystem nicht mehr als ein Sauerstoffatom, nicht mehr als ein Schwefelatom und 1, 2, 3 oder 4 Stickstoffatome enthält, wobei nicht gleichzeitig ein Sauerstoff- und Schwefelatom vorkommen, und wobei das durch N, T, V, W und zwei Kohlenstoffatome gebildete Ringsystem unsubstituiert ist oder ein-, zwei oder dreifach unabhängig voneinander substituiert ist durch die oben unter 1.1. bis 1.18. definierten Substituenten, und
die jeweils anderen Substituenten R¹, R², R³ und R⁴ unabhängig voneinander für
1. Wasserstoffatom,
2. Halogen,
3. (C₁-C₄)-Alkyl,
4. Heteroaryl mit 5 bis 14 Ringgliedern, worin Heteroaryl wie oben definiert ist und unsubstituiert oder wie oben substituiert ist,
5. Heterocyclus mit 5 bis 12 Ringgliedern, worin Heterocyclus wie oben definiert ist und unsubstituiert oder wie oben substituiert ist,
6. (C₁-C₆)-Alkyl,
7. -CN,
8. -O-(C₀-C₄)-Alkyl-Aryl, worin Aryl wie oben definiert ist,
9. -O-(C₁-C₄)-Alkyl,
10. -OR¹¹,
11. -N(R¹¹)₂,
12. -S(O)ₓ-R¹¹, worin x die ganze Zahl Null, 1 oder 2 ist,
13. -NO₂ oder
14. -CF₃ stehen,
R⁵ für
1. Wasserstoffatom,
2. -OH oder
3. =O steht, und
R⁶ für
1. Aryl, worin Aryl wie oben definiert ist und unsubstituiert oder wie oben substituiert ist,
2. Phenyl, ein- oder zweifach substituiert durch
2.1 -N(R¹¹)₂ ,
2.2 -NH-C(O)-R¹¹,
2.3 -S(O)ₓ-R¹¹, worin x die ganze Zahl Null, 1 oder 2 ist,
2.4 -C(O)-R¹¹ oder
2.5 -(C₁-C₄)-Alkyl-NH₂,
3. Heteroaryl mit 5 bis 14 Ringgliedern, worin Heteroaryl wie oben definiert ist und unsubstituiert oder durch -N-(C₃-C₆)-Cycloalkyl oder wie oben substituiert ist oder
4. Heterocyclus mit 5 bis 12 Ringgliedern, worin Heterocyclus wie oben definiert ist und unsubstituiert oder durch -NH-(C₁-C₆)-Alkyl, -NH-Phenyl, -NH-(C₃-C₆)-Cycloalkyl oder ein-, zwei- oder dreifach wie oben substituiert ist, steht.

6. Verwendung gemäß Anspruch 5, **dadurch gekennzeichnet, dass** einer der Substituenten R¹, R², R³ und R⁴ für einen Rest der Formel II steht, worin
R⁸ für Wasserstoffatom steht,
R⁹ für
1. charakteristischen Rest einer Aminosäure, der sich von einer natürlich vorkommenden α-Aminosäure aus der Gruppe Histidin, Tryptophan, Serin, Threonin, Cystein, Methionin, Asparagin, Glutamin, Lysin, Arginin und Glutaminsäure ableitet oder
2. (C₁-C₆)-Alkyl, worin Alkyl gerade oder verzweigt ist und unsubstituiert oder ein- oder zweifach substituiert ist durch einen Rest aus der Gruppe Pyrrol, Pyrrol ein- oder zweifach substituiert durch -(C₁-C₄)-Alkyl, Pyrazol, Phenyl, Imidazol, Triazol, Thiophen, Thiazol, Oxazol, Isoxazol, Pyridin, Pyrimidin, Indol, Benzothiophen, Benzimidazol, Benzoxazol, Benzothiazol, Azetidin, Pyrrolin, Pyrrolidin, Piperidin, Isothiazol, Diazepin, Thiomorpholin, -CN, Morpholin, Azepin, 1,3,4-Oxadiazol, Pyrazin, -N(R¹³)-Phenyl, (C₃-C₆)-Cycloalkyl, -OR¹¹, -NH(R¹¹), worin R¹¹ jeweils wie in Anspruch 1 definiert ist, -S(O)ₓ-R¹², worin x für Null, 1 oder 2 und R¹² Naphthyl, Pyrimidinyl, Morpholinyl oder Phenyl bedeutet, welche unsubstituiert oder ein- oder zweifach substituiert sind durch -OH, (C₁-C₄)-Alkyl, -CF₃, Halogen, -O-(C₁-C₄)-Alkyl, -COOH, -C(O)-O-(C₁-C₄)-Alkyl, -NH₂ oder -NH-C(O)-(C₁-C₄)-Alkyl,
Z für -C(O)-R¹⁰, Tetrazol, (C₁-C₆)-Alkyl, worin Alkyl gerade oder verzweigt ist und unsubstituiert oder ein- oder zweifach substituiert ist durch Phenyl, oder -OH, oder 1,3,4-Oxadiazol, worin 1,3,4-Oxadiazol unsubstituiert oder einfach substituiert ist durch -NH₂, -NH(C₁-C₄)-Alkyl, -N-[(C₁-C₄)-Alkyl]₂, -NH-C(O)-(C₁-C₄)-Alkyl,- NH-C(O)-NH-(C₁-C₄)-Alkyl, -NH-C(O)-NH-(C₃-C₇)-Cyloalkyl, -NH-C(O)-NH-Aryl, worin Aryl ausgewählt ist aus der Gruppe Phenyl, 1-Naphthyl, 2-Naphthyl, 2-Biphenylyl, 3-Biphenylyl, 4-Biphenylyl, Anthryl und Fluorenyl, -NH-C(O)-NH-Phenyl, -NH-SO₂-Aryl, worin Aryl wie oben definiert ist ,-NH-SO₂-(C₁-C₄)-Alkyl, -OH oder -(C₁-C₄)-Alkyl, steht, worin
R¹⁰ für -O-R¹¹, Phenyl, Pyrimidin, -OH, Morpholinyl, -N(R¹¹)₂ oder -NH₂ steht,
R¹¹ für
1. -(C₁-C₄)-Alkyl,
2. R¹³ oder
3. -N(R¹³)₂, worin
R¹³ unabhängig voneinander
a) Wasserstoffatom,
b) -(C₁-C₆)-Alkyl,
c) -(C₁-C₄)-Alkyl-O-(C₁-C₄)-Alkyl,
d) -(C₁-C₆)-Alkyl-N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -COOH substituiert ist,
e) Halogen oder
f) -(C₀-C₄)-Alkyl, ein- oder zweifach substituiert durch Aryl, worin Aryl
wie oben definiert ist, Imidazolyl oder Morpholinyl, steht oder
R⁸ und R⁹ bilden zusammen mit dem Stickstoff- und Kohlenstoffatom an das sie jeweils gebunden sind einen Ring der Formel IIa aus der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, Imidazolidin, oxazol, Isoxazol, 2-Isoxazolidin, Isoxazolidin, Morpholin, lsothiazol, Thiazol, Tetrazol, 1,2,3,5-Oxathiadiazol-2-Oxide, Oxadiazolone, Isoxazolone, Triazolone, Oxadiazolidindione, Triazole, welche durch F, CN, CF₃ oder COO-(C₁-C₄)-Alkyl substituert sind, 3-Hydroxypyrro-2,4-dione, 5-Oxo-1,2,4-Thiadiazole, 1,3,4-Oxadiazol, Isothiazolidin, Thiomorpholin, Indazol, Thiadiazol, Benzimidazol, Chinolin, Triazol, Phthalazin, Chinazolin, Chinoxalin, Purin, Pteridin, Indol, Tetrahydrochinolin, Tetrahydroisochinolin und Isochinolin, oder R⁹ und Z bilden zusammen mit den Kohlenstoffatomen an das sie jeweils gebunden sind einen Ring der Formel IIc aus der Gruppe Pyrrol, Pyrrolin, Pyrrolidin, Pyridin, Piperidin, Piperylen, Pyridazin, Pyrimidin, Pyrazin, Piperazin, Pyrazol, imidazol, Pyrazolin, imidazolin, Pyrazolidin, Imidazolidin, oxazol, Isoxazol, 2-Isoxazolidin, Isoxazolidin, Morpholin, Isothiazol, Thiazol, Isothiazolidin, Thiomorpholin, Indazol, Thiadiazol, Benzimidazol, Chinolin, Triazol, Phthalazin, Chinazolin, Chinoxalin, Purin, Pteridin, Indol, Tetrahydrochinolin, Tetrahydroisochinolin, Isochinolin, Tetrazol, 1,2,3,5-Oxathiadiazol-2-Oxide, Oxadiazolone, Isoxazolone, Triazolone, Oxadiazolidindione, Triazole, welche durch F, CN, CF₃ oder COO-(C₁-C₄)-Alkyl substituert sind, 3-Hydroxypyrro-2,4-dione, 1,3,4-Oxadiazol und 5-Oxo-1,2,4-Thiadiazole und
die jeweils anderen Substituenten R¹, R², R³ und R⁴ unabhängig voneinander für
1. Wasserstoffatom,
2. Halogen,
3. (C₁-C₄)-Alkyl,
4. -CN,
S. -NO₂,
6. -O-(C₀-C₄)-Alkyl-Aryl, worin Aryl wie oben definiert ist,
7. -O-(C₁-C₄)-Alkyl,
8. -N-(C₀-C₄)-Alkyl-Aryl, worin Aryl wie oben definiert ist,
9. -N-(C₁-C₄)-Alkyl oder
10. -CF₃ stehen,
R⁵ für
1. Wasserstoffatom,
2. -OH oder
3. =O steht, und
R⁶ für
1. Phenyl, ein oder zweifach substituiert durch
1.1 -CN,
1.2 -NO₂,
1.3 -O-(C₁-C₄)-Alkyl,
1.4 -NH₂ oder
1.5 -(C₁-C₄)-Alkyl-NH₂
2. Heteroaryl mit 5 bis 14 Ringgliedern, worin Heteroaryl für einen Rest steht aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen, und worin Heteroaryl unsubstituiert oder ein- bis dreifach substituiert durch -N-R¹⁴, worin R¹⁴ -(C₁-C₆)-Alkyl, -(C₃-C₆)-Cycloalkyl oder Phenyl bedeutet, Halogen, -OH oder -(C₁-C₄)-Alkyl, oder
3. Heterocyclus mit 5 bis 12 Ringgliedern, worin Heterocyclus für einen monocyclischen oder bicyclischen 5-gliedrigen bis 12-gliedrigen heterocyclischen Ring steht, der teilweise gesättigt oder vollständig gesättigt aus der Gruppe Pyrrol, Furan, Thiophen, Imidazol, Pyrazol, Oxazol, Isoxazol, Thiazol, Isothiazol, Tetrazol, 1,3,4-Oxadiazol, 1,2,3,5-Oxathiadiazol-2-Oxide, Triazolone, Oxadiazolone, Isoxazolone, Oxadiazolidindione, Triazole, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen und worin Heterocyclus unsubstituiert oder ein- bis dreifach substituiert durch -N-R¹⁴, worin R¹⁴ -(C₁-C₆)-Alkyl, -(C₃-C₆)-Cyctoalkyl oder Phenyl bedeutet, Halogen, -OH oder -(C₁-C₄)-Alkyl, steht.

7. Verfahren zur Herstellung eines Arzneimittels , **dadurch gekennzeichnet, dass** man mindestens eine Verbindung der Formel I gemäß einem oder mehreren der Ansprüche 1 und 2 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. A compound of the formula I 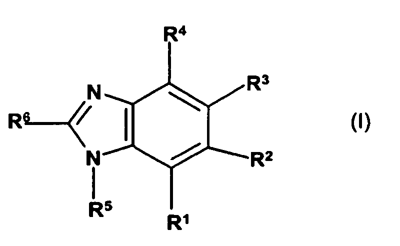 and/or a stereoisomeric form of the compound of the formula I and/or a physiologically tolerable salt of the compound of the formula I, where one of the substituents R¹, R², R³ and R⁴ is a radical of the formula II 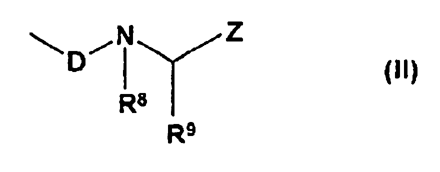 in which
D is -C(O)-, -S(O)- or -S(O)₂-,
R⁸ is hydrogen or (C₁-C₄)-alkyl,
R⁹ is
1. a characteristic radical of a native α-amino acid from the group consisting of alanine, valine, leucine, isoleucine, phenylalanine, tyrosine, tryptophan, serine, threonine, cysteine, methionine, asparagine, glutamine, lysine, histidine, arginine and glutamic acid,
2. a characteristic radical of nonnative amino acid from the group consisting of 2-aminoadipic acid, 2-aminobutyric acid, 2-aminoisobutyric acid, 2,3-diaminopropionic acid, 2,4-diaminobutyric acid, 1,2,3,4-tetrahydroisoquinoline-1-carboxylic acid, 1,2,3,4-tetrahydroisoquinoline-3-carboxylic acid, 2-aminopimelic acid, phenylglycine, 3-(2-thienyl)alanine, 3-(3-thienyl)alanine, sarcosine, 2-(2-thienyl)glycine, 2-aminoheptanoic acid, pipecolic acid, hydroxyllysine, N-methylisoleucine, 6-N-methyllysine, N-methylvaline, norvaline, norleucine, ornithine, alloisoleucine, 4-hydroxyproline, allohydroxylysine, allothreonine, 3-hydroxyproline, 3-(2-naphthyl)alanine, 3-(1-naphthylalanine), homophenylalanine, homocysteine, 2-amino-3-phenylaminoethylpropionic acid, homocysteic acid, homotryptophan, cysteic acid, 3-(2-pyridyl)alanine, 3-(3-pyridyl)alanine, 3-(4-pyridyl)alanine, phosphinothricine, 4-fluorophenylalanine, 3-fluorophenylalanine, 2-fluorophenylalanine, 4-chlorophenylalanine, 4-nitrophenylalanine, 4-aminophenylalanine, cyclohexylalanine, citrulline, 5-fluorotryptophan, 5-methoxytryptophan, 2-amino-3-phenylaminopropionic acid, methionine sulfone, methionine sulfoxide or -NH-NR¹¹-CON(R¹¹)₂,
3. (C₁-C₆)-alkyl, in which alkyl is linear or branched and is mono-, di- or trisubstituted independently of one another by
3.1 aryl, in which aryl is an aromatic carbon radical having 6 to 14 carbon atoms in the ring, from the group consisting of phenyl, 1-naphthyl, 2-naphthyl, 2-biphenylyl, 3-biphenylyl, 4-biphenylyl, anthryl or fluoroenyl, aryl being unsubstituted or substituted by one, two or three identical or different radicals from the group consisting of (C₁-C₈)-alkyl, (C₁-C₈)-alkoxy, halogen, nitro, amino, trifluoromethyl, hydroxyl, hydroxymethyl, 1-hydroxyethyl, 2-hydroxyethyl, methylenedioxy, ethylenedioxy, formyl, acetyl, cyano, hydroxycarbonyl, aminocarbonyl, (C₁-C₄)-alkoxycarbonyl, phenyl, phenoxy, benzyl, benzyloxy or tetrazolyl,
3.2 heteroaryl having 5 to 14 ring members, in which heteroaryl is a radical from the group consisting of pyrrole, furan, thiophen, imidazole, pyrazole, oxazole, isoxazole, thiazole, isothiazole, tetrazole, 1,3,4-oxadiazole, 1,2,3,5-oxathiadiazole-2-oxides, triazolones, oxadiazolones, isoxazolones, oxadiazolidinediones, triazoles, 3-hydroxypyrro-2,4-diones, 5-oxo-1,2,4-thiadiazoles, pyridine, pyrazine, pyrimidine, indole, isoindole, indazole, phthalazine, quinoline, isoquinoline, quinoxaline, quinazoline, cinnoline, -carboline and benzo-fused, cyclopenta-, cyclohexa- or cyclohepta-fused derivatives of these heterocycles, heteroaryl being unsubstituted or substituted by one, two or three identical or different radicals from the group consisting of (C₁-C₈)-alkyl, (C₁-C₈)-alkoxy, halogen, nitro, N(R¹¹)₂, trifluoromethyl, hydroxyl, hydroxymethyl, 1-hydroxyethyl, 2-hydroxyethyl, methylenedioxy, formyl, acetyl, cyano, hydroxycarbonyl, aminocarbonyl, (C₁-C₄)-alkoxycarbonyl, phenyl, phenoxy, benzyl, benzyloxy or tetrazolyl,
3.3 a radical from the group consisting of azetidine, pyrroline, pyrrolidine, piperidine, isothiazole, diazepine, thiomorpholine, morpholine or azepine,
3.4 -O-R¹¹,
3.5 =O,
3.6 halogen,
3.7 -CN,
3.8 -CF₃,
3.9 -S(O)ₓ-R¹¹, in which x is the integer zero, 1 or 2,
3.10 -C(O)-N(R¹¹)₂,
3.11 -N(R¹¹)₂,
3.12 (C₃-C₆)-cycloalkyl,
3.13 a radical of the formula 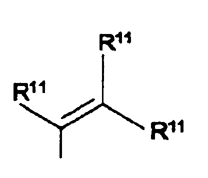 or
3.14 a radical of the formula 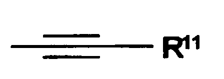
in which
R¹¹ is
a) a hydrogen atom,
b) (C₁-C₆)-alkyl, in which alkyl is unsubstituted or mono-, di- or trisubstituted
1. aryl, in which aryl is as defined above and is unsubstituted or substituted as above,
2. heteroaryl having 5 to 14 ring members in which heteroaryl is as defined above,
3. halogen,
4. -N-(C₁-C₆)ₙ-alkyl, in which n is the integer zero, 1 or 2 and alkyl is unsubstituted or mono-, di- or trisubstituted independently of one another by halogen or by -COOH,
5. -O-(C₁-C₆)-alkyl or
6. -COOH,
c) aryl, in which aryl is as defined above and is unsubstituted or substituted as above,
d) heteroaryl having 5 to 14 ring members, in which heteroaryl is as defined above,
e) halogen or
f) -N(R¹³)₂, in which each
R¹³ is independently
f)a) hydrogen,
f)b) -(C₁-C₆)-alkyl,
f)c) -(C₁-C₄)-alkyl-O-(C₁-C₄)-alkyl,
f)d) -(C₁-C₆)-alkyl-N-(C₁-C₆)ₙ-alkyl, in which n is zero, 1 or 2 and each alkyl is unsubstituted or independently mono-, di- or trisubstituted by halogen or by -COOH,
f) e) halogen, or
f) f) -(C₀-C₄)-alkyl, mono- or disubstituted by phenyl, 1-naphthyl, 2-naphthyl, 2-biphenylyl, 3-biphenylyl, 4-biphenylyl, anthryl, fluorenyl, imidazolyl or morpholinyl, and
in the case of (R¹¹)₂, R¹¹ independently of one another has the meaning of a) to f)
Z is
1. aryl, in which aryl is as defined above and is unsubstituted or substituted as above,
2. heteroaryl having 5 to 14 ring members in which heteroaryl is as defined above and is unsubstituted or substituted as above,
3. heterocycle having 5 to 12 ring members in which the heterocycle is a monocyclic or bicyclic 5-membered to 12-membered heterocyclic ring which is partially saturated or completely saturated and selected from the group consisting of pyrrole, furan, thiophene, imidazole, pyrazole, oxazole, isoxazole, thiazole, isothiazole, tetrazole, 1,3,4-oxadiazole, 1,2,3,5-oxathiadiazole 2-oxide, triazolones, oxadiazolones, isoxazolones, oxadiazolidinediones, triazoles, 3-hydroxypyrro-2,4-diones, 5-oxo-1,2,4-thiadiazoles, pyridine, pyrazine, pyrimidine, indole, isoindole, indazole, phthalazine, quinoline, isoquinoline, quinoxaline, quinazoline, cinnoline, -carboline and benzo-fused, cyclopenta-, cyclohexa- or cyclohepta-fused derivatives of these heterocycles, heterocycle being unsubstituted or substituted by one, two, three or four identical or different radicals from the group consisting of (C₁-C₈)-alkyl, (C₁-C₈)-alkoxy, phenyl-(C₁-C₄)-alkoxy, hydroxyl, oxo, halogen, nitro, amino or trifluoromethyl, it also being possible for the nitrogen heterocycles to be present as N-oxides or as quaternary salts,
4. 1,3,4-oxadiazole, in which 1,3,4-oxadiazole is unsubstituted or monosubstituted by -NH-C(O)-(C₁-C₄)-alkyl, -NH-C(O)-NH-(C₁-C₄)-alkyl, -NH-C(O)-NH-(C₃-C₇)-cycloalkyl, -NH-C(O)-NH-aryl, in which aryl is as defined above, -NH-C(O)-NH-phenyl, -NH-SO₂-aryl, in which aryl is as defined above, or -NH-SO₂-(C₁-C₄)-alkyl,
5. -(C₁-C₆)-alkyl, in which alkyl is linear or branched and is unsubstituted or mono- or disubstituted by phenyl or -OH, or
6. -C(O)-R¹⁰, in which
R¹⁰ is
1. -O-R¹¹,
2. -N(R¹¹)₂,
3. phenyl,
4. pyrimidinyl or
5. morpholinyl, or
R⁸ and R⁹ form, together with the nitrogen atom and carbon atom to which they are each bonded, a heterocyclic ring of the formula IIa 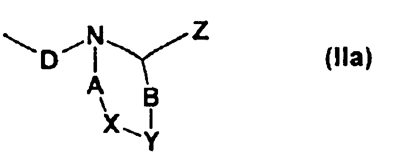 in which D and Z are as defined in formula II,
A is a nitrogen atom or the radical -CH₂-,
B is an oxygen atom, sulfur atom, nitrogen atom or the radical -CH₂-,
X is an oxygen atom, sulfur atom, nitrogen atom or the radical -CH₂-,
Y is absent or is an oxygen atom, sulfur atom, nitrogen atom or the radical -CH₂-, or
X and Y together form a phenyl, 1,2-diazine, 1,3-diazine or a 1,4-diazine radical,
where the ring system formed by N, A, X, Y, B and the carbon atom contains not more than one oxygen atom, X is not an oxygen atom, sulfur atom or nitrogen atom if A is a nitrogen atom, contains not more than one sulfur atom, contains 1, 2, 3 or 4 nitrogen atoms and where an oxygen and sulfur atom do not occur at the same time,
where the ring system formed by N, A, X, Y, B and the carbon atom is unsubstituted or mono-, di- or trisubstituted independently of one another by (C₁-C₈)-alkyl, in which alkyl is unsubstituted or mono- or disubstituted by
1.1. -OH
1.2. (C₁-C₈)-alkoxy,
1.3. halogen,
1.4. -NO₂,
1.5. -NH₂,
1.6. -CF₃,
1.6. -OH,
1.7. methylenedioxy,
1.8. -C(O)-CH₃,
1.9. -CH(O),
1.10. -CN,
1.11. -COOH,
1.12. -C(O)-NH₂,
1.13. (C₁-C₄)-alkoxycarbonyl,
1.14. phenyl,
1.15. phenoxy,
1.16. benzyl,
1.17. benzyloxy or
1.18. tetrazolyl, or
R⁹ and Z form together with the carbon atoms to which they each are bonded a heterocyclic ring of the formula IIc 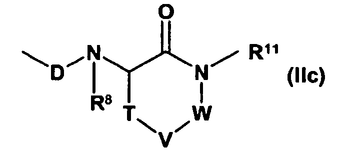 in which D, R⁸ and R¹¹ are as defined in formula II,
T is an oxygen atom, sulfur atom, nitrogen atom or the radical -CH₂-,
W is an oxygen atom, sulfur atom, nitrogen atom or the radical -CH₂-,
V is absent or is an oxygen atom, sulfur atom, nitrogen atom or the radical -CH₂-, or
T and V or V and W together form a phenyl, 1,2-diazine, 1,3-diazine or a 1,4-diazine radical, where the ring system formed by N, T, V, W and two carbon atoms contains not more than one oxygen atom, not more than one sulfur atom and 1, 2, 3 or 4 nitrogen atoms, where an oxygen atom and sulfur atom do not occur at the same time, and where the ring system formed by N, T, V, W and two carbon atoms is unsubstituted or mono-, di- or trisubstituted independently of one another by the substituents defined above under 1.1. to 1.18., and
the other substituents R¹, R², R³ and R⁴ in each case independently of one another are
1. a hydrogen atom,
2. halogen,
3. (C₁-C₄)-alkyl,
4. heteroaryl having 5 to 14 ring members, in which heteroaryl is as defined above and is unsubstituted or substituted as above,
5. a heterocycle having 5 to 12 ring members, in which the heterocycle is as defined above and is unsubstituted or substituted as above,
6. (C₁-C₆)-alkyl,
7. -CN,
8. -O-(C₀-C₄)-alkyl-aryl, in which aryl is as defined above,
9. -O-(C₁-C₄)-alkyl,
10. -OR¹¹,
11. -N(R¹¹)₂,
12. -S(O)ₓR¹¹, in which x is the integer zero, 1 or 2,
13. -NO₂ or
14 . -CF₃,
R⁵ is
1. a hydrogen atom,
2. -OH or
3. =O, and
R⁶ is
1. aryl, in which aryl is as defined above and is unsubstituted or substituted as above,
2. phenyl, mono- or disubstituted by
2.1 -N(R¹¹)₂,
2.2 -NH-C(O)-R¹¹,
2.3 -S(O)ₓ-R¹¹, in which x is the integer zero, 1 or 2,
2.4 -C(O)-R¹¹, or
2.5 -(C₁-C₄)-alkyl-NH₂,
3. heteroaryl having 5 to 14 ring members, in which heteroaryl is as defined above and is unsubstituted or substituted by -N-(C₃-C₆)-cycloalkyl or substituted as above or
4. a heterocycle having 5 to 12 ring members, in which the heterocycle is as defined above and is unsubstituted or substituted by -NH-(C₁-C₆)-alkyl, -NH-phenyl, -NH-(C₃-C₆)-cycloalkyl or is monosubstituted, disubstituted or trisubstituted as above.

2. A compound of the formula I as claimed in claim 1, wherein one of the substituents R₁, R₂, R₃ and R₄ is a radical of the formula II, in which
R⁸ is a hydrogen atom,
R⁹ is
1. a characteristic radical of an amino acid, which is derived from a naturally occurring α-amino acid from the group consisting of histidine, tryptophan, serine, threonine, cysteine, methionine, asparagines, glutamine, lysine, arginine and glutamic acid, or,
2. (C₁-C₆)-alkyl, in which alkyl is linear or branched and is unsubstituted or pyrrole mono- or pyrrole mono- or disubstituted by a radical from the group consisting of pyrrole, pyrrole mono- or disubstituted by-(C₁-C₄)-alkyl, pyrazole, phenyl, imidazole, triazole, thiophene, thiazole, oxazole, isoxazole, pyridine, pyrimidine, indole, benzothiophene, benzimidazole, benzoxazole, benzothiazole, azetidine, pyrroline, pyrrolidine, piperidine, isothiazole, diazepine, thiomorpholine, -CN, morpholine, azepine, 1,3,4-oxadiazole, pyrazine, -N(R¹³)-phenyl, (C₃-C₆)-cycloalkyl, -OR¹¹, -NH(R¹¹), in which R¹¹ is in each case as defined in claim 1, -S(O)ₓ-R¹², in which x is zero, 1 or 2 and R¹² is naphthyl, pyrimidinyl, morpholinyl or phenyl, which are unsubstituted or mono- or disubstituted by -OH, (C₁-C₄)-alkyl, -CF₃, halogen, -O-(C₁-C₄)-alkyl, -COOH, -C(O)-O-(C₁-C₄)-alkyl, -NH₂ or -NH-C(O)- (C₁-C₄)-alkyl
Z is -C (O) -R¹⁰, tetrazole, (C₁-C₆)-alkyl, in which alkyl is linear or branched and is unsubstituted or mono- or disubstituted by phenyl or -OH, or 1,3,4-oxadiazole, in which 1,3,4-oxadiazole is unsubstituted or monosubstituted by -NH₂, -NH(C₁-C₄)-alkyl, -N-[(C₁-C₄)-alkyl] ₂, -NH-C(O)- (C₁-C₄) -alkyl, -NH-C(O)-NH-(C₁-C₄)-alkyl, -NH-C(O)-NH-(C₃-C₇)-cycloalkyl, -NH-C(O)-NH-aryl, in which aryl is chosen from the group consisting of phenyl, 1-naphthyl, 2-naphthyl, 2-biphenylyl, 3-biphenylyl, 4-biphenylyl, anthryl and fluorenyl, -NH-C(O)-NH-phenyl, -NH-SO₂-aryl, in which aryl is as defined above, -NH-SO₂-(C₁-C₄)-alkyl, -OH or -(C₁-C₄)-alkyl, in which
R¹⁰ is -O-R¹¹, phenyl, pyrimidine, -OH, morpholinyl, -N(R¹¹)₂ or -NH₂,
R¹¹ is
1. -(C₁-C₄)-alkyl,
2. R¹³ or
3. -N(R¹³)₂, in which
R¹³ independently of one another is
a) a hydrogen atom,
b) -(C₁-C₆)-alkyl,
c) -(C₁-C₄)-alkyl-O-(C₁-C₄)-alkyl,
d) -(C₁-C₆)-alkyl-N-(C₁-C₆)ₙ-alkyl, in which n is the integer zero, 1 or 2 and alkyl is unsubstituted or mono-, di- or trisubstituted independently of one another by halogen or by -COOH,
e) halogen or
f) -(C₀-C₄)-alkyl, mono- or disubstituted by aryl, in which aryl is as defined above, imidazolyl or morpholinyl, or
R⁸ and R⁹, together with the nitrogen atom and carbon atom to which they are each bonded, form a ring of the formula IIa from the group consisting of pyrrole, pyrroline, pyrrolidine, pyridine, piperidine, piperylene, pyridazine, pyrimidine, pyrazine, piperazine, pyrazole, imidazole, pyrazoline, imidazoline, pyrazolidine, imidazolidine, oxazole, isoxazole, 2-isoxazolidine, isoxazolidine, morpholine, isothiazole, thiazole, tetrazole, 1,2,3,5-oxathiadiazole-2-oxides, oxadiazolones, isoxazolones, triazolones, oxadiazolidinediones, triazoles which are substituted by F, CN, CF₃ or COO-(C₁-C₄)-alkyl, 3-hydroxypyrro-2,4-diones, 5-oxo-1,2,4-thiadiazoles, 1,3,4-oxadiazole, isothiazolidine, thiomorpholine, indazole, thiadiazole, benzimidazole, quinoline, triazole, phthalazine, quinazoline, quinoxaline, purine, pteridine, indole, tetrahydroquinoline, tetrahydroisoquinoline and isoquinoline, or
R⁹ and Z, together with the carbon atoms to which they are each bonded, form a ring of the formula IIc from the group consisting of pyrrole, pyrroline, pyrrolidine, pyridine, piperidine, piperylene, pyridazine, pyrimidine, pyrazine, piperazine, pyrazole, imidazole, pyrazoline, imidazoline, pyrazolidine, imidazolidine, oxazole, isoxazole, 2-isoxazolidine, isoxazolidine, morpholine, isothiazole, thiazole, isothiazolidine, thiomorpholine, indazole, thiadiazole, benzimidazole, quinoline, triazole, phthalazine, quinazoline, quinoxaline, purine, pteridine, indole, tetrahydroquinoline, tetrahydroisoquinoline, isoquinoline, tetrazole, 1,2,3,5-oxathiadiazole-2-oxides, oxadiazolones, isoxazolones, triazolones, oxadiazolidinediones, triazoles which are substituted by F, CN, CF₃ or COO-(C₁-C₄)-alkyl, 3-hydroxypyrrole-2,4-diones, 1,3,4-oxadiazole and 5-oxo-1,2,4-thiadiazoles and the other substituents R¹, R², R³ and R⁴ in each case independently of one another are
1. a hydrogen atom,
2. halogen,
3. (C₁-C₄) -alkyl,
4. -CN,
5. -NO₂,
6. -O-(C₀-C₄)-alkyl-aryl, in which aryl is as defined above,
7. -O-(C₁-C₄)-alkyl,
8. -N-(C₀-C₄)-alkyl-aryl, in which aryl is as defined above,
9. -N-(C₁-C₄)-alkyl or
10. -CF₃,
R⁵ is
1. a hydrogen atom,
2. -OH or
3. =O, and
R⁶ is
1. phenyl, mono- or disubstituted by
1.1 -CN,
1.2 -NO₂,
1.3 -O-(C₁-C₄)-alkyl, or
1.4 -NH₂ or
1.5 -(C₁-C₄)-alkyl-NH₂
2. heteroaryl having 5 to 14 ring members, in which heteroaryl is a radical from the group consisting of pyrrole, furan, thiophen, imidazole, pyrazole, oxazole, isoxazole, thiazole, isothiazole, tetrazole, 1,3,4-oxadiazole, 1,2,3,5-oxathiadiazol-2-oxides, triazolones, oxadiazolones, isoxazolones, oxaxiazolidindiones, triazoles, 3-hydfoxypyrro-2,4-diones, 5-oxo-1,2,4-thiadiazoles, pyridine, pyrazine, pyrimidine, indole, isoindole, indazole, quinoline, isoquinoline, quinoxaline, quinazoline, phthalazine, cinnoline, -carboline and benzo-fused, cyclopenta-, cyclohexa- or cyclohepta-fused derivatives of these heterocycles, and in which heteroaryl is unsubstituted or mono- to trisubstituted by -N-R¹⁴, in which R¹⁴ is -(C₁-C₆)-alkyl, -(C₃-C₆)-cycloalkyl of phenyl, halogen, -OH or -(C₁-C₄)-alkyl, or
3. a heterocycle having 5 to 12 ring members in which the heterocycle is a monocyclic or bicyclic 5-membered to 12-membered heterocyclic ring, which is partially or completely saturated, from the group consisting of pyrrole, furan, thiophen, imidazole, pyrazole, oxazole, isoxazole, thiazole, isothiazole, tetrazole, 1,3,4-oxadiazole, 1,2,3,5-oxathiadiazole-2-oxides, triazolones, oxadiazolones, isoxazolones, oxadiazolidinediones, triazoles, 3-hydroxypyrro-2,4-diones, 5-oxo-1,2,4-thiadiazoles, pyridine, pyrazine, pyrimidine, indole, isoindole, indazole, phthalazine, quinoline, isoquinoline, quinoxaline, quinazoline, cinnoline, -carboline and benzo-fused, cyclopenta-, cyclohexa- or cyclohepta-fused derivatives of these heterocycles, and in which the heterocycle is unsubstituted or mono- to trisubstituted by -N-R¹⁴, in which R¹⁴ is - (C₁-C₆)-alkyl, -(C₁-C₆)-cycloalkyl or phenyl, halogen, -OH or -(C₁-C₄)-alkyl.

3. A process for the preparation of the compounds of the formula I as claimed in one or more of claims 1 and 2, which comprises
a) reacting a compound of the formula IV 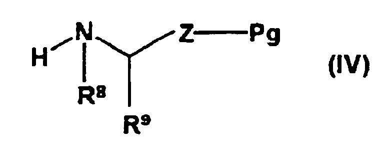 in which Pg is a suitable protective group (e.g. methyl ester), an amide group or a hydroxyl group and Z, R⁸ and R⁹ are as defined in formula II, with an acid chloride or an activated ester of the compound of the formula III 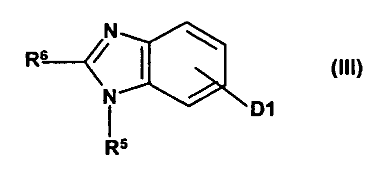 where D1 is -COOH or sulfonylhalogen and R⁵ and R⁶ are as defined in formula I, in the presence of a base or, if appropriate, of a dehydrating agent in solution and, after removal of the protective group, converting into a compound of the formula I, or
b) reacting a compound of the formula IVa 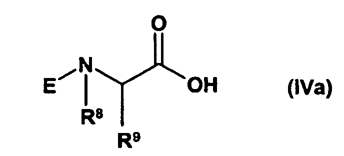 in which R⁸ and R⁹ are as defined in formula II and E is an N-amino protective group, with its carboxyl group coupled via an intermediate chain L to a polymeric resin of the formula PS, a compound of the formula V 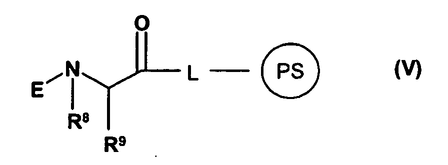 resulting, which, after selective removal of the protective group E, is reacted with a compound of the formula III, where R⁵ and R⁶ are as defined in formula I, in the presence of a base or, if appropriate, of a dehydrating agent to give a compound of the formula VI 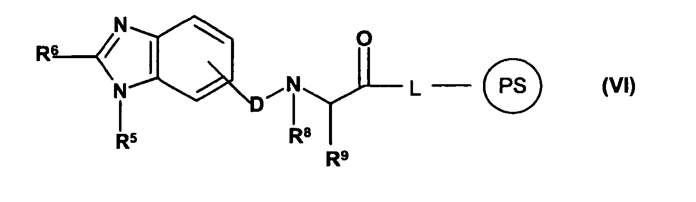 and converting the compound of the formula VI, after removal of the support material, into a compound of the formula I, or
c) reacting a compound of the formula V, after selective removal of the protective group E, with a compound of the formula VII 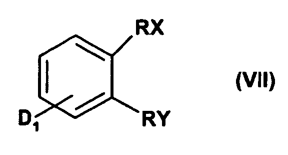 where D₁ is -COOH or sulfonylhalogen and RX is halogen and RY is a radical -NO₂ or -NH-E and E is a protective group, to give a compound of the formula VIII 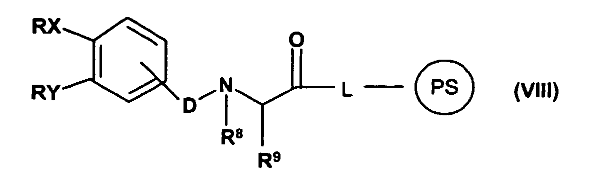 and then reacting the compound of the formula VIII with a compound of the formula IX
NH₂-R⁶ (IX)
in which R⁶ is as defined in the compound of the formula I, to give an intermediate compound of the formula VIa 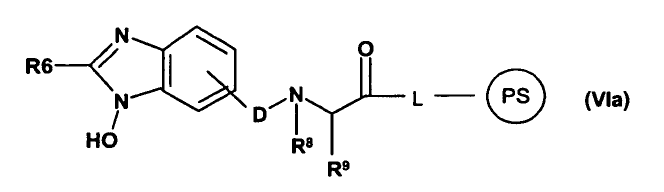 then either converting the intermediate compound of the formula VIa into a compound of the formula I after removal of the support material or reducing it, for example, with tributylphosphine to give a compound of the formula VI and converting into a compound of the formula I after removal of the support material, or
d) converting a compound of the formula I into a physiologically tolerable salt.

4. A pharmaceutical comprising an efficacious amount of at least one compound of the formula I as claimed in one or more of claims 1 and 2 together with a pharmaceutically suitable and physiologically tolerable excipient, additive and/or other active compounds and auxiliaries.

5. The use of at least one compound of the formula I 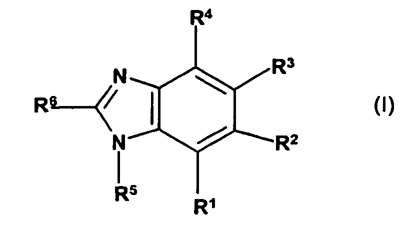 and/or a stereoisomeric form of the compounds of the formula I and/or a physiologically tolerable salt of the compounds of the formula I, for the production of pharmaceuticals for the prophylaxis and therapy of disorders from among asthma, rheumatoid arthritis, osteoarthritis, Alzheimer's disease, carcinomatous disorders, cardiac infarct, cardiac insufficiency, acute coronary syndrome, instable angina pectoris, septic shock, acute and chronic renal failure, stroke or atherosclerosis, where
one of the substituents R¹, R², R³ and R⁴ is a radical of the formula II 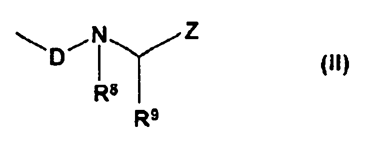 in which
D is -C(O)-, -S(O)- or -S(O)₂-,
R⁸ is hydrogen or (C₁-C₄)-alkyl,
R⁹ is
1. a characteristic radical of an amino acid, a native α-amino acid from the group consisting of glycine, alanine, valine, leucine, isoleucine, phenylalanine, tyrosine, tryptophan, serine, threonine, cysteine, methionine, asparagine, glutamine, lysine, histidine, arginine and glutamic acid, or
of a nonnative amino acid from the group consisting of 2-aminoadipic acid, 2-aminobutyric acid, 2-aminoisobutyric acid, 2,3-diaminopropionic acid, 2,4-diaminobutyric acid, 1,2,3,4-tetrahydroisoquinoline-1-carboxylic acid, 1,2,3,4-tetrahydroisoquinoline-3-carboxylic acid, 2-aminopimelic acid, phenylglycine, 3-(2-thienyl)alanine, 3-(3-thienyl)alanine, sarcosine, 2-(2-thienyl)glycine, 2-aminoheptanoic acid, pipecolic acid, hydroxyllysine, N-methylisoleucine, 6-N-methyllysine, N-methylvaline, norvaline, norleucine, ornithine, alloisoleucine, 4-hydroxyproline, allohydroxylysine, allothreonine, 3-hydroxyproline, 3-(2-naphthyl)alanine, 3-(1-naphthy1-alanine), homophenylalanine, homocysteine, 2-amino-3-phenylaminoethylpropionic acid, homocysteic acid, homotryptophan, cysteic acid, 3-(2-pyridyl)alanine, 3-(3-pyridyl)-alanine, 3-(4-pyridyl)alanine, phosphinothricine, 4-fluorophenylalanine, 3-fluorophenylalanine, 2-fluorophenylalanine, 4-chlorophenylalanine, 4-nitrophenylalanine, 4-aminophenylalanine, cyclohexylalanine, citrulline, 5-fluorotryptophan, 5-methoxytryptophan, 2-amino-3-phenylaminopropionic acid, methionine sulfone, methionine sulfoxide or -NH-NR¹¹-CON(R¹¹)₂,
2. aryl, in which aryl is an aromatic carbon radical having 6 to 14 carbon atoms in the ring from the group consisting of 1-naphthyl, 2-naphthyl, 2-biphenylyl, 3-biphenylyl, 4-biphenylyl, anthryl and fluorenyl,
aryl being unsubstituted or substituted by one, two or three identical or different radicals from the group consisting of (C₁-C₈)-alkyl, (C₁-C₈)-alkoxy, halogen, nitro, amino, trifluoromethyl, hydroxyl, hydroxymethyl, 1-hydroxyethyl, 2-hydroxyethyl, methylenedioxy, ethylenedioxy, formyl, acetyl, cyano, hydroxycarbonyl, aminocarbonyl, (C₁-C₄)-alkoxycarbonyl, phenyl, phenoxy, benzyl, benzyloxy or tetrazoyl,
3. heteroaryl having 5 to 14 ring members, in which heteroaryl is a radical from the group consisting of pyrrole, furan, thiophen, imidazole, pyrazole, oxazole, isoxazole, thiazole, isothiazole, tetrazole, 1,3,4-oxadiazole, 1,2,3,5-oxathiadiazole-2-oxides, triazolones, oxadiazolones, isoxazolones, oxadiazolidinediones, triazoles, 3-hydroxypyrro-2,4-diones, 5-oxo-1,2,4-thiadiazoles, pyridine, pyrazine, pyrimidine, indole, isoindole, indazole, phthalazine, quinoline, isoquinoline, quinoxaline, quinazoline, cinnoline, -carboline and benzo-fused, cyclopenta-, cyclohexa- or cyclohepta-fused derivatives of these heterocycles,
heteroaryl being unsubstituted or substituted by one, two or three identical or different radicals from the group consisting of (C₁-C₈)-alkyl, (C₁-C₈)-alkoxy, halogen, nitro, -N(R¹¹)₂, trifluoromethyl, hydroxyl, hydroxymethyl, 1-hydroxyethyl, 2-hydroxyethyl, methylenedioxy, formyl, acetyl, cyano, hydroxycarbonyl, aminocarbonyl, (C₁-C₄)-alkoxycarbonyl, phenyl, phenoxy, benzyl, benzyloxy or tetrazolyl,
4. heterocycle having 5 to 12 ring members, in which heterocycle is a monocyclic or bicyclic 5-membered to 12-membered heterocyclic ring which is partially saturated or completely saturated and selected from the group consisting of pyrrole, furan, thiophen, imidazole, pyrazole, oxazole, isoxazole, thiazole, isothiazole, tetrazole, 1,3,4-oxadiazole, 1,2,3,5-oxathiadiazole-2-oxides,
triazolones, oxadiazolones, isoxazolones, oxadiazolidinediones, triazoles, 3-hydroxypyrro-2,4-diones, 5-oxo-1,2,4-thiadiazoles, pyridine, pyrazine, pyrimidine, indole, isoindole, indazole, phthalazine, quinoline, isoquinoline, quinoxaline, quinazoline, cinnoline, -carboline and benzo-fused, cyclopenta-, cyclohexa- or cyclohepta-fused derivatives of these heterocycles,
heterocycle being unsubstituted or substituted by one, two, three or four identical or different radicals from the group consisting of (C₁-C₈)-alkyl, (C₁-C₈)-alkoxy, phenyl-(C₁-C₄)-alkoxy, hydroxyl, oxo, halogen, nitro, amino or trifluoromethyl, it also being possible for the nitrogen heterocycles to be present as N-oxides or as quaternary salts,
5. (C₁-C₆)-alkyl, in which alkyl is linear or branched and is unsubstituted or mono-, di- or trisubstituted independently of one another by
5.1 aryl, in which aryl is as defined above and is unsubstituted or substituted as above,
5.2 heteroaryl having 5 to 14 ring members, in which heteroaryl is as defined above and is unsubstituted or substituted as above,
5.3 a heterocycle having 5 to 12 ring members, in which the heterocycle is as defined above and is unsubstituted or substituted as above,
5.4 a radical from the group consisting of azetidine, pyrroline, piperidine, isothiazole, diazepin, thiomorpholine, morpholine or azepine,
5.5 -O-R¹¹,
5.6 =O,
5.7 halogen,
5.8 -CN,
5.9 -CF₃,
5.10 -S(O)ₓ-R¹¹, in which x is the integer zero, 1 or 2,
5.12 -C(O)-N(R¹¹)₂,
5.13 -N (R¹¹)₂,
5.14 (C₃-C₆)-cycloalkyl,
5.15 a radical of the formula 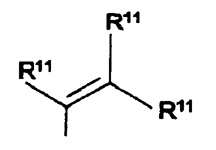 or
5.16 a radical of the formula 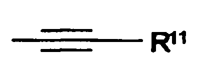
in which
R¹¹ is
a) a hydrogen atom,
b) (C₁-C₆)-alkyl, in which alkyl is unsubstituted or mono-, di- or trisubstituted by
1. aryl, in which aryl is as defined above and is unsubstituted or substituted as above,
2. heteroaryl having 5 to 14 ring members in which heteroaryl is as defined above,
3. halogen,
4. -N-(C₁-C₆)ₙ-alkyl, in which n is the integer zero, 1 or 2 and alkyl is unsubstituted or mono-, di- or trisubstituted independently of one another by halogen or by -COOH,
5. -O-(C₁-C₆)-alkyl or
6. -COOH,
c) aryl, in which aryl is as defined above and is unsubstituted or substituted as above,
d) heteroaryl having 5 to 14 ring members in which heteroaryl is as defined above,
e) halogen or
f) -N(R¹³)₂, in which each
R¹³ is independently
f)a) hydrogen,
f)b) -(C₁-C₆)-alkyl,
f)c) -(C₁-C₄)-alkyl-O-(C₁-C₄)-alkyl,
f)d) -(C₁-C₆)-alkyl-N-(C₁-C₆)ₙ-alkyl, in which n is zero, 1 or 2 and each alkyl is unsubstituted or independently mono-, di- or trisubstituted by halogen or by -COOH,
f)e) halogen, or
f)f) -(C₀-C₄)-alkyl, mono- or disubstituted by phenyl, 1-naphthyl, 2-naphthyl, 2-biphenylyl, 3-biphenylyl, 4-biphenylyl, anthryl, fluorenyl, imidazolyl or morpholinyl, and
in the case of (R¹¹)₂, R¹¹ independently of one another has the meaning of a) to f)
Z is
1. aryl, in which aryl is as defined above and is unsubstituted or substituted as above,
2. heteroaryl having 5 to 14 ring members in which heteroaryl is as defined above and is unsubstituted or substituted as above,
3. heterocycle having 5 to 12 ring members in which heterocycle is as defined above and is unsubstituted or substituted as above,
4. 1,3,4-oxadiazole, in which 1,3,4-oxadiazole is unsubstituted or monosubstituted by -NH-C(O)-(C₁-C₄)-alkyl, -NH-C(O)-NH-(C₁-C₄)-alkyl, -NH-C(O)-NH-(C₃-C₇)-cycloalkyl, -NH-C(O)-NH-aryl, in which aryl is as defined above, -NH-C(O)-NH-phenyl, -NH-SO₂-aryl, in which aryl is as defined above, or -NH-SO₂-(C₁-C₄)-alkyl,
5. -(C₁-C₆)-alkyl, in which alkyl is linear or branched and is unsubstituted or mono- or disubstituted by phenyl or -OH, or
6. -C(O)-R¹⁰, in which
R¹⁰ is
1. -O-R¹¹
2. -N(R¹¹)₂,
3.phenyl,
4. pyrimidinyl or
5. morpholinyl, or
R⁸ and R⁹ form, together with the nitrogen atom and carbon atom to which they are each bonded, a heterocyclic ring of the formula IIa 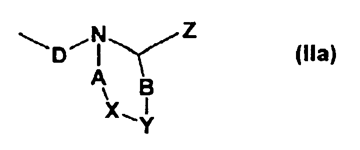 in which D and Z are as defined in formula II,
A is a nitrogen atom or the radical -CH₂-,
B is an oxygen atom, sulfur atom, nitrogen atom or the radical -CH₂-,
X is an oxygen atom, sulfur atom, nitrogen atom or the radical -CH₂-,
Y is absent or is an oxygen atom, sulfur atom, nitrogen atom or the radical -CH₂-, or
X and Y together form a phenyl, 1,2-diazine, 1,3-diazine or a 1,4-diazine radical,
where the ring system formed by N, A, X, Y, B and the carbon atom contains not more than one oxygen atom, X is not an oxygen atom, sulfur atom or nitrogen atom if A is a nitrogen atom, contains not more than one sulfur atom, contains 1, 2, 3 or 4 nitrogen atoms and where an oxygen atom and sulfur atom do not occur at the same time,
where the ring system formed by N, A, X, Y, B and the carbon atom is unsubstituted or mono-, di- or trisubstituted independently of one another by (C₁-C₈)-alkyl, in which alkyl is unsubstituted or mono- or disubstituted by
1.1. -OH
1.2. (C₁-C₈)-alkoxy,
1.3. halogen,
1.4. -NO₂,
1.5. -NH₂,
1.6. -CF₃,
1.6. -OH,
1.7. methylenedioxy,
1.8. -C(O)-CH₃,
1.9. -CH(O),
1.10. -CN,
1.11. -COOH,
1.12. -C(O)-NH₂,
1.13. (C₁-C₄)-alkoxycarbonyl,
1.14. phenyl,
1.15. phenoxy,
1.16. benzyl,
1.17. benzyloxy or
1.18. tetrazolyl, or
R⁹ and Z together with the carbon atoms to which they each are bonded form a heterocyclic ring of the formula IIc 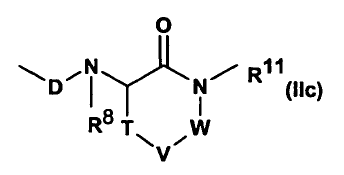 in which
D, R⁸ and R¹¹ are as defined in formula II,
T is an oxygen atom, sulfur atom, nitrogen atom or the radical -CH₂-,
W is an oxygen atom, sulfur atom, nitrogen atom or the radical -CH₂-,
V is absent or is an oxygen atom, sulfur atom, nitrogen atom or the radical -CH₂-, or
T and V or V and W together form a phenyl, 1,2-diazine, 1,3-diazine or a 1,4-diazine radical,
where the ring system formed by N, T, V, W and two carbon atoms contains not more than one oxygen atom, not more than one sulfur atom and 1, 2, 3 or 4 nitrogen atoms, where an oxygen atom and sulfur atom do not occur at the same time, and where the ring system formed by N, T, V, W and two carbon atoms is unsubstituted or mono-, di- or trisubstituted independently of one another by the substituents defined above under 1.1. to 1.18., and
the other substituents R¹, R², R³ and R⁴ in each case independently of one another are
1. a hydrogen atom,
2. halogen,
3. (C₁-C₄)-alkyl
4. a heteroaryl having 5 to 14 ring members, in which heteroaryl is as defined above and is unsubstituted or substituted as above,
5. a heterocycle having 5 to 12 ring members, in which the heterocycle is as defined above and is unsubstituted or substituted as above, or
6. (C₁-C₆)-alkyl,
7. -CN,
8. -O-(C₀-C₄)-alkyl-aryl, in which aryl is as defined above,
9. -O-(C₁-C₄)-alkyl,
10. -OR¹¹',
11. -N(R¹¹)₂,
12. -S(O)ₓ-R¹¹, in which x is the integer zero, 1 or 2,
13. NO₂ or
14. -CF₃,
R⁵ is
1. a hydrogen atom,
2. -OH or
3. =O, and
R⁶ is
1. aryl, in which aryl is as defined above and is unsubstituted or substituted as above,
2. phenyl, mono- or disubstituted by
2.1 -N(R¹¹)₂,
2.2 -NH-C(O)-R¹¹,
2.3 -S(O)ₓ-R¹¹, in which x is the integer zero, 1 or 2,
2.4 -C(O)-R¹¹ or
2.5 -(C₁-C₄)-alkyl-NH₂,
3. heteroaryl having 5 to 14 ring members, in which heteroaryl is as defined above and is unsubstituted or substituted by -N-(C₃-C₆)-cycloalkyl or substituted as above or
4. a heterocycle having 5 to 12 ring members, in which the heterocycle is as defined above and is unsubstituted or substituted by -NH-(C₁-C₆)-alkyl, -NH-phenyl, -NH-(C₃-C₆)-cycloalkyl or mono-, di- or trisubstituted as above.

6. The use as claimed in claim 5, wherein one of the substituents R¹, R², R³ and R⁴ is a radical of the formula II in which
R⁸ is a hydrogen atom,
R⁹ is
1. a characteristic radical of a native α-amino acid from the group consisting of histidine, tryptophan, serine, threonine, cysteine, methionine, asparagine, glutamine, lysine, arginine and glutamic acid, or
2. (C₁-C₆)-alkyl, in which alkyl is linear or branched and is unsubstituted or mono- or disubstituted by a radical from the group consisting of
pyrrole, pyrrole mono- or disubstituted by -(C₁-C₄)-alkyl, pyrazole, phenyl, imidazole, triazole, thiophene, thiazole, oxazole, isoxazole, pyridine, pyrimidine, indole, benzothiophene, benzimidazole, benzoxazole, benzothiazole, azetidine, pyrroline, pyrrolidine, piperidine, isothiazole, diazepine, thiomorpholine, -CN, morpholine, azepine, 1,3,4-oxadiazole, pyrazine, -N(R¹³)-phenyl, (C₃-C₆)-cycloalkyl, -OR¹¹, -NH(R¹¹), in which R¹¹ is in each case as defined in claim 1 -S(O)ₓ-R¹², in which x is zero, 1 or 2 and R¹² is naphthyl, pyrimidinyl, morpholinyl or phenyl, which are unsubstituted or mono- or disubstituted by -OH, (C₁-C₄)-alkyl, -CF₃, halogen, -O-(C₁-C₄)-alkyl, -COOH, -C(O)-O-(C₁-C₄)-alkyl, -NH₂ or -NH-C(O)-(C₁-C₄)-alkyl,
Z is -C(O)-R¹⁰, tetrazole, (C₁-C₆)-alkyl, in which alkyl is linear or branched and is unsubstituted or mono- or disubstituted by phenyl or -OH, or 1,3,4-oxadiazole, in which 1,3,4-oxadiazole is unsubstituted or monosubstituted by
-NH₂, -NH(C₁-C₄)-alkyl, -N-[(C₁-C₄)-alkyl]₂, -NH-C(O)- (C₁-C₄) -alkyl, -NH-C(O)-NH-(C₁-C₄)-alkyl, -NH-C(O)-NH-(C₃-C₇)-cycloalkyl, -NH-C(O)-NH-aryl, in which aryl is chosen from the group consisting of phenyl, 1-naphthyl, 2-naphthyl, 2-biphenylyl, 3-biphenylyl, 4-biphenylyl, anthryl and fluorenyl, -NH-C(O)-NH-phenyl, -NH-SO₂-aryl, in which aryl is as defined above, -NH-SO₂-(C₁-C₄)-alkyl, -OH or -(C₁-C₄) -alkyl, in which
R¹⁰ is -O-R¹¹, phenyl, pyrimidine, -OH, morpholinyl, -N(R¹¹)₂ or -NH₂,
R¹¹ is
1. -(C₁-C₄)-alkyl,
2. R¹³ or
3. -N(R¹³)₂, in which R¹³ independently of one another is
a) a hydrogen atom,
b) -(C₁-C₆)-alkyl,
c) -(C₁-C₄)-alkyl-O-(C₁-C₄)-alkyl,
d) -(C₁-C₆)-alkyl-N-(C₁-C₆)ₙ-alkyl, in which n is the integer zero, 1 or 2 and alkyl is unsubstituted or mono-, di-, or trisubstituted independently of each other by halogen or by -COOH,
e) halogen or
f) - (C₀-C₄)-alkyl, mono- or disubstituted by aryl, in which aryl is as defined above, imidazolyl or morpholinyl, or
R⁸ and R⁹ together with the nitrogen atom and carbon atom to which they are each bonded, form a ring of the formula IIa from the group consisting of pyrrole, pyrroline, pyrrolidine, pyridine, piperidine, piperylene, pyridazine, pyrimidine, pyrazine, piperazine, pyrazole, imidazole, pyrazoline, imidazoline, pyrazolidine, imidazolidine, oxazole, isoxazole, 2-isoxazolidine, isoxazolidine, morpholine, isothiazole, thiazole, tetrazole, 1,2,3,5-oxathiadiazole-2-oxides, oxadiazolones, isoxazolones, triazolones, oxadiazolidinediones, triazoles which are substituted by F, CN, CF₃ or COO-(C₁-C₄)-alkyl, 3-hydroxypyrro-2,4-diones, 5-oxo-1,2,4-thiadiazoles, 1,3,4-oxadiazole, isothiazolidine, thiomorpholine, indazole, thiadiazole, benzimidazole, quinoline, triazole, phthalazine, quinazoline, quinoxaline, purine, pteridine, indole, tetrahydroquinoline, tetrahydroisoquinoline and isoquinoline, or
R⁹ and Z, together with the carbon atoms to which they are each bonded, form a ring of the formula IIc from the group consisting of pyrrole, pyrroline, pyrrolidine, pyridine, piperidine, piperylene, pyridazine, pyrimidine, pyrazine, piperazine, pyrazole, imidazole, pyrazoline, imidazoline, pyrazolidine, imidazolidine, oxazole, isoxazole, 2-isoxazolidine, isoxazolidine, morpholine, isothiazole, thiazole, isothiazolidine, thiomorpholine, indazole, thiadiazole, benzimidazole, quinoline, triazole, phthalazine, quinazoline, quinoxaline, purine, pteridine, indole, tetrahydroquinoline, tetrahydroisoquinoline, isoquinoline, tetrazole, 1,2,3,5-oxathiadiazole-2-oxides, oxadiazolones, isoxazolones, triazolones, oxadiazolidinediones, triazoles which are substituted by F, CN, CF₃ or COO-(C₁-C₄)-alkyl, 3-hydroxypyrrole-2,4-diones, 1,3,4-oxadiazole and 5-oxo-1,2,4-thiadiazoles and
the other substituents R¹, R², R³ and R⁴ in each case independently of one another are
1. a hydrogen atom,
2. halogen,
3. (C₁-C₄)-alkyl,
4. -CN,
5. -NO₂,
6. -O-(C₀-C₄)-alkyl-aryl, in which aryl is as defined above,
7. -O-(C₁-C₄)-alkyl,
8. -N-(C₀-C₄)-alkyl-aryl, in which aryl is as defined above,
9. -N-(C₁-C₄)-alkyl or
10. -CF₃,
R⁵ is
1. a hydrogen atom,
2. -OH or
3. =O, and
R⁶ is
1. phenyl, mono- or disubstituted by
1.1 -CN,
1.2 -NO₂,
1.3 -O- (C₁-C₄) -alkyl,
1.4 -NH₂ or
1.5 -(C₁-C₄)-alkyl-NH₂
2. heteroaryl having 5 to 14 ring members, in which heteroaryl is a radical from the group consisting of pyrrole, furan, thiophen, imidazole, pyrazole, oxazole, isoxazole, thiazole, isothiazole, tetrazole, 1,3,4-oxadiazole, 1,2,3,5-oxathiadiazole-2-oxides, triazolones, oxadiazolones, isoxazolones, oxadiazolidinediones, triazoles, 3-hydroxypyrro-2,4-diones, 5-oxo-1,2,4-thiadiazoles, pyridine, pyrazine, pyrimidine, indole, isoindole, indazole, phthalazine, quinoline, isoquinoline, quinoxaline, quinazoline, cinnoline, -carboline and benzo-fused, cyclopenta-, cyclohexa- or cyclohepta-fused derivatives of these heterocycles, and in which heteroaryl is unsubstituted or mono- to trisubstituted by N-R¹⁴, in which R¹⁴ is -(C₁-C₆)-alkyl, -(C₃-C₆)-cycloalkyl or phenyl, halogen, -OH or -(C₁-C₄)-alkyl, or
3. a heterocycle having 5 to 12 ring members in which the heterocycle is a monocyclic or bicyclic 5-membered to 12-membered heterocyclic ring, which is partially saturated or completely saturated, from the group consisting of pyrrole, furan, thiophen, imidazole, pyrazole, oxazole, isoxazole, thiazole, isothiazole, tetrazole, 1,3,4-oxadiazole, 1,2,3,5-oxathiadiazole-2-oxides, triazolones, oxadiazolones, isoxazolones, oxadiazolidinediones, triazoles, 3-hydroxypyrro-2,4-diones, 5-oxo-1,2,4-thiadiazoles, pyridine, pyrazine, pyrimidine, indole, isoindole, indazole, phthalazine, quinoline, isoquinoline, quinoxaline, quinazoline, cinnoline, -carboline and benzo-fused, cyclopenta-, cyclohexa- or cyclohepta-fused derivatives of these heterocycles, and in which the heterocycle is unsubstituted or mono- to trisubstituted by -N-R¹⁴, in which R¹⁴ is -(C₁-C₆)-alkyl, -(C₃-C₆)-cycloalkyl or phenyl, halogen, -OH or (C₁-C₄)-alkyl.

7. A process for the production of a pharmaceutical, which comprises bringing at least one compound of the formula I as claimed in one or more of claims 1 and 2 into a suitable administration form using a pharmaceutically suitable and physiologically tolerable excipient and, if appropriate, further suitable active compounds, additives or auxiliaries.

## Revendications

1. Composé de formule I 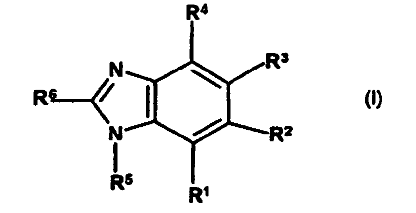 et/ou une forme stéréo-isomère du composé de formule I et/ou un sel physiologiquement acceptable du composé de formule I, un des substituants R¹, R², R³ et R⁴ représentant un radical de formule II, 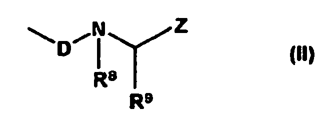 dans laquelle
D représente -C(O)-, -S(O)- ou -S(O)₂-,
R⁸ représente un atome d'hydrogène ou alkyle en C₁ à C₄,
R⁹ représente
1. un radical caractéristique d'un acide aminé, dérivé d'un acide α-aminé existant à l'état naturel du groupe constitué par l'alanine, la valine, la leucine, l'isoleucine, la phénylalanine, la tyrosine, le tryptophane, la sérine, la thréonine, la cystéine, la méthionine, l'asparagine, la glutamine, la lysine, l'histidine, l'arginine et l'acide glutamique,
2. un radical caractéristique d'un acide aminé, qui est dérivé d'un acide aminé qui n'existe pas à l'état naturel du groupe constitué par l'acide 2-aminoadipique, l'acide 2-aminobutyrique, l'acide 2-aminoisobutyrique, l'acide 2,3-diaminopropionique, l'acide 2,4-diaminobutyrique, l'acide 1,2,3,4-tétrahydroisoquinoléine-1-carboxylique, l'acide 1,2,3,4-tétrahydroisoquinoléine-3-carboxylique, l'acide 2-aminopimélique, la phénylglycine, la 3-(2-thiényl)-alanine, la 3-(3-thiényl)-alanine, la sarcosine, la 2-(2-thiényl)-glycine, l'acide 2-aminoheptanoïque, l'acide pipécolique, l'hydroxylysine, la N-méthylisoleucine, la 6-N-méthyllysine, la N-méthylvaline, la norvaline, la norleucine, l'ornithine, l'allo-isoleucine, la 4-hydroxyproline, l'allohydroxylysine, l'allo-thréonine, la 3-hydroxyproline, la 3-(2-naphtylalanine), la 3-(1-naphtylalanine), l'homophénylalanine, l'homocystéine, l'acide 2-amino-3-phénylaminoéthylpropionique, l'acide homocystéique, l'homotryptophane, l'acide cystéique, la 3-(2-pyridyl)-alanine, la 3-(3-pyridyl)-alanine, la 3-(4-pyridyl)-alanine, la phosphinothricine, la 4-fluorophénylalanine, la 3-fluorophénylalanine, la 2-fluorophénylalanine, la 4-chlorophénylalanine, la 4-nitrophénylalanine, la 4-aminophénylalanine, la cyclohexylalanine, la citrulline, le 5-fluorotryptophane, le 5-méthoxytryptophane, l'acide 2-amino-3-phénylaminopropionique, la méthionine-sulfone, le méthioninesulfoxyde ou -NH-NR¹¹-CON(R¹¹)₂,
3. alkyle en C₁ à C₆, où alkyle est linéaire ou ramifié et monosubstitué, disubstitué ou trisubstitué, indépendamment l'un de l'autre, par
3.1 aryle, où aryle représente un radical carboné aromatique comprenant 6 à 14 atomes de carbone dans le cycle du groupe constitué par phényle, 1-naphtyle, 2-naphtyle, 2-biphénylyle, 3-biphénylyle, 4-biphénylyle, anthryle ou fluorényle,
où aryle est non substitué ou monosubstitué, disubstitué ou trisubstitué par des radicaux identiques ou différents de la série constituée par alkyle en C₁ à C₈, alcoxy en C₁ à C₈, halogène, nitro, amino, trifluorométhyle, hydroxy, hydroxyméthyle, 1-hydroxyéthyle, 2-hydroxyéthyle, méthylènedioxy, éthylènedioxy, formyle, acétyle, cyano, hydroxycarbonyle, aminocarbonyle, (alcoxy en C₁ à C₄)-carbonyle, phényle, phénoxy, benzyle, benzyloxy ou tétrazolyle,
3.2 hétéroaryle comprenant 5 à 14 éléments de cycle, où hétéroaryle représente un radical du groupe constitué par le pyrrole, le furanne, le thiophène, l'imidazole, le pyrazole, l'oxazole, l'isoxazole, le thiazole, l'isothiazole, le tétrazole, le 1,3,4-oxadiazole, les 2-oxydes de 1,2,3,5-oxathiadiazole, les triazolones, les oxadiazolones, les isoxazolones, les oxadiazolidinediones, les triazoles, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoléine, l'isoquinoléine, la quinoxaline, la quinazoline, la cinnoline, la carboline et les dérivés annelés en benzène, cyclopenta, cyclohexa ou cyclohepta de ces hétérocycles,
où hétéroaryle est non substitué ou monosubstitué, disubstitué ou trisubstitué par des radicaux identiques ou différents de la série alkyle en C₁ à C₈, alcoxy en C₁ à C₈, halogène, nitro, -N(R¹¹)₂, trifluorométhyle, hydroxy, hydroxyméthyle, 1-hydroxyéthyle, 2-hydroxyéthyle, méthylènedioxy, formyle, acétyle, cyano, hydroxycarbonyle, aminocarbonyle, (alcoxy en C₁ à C₄)carbonyle, phényle, phénoxy, benzyle, benzyloxy ou tétrazolyle,
3.3 un radical du groupe constitué par l'azétidine, la pyrroline, la pyrrolidine, la pipéridine, l'isothiazole, la diazépine, la thiomorpholine, la morpholine ou l'azépine,
3.4 -O-R¹¹,
3.5 =O,
3.6 halogène,
3.7 -CN,
3.8 -CF₃,
3.9 -S(O)ₓ-R¹¹, dans laquelle x vaut le nombre entier zéro, 1 ou 2,
3.10 -C(O)-N (R¹¹)₂,
3.11 -N(R¹¹)₂,
3.12 cycloalkyle en C₃ à C₆,
3.13 un radical de formule 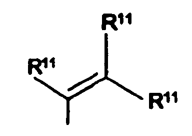 ou
3.14 un radical de formule 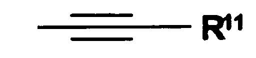
dans lesquelles
R¹¹ représente
a) un atome d'hydrogène,
b) alkyle en C₁ à C₆, où alkyle est non substitué ou monosubstitué, disubstitué ou trisubstitué par
1. aryle, où aryle est défini comme ci-dessus et est non substitué ou substitué comme ci-dessus,
2. hétéroaryle comprenant 5 à 14 éléments de cycle, où hétéroaryle est défini comme ci-dessus,
3. halogène,
4. -N-(alkyle en C₁ à C₆)ₙ, où n signifie le nombre entier zéro, 1 ou 2 et alkyle est non substitué ou monosubstitué, disubstitué ou trisubstitué, indépendamment l'un de l'autre, par halogène ou par -COOH,
5. -O-(alkyle en C₁ à C₆) ou
6. -COOH,
c) aryle, où aryle est défini comme ci-dessus et est non substitué ou substitué comme ci-dessus,
d) hétéroaryle comprenant 5 à 14 éléments de cycle, où hétéroaryle est défini comme ci-dessus,
e) halogène ou
f) -N(R¹³)₂, où
R¹³ représente, indépendamment l'un de l'autre
f) a) un atome d'hydrogène,
f) b) -alkyle en C₁ à C₆,
f) c) -(alkyle en C₁ à C₄)-O-(alkyle en C₁ à C₄),
f) d) -(alkyle en C₁ à C₆)-N-(alkyle en C₁ à C₆)ₙ, où n signifie le nombre entier zéro, 1 ou 2 et alkyle et peut être non substitué ou monosubstitué, disubstitué ou trisubstitué, indépendamment l'un de l'autre, par halogène ou par -COOH,
f) e) halogène ou
f) f) -alkyle en C₀ à C₄, monosubstitué ou disubstitué par phényle, 1-naphtyle, 2-naphtyle, 2-biphénylyle, 3-biphénylyle, 4-biphénylyle, anthryle, fluorényle, imidazolyle ou morpholinyle, et
dans le cas de (R¹¹)₂, R¹¹ présente, indépendamment l'un de l'autre, la signification de a) à f)
Z représente
1. aryle, où aryle est défini comme ci-dessus et est non substitué ou substitué comme ci-dessus,
2. hétéroaryle comprenant 5 à 14 éléments de cycle, où hétéroaryle est défini comme ci-dessus et est non substitué ou substitué comme ci-dessus,
3. hétérocycle comprenant 5 à 12 éléments de cycle, où hétérocycle représente un hétérocycle monocyclique ou bicyclique de 5 à 12 éléments, qui est partiellement ou complètement saturé du groupe constitué par le pyrrole, le furanne, le thiophène, l'imidazole, le pyrazole, l'oxazole, l'isoxazole, le thiazole, l'isothiazole, le tétrazole, le 1,3,4-oxadiazole, les 2-oxydes de 1,2,3,5-oxathiadiazole, les triazolones, les oxadiazolones, les isoxazolones, les oxadiazolidinediones, les triazoles, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoléine, l'isoquinoléine, la quinoxaline, la quinazoline, la cinnoline, la carboline et les dérivés annelés en benzène, cyclopenta, cyclohexa ou cyclohepta de ces hétérocycles,
où l'hétérocycle est non substitué ou monosubstitué, disubstitué, trisubstitué ou tétrasubstitué par des radicaux identiques ou différents de la série constituée par alkyle en C₁ à C₈, alcoxy en C₁ à C₈, phényl(alcoxy en C₁ à C₄), hydroxy, oxo, halogène, nitro, amino ou trifluorométhyle, les hétérocycles azotés pouvant également se trouver sous forme de N-oxydes ou de sels quaternaires,
4. 1,3,4-oxadiazole, où 1,3,4-oxadiazole est non substitué ou monosubstitué par -NH-C(O)-(alkyle en C₁ à C₄), -NH-C(O)-NH- (alkyle en C₁ à C₄), -NH-C (O)-NH-(cycloalkyle en C₃ à C₇), -NH-C(O)-NH-aryle, où aryle est défini comme ci-dessus, -NH-C(O)-NH-phényle, -NH-SO₂-aryle, où aryle est défini comme ci-dessus, ou -NH-SO₂-(alkyle en C₁ à C₄),
5. -alkyle en C₁ à C₆, où alkyle est linéaire ou ramifié et est non substitué ou monosubstitué ou disubstitué par phényle ou -OH, ou
6. -C(O)-R¹⁰, où
R¹⁰ représente
1. -O-R¹¹,
2. -N(R¹¹)₂,
3. phényle,
4. pyrimidinyle ou
5. morpholinyle, ou
R⁸ et R⁹ forment ensemble avec l'atome d'azote et l'atome de carbone auxquels ils sont à chaque fois liés, un hétérocycle de formule IIa, 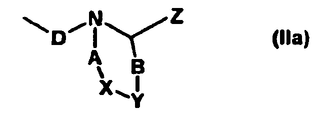 où D et Z sont définis comme dans la formule II,
A représente l'atome d'azote ou le radical -CH₂-,
B représente l'atome d'oxygène, de soufre, d'azote ou le radical -CH₂-,
X représente l'atome d'oxygène, de soufre, d'azote ou le radical -CH₂-,
Y est absent ou représente l'atome d'oxygène, de soufre, d'azote ou le radical -CH₂-, ou
X et Y forment ensemble un radical phényle, 1,2-diazine, 1,3-diazine ou 1,4-diazine,
le système cyclique formé par N, A, X, Y, B et l'atome de carbone ne contenant pas plus d'un atome d'oxygène, X ne représentant pas l'atome d'oxygène, de soufre ni d'azote lorsque A représente l'atome d'azote, ne contenant pas plus d'un atome de soufre, contenant 1, 2, 3 ou 4 atomes d'azote et un atome d'oxygène et de soufre n'étant pas présents simultanément,
le système cyclique formé par N, A, X, Y, B et l'atome de carbone étant non substitué ou monosubstitué, disubstitué ou trisubstitué, indépendamment l'un de l'autre, par alkyle en C₁ à C₈, où alkyle est non substitué ou monosubstitué ou disubstitué par
1.1. -OH,
1.2. alcoxy en C₁ à C₈,
1.3. halogène,
1.4. -NO₂,
1.5. -NH₂,
1.6. -CF₃,
1.6. -OH,
1.7. méthylènedioxy,
1.8. -C(O)-CH₃,
1.9. -CH(O),
1.10. -CN,
1.11. -COOH,
1.12. -C(O)-NH₂,
1.13. (alcoxy en C₁ à C₄) carbonyle,
1.14. phényle,
1.15. phénoxy,
1.16. benzyle,
1.17. benzyloxy ou
1.18. tétrazolyle, ou
R⁹ et Z forment ensemble avec les atomes de carbone auxquels ils sont à chaque fois liés un hétérocycle de formule IIc, 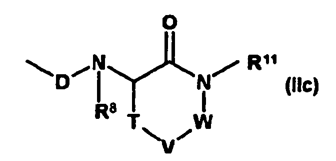 dans laquelle D, R⁸ et R¹¹ sont définis comme dans la formule II,
T représente l'atome d'oxygène, de soufre, d'azote ou le radical -CH₂-,
W représente l'atome d'oxygène, de soufre, d'azote ou le radical -CH₂-,
V est absent ou représente l'atome d'oxygène, de soufre, d'azote ou le radical -CH₂-, ou
T et V ou V et W forment ensemble un radical phényle, 1,2-diazine, 1,3-diazine ou 1,4-diazine,
le système cyclique formé par N, T, V, W et deux atomes de carbone ne contenant pas plus d'un atome d'oxygène, pas plus d'un atome de soufre et contenant 1, 2, 3 ou 4 atomes d'azote, un atome d'oxygène et un atome de soufre n'étant pas être présents simultanément et le système cyclique formé par N, T, V, W et deux atomes de carbone étant non substitué ou monosubstitué, disubstitué ou trisubstitué, indépendamment l'un de l'autre, par les substituants définis ci-dessus aux points 1.1. à 1.18., et
les autres substituants R¹, R², R³ et R⁴ représentent à chaque fois, indépendamment l'un de l'autre,
1. un atome d'hydrogène,
2. halogène,
3. alkyle en C₁ à C₄,
4. hétéroaryle comprenant 5 à 14 éléments de cycle, où hétéroaryle est défini comme ci-dessus et est non substitué ou substitué comme ci-dessus,
5. hétérocycle comprenant 5 à 12 éléments de cycle, où hétérocycle est défini comme ci-dessus et est non substitué ou substitué comme ci-dessus,
6. alkyle en C₁ à C₆,
7. -CN,
8. -O- (alkyle en C₀ à C₄) -aryle, où aryle est défini comme ci-dessus,
9. -O-(alkyle en C₁ à C₄),
10. -O-R¹¹,
11. -N(R¹¹)₂,
12. -S(O)ₓ-R¹¹, où x représente le nombre entier zéro, 1 ou 2,
13. -NO₂ ou
14. -CF₃,
R⁵ représente
1. un atome d'hydrogène,
2. -OH ou
3. =O, et
R⁶ représente
1. aryle, où aryle est défini comme ci-dessus et est non substitué ou substitué comme ci-dessus,
2. phényle, monosubstitué ou disubstitué par
2.1 -N(R¹¹)₂,
2.2 -NH-C(O)-R¹¹,
2.3 -S(O)ₓ-R¹¹, où x représente le nombre entier zéro, 1 ou 2,
2.4 -C(O)-R¹¹ ou
2.5 -(alkyle en C₁ à C₄)-NH₂,
3. hétéroaryle comprenant 5 à 14 éléments de cycle, où hétéroaryle est défini comme ci-dessus et est non substitué ou substitué par -N-(cycloalkyle en C₃ à C₆) ou comme ci-dessus ou
4. hétérocycle comprenant 5 à 12 éléments de cycle, où hétérocycle est défini comme ci-dessus et est non substitué ou substitué par -NH-(alkyle en C₁ à C₆), -NH-phényle, -NH-(cycloalkyle en C₃ à C₆) ou monosubstitué, disubstitué ou trisubstitué comme ci-dessus.

2. Composé de formule I selon la revendication 1, **caractérisé en ce qu'**un des substituants R¹, R², R³ et R⁴ représente un radical de formule II, dans laquelle
R⁸ représente un atome d'hydrogène,
R⁹ représente
1. le radical caractéristique d'un acide aminé, qui est dérivé d'un acide α-aminé existant à l'état naturel du groupe constitué par l'histidine, le tryptophane, la sérine, la thréonine, la cystéine, la méthionine, l'asparagine, la glutamine, la lysine, l'arginine et l'acide glutamique ou
2. alkyle en C₁ à C₆, où alkyle est linéaire ou ramifié et est non substitué ou monosubstitué ou disubstitué par un radical du groupe constitué par pyrrole, pyrrole monosubstitué ou disubstitué par -alkyle en C₁ à C₄, pyrazole, phényle, imidazole, triazole, thiophène, thiazole, oxazole, isoxazole, pyridine, pyrimidine, indole, benzothiophène, benzimidazole, benzoxazole, benzothiazole, azétidine, pyrroline, pyrrolidine, pipéridine, isothiazole, diazépine, thiomorpholine, -CN, morpholine, azépine, 1,3,4-oxadiazole, pyrazine, -N(R¹³) -phényle, (cycloalkyle en C₃ à C₆), -OR¹¹, -NH(R¹¹), où R¹¹ est à chaque fois défini comme dans la revendication 1, -S(O)ₓ-R¹², où x représente zéro, 1 ou 2 et R¹² représente naphtyle, pyrimidinyle, morpholinyle ou phényle, qui sont non substitués ou monosubstitués ou disubstitués par -OH, alkyle en C₁ à C₄, -CF₃, halogène, -O-(alkyle en C₁ à C₄), -COOH, -C(O)-O-(alkyle en C₁ à C₄), -NH₂ ou -NH-C(O)-(alkyle en C₁ à C₄),
Z représente -C(O)-R¹⁰, tétrazole, alkyle en C₁ à C₆, où alkyle est linéaire ou ramifié et est non substitué ou monosubstitué ou disubstitué par phényle, ou -OH, ou 1,3,4-oxadiazole, où 1,3,4-oxadiazole est non substitué ou monosubstitué par
-NH₂, -NH- (alkyle en C₁ à C₄), -N-(alkyle en C₁ à C₄) ₂, -NH-C(O)-(alkyle en C₁ à C₄), -NH-C(O) -NH- (alkyle en C₁ à C₄), -NH-C(O)-NH-(cycloalkyle en C₃ à C₇), -NH-C(O)-NH-aryle, où aryle est choisi dans le groupe constitué par phényle, 1-naphtyle, 2-naphtyle, 2-biphénylyle, 3-biphénylyle, 4-biphénylyle, anthryle et fluorényle, -NH-C(O)-NH-phényle, -NH-SO₂-aryle, où aryle est défini comme ci-dessus, -NH-SO₂-(alkyle en C₁ à C₄), -OH ou -alkyle en C₁ à C₄, où
R¹⁰ représente -O-R¹¹, phényle, pyrimidine, -OH, morpholinyle, -N(R¹¹)₂ ou -NH₂,
R¹¹ représente
1. -alkyle en C₁ à C₄,
2. R¹³ ou
3. -N(R¹³)₂, où R¹³ représente, indépendamment l'un de l'autre
a) un atome d'hydrogène,
b) -alkyle en C₁ à C₆,
c) -(alkyle en C₁ à C₄)-O-(alkyle en C₁ à C₄),
d) -(alkyle en C₁ à C₆)-N-(alkyle en C₁ à C₆)ₙ, où n signifie le nombre entier zéro, 1 ou 2 et alkyle est non substitué ou monosubstitué, disubstitué ou trisubstitué, indépendamment l'un de l'autre, par halogène ou par -COOH,
e) halogène ou
f) -alkyle en C₀ à C₄, monosubstitué ou disubstitué par aryle, où aryle est défini comme ci-dessus, imidazolyle
ou morpholinyle, ou
R⁸ et R⁹ forment ensemble avec l'atome d'azote et l'atome de carbone auxquels ils sont à chaque fois liés un cycle de formule IIa du groupe constitué par le pyrrole, la pyrroline, la pyrrolidine, la pyridine, la pipéridine, le pipérylène, la pyridazine, la pyrimidine, la pyrazine, la pipérazine, le pyrazole, l'imidazole, la pyrazoline, l'imidazoline, la pyrazolidine, l'imidazolidine, l'oxazole, l'isoxazole, la 2-isoxazolidine, l'isoxazolidine, la morpholine, l'isothiazole, le thiazole, le tétrazole, les 2-oxydes de 1,2,3,5-oxathiadiazole, les oxadiazolones, les isoxazolones, les triazolones, les oxadiazolidinediones, les triazoles, qui sont substitués par F, CN, CF₃ ou COO-(alkyle en C₁ à C₄), les 3-hydroxypyrro-2,4-diones, les 5-oxo-1,2,4-thiadiazoles, le 1,3,4-oxadiazole, l'isothiazolidine, la thiomorpholine, l'indazole, le thiadiazole, le benzimidazole, la quinoléine, le triazole, la phtalazine, la quinazoline, la quinoxaline, la purine, la ptéridine, l'indole, la tétrahydroquinoléine, la tétrahydroisoquinoléine et l'isoquinoléine, ou
R⁹ et Z forment ensemble avec les atomes de carbone auxquels ils sont à chaque fois liés un cycle de formule IIc du groupe constitué par le pyrrole, la pyrroline, la pyrrolidine, la pyridine, la pipéridine, le pipérylène, la pyridazine, la pyrimidine, la pyrazine, la pipérazine, le pyrazole, l'imidazole, la pyrazoline, l'imidazoline, la pyrazolidine, l'imidazolidine, l'oxazole, l'isoxazole, la 2-isoxazolidine, l'isoxazolidine, la morpholine, l'isothiazole, le thiazole, l'isothiazolidine, la thiomorpholine, l'indazole, le thiadiazole, le benzimidazole, la quinoléine, le triazole, la phtalazine, la quinazoline, la quinoxaline, la purine, la ptéridine, l'indole, la tétrahydroquinoléine, la tétrahydroisoquinoléine, l'isoquinoléine, le tétrazole, les 2-oxydes de 1,2,3,5-oxathiadiazole, les oxadiazolones, les isoxazolones, les triazolones, les oxadiazolidinediones, les triazoles, qui sont substitués par F, CN, CF₃ ou COO-(alkyle en C₁ à C₄), les 3-hydroxypyrro-2,4-diones, le 1,3,4-oxadiazole et les 5-oxo-1,2,4-thiadiazoles et
les autres substituants R¹, R², R³ et R⁴ représentent à chaque fois, indépendamment l'un de l'autre,
1. un atome d'hydrogène,
2. halogène,
3. alkyle en C₁ à C₄,
4. -CN,
5 . -NO₂,
6. -O-(alkyle en C₀ à C₄)-aryle, où aryle est défini comme ci-dessus,
7. -O-(alkyle en C₁ à C₄),
8. -N-(alkyle en C₀ à C₄) -aryle, où aryle est défini comme ci-dessus,
9. -N- (alkyle en C₁ à C₄),
10. -CF₃,
R⁵ représente
1. un atome d'hydrogène,
2. -OH ou
3. =O, et
R⁶ représente
1. phényle, monosubstitué ou disubstitué par
1.1 -CN,
1.2 -NO₂,
1.3 -O-(alkyle en C₁ à C₄),
1.4 -NH₂ ou
1.5 -(alkyle en C₁ à C₄)-NH₂
2. hétéroaryle comprenant 5 à 14 éléments de cycle, où hétéroaryle représente un radical du groupe constitué par le pyrrole, le furanne, le thiophène, l'imidazole, le pyrazole, l'oxazole, l'isoxazole, le thiazole, l'isothiazole, le tétrazole, le 1,3,4-oxadiazole, les 2-oxydes de 1,2,3,5-oxathiadiazole, les triazolones, les oxadiazolones, les isoxazolones, les oxadiazolidinediones, les triazoles, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoléine, l'isoquinoléine, la quinoxaline, la quinazoline, la cinnoline, la carboline et les dérivés annelés en benzène, cyclopenta, cyclohexa ou cyclohepta de ces hétérocycles, et où hétéroaryle est non substitué ou monosubstitué à trisubstitué par -N-R¹⁴, où R¹⁴ signifie -alkyle en C₁ à C₆, -cycloalkyle en C₃ à C₆ ou phényle, halogène, -OH ou -alkyle en C₁ à C₄, ou
3. hétérocycle comprenant 5 à 12 éléments de cycle, où hétérocycle représente un hétérocycle monocyclique ou bicyclique comprenant 5 à 12 éléments, qui est partiellement ou totalement saturé, du groupe constitué par le pyrrole, le furanne, le thiophène, l'imidazole, le pyrazole, l'oxazole, l'isoxazole, le thiazole, l'isothiazole, le tétrazole, le 1,3,4-oxadiazole, les 2-oxydes de 1,2,3,5-oxa'thiadiazole, les triazolones, les oxadiazolones, les isoxazolones, les oxadiazolidinediones, les triazoles, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoléine, l'isoquinoléine, la quinoxaline, la quinazoline, la cinnoline, la carboline et les dérivés annelés en benzène, cyclopenta, cyclohexa ou cyclohepta de ces hétérocycles et où hétérocycle est non substitué ou monosubstitué à trisubstitué par -N-R¹⁴, où R¹⁴ signifie -alkyle en C₁ à C₆, -cycloalkyle en C₃ à C₆, ou phényle, halogène, -OH ou -alkyle en C₁ à C₄.

3. Procédé pour la préparation du composé de formule I selon l'une ou plusieurs des revendications 1 et 2, **caractérisé**
a) en ce qu'on fait réagir un composé de formule IV, 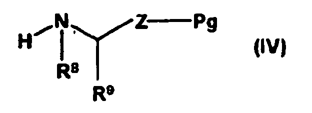 où Pg représente un groupe de protection approprié (par exemple l'ester méthylique), un groupe amide ou un groupe hydroxy et Z, R⁸ et R⁹ sont définis comme dans la formule II, avec un chlorure d'acide ou un ester activé du composé de formule III, 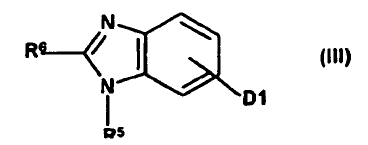 D1 signifiant -COOH ou sulfonylhalogène et R⁵ et R⁶ étant définis comme dans la Formule I, en présence d'une base ou le cas échéant d'un agent soustrayant l'eau, en solution, et on transforme, après dissociation du groupe de protection, en un composé de formule I, ou
b) en ce qu'on couple un composé de formule IVa 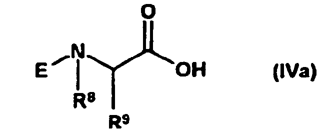 dans laquelle R⁸ et R⁹ sont définis comme dans la formule II et E représente un groupe de protection N-amino, avec son groupe carboxyle via une chaîne intermédiaire L à une résine polymère de formule générale PS avec formation d'un composé de formule V 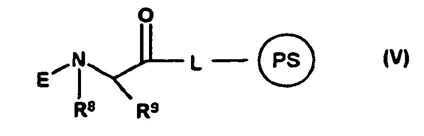 qu'on fait réagir, après dissociation sélective du groupe de protection E, avec un composé de formule III, R⁵ et R⁶ étant définis comme dans la formule I, en présence d'une base ou le cas échéant d'un agent soustrayant l'eau, en un composé de formule VI 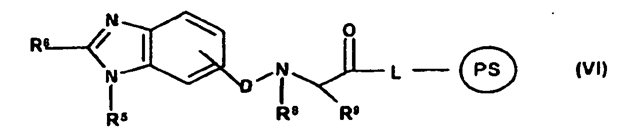 et on transforme le composé de formule VI après dissociation du matériau support en un composé de formule I ou
c) en ce qu'on fait réagir un composé de formule V, après dissociation sélective du groupe de protection E, avec un composé de formule VII, 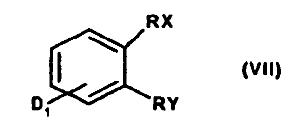 D₁ représentant -COOH ou sulfonylhalogène et RX représentant halogène et RY représentant un radical -NO₂
ou -NH-E et E signifiant un groupe de protection, en un composé de formule VIII 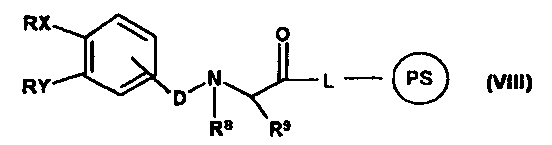 puis on fait réagir le composé de formule VIII avec un composé de formule IX
NH₂-R⁶ (IX)
où R⁶ est défini comme dans la formule I,
en un composé intermédiaire de formule VIa, 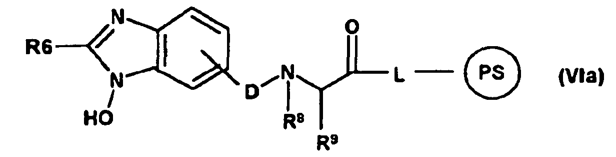 puis on transforme le composé intermédiaire de formule VIa, après dissociation du matériau support, en un composé de formule I ou on réduit, par exemple avec de la tributylphosphine, en un composé de formule VI et on transforme, après dissociation du matériau support en un composé de formule I, ou
d) en ce qu'on transforme un composé de formule I en un sel physiologiquement compatible.

4. Médicament, **caractérisé par** une teneur efficace en au moins un composé de formule I selon l'une ou plusieurs des revendications 1 et 2 avec un support, un additif et/ou d'autres substances actives et d'autres adjuvants, pharmaceutiquement appropriés et physiologiquement acceptables.

5. Utilisation d'au moins un composé de formule I 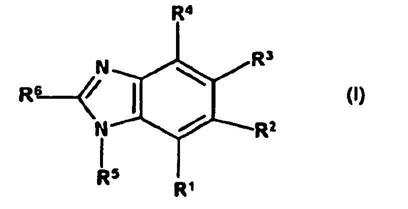 et/ou d'une forme stéréo-isomère du composé de formule I et/ou d'un sel physiologiquement acceptable du composé de formule I pour la préparation de médicaments destinés à la prophylaxie et la thérapie de maladies de la série constituée par l'asthme, l'arthrite rhumatoïde, l'ostéoarthrite, la maladie d'Alzheimer, les maladies cancéreuses, l'infarctus du myocarde, l'insuffisance cardiaque, le syndrome coronaire aigu, l'angine de poitrine instable, le choc septique, l'insuffisance rénale aiguë et chronique, une attaque
ou l'athérosclérose,
un des substituants R¹, R², R³ et R⁴ représentant un radical de formule II, 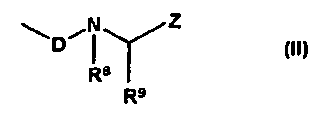 dans laquelle
D représente -C(O)-, -S(O)- ou -S(O)₂-,
R⁸ représente un atome d'hydrogène ou alkyle en C₁ à C₄,
R⁹ représente
1. un radical caractéristique d'un acide aminé, dérivé d'un acide α-aminé existant à l'état naturel du groupe constitué par la glycine, l'alanine, la valine, la leucine, l'isoleucine, la phénylalanine, la tyrosine, le tryptophane, la sérine, la thréonine, la cystéine, la méthionine, l'asparagine, la glutamine, la lysine, l'histidine, l'arginine et l'acide glutamique ou dérivé d'un acide aminé qui n'existe pas à l'état naturel du groupe constitué par l'acide 2-aminoadipique, l'acide 2-aminobutyrique, l'acide 2-aminoisobutyrique, l'acide 2,3-diaminopropionique, l'acide 2,4-diaminobutyrique, l'acide 1,2,3,4-tétra-hydroisoquinoléine-1-carboxylique, l'acide 1,2,3,4-tétrahydroisoquinoléine-3-carboxylique, l'acide 2-aminopimélique, la phénylglycine, la 3-(2-thiényl)-alanine, la 3-(3-thiényl)-alanine, la sarcosine, la 2-(2-thiényl)-glycine, l'acide 2-aminoheptanoique, l'acide pipécolique, l'hydroxylysine, la N-méthylisoleucine, la 6-N-méthyllysine, la N-méthylvaline, la norvaline, la norleucine, l'ornithine, l'allo-isoleucine, la 4-hydroxyproline, l'allohydroxylysine, l'allo-thréonine, la 3-hydroxyproline, la 3-(2-naphtylalanine), la 3-(1-naphtylalanine), l'homophénylalanine, l'homocystéine, l'acide 2-amino-3-phénylaminoéthylpropionique, l'acide, homocystéique, l'homotryptophane, l'acide cystéique, la 3-(2-pyridyl)-alanine, la 3-(3-pyridyl)-alanine, la 3-(4-pyridyl)-alanine, la phosphinothricine, la 4-fluorophénylalanine, la 3-fluorophénylalanine, la 2-fluorophénylalanine, la 4-chlorophénylalanine, la 4-nitrophénylalanine, la 4-aminophénylalanine, la cyclohexylalanine, la citrulline, le 5-fluorotryptophane, le 5-méthoxytryptophane, l'acide 2-amino-3-phénylaminopropionique, la méthionine-sulfone, le méthioninesulfoxyde ou -NH-NR¹¹-CON(R¹¹)₂,
2. aryle, où aryle représente un radical carboné aromatique comprenant 6 à 14 atomes de carbone dans le cycle du groupe constitué par phényle, 1-naphtyle, 2-naphtyle, 2-biphénylyle, 3-biphénylyle, 4-biphénylyle, anthryle ou fluorényle,
où aryle est non substitué ou monosubstitué, disubstitué ou trisubstitué par des radicaux identiques ou différents de la série constituée par alkyle en C₁ à C₈, alcoxy en C₁ à C₈, halogène, nitro, amino, trifluorométhyle, hydroxy, hydroxyméthyle, 1-hydroxyéthyle, 2-hydroxyéthyle, méthylènedioxy, éthylènedioxy, formyle, acétyle, cyano, hydroxycarbonyle, aminocarbonyle, (alcoxy en C₁ à C₄)-carbonyle, phényle, phénoxy, benzyle, benzyloxy ou tétrazolyle,
3. hétéroaryle comprenant 5 à 14 éléments de cycle, où hétéroaryle représente un radical du groupe constitué par le pyrrole, le furanne, le thiophène, l'imidazole, le pyrazole, l'oxazole, l'isoxazole, le thiazole, l'isothiazole, le tétrazole, le 1,3,4-oxadiazole, les 2-oxydes de 1,2,3,5-oxathiadiazole, les triazolones, les oxadiazolones, les isoxazolones, les oxadiazolidinediones, les triazoles, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoléine, l'isoquinoléine, la quinoxaline, la quinazoline, la cinnoline, la carboline et les dérivés annelés en benzène, cyclopenta, cyclohexa ou cyclohepta de ces hétérocycles,
où hétéroaryle est non substitué ou monosubstitué, disubstitué ou trisubstitué par des radicaux identiques ou différents de la série alkyle en C₁ à C₈, alcoxy en C₁ à C₈, halogène, nitro, -N(R¹¹)₂, trifluorométhyle, hydroxy, hydroxyméthyle, 1-hydroxyéthyle, 2-hydroxyéthyle, méthylènedioxy, formyle, acétyle, cyano, hydroxycarbonyle, aminocarbonyle, (alcoxy en C₁ à C₄)carbonyle, phényle, phénoxy, benzyle, benzyloxy ou tétrazolyle,
4. hétérocycle comprenant 5 à 12 éléments de cycle, où hétérocycle représente un hétérocycle monocyclique ou bicyclique de 5 à 12 éléments, qui est partiellement ou complètement saturé du groupe constitué par le pyrrole, le furanne, le thiophène, l'imidazole, le pyrazole, l'oxazole, l'isoxazole, le thiazole, l'isothiazole, le tétrazole, le 1,3,4-oxadiazole, les 2-oxydes de 1,2,3,5-oxathiadiazole, les triazolones, les oxadiazolones, les isoxazolones, les oxadiazolidinediones, les triazoles, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoléine, l'isoquinoléine, la quinoxaline, la quinazoline, la cinnoline, la carboline et les dérivés annelés en benzène, cyclopenta, cyclohexa ou cyclohepta de ces hétérocycles,
où l'hétérocycle est non substitué ou monosubstitué, disubstitué, trisubstitué ou tétrasubstitué par des radicaux identiques ou différents de la série constituée par alkyle en C₁ à C₈, alcoxy en C₁ à C₈, phényl(alcoxy en C₁ à C₄), hydroxy, oxo, halogène, nitro, amino ou trifluorométhyle, les hétérocycles azotés pouvant également se trouver sous forme de N-oxydes ou de sels quaternaires,
5. alkyle en C₁ à C₆, où alkyle est linéaire ou ramifié et est non substitué ou monosubstitué, disubstitué ou trisubstitué, indépendamment l'un de l'autre, par
5.1 aryle, où aryle est défini comme ci-dessus et est non substitué ou substitué comme ci-dessus,
5.2 hétéroaryle comprenant 5 à 14 éléments de cycle, où hétéroaryle est défini comme ci-dessus et est non substitué ou substitué comme ci-dessus,
5.3 hétérocycle comprenant 5 à 12 éléments de cycle, où hétérocycle est défini comme ci-dessus et est non substitué ou substitué comme ci-dessus
5.4 un radical du groupe constitué par l'azétidine, la pyrroline, la pyrrolidine, la pipéridine, l'isothiazole, la diazépine, la thiomorpholine, la morpholine ou l'azépine,
5.5 -O-R¹¹,
5.6 =O,
5.7 halogène,
5.8 -CN,
5.9 -CF₃,
5.10 -S(O)ₓ-R¹¹, où x représente le nombre entier zéro, 1 ou 2,
5.12 -C(O)-N(R¹¹)₂,
5.13 -N(R¹¹)₂,
5.14 cycloalkyle en C₃ à C₆,
5.15 un radical de formule 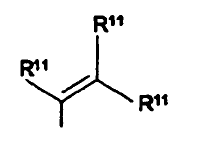 ou
5.16 un radical de formule 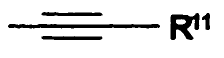
dans lesquelles
R¹¹ représente
a) un atome d'hydrogène,
b) alkyle en C₁ à C₆, où alkyle est non substitué ou monosubstitué, disubstitué ou trisubstitué par
1. aryle, où aryle est défini comme ci-dessus et est non substitué ou substitué comme ci-dessus,
2. hétéroaryle comprenant 5 à 14 éléments de cycle, où hétéroaryle est défini comme ci-dessus,
3. halogène,
4. -N-(alkyle en C₁ à C₆)ₙ, où n représente le nombre entier zéro, 1 ou 2 et alkyle est non substitué ou monosubstitué, disubstitué ou trisubstitué indépendamment l'un de l'autre par halogène ou par -COOH,
5. -O-(alkyle en C₁ à C₆) ou
6. -COOH,
c) aryle, où aryle est défini comme ci-dessus et est non substitué ou substitué comme ci-dessus,
d) hétéroaryle comprenant 5 à 14 éléments de cycle, où hétéroaryle est défini comme ci-dessus,
e) halogène ou
f) -N(R¹³)₂, où
R¹³ représente, indépendamment l'un de l'autre,
f) a) un atome d'hydrogëne,
f) b) -alkyle en C₁ à C₆,
f) c) -(alkyle en C₁ à C₄)-O-(alkyle en C₁ à C₄),
f) d) -(alkyle en C₁ à C₆)-N-(alkyle en C₁ à C₆)ₙ, où n signifie le nombre entier zéro, 1 ou 2 et alkyle est non substitué ou monosubstitué, disubstitué ou trisubstitué, indépendamment l'un de l'autre, par halogène ou par -COOH,
f) e) halogène ou
f) f) -(alkyle en C₀ à C₄), monosubstitué ou disubstitué par phényle, 1-naphtyle, 2-naphtyle, 2-biphénylyle, 3-biphénylyle, 4-biphénylyle, anthryle, fluorényle, imidazolyle ou morpholinyle, et
dans le cas de (R¹¹)₂, R¹¹ a la signification, indépendamment l'un de l'autre, de a) à f)
Z représente
1. aryle, où aryle est défini comme ci-dessus et est non substitué ou substitué comme ci-dessus,
2. hétéroaryle comprenant 5 à 14 éléments de cycle, où hétéroaryle est défini comme ci-dessus et est non substitué ou substitué comme ci-dessus,
3. hétérocycle comprenant 5 à 12 éléments de cycle, où hétérocycle est défini comme ci-dessus et est non substitué ou substitué comme ci-dessus,
4. 1,3,4-oxadiazole, où 1,3,4-oxadiazole est non substitué ou monosubstitué par -NH-C(O)-(alkyle en C₁ à C₄), NH-C(O)-NH-(alkyle en C₁ à C₄), -NH-C(O)-NH-(cycloalkyle en C₃ à C₇), -NH-C(O)-NH-aryle, où aryle est défini comme ci-dessus, -NH-C(O)-NH-phényle, -NH-SO₂-aryle, où aryle est défini comme ci-dessus, ou -NH-SO₂-(alkyle en C₁ à C₄),
5. -alkyle en C₁ à C₆, où alkyle est linéaire ou ramifié et est non substitué ou monosubstitué ou disubstitué par phényle ou -OH, ou
6. -C(O)-R¹⁰,
où R¹⁰ représente
1. -O-R¹¹,
2. -N(R¹¹)₂,
3. phényle,
4. pyrimidinyle ou
5. morpholinyle, ou
R⁸ et R⁹ forment ensemble avec l'atome d'azote et l'atome de carbone auxquels ils sont à chaque fois liés un hétérocycle de formule IIa, 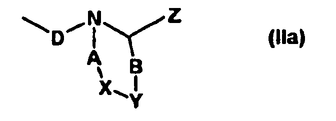 dans laquelle D et Z sont définis comme dans la formule II,
A représente l'atome d'azote ou le radical -CH₂-,
B représente l'atome d'oxygène, de soufre, d'azote ou le radical -CH₂-,
X représente l'atome d'oxygène, de soufre, d'azote ou le radical -CH₂-,
Y est absent ou représente l'atome d'oxygène, de soufre, d'azote ou le radical -CH₂-, ou
X et Y forment ensemble un radical phényle, 1,2-diazine, 1,3-diazine ou 1,4-diazine,
le système cyclique formé par N, A, X, Y, B et l'atome de carbone ne contenant pas plus d'un atome d'oxygène, X ne représentant pas un atome d'oxygène, de soufre ni d'azote lorsque A est un atome d'azote, ne contenant pas plus d'un atome de soufre, contenant 1, 2, 3 ou 4 atomes d'azote et un atome d'oxygène et un atome de soufre n'étant pas présents simultanément,
le système cyclique formé par N, A, X, Y, B et l'atome de carbone étant non substitué ou monosubstitué, disubstitué ou trisubstitué, indépendamment l'un de l'autre, par alkyle en C₁ à C₈, où alkyle est non substitué ou monosubstitué ou disubstitué par
1.1. -OH,
1.2. alcoxy en C₁ à C₈,
1.3. halogène,
1.4. -NO₂,
1.5. -NH₂,
1.6. -CF₃,
1.6. -OH,
1.7. méthylènedioxy,
1.8. -C(O)-CH₃,
1. 9. -CH(O),
1.10. -CN,
1.11. -COOH,
1.12. -C(O)-NH₂,
1.13. (alcoxy en C₁ à C₄) -carbonyle,
1.14. phényle,
1.15. phénoxy,
1.16. benzyle,
1.17. benzyloxy ou
1.18. tétrazolyle, ou
R⁹ et Z forment ensemble avec les atomes de carbone auxquels ils sont à chaque fois liés un hétérocycle de formule IIc, 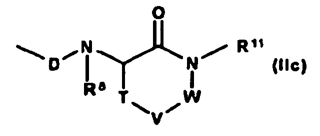 dans laquelle D, R⁸ et R¹¹ sont comme définis dans la formule II,
T représente l'atome d'oxygène, de soufre, d'azote ou le radical -CH₂-,
W représente l'atome d'oxygène, de soufre, d'azote ou le radical -CH₂-,
V manque ou représente l'atome d'oxygène, de soufre, d'azote ou le radical -CH₂-, ou
T et V ou V et W forment ensemble un radical phényle, 1,2-diazine, 1,3-diazine ou 1,4-diazine,
le système cyclique formé par N, T, V, W et deux atomes de carbone ne contenant pas plus d'un atome d'oxygène, pas plus d'un atome de soufre et contenant 1, 2, 3 ou 4 atomes d'azote, un atome d'oxygène et de soufre n'étant pas présents simultanément et le système cyclique formé par N, T, V, W et deux atomes de carbone étant non substitué ou monosubstitué, disubstitué ou trisubstitué, indépendamment l'un de l'autre, par les substituants définis ci-dessus aux points 1.1. à 1.18., et
les autres substituants R¹, R², R³ et R⁴ représentent, indépendamment l'un de l'autre,
1. un atome d'hydrogène,
2. halogène,
3. alkyle en C₁ à C₄,
4. hétéroaryle comprenant 5 à 14 éléments de cycle, où hétéroaryle est défini comme ci-dessus et est non substitué ou substitué comme ci-dessus,
5. hétérocycle comprenant 5 à 12 éléments de cycle, où hétérocycle est défini comme ci-dessus et est non substitué ou substitué comme ci-dessus,
6. alkyle en C₁ à C₆,
7. -CN,
8. -O-(alkyle en C₀ à C₄)-aryle, où aryle est défini comme ci-dessus,
9. -O-(alkyle en C₁ à C₄),
10. -OR¹¹,
11. -N(R¹¹)₂,
12. -S(O)ₓR¹¹, où x représente le nombre entier zéro, 1 ou 2,
13. -NO₂ ou
14. -CF₃,
R⁵ représente
1. un atome d'hydrogène,
2. -OH ou
3. =O, et
R⁶ représente
1. aryle, où aryle est défini comme ci-dessus et est non substitué ou substitué comme ci-dessus,
2. phényle, monosubstitué ou disubstitué par
2.1 -N(R¹¹)₂ ,
2 .2 -NH-C (O)-R¹¹.
2 . 3 -S(O)ₓR¹¹, où x représente le nombre entier zéro, 1 ou 2,
2.4 -C(O)-R¹¹ ou
2 .5 - (alkyle en C₁ à C₄) -NH₂,
3. hétéroaryle comprenant 5 à 14 éléments de cycle, où hétéroaryle est défini comme ci-dessus et est non substitué ou substitué par -N-(cycloalkyle en C₃ à C₆) ou comme ci-dessus ou
4. hétérocycle comprenant 5 à 12 éléments de cycle, où hétérocycle est défini comme ci-dessus et est non substitué ou substitué -NH-(alkyle en C₁ à C₆), -NH-phényle, -NH-(cycloalkyle en C₃ à C₆) ou monosubstitué, disubstitué ou trisubstitué comme ci-dessus.

6. Utilisation selon la revendication 5, **caractérisée en ce qu'**un des substituants R¹, R², R³ et R⁴ représente un radical de formule II, dans laquelle
R⁸ représente un atome d'hydrogène,
R⁹ représente
1. le radical caractéristique d'un acide aminé, qui est dérivé d'un acide α-aminé existant à l'état naturel du groupe constitué par l'histidine, le tryptophane, la sérine, la thréonine, la cystéine, la méthionine, l'asparagine, la glutamine, la lysine, l'arginine et l'acide glutamique ou
2. alkyle en C₁ à C₆, où alkyle est linéaire ou ramifié et est non substitué ou monosubstitué ou disubstitué par un radical du groupe constitué par pyrrole, pyrrole monosubstitué ou disubstitué par -alkyle en C₁ à C₄, pyrazole, phényle, imidazole, triazole, thiophène, thiazole, oxazole, isoxazole, pyridine, pyrimidine, indole, benzothiophène, benzimidazole, benzoxazole, benzothiazole, azétidine, pyrroline, pyrrolidine, pipéridine, isothiazole, diazépine, thiomorpholine, -CN, morpholine, azépine, 1,3,4-oxadiazole, pyrazine, -N (R¹³) -phényle, (cycloalkyle en C₃ à C₆), -OR¹¹, -NH(R¹¹), où R¹¹ est à chaque fois défini comme dans la revendication 1, -S(O)ₓ-R¹², où x représente zéro, 1 ou 2 et R¹² représente naphtyle, pyrimidinyle, morpholinyle ou phényle, qui sont non substitués ou monosubstitués ou disubstitués par -OH, alkyle en C₁ à C₄, -CF₃, halogène, -O-(alkyle en C₁ à C₄), -COOH, -C(O)-O-(alkyle en C₁ à C₄), -NH₂ ou -NH-C(O)-(alkyle en C₁ à C₄),
Z représente -C(O)-R¹⁰, tétrazole, alkyle en C₁ à C₆, où alkyle est linéaire ou ramifié et est non substitué ou monosubstitué ou disubstitué par phényle, ou -OH, ou 1,3,4-oxadiazole, où 1,3,4-oxadiazole est non substitué ou monosubstitué par
-NH₂, -NH-(alkyle en C₁ à C₄), -N-(alkyle en C₁ à C₄)₂, -NH-C(O)-(alkyle en C₁ à C₄), -NH-C(O)-NH- (alkyle en C₁ à C₄), -NH-C(O)-NH-(cycloalkyle en C₃ à C₇), -NH-C(O)-NH-aryle, où aryle est choisi dans le groupe constitué par phényle, 1-naphtyle, 2-naphtyle, 2-biphénylyle, 3-biphénylyle, 4-biphénylyle, anthryle et fluorényle, -NH-C(O)-NH-phényle, -NH-SO₂-aryle, où aryle est défini comme ci-dessus, -NH-SO₂-(alkyle en C₁ à C₄), -OH ou -alkyle en C₁ à C₄, où
R¹⁰ représente -O-R¹¹, phényle, pyrimidine, -OH, morpholinyle, -N(R¹¹)₂ ou -NH₂,
R¹¹ représente
1. alkyle en C₁ à C₄,
2. R¹³ ou
3. -N(R¹³)₂, où R¹³ représente, indépendamment l' un de l'autre,
a) un atome d'hydrogène,
b) -alkyle en C₁ à C₆,
c) -(alkyle en C₁ à C₄)-O-(alkyle en C₁ à C₄),
d) -(alkyle en C₁ à C₆)-N-(alkyle en C₁ à C₆)ₙ, où n signifie le nombre entier zéro, 1 ou 2 et alkyle est non substitué ou monosubstitué, disubstitué ou trisubstitué, indépendamment l'un de l'autre, par halogène ou par -COOH,
e) halogène ou
f) -alkyle en C₀ à C₄, monosubstitué ou disubstitué par aryle, où aryle est défini comme ci-dessus, imidazolyle ou morpholinyle, ou
R⁸ et R⁹ forment ensemble avec l'atome d'azote et l'atome de carbone auxquels ils sont à chaque fois liés un cycle de formule IIa du groupe constitué par le pyrrole, la pyrroline, la pyrrolidine, la pyridine, la pipéridine, le pipérylène, la pyridazine, la pyrimidine, la pyrazine, la pipérazine, le pyrazole, l'imidazole, la pyrazoline, l'imidazoline, la pyrazolidine, l'imidazolidine, l'oxazole, l'isoxazole, la 2-isoxazolidine, l'isoxazolidine, la morpholine, l'isothiazole, le thiazole, le tétrazole, les 2-oxydes de 1,2,3,5-oxathiadiazole, les oxadiazolones, les isoxazolones, les triazolones, les oxadiazolidinediones, les triazoles, qui sont substitués par F, CN, CF₃ ou COO-(alkyle en C₁ à C₄), les 3-hydroxypyrro-2,4-diones, les 5-oxo-1,2,4-thiadiazoles, le 1,3,4-oxadiazole, l'isothiazolidine, la thiomorpholine, l'indazole, le thiadiazole, le benzimidazole, la quinoléine, le triazole, la phtalazine, la quinazoline, la quinoxaline, la purine, la ptéridine, l'indole, la tétrahydroquinoléine, la tétrahydroisoquinoléine et l'isoquinoléine, ou
R⁹ et Z forment ensemble avec les atomes de carbone auxquels ils sont à chaque fois liés un cycle de formule IIc du groupe constitué par le pyrrole, la pyrroline, la pyrrolidine, la pyridine, la pipéridine, le pipérylène, la pyridazine, la pyrimidine, la pyrazine, la pipérazine, le pyrazole, l'imidazole, la pyrazoline, l'imidazoline, la pyrazolidine, l'imidazolidine, l'oxazole, l'isoxazole, la 2-isoxazolidine, l'isoxazolidine, la morpholine, l'isothiazole, le thiazole, l'isothiazolidine, la thiomorpholine, l'indazole, le thiadiazole, le benzimidazole, la quinoléine, le triazole, la phtalazine, la quinazoline, la quinoxaline, la purine, la ptéridine, l'indole, la tétrahydroquinoléine, la tétrahydroisoquinoléine, l'isoquinoléine, le tétrazole, les 2-oxydes de 1,2,3,5-oxathiadiazole, les oxadiazolones, les isoxazolones, les triazolones, les oxadiazolidinediones, les triazoles, qui sont substitués par F, CN, CF₃ ou COO- (alkyle en C₁ à C₄), les 3-hydroxypyrro-2,4-diones, le 1,3,4-oxadiazole et les 5-oxo-1,2,4-thiadiazoles et
les autres substituants R¹, R², R³ et R⁴ représentent à chaque fois, indépendamment l'un de l'autre,
1. un atome d'hydrogène,
2. halogène,
3. alkyle en C₁ à C₄,
4. -CN,
5. -NO₂,
6. -O-(alkyle en C₀ à C₄)-aryle, où aryle est défini comme ci-dessus,
7. -O-(alkyle en C₁ à C₄),
8. -N-(alkyle en C₀ à C₄)-aryle, où aryle est défini comme ci-dessus,
9. -N- (alkyle en C₁ à C₄),
10. -CF₃,
R⁵ représente
1. un atome d'hydrogène,
2. -OH ou
3. =O, et
R⁶ représente
1. phényle, monosubstitué ou disubstitué par
1.1 -CN,
1.2 -NO₂ ,
1.3 -O-(alkyle en C₁ à C₄),
1.4 -NH₂ ou
1.5 -(alkyle en C₁ à C₄) -NH₂
2. hétéroaryle comprenant 5 à 14 éléments de cycle, où hétéroaryle représente un radical du groupe constitué par le pyrrole, le furanne, le thiophène, l'imidazole, le pyrazole, l'oxazole, l'isoxazole, le thiazole, l'isothiazole, le tétrazole, le 1,3,4-oxadiazole, les 2-oxydes de 1,2,3,5-oxathiadiazole, les triazolones, les oxadiazolones, les isoxazolones, les oxadiazolidinediones, les triazoles, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoléine, l'isoquinoléine, la quinoxaline, la quinazoline, la cinnoline, la carboline et les dérivés annelés en benzène, cyclopenta, cyclohexa ou cyclohepta de ces hétérocycles, et où hétéroaryle est non substitué ou monosubstitué à trisubstitué par -N-R¹⁴, où R¹⁴ signifie -alkyle en C₁ à C₆, -cycloalkyle en C₃ à C₆ ou phényle, halogène, -OH ou -alkyle en C₁ à C₄, ou
3. hétérocycle comprenant 5 à 12 éléments de cycle, où hétérocycle représente un hétérocycle monocyclique ou bicyclique comprenant 5 à 12 éléments, qui est partiellement ou totalement saturé, du groupe constitué par le pyrrole, le furanne, le thiophène, l'imidazole, le pyrazole, l'oxazole, l'isoxazole, le thiazole, l'isothiazole, le tétrazole, le 1,3,4-oxadiazole, les 2-oxydes de 1,2,3,5-oxathiadiazole, les triazolones, les oxadiazolones, les isoxazolones, les oxadiazolidinediones, les triazoles, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoléine, l'isoquinoléine, la quinoxaline, la quinazoline, la cinnoline, la carboline et les dérivés annelés en benzène, cyclopenta, cyclohexa ou cyclohepta de ces hétérocycles et où hétérocycle est non substitué ou monosubstitué à trisubstitué par -N-R¹⁴, où R¹⁴ signifie -alkyle en C₁ à C₆, -cycloalkyle en C₃ à C₆, ou phényle, halogène, -OH ou -alkyle en C₁ à C₄.

7. Procédé pour la préparation d'un médicament, **caractérisé en ce qu'**on amène au moins un composé de formule I selon l'une ou plusieurs des revendications 1 et 2 avec un support pharmaceutiquement approprié et physiologiquement acceptable et, le cas échéant, d'autres substances actives, substances support ou d'autres adjuvants dans une forme d'administration appropriée.
